# EUROPEAN PATENT SPECIFICATION

(11) **EP 3 962 909 B1**
(45) Date of publication and mention of the grant of the patent: **04.10.2023**
(21) Application number: 20721247.3
(22) Date of filing: 29.04.2020
(51) Int. Cl.: C07D 403/06, C07D 209/42, C07D 405/06, C07D 471/04, A61K 31/496, A61P 31/00

(54) **NOVEL OXALYL PIPERAZINES ACTIVE AGAINST THE HEPATITIS B VIRUS (HBV)**
NEUARTIGE OXALYLPIPERAZINE MIT WIRKSAMKEIT GEGEN DAS HEPATITIS-B-VIRUS (HBV)
NOUVELLES PIPÉRAZINES D'OXALYLE ACTIVES CONTRE LE VIRUS DE L'HÉPATITE B (VHB)

(30) Priority: 30.04.2019 EP 19172008; 02.05.2019 EP 19172402
(43) Date of publication of application: 09.03.2022
(73) Proprietor: AiCuris GmbH & Co. KG, 42117 Wuppertal (DE)
(72) Inventor: BONSMANN, Susanne, 50769 Köln (DE); DONALD, Alastair, 42111 Wuppertal (DE); URBAN, Andreas, 45549 Sprockhövel (DE); GOLDNER, Thomas, 42553 Velbert (DE); PERICÀS BRONDO, Miquel Àngel, 08950 Esplugues de Llobregat (ES); BARRIOS, Esther Alza, 43003 Tarragona (ES); DETTA, Elena, 42105 Wuppertal (DE); RAYMOND, Justine, 42103 Wuppertal (DE)
(74) Representative: Kilger, Ute
(86) International application number: PCT/EP2020/061920
(87) International publication number: WO 2020/221811

(56) References cited:
- WO-A1-2018/011163
- DATABASE REGISTRY [Online] CHEMICAL ABSTRACTS SERVICE, COLUMBUS, OHIO, US; 7 December 2008 (2008-12-07), XP002793194, retrieved from STN Database accession no. 1081121-93-8

## Description

### Technical Field

The present invention relates generally to novel antiviral agents. Specifically, the present invention relates to compounds which can inhibit the protein(s) encoded by hepatitis B virus (HBV) or interfere with the function of the HBV replication cycle, compositions comprising such compounds, methods for inhibiting HBV viral replication, methods for treating or preventing HBV infection, and processes for making the compounds.

### Background of the Invention

Chronic HBV infection is a significant global health problem, affecting over 5% of the world population (over 350 million people worldwide and 1.25 million individuals in the US). Despite the availability of a prophylactic HBV vaccine, the burden of chronic HBV infection continues to be a significant unmet worldwide medical problem, due to suboptimal treatment options and sustained rates of new infections in most parts of the developing world. Current treatments do not provide a cure and are limited to only two classes of agents (interferon alpha and nucleoside analogues/inhibitors of the viral polymerase); drug resistance, low efficacy, and tolerability issues limit their impact.

The low cure rates of HBV are attributed at least in part to the fact that complete suppression of virus production is difficult to achieve with a single antiviral agent, and to the presence and persistence of covalently closed circular DNA (cccDNA) in the nucleus of infected hepatocytes. However, persistent suppression of HBV DNA slows liver disease progression and helps to prevent hepatocellular carcinoma (HCC).

Current therapy goals for HBV-infected patients are directed to reducing serum HBV DNA to low or undetectable levels, and to ultimately reducing or preventing the development of cirrhosis and HCC.

The HBV is an enveloped, partially double-stranded DNA (dsDNA) virus of the hepadnavirus family (Hepadnaviridae). HBV capsid protein (HBV-CP) plays essential roles in HBV replication. The predominant biological function of HBV-CP is to act as a structural protein to encapsidate pre-genomic RNA and form immature capsid particles, which spontaneously selfassemble from many copies of capsid protein dimers in the cytoplasm.

HBV-CP also regulates viral DNA synthesis through differential phosphorylation states of its C-terminal phosphorylation sites. Also, HBV-CP might facilitate the nuclear translocation of viral relaxed circular genome by means of the nuclear localization signals located in the arginine-rich domain of the C-terminal region of HBV-CP.

In the nucleus, as a component of the viral cccDNA mini-chromosome, HBV-CP could play a structural and regulatory role in the functionality of cccDNA mini-chromosomes. HBV-CP also interacts with viral large envelope protein in the endoplasmic reticulum (ER), and triggers the release of intact viral particles from hepatocytes.

HBV-CP related anti-HBV compounds have been reported. For example, phenylpropenamide derivatives, including compounds named AT-61 and AT-130 (Feld J. et al. Antiviral Res. 2007, 76, 168), and a class of thiazolidin-4-ones from Valeant (WO2006/033995), have been shown to inhibit pre-genomic RNA (pgRNA) packaging.

F. Hoffmann-La Roche AG have disclosed a series of 3-substituted tetrahydro-pyrazolo[1,5-a]pyrazines for the therapy of HBV (WO2016/113273, WO2017/198744, WO2018/011162, WO2018/011160, WO2018/011163).

Shanghai Hengrui Pharma have disclosed a series of heteroaryl piperazines for HBV therapy (WO2019/020070). Shanghai Longwood Biopharmaceuticals have disclosed a series of bicyclic heterocycles active against HBV (WO2018/202155).

Zhimeng Biopharma have disclosed pyrazole-oxazolidinone compounds as being active against HBV (WO2017/173999).

Heteroaryldihydropyrimidines (HAPs) were discovered in a tissue culture-based screening (Weber et al., Antiviral Res. 2002, 54, 69). These HAP analogs act as synthetic allosteric activators and are able to induce aberrant capsid formation that leads to degradation of HBV-CP (WO 99/54326, WO 00/58302, WO 01/45712, WO 01/6840). Further HAP analogs have also been described (J. Med. Chem. 2016, 59 (16), 7651-7666).

A subclass of HAPs from F. Hoffman-La Roche also shows activity against HBV (WO2014/184328, WO2015/132276, and WO2016/146598). A similar subclass from Sunshine Lake Pharma also shows activity against HBV (WO2015/144093). Further HAPs have also been shown to possess activity against HBV (WO2013/102655, Bioorg. Med. Chem. 2017, 25(3) pp. 1042-1056, and a similar subclass from Enanta Therapeutics shows similar activity (WO2017/011552). A further subclass from Medshine Discovery shows similar activity (WO2017/076286). A further subclass (Janssen Pharma) shows similar activity (WO2013/102655).

A subclass of pyridazones and triazinones (F. Hoffman-La Roche) also show activity against HBV (WO2016/023877), as do a subclass of tetrahydropyridopyridines (WO2016/177655). A subclass of tricyclic 4-pyridone-3-carboxylic acid derivatives from Roche also show similar anti-HBV activity (WO2017/013046).

A subclass of sulfamoyl-arylamides from Novira Therapeutics (now part of Johnson & Johnson Inc.) also shows activity against HBV (WO2013/006394, WO2013/096744, WO2014/165128, WO2014/184365, WO2015/109130, WO2016/089990, WO2016/109663, WO2016/109684, WO2016/109689, WO2017/059059). A similar subclass of thioetherarylamides (also from Novira Therapeutics) shows activity against HBV (WO2016/089990). Additionally, a subclass of aryl-azepanes (also from Novira Therapeutics) shows activity against HBV (WO2015/073774). A similar subclass of arylamides from Enanta Therapeutics show activity against HBV (WO2017/015451).

Sulfamoyl derivatives from Janssen Pharma have also been shown to possess activity against HBV (WO2014/033167, WO2014/033170, WO2017/001655, J. Med. Chem, 2018, 61(14) 6247-6260).

A subclass of glyoxamide substituted pyrrolamide derivatives also from Janssen Pharma have also been shown to possess activity against HBV (WO2015/011281). A similar class of glyoxamide substituted pyrrolamides (Gilead Sciences) has also been described (WO2018/039531).

A subclass of sulfamoyl- and oxalyl-heterobiaryls from Enanta Therapeutics also show activity against HBV (WO2016/161268, WO2016/183266, WO2017/015451, WO2017/136403 & US20170253609).

A subclass of aniline-pyrimidines from Assembly Biosciences also show activity against HBV (WO2015/057945, WO2015/172128). A subclass of fused tri-cycles from Assembly Biosciences (dibenzo-thiazepinones, dibenzo-diazepinones, dibenzo-oxazepinones) show activity against HBV (WO2015/138895, WO2017/048950). A further series from Assembly Biosciences (WO2016/168619) also show anti-HBV activity.

A series of cyclic sulfamides has been described as modulators of HBV-CP function by Assembly Biosciences (WO2018/160878).

Arbutus Biopharma have disclosed a series of benzamides for the therapy of HBV (WO2018/052967, WO2018/172852). Also disclosed are compositions and uses of similar compounds in combination with a CYP3A inhibitor (WO2019/046287).

A series of thiophene-2-carboxamides from the University of Missouri have been described as HBV inhibitors (US2019/0092742).

It was also shown that the small molecule bis-ANS acts as a molecular 'wedge' and interferes with normal capsid-protein geometry and capsid formation (Zlotnick A et al. J. Virol. 2002, 4848).

Problems that HBV direct acting antivirals may encounter are toxicity, mutagenicity, lack of selectivity, poor efficacy, poor bioavailability, low solubility and difficulty of synthesis. There is a thus a need for additional inhibitors for the treatment, amelioration or prevention of HBV that may overcome at least one of these disadvantages or that have additional advantages such as increased potency or an increased safety window.

Administration of such therapeutic agents to an HBV infected patient, either as monotherapy or in combination with other HBV treatments or ancillary treatments, will lead to significantly reduced virus burden, improved prognosis, diminished progression of the disease and/or enhanced seroconversion rates.

### Summary of the invention

Provided herein are compounds useful for the treatment or prevention of HBV infection in a subject in need thereof, and intermediates useful in their preparation. The subject matter of the invention is a compound of Formula I 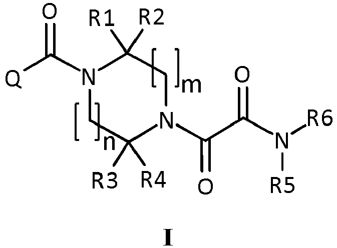 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
- indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
   wherein heteroaryl and heterocycloalkyl each has 1 or 2 heteroatoms each independently selected from N, O and S for use in the prevention or treatment of an HBV infection in a subject
- In one embodiment of the invention subject matter of the invention is a compound of Formula I in whichR1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; wherein heteroaryl and heterocycloalkyl each has 1 or 2 heteroatoms each independently selected from N, O and S for use in the prevention or treatment of an HBV infection in a subject.
- In one embodiment of the invention subject matter of the invention is a compound of Formula I in whichR1, R2, R3, and R4 are for each position independently selected from the group comprising H, D, and C1-C6-alkyl
- R5 and R6 are independently selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1 -C2-alkyl-C3 -C5-cycloalkyl, C1-C2-alkyl-C3-CS-heteroaryl, and C1-C2-alkyl-C3-CS-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
   indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula I in which
- R1, R2, R3, and R4 are for each position independently selected from the group comprising H, D, and C1-C6-alkyl
- R5 and R6 are independently selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1 -C2-alkyl-C3 -C5-cycloalkyl, C1-C2-alkyl-C3-CS-heteroaryl, and C1-C2-alkyl-C3-CS-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula I in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy.
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
- indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula I in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy.
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
- indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention are stereoisomers of a compound of Formula I 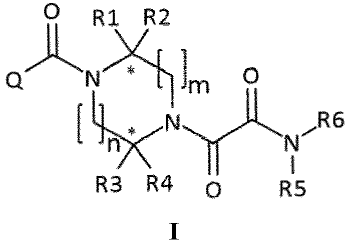 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy.
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
- indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
   wherein heteroaryl and heterocycloalkyl each has 1 or 2 heteroatoms each independently selected from N, O and S for use in the prevention or treatment of an HBV infection in a subject.

One embodiment of the invention is a compound of Formula I or a pharmaceutically acceptable salt thereof according to the invention, for use in the prevention or treatment of an HBV infection in subject.

One embodiment of the invention is a pharmaceutical composition comprising a compound of Formula I or a pharmaceutically acceptable salt thereof according to the present invention, together with a pharmaceutically acceptable carrier for use in the prevention or treatment of an HBV infection in a subject.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

One embodiment of the invention is a method of treating an HBV infection in an individual in need thereof, comprising administering to the individual a therapeutically effective amount of a compound of Formula I or a pharmaceutically acceptable salt thereof according to the present invention.

A further embodiment of the invention is a compound of Formula I or a pharmaceutically acceptable salt thereof according to the invention, for use in the prevention or treatment of an HBV infection in subject in need thereof.

A further embodiment of the invention is a compound of Formula II or a pharmaceutically acceptable salt thereof according to the invention, for use in the prevention or treatment of an HBV infection in subject in need thereof 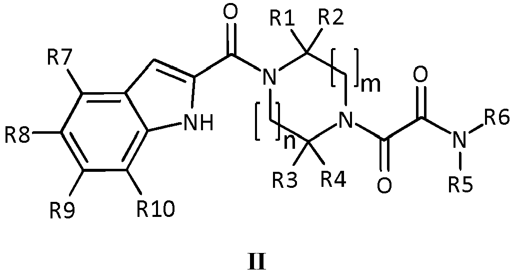 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH3 and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S.

In one embodiment of the invention subject matter of the invention is a compound of Formula II in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH3 and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula II in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula II in which
- R1, R2, R3, and R4 are for each position independently selected from the group comprising H, D, and C1-C6-alkyl
- R5 and R6 are independently selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1 -C2-alkyl-C3 -C5-cycloalkyl, C1-C2-alkyl-C3-CS-heteroaryl, and C1-C2-alkyl-C3-CS-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

One embodiment of the invention is a compound of Formula II or a pharmaceutically acceptable salt thereof according to the invention, for use in the prevention or treatment of an HBV infection in subject.

One embodiment of the invention is a pharmaceutical composition comprising a compound of Formula II or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula II or a pharmaceutically acceptable salt thereof according to the present invention.

A further embodiment of the invention is a compound of Formula II or a pharmaceutically acceptable salt thereof according to the invention, for use in the prevention or treatment of an HBV infection in subject in need thereof.

A further embodiment of the invention is a compound of Formula IIa or a pharmaceutically acceptable salt thereof according to the invention, for use in the prevention or treatment of an HBV infection in subject in need thereof 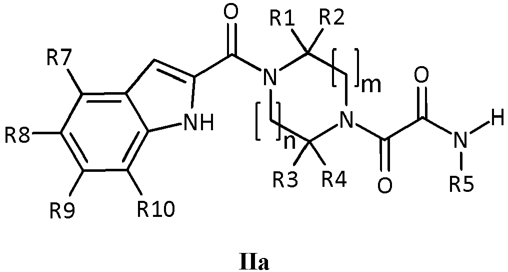 in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, C1-C2-alkyl-C3-CS-heteroaryl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S.

In one embodiment of the invention subject matter of the invention is a compound of Formula IIa in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, C1-C2-alkyl-C3-CS-heteroaryl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula IIa in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH3 and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula IIa in which
- R1, R2, R3, and R4 are for each position independently selected from the group comprising H, D, and C1-C6-alkyl
- R5 is selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-C5-heteroaryl, and C1-C2-alkyl-C3-CS-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

One embodiment of the invention is a compound of Formula IIa or a pharmaceutically acceptable salt thereof according to the invention, for use in the prevention or treatment of an HBV infection in subject.

One embodiment of the invention is a pharmaceutical composition comprising a compound of Formula IIa or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula IIa or a pharmaceutically acceptable salt thereof according to the present invention.

A further embodiment of the invention is a compound of Formula IIa or a pharmaceutically acceptable salt thereof according to the invention, for use in the prevention or treatment of an HBV infection in subject in need thereof.

A further embodiment of the invention is a compound of Formula IIb or a pharmaceutically acceptable salt thereof according to the invention, for use in the prevention or treatment of an HBV infection in subject in need thereof 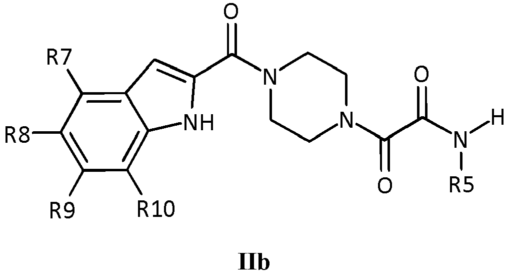 in which
- R5 is selected from the group comprising H, C6-aryl, C3-CS-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, C1-C2-alkyl-C3-CS-heteroaryl and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S.

In one embodiment of the invention subject matter of the invention is a compound of Formula IIb in which
- R5 is selected from the group comprising H, C6-aryl, C3-CS-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, C1-C2-alkyl-C3-CS-heteroaryl and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula IIb in which
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy.
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

In one embodiment of the invention subject matter of the invention is a compound of Formula IIb in which
- R5 is selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-C5-heteroaryl, and C1-C2-alkyl-C3-CS-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃,
- R7, R8, R9 and R10 are independently selected from the group comprising H, D, F, Cl, Br, I, CF₃, CF₂H, C1-C4-alkyl, CF₂CH₃, cyclopropyl and cyano,
   wherein heteroaryl and heterocycloalkyl each has 1 or 2 heteroatoms each independently selected from N, O and S for use in the prevention or treatment of an HBV infection in a subject.

One embodiment of the invention is a compound of Formula IIb or a pharmaceutically acceptable salt thereof according to the invention, for use in the prevention or treatment of an HBV infection in subject.

One embodiment of the invention is a pharmaceutical composition comprising a compound of Formula IIb or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula IIb or a pharmaceutically acceptable salt thereof according to the present invention.

A further embodiment of the invention is a compound of Formula IIb or a pharmaceutically acceptable salt thereof according to the invention, for use in the prevention or treatment of an HBV infection in subject in need thereof.

A further embodiment of the invention is a compound of Formula III or a pharmaceutically acceptable salt thereof according to the invention, for use in the prevention or treatment of an HBV infection in subject in need thereof 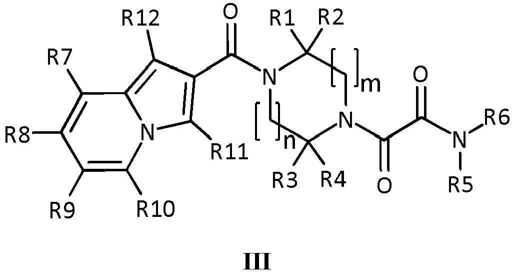 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy.
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S.

In one embodiment of the invention subject matter of the invention is a compound of Formula III in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH3 and carboxy.
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S for use in the prevention or treatment of an HBV infection in a subject.

One embodiment of the invention is a compound of Formula III or a pharmaceutically acceptable salt thereof according to the invention, for use in the prevention or treatment of an HBV infection in subject.

One embodiment of the invention is a pharmaceutical composition comprising a compound of Formula III or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula III or a pharmaceutically acceptable salt thereof according to the present invention.

A further embodiment of the invention is a compound of Formula III or a pharmaceutically acceptable salt thereof according to the invention, for use in the prevention or treatment of an HBV infection in subject in need thereof.

A further embodiment of the invention is a compound of Formula IIIa or a pharmaceutically acceptable salt thereof according to the invention, for use in the prevention or treatment of an HBV infection in subject in need thereof 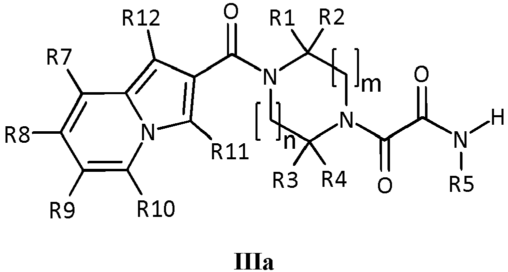 in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S.

In one embodiment of the invention subject matter of the invention is a compound of Formula IIIa in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S for use in the prevention or treatment of an HBV infection in a subject.

One embodiment of the invention is a compound of Formula IIIa or a pharmaceutically acceptable salt thereof according to the invention, for use in the prevention or treatment of an HBV infection in subject.

One embodiment of the invention is a pharmaceutical composition comprising a compound of Formula IIIa or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula IIIa or a pharmaceutically acceptable salt thereof according to the present invention.

A further embodiment of the invention is a compound of Formula IIIa or a pharmaceutically acceptable salt thereof according to the invention, for use in the prevention or treatment of an HBV infection in subject in need thereof.

A further embodiment of the invention is a compound of Formula IIIb or a pharmaceutically acceptable salt thereof according to the invention, for use in the prevention or treatment of an HBV infection in subject in need thereof 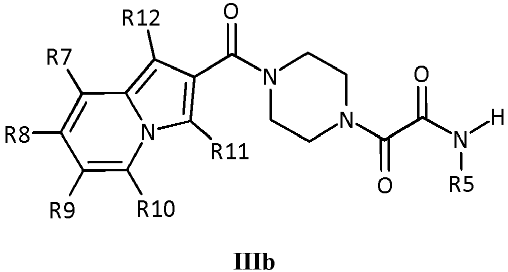 in which
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S.

In one embodiment of the invention subject matter of the invention is a compound of Formula IIIb in which
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-CS-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S for use in the prevention or treatment of an HBV infection in a subject.

One embodiment of the invention is a compound of Formula IIIb or a pharmaceutically acceptable salt thereof according to the invention, for use in the prevention or treatment of an HBV infection in subject.

One embodiment of the invention is a pharmaceutical composition comprising a compound of Formula IIIb or a pharmaceutically acceptable salt thereof according to the present invention, together with a pharmaceutically acceptable carrier for use in the prevention or treatment of an HBV infection in a subject.

One embodiment of the invention is a method of treating an HBV infection in an individual in need thereof, comprising administering to the individual a therapeutically effective amount of a compound of Formula IIIb or a pharmaceutically acceptable salt thereof according to the present invention.

A further embodiment of the invention is a compound of Formula IIIb or a pharmaceutically acceptable salt thereof according to the invention, for use in the prevention or treatment of an HBV infection in subject in need thereof.

In some embodiments, the dose of a compound of the invention is from about 1 mg to about 2,500 mg. In some embodiments, a dose of a compound of the invention used in compositions described herein is less than about 10,000 mg, or less than about 8,000 mg, or less than about 6,000 mg, or less than about 5,000 mg, or less than about 3,000 mg, or less than about 2,000 mg, or less than about 1,000 mg, or less than about 500 mg, or less than about 200 mg, or less than about 50 mg. Similarly, in some embodiments, a dose of a second compound (i.e., another drug for HBV treatment) as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof. All before mentioned doses refer to daily doses per patient.

In general it is contemplated that an antiviral effective daily amount would be from about 0.01 to about 50 mg/kg, or about 0.01 to about 30 mg/kg body weight. It may be appropriate to administer the required dose as two, three, four or more sub-doses at appropriate intervals throughout the day. Said sub-doses may be formulated as unit dosage forms, for example containing about 1 to about 500 mg, or about 1 to about 300 mg or about 1 to about 100 mg , or about 2 to about 50 mg of active ingredient per unit dosage form.

The compounds of the invention may, depending on their structure, exist as salts, solvates or hydrates. The invention therefore also encompasses the salts, solvates or hydrates and respective mixtures thereof.

The compounds of the invention may, depending on their structure, exist in tautomeric or stereoisomeric forms (enantiomers, diastereomers). The invention therefore also encompasses the tautomers, enantiomers or diastereomers and respective mixtures thereof. The stereoisomerically uniform constituents can be isolated in a known manner from such mixtures of enantiomers and/or diastereomers.

Subject-matter of the present invention is a compound of Formula I, II, IIa, IIb, III, IIIa, IIIb or a pharmaceutically acceptable salt thereof or a solvate or a hydrate of said compound or a pharmaceutically acceptable salt of said solvate or hydrate or a prodrug as defined in the claims of said compound or a pharmaceutically acceptable salt of said prodrug or a solvate or a hydrate of said prodrug or a pharmaceutically acceptable salt of said solvate or a hydrate of said prodrug for use in the prevention or treatment of an HBV infection in subject.

Subject matter of the present invention is also a pharmaceutical composition comprising a compound of Formula I, II, IIa, IIb, III, IIIa, IIIb or a pharmaceutically acceptable salt thereof or a solvate or a hydrate of said compound or a pharmaceutically acceptable salt of said solvate or hydrate or a prodrug of said compound or a pharmaceutically acceptable salt of said prodrug or a solvate or a hydrate of said prodrug or a pharmaceutically acceptable salt of said solvate or a hydrate of said prodrug , together with a pharmaceutically acceptable carrier for use in the prevention or treatment of an HBV infection in a subject..

Subject matter of the present invention is also a method of treating an HBV infection in an individual in need thereof, comprising administering to the individual a therapeutically effective amount of a compound of Formula I, II, IIa, IIb, III, IIIa, IIIb or a pharmaceutically acceptable salt thereof or a solvate or a hydrate of said compound or a pharmaceutically acceptable salt of said solvate or hydrate or a prodrug of said compound or a pharmaceutically acceptable salt of said prodrug or a solvate or a hydrate of said prodrug or a pharmaceutically acceptable salt of said solvate or a hydrate of said prodrug.

Subject matter of the present invention is also a method of preparing the compounds of the present invention. Subject matter of the invention is, thus, a method for the preparation of a compound of Formula I according to the present invention by reacting a compound of Formula IV 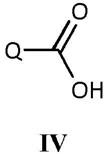 in which Q is as above-defined, with a compound of Formula V 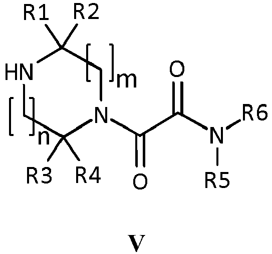 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R5 and R6 are optionally connected to form a C4-C8-heterocyclyl ring
- n is 0, 1, or 2
- m is 0, 1, or 2.

In one embodiment subject matter of the invention is a method for the preparation of a compound of Formula I according to the present invention by reacting a compound of Formula IV 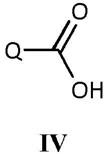 in which Q is as above-defined, with a compound of Formula V 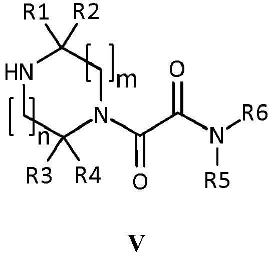 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R5 and R6 are optionally connected to form a C4-C8-heterocyclyl ring
- n is 0, 1, or 2
- m is 0, 1, or 2.

### Definitions

Listed below are definitions of various terms used to describe this invention. These definitions apply to the terms as they are used throughout this specification and claims unless otherwise limited in specific instances either individually or as part of a larger group.

Unless defined otherwise all technical and scientific terms used herein generally have the same meaning as commonly understood by one of ordinary skill in the art to which this invention belongs. Generally the nomenclature used herein and the laboratory procedures in cell culture, molecular genetics, organic chemistry and peptide chemistry are those well-known and commonly employed in the art.

As used herein the articles "a" and "an" refer to one or to more than one (i.e. to at least one) of the grammatical object of the article. By way of example, "an element" means one element or more than one element. Furthermore, use of the term "including" as well as other forms such as "include", "includes" and "included", is not limiting.

As used herein the term "capsid assembly modulator" refers to a compound that disrupts or accelerates or inhibits or hinders or delays or reduces or modifies normal capsid assembly (e.g. during maturation) or normal capsid disassembly (e.g. during infectivity) or perturbs capsid stability, thereby inducing aberrant capsid morphology or aberrant capsid function. In one embodiment, a capsid assembly modulator accelerates capsid assembly or disassembly thereby inducing aberrant capsid morphology. In another embodiment a capsid assembly modulator interacts (e.g. binds at an active site, binds at an allosteric site or modifies and/or hinders folding and the like), with the major capsid assembly protein (HBV-CP) , thereby disrupting capsid assembly or disassembly. In yet another embodiment a capsid assembly modulator causes a perturbation in the structure or function of HBV-CP (e.g. the ability of HBV-CP to assemble, disassemble, bind to a substrate, fold into a suitable conformation or the like which attenuates viral infectivity and/or is lethal to the virus).

As used herein the term "treatment" or "treating" is defined as the application or administration of a therapeutic agent i.e., a compound of the invention (alone or in combination with another pharmaceutical agent) to a patient, or application or administration of a therapeutic agent to an isolated tissue or cell line from a patient (e.g. for diagnosis or ex vivo applications) who has an HBV infection, a symptom of HBV infection, or the potential to develop an HBV infection with the purpose to cure, heal, alleviate, relieve, alter, remedy, ameliorate, improve or affect the HBV infection, the symptoms of HBV infection or the potential to develop an HBV infection. Such treatments may be specifically tailored or modified based on knowledge obtained from the field of pharmacogenomics.

As used herein the term "prevent" or "prevention" means no disorder or disease development if none had occurred, or no further disorder or disease development if there had already been development of the disorder or disease. Also considered is the ability of one to prevent some or all of the symptoms associated with the disorder or disease.

As used herein the term "patient", "individual" or "subject" refers to a human or a non-human mammal. Non-human mammals include for example livestock and pets such as ovine, bovine, porcine, feline, and murine mammals. Preferably the patient, subject, or individual is human.

As used herein the terms "effective amount", "pharmaceutically effective amount", and "therapeutically effective amount" refer to a nontoxic but sufficient amount of an agent to provide the desired biological result. That result may be reduction and/or alleviation of the signs, symptoms, or causes of a disease, or any other desired alteration of a biological system. An appropriate therapeutic amount in any individual case may be determined by one of ordinary skill in the art using routine experimentation.

As used herein the term "pharmaceutically acceptable" refers to a material such as a carrier or diluent which does not abrogate the biological activity or properties of the compound and is relatively non-toxic i.e. the material may be administered to an individual without causing undesirable biological effects or interacting in a deleterious manner with any of the components of the composition in which it is contained.

As used herein the term "pharmaceutically acceptable salt" refers to derivatives of the disclosed compounds wherein the parent compound is modified by converting an existing acid or base moiety to its salt form. Examples of pharmaceutically acceptable salts include but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent or in a mixture of the two; generally non-aqueous media like ether, ethyl acetate, ethanol, isopropanol, or acetonitrile are preferred. Lists of suitable salts are found in Remington's Pharmaceutical Sciences 17th ed. Mack Publishing Company, Easton, Pa., 1985 p.1418 and Journal of Pharmaceutical Science, 66, 2 (1977), each of which is incorporated herein by reference in its entirety. Pharmaceutically acceptable salts of the compounds according to the invention include acid addition salts, for example, but not limited to, salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, trifluoroacetic acid, propionic acid, lactic acid, tartaric acid, malic acid, citric acid, fumaric acid, maleic acid and benzoic acid. Pharmaceutically acceptable salts of the compounds according to the invention also include salts of customary bases, for example, but not limited to, alkali metal salts (for example sodium and potassium salts), alkaline earth metal salts (for example calcium and magnesium salts) and ammonium salts derived from ammonia or organic amines having 1 to 16 carbon atoms, such as, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

As used herein, the term "solvate" refers to compounds which form a complex in the solid or liquid state by coordination with solvent molecules. Suitable solvents include, but are not limited to, methanol, ethanol, acetic acid and water. Hydrates are a special form of solvates in which the coordination takes place with water.

As used herein the term "composition" or "pharmaceutical composition" refers to a mixture of at least one compound useful within the invention with a pharmaceutically acceptable carrier. The pharmaceutical composition facilitates administration of the compound to a patient or subject. Multiple techniques of administering a compound exist in the art including but not limited to intravenous, oral, aerosol, rectal, parenteral, ophthalmic, pulmonary and topical administration.

As used herein the term "pharmaceutically acceptable carrier" means a pharmaceutically acceptable material, composition or carrier such as a liquid or solid filler, stabilizer, dispersing agent, suspending agent, diluent, excipient, thickening agent, solvent or encapsulating material involved in carrying or transporting a compound useful within the invention within or to the patient such that it may perform its intended function. Typically such constructs are carried or transported from one organ, or portion of the body, to another organ or portion of the body. Each carrier must be "acceptable" in the sense of being compatible with the other ingredients of the formulation including the compound use within the invention and not injurious to the patient. Some examples of materials that may serve as pharmaceutically acceptable carriers include: sugars, such as lactose, glucose and sucrose; starches such as corn starch and potato starch; cellulose and its derivatives such as sodium carboxymethyl cellulose, ethyl cellulose and cellulose acetate; powdered tragacanth; malt, gelatin, talc; excipients such as cocoa butter and suppository waxes; oils such as peanut oil, cottonseed oil, safflower oil, sesame oil, olive oil, corn oil and soybean oil; glycols such as propylene glycol; polyols such as glycerin, sorbitol, mannitol and polyethylene glycol; esters such as ethyl oleate and ethyl laurate; agar; buffering agents, such as magnesium hydroxide and aluminium hydroxide; surface active agents; alginic acid; pyrogen-free water; isotonic saline; Ringer's solution; ethyl alcohol; phosphate buffer solutions and other non-toxic compatible substances employed in pharmaceutical formulations.

As used herein "pharmaceutically acceptable carrier" also includes any and all coatings, antibacterial and antifungal agents and absorption delaying agents and the like that are compatible with the activity of the compound useful within the invention and are physiologically acceptable to the patient. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described for example in Remington's Pharmaceutical Sciences (Genaro, Ed., Mack Publishing Company, Easton, Pa., 1985) which is incorporated herein by reference.

As used herein, the term "substituted" means that an atom or group of atoms has replaced hydrogen as the substituent attached to another group.

As used herein, the term "comprising" also encompasses the option "consisting of'.

As used herein, the term "alkyl" by itself or as part of another substituent means, unless otherwise stated, a straight or branched chain hydrocarbon having the number of carbon atoms designated (i.e. C1-C6-alkyl means one to six carbon atoms) and includes straight and branched chains. Examples include methyl, ethyl, propyl, isopropyl, butyl, isobutyl, tert-butyl, pentyl, neopentyl, and hexyl. In addition, the term "alkyl" by itself or as part of another substituent can also mean a C1-C3 straight chain hydrocarbon substituted with a C3-C5-carbocylic ring. Examples include (cyclopropyl)methyl, (cyclobutyl)methyl and (cyclopentyl)methyl. For the avoidance of doubt, where two alkyl moieties are present in a group, the alkyl moieties may be the same or different.

As used herein the term "alkenyl" denotes a monovalent group derived from a hydrocarbon moiety containing at least two carbon atoms and at least one carbon-carbon double bond of either E or Z stereochemistry. The double bond may or may not be the point of attachment to another group. Alkenyl groups (e.g. C2-C8-alkenyl) include, but are not limited to for example ethenyl, propenyl, prop-1-en-2-yl, butenyl, methyl-2-buten-1-yl, heptenyl and octenyl. For the avoidance of doubt, where two alkenyl moieties are present in a group, the alkyl moieties may be the same or different.

As used herein, a C2-C6-alkynyl group or moiety is a linear or branched alkynyl group or moiety containing from 2 to 6 carbon atoms, for example a C2-C4 alkynyl group or moiety containing from 2 to 4 carbon atoms. Exemplary alkynyl groups include -C≡CH or -CH₂-C=C, as well as 1- and 2-butynyl, 2-pentynyl, 3-pentynyl, 4-pentynyl, 2-hexynyl, 3-hexynyl, 4-hexynyl and 5-hexynyl. For the avoidance of doubt, where two alkynyl moieties are present in a group they may be the same or different.

As used herein, the term "halo" or "halogen" alone or as part of another substituent means unless otherwise stated a fluorine, chlorine, bromine, or iodine atom, preferably fluorine, chlorine, or bromine, more preferably fluorine or chlorine. For the avoidance of doubt, where two halo moieties are present in a group, they may be the same or different.

As used herein, a C1-C6-alkoxy group or C2-C6-alkenyloxy group is typically a said C1-C6-alkyl (e.g. a C1-C4 alkyl) group or a said C2-C6-alkenyl (e.g. a C2-C4 alkenyl) group respectively which is attached to an oxygen atom.

As used herein, the term "carboxy" and by itself or as part of another substituent means, unless otherwise stated, a group of formula C(=O)OH.

As used herein, the term "cyano" by itself or as part of another substituent means, unless otherwise stated, a group of formula C=N.

As used herein, the term "nitro" by itself or as part of another substituent means, unless otherwise stated, a group of formula NO₂.

As used herein, the term "carboxyl ester" by itself or as part of another substituent means, unless otherwise stated, a group of formula C(=O)OX, wherein X is selected from the group consisting of C1-C6-alkyl, C3-C7-cycloalkyl, and aryl.

As used herein, a carboxyphenyl group is typically a said phenyl group substituted with a said carboxy group.

As used herein the term "aryl" employed alone or in combination with other terms, means unless otherwise stated a carbocyclic aromatic system containing one or more rings (typically one, two or three rings) wherein such rings may be attached together in a pendant manner such as a biphenyl, or may be fused, such as naphthalene. Examples of aryl groups include phenyl, anthracyl, and naphthyl. Preferred examples are phenyl (e.g. C6-aryl) and biphenyl (e.g. C12-aryl). In some embodiments aryl groups have from six to sixteen carbon atoms. In some embodiments aryl groups have from six to twelve carbon atoms (e.g. C6-C12-aryl). In some embodiments, aryl groups have six carbon atoms (e.g. C6-aryl).

As used herein the terms "heteroaryl" and "heteroaromatic" refer to a heterocycle having aromatic character containing one or more rings (typically one, two or three rings), that contains one to four ring heteroatoms each independently selected from oxygen, sulfur and nitrogen. Heteroaryl substituents may be defined by the number of carbon atoms e.g. Cl-C9-heteroaryl indicates the number of carbon atoms contained in the heteroaryl group without including the number of heteroatoms. For example a C1-C9-heteroaryl will include an additional one to four heteroatoms. A polycyclic heteroaryl may include one or more rings that are partially saturated. Non-limiting examples of heteroaryls include:

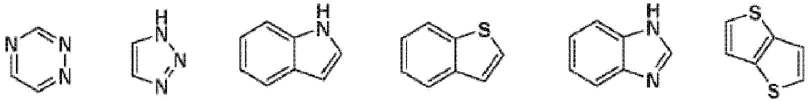

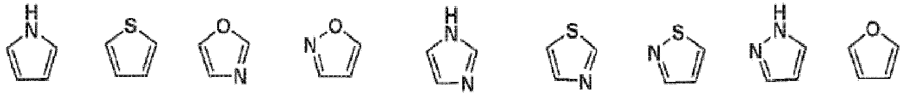

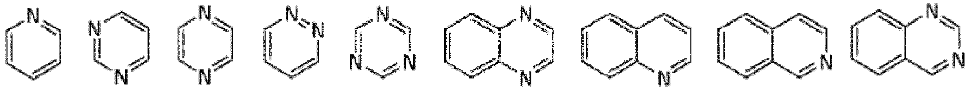

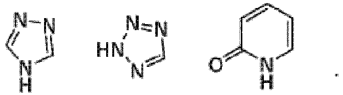

Additional non-limiting examples of heteroaryl groups include pyridyl, pyrazinyl, pyrimidinyl (including e.g. 2-and 4-pyrimidinyl), pyridazinyl, thienyl, furyl, pyrrolyl (including e.g., 2-pyrrolyl), imidazolyl, thiazolyl, oxazolyl, pyrazolyl (including e.g. 3- and 5-pyrazolyl), isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyland 1,3,4-oxadiazolyl. Non-limiting examples of polycyclic heterocycles and heteroaryls include indolyl (including 3-, 4-, 5-, 6- and 7-indolyl), indolinyl, quinolyl, tetrahydroquinolyl, isoquinolyl (including, e.g. 1-and 5-isoquinolyl), 1,2,3,4-tetrahydroisoquinolyl, cinnolinyl, quinoxalinyl (including, e .g 2- and 5-quinoxalinyl), quinazolinyl, phthalazinyl, 1,8-naphthyridinyl, 1,4-benzodioxanyl, coumarin, dihydrocoumarin, 1,5-naphthyridinyl, benzofuryl (including, e .g. 3-, 4-, 5-, 6-, and 7-benzofuryl), 2,3-dihydrobenzofuryl, 1,2-benzisoxazolyl, benzothienyl (including e.g. 3-, 4-, 5-, 6-, and 7-benzothienyl), benzoxazolyl, benzothiazolyl (including e.g. 2-benzothiazolyl and 5-benzothiazolyl), purinyl, benzimidazolyl (including e.g., 2-benzimidazolyl), benzotriazolyl, thioxanthinyl, carbazolyl, carbolinyl, acridinyl, pyrrolizidinyl and quinolizidinyl.

As used herein the term "haloalkyl" is typically a said alkyl, alkenyl, alkoxy or alkenoxy group respectively wherein any one or more of the carbon atoms is substituted with one or more said halo atoms as defined above. Haloalkyl embraces monohaloalkyl, dihaloalkyl, and polyhaloalkyl radicals. The term "haloalkyl"includes but is not limited to fluoromethyl, 1-fluoroethyl, difluoromethyl, 2,2-difluoroethyl, 2,2,2-trifluoroethyl, trifluoromethyl, chloromethyl, dichloromethyl, trichloromethyl, pentafluoroethyl, difluoromethoxy, and trifluoromethoxy.

As used herein, a C1-C6-hydroxyalkyl group is a said C1-C6 alkyl group substituted by one or more hydroxy groups. Typically, it is substituted by one, two or three hydroxyl groups. Preferably, it is substituted by a single hydroxy group.

As used herein, a C1-C6-aminoalkyl group is a said C1-C6 alkyl group substituted by one or more amino groups. Typically, it is substituted by one, two or three amino groups. Preferably, it is substituted by a single amino group.

As used herein, a C1-C4-carboxyalkyl group is a said C1-C4 alkyl group substituted by carboxyl group.

As used herein, a C1-C4-carboxamidoalkyl group is a said C1-C4 alkyl group substituted by a substituted or unsubstituted carboxamide group.

As used herein, a C1-C4-acylsulfonamido-alkyl group is a said C1-C4 alkyl group substituted by an acylsulfonamide group of general formula C(=O)NHSO₂CH₃ or C(=O)NHSO₂-c-Pr.

As used herein the term "cycloalkyl" refers to a monocyclic or polycyclic nonaromatic group wherein each of the atoms forming the ring (i.e. skeletal atoms) is a carbon atom. In one embodiment, the cycloalkyl group is saturated or partially unsaturated. In another embodiment, the cycloalkyl group is fused with an aromatic ring. Cycloalkyl groups include groups having 3 to 10 ring atoms (C3-C10-cycloalkyl), groups having 3 to 8 ring atoms (C3-C8-cycloalkyl), groups having 3 to 7 ring atoms (C3-C7-cycloalkyl) and groups having 3 to 6 ring atoms (C3-C6-cycloalkyl). Illustrative examples of cycloalkyl groups include, but are not limited to the following moieties:

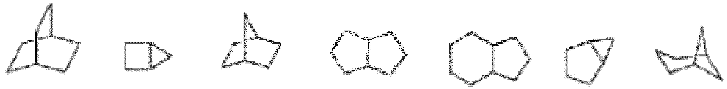

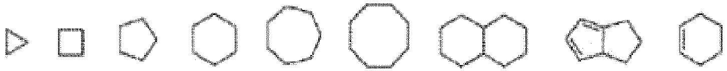

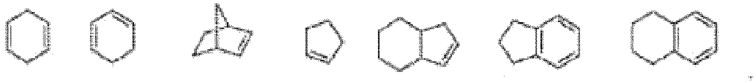

Monocyclic cycloalkyls include but are not limited to cyclopropyl, cyclobutyl, cyclopentyl, cyclohexyl, cycloheptyl, and cyclooctyl. Dicyclic cycloalkyls include but are not limited to tetrahydronaphthyl, indanyl, and tetrahydropentalene. Polycyclic cycloalkyls include adamantine and norbornane. The term cycloalkyl includes "unsaturated nonaromatic carbocyclyl" or "nonaromatic unsaturated carbocyclyl" groups both of which refer to a nonaromatic carbocycle as defined herein which contains at least one carbon-carbon double bond or one carbon-carbon triple bond.

As used herein the term "halo-cycloalkyl" is typically a said cycloalkyl wherein any one or more of the carbon atoms is substituted with one or more said halo atoms as defined above. Halo-cycloalkyl embraces monohaloalkyl, dihaloalkyl, and polyhaloalkyl radicals. Halo-cycloalkyl embraces 3,3-difluoro-cyclobutyl, 3-fluorocyclobutyl, 2-fluorocyclobutyl, 2,2-difluorocyclobutyl, and 2,2-difluorocyclopropyl.

As used herein the terms "heterocycloalkyl" and "heterocyclyl" refer to a heteroalicyclic group containing one or more rings (typically one, two or three rings), that contains one to four ring heteroatoms each selected from oxygen, sulfur and nitrogen. In one embodiment each heterocyclyl group has from 3 to 10 atoms in its ring system with the proviso that the ring of said group does not contain two adjacent oxygen or sulfur atoms. In one embodiment each heterocyclyl group has a fused bicyclic ring system with 3 to 10 atoms in the ring system, again with the proviso that the ring of said group does not contain two adjacent oxygen or sulfur atoms. In one embodiment each heterocyclyl group has a bridged bicyclic ring system with 3 to 10 atoms in the ring system, again with the proviso that the ring of said group does not contain two adjacent oxygen or sulfur atoms. In one embodiment each heterocyclyl group has a spiro-bicyclic ring system with 3 to 10 atoms in the ring system, again with the proviso that the ring of said group does not contain two adjacent oxygen or sulfur atoms. Heterocyclyl substituents may be alternatively defined by the number of carbon atoms e.g. C2-C8-heterocyclyl indicates the number of carbon atoms contained in the heterocyclic group without including the number of heteroatoms. For example a C2-C8-heterocyclyl will include an additional one to four heteroatoms. In another embodiment the heterocycloalkyl group is fused with an aromatic ring. In another embodiment the heterocycloalkyl group is fused with a heteroaryl ring. In one embodiment the nitrogen and sulfur heteroatoms may be optionally oxidized and the nitrogen atom may be optionally quaternized. The heterocyclic system may be attached, unless otherwise stated, at any heteroatom or carbon atom that affords a stable structure. An example of a 3-membered heterocyclyl group includes and is not limited to aziridine. Examples of 4-membered heterocycloalkyl groups include, and are not limited to azetidine and a beta-lactam. Examples of 5-membered heterocyclyl groups include, and are not limited to pyrrolidine, oxazolidine and thiazolidinedione. Examples of 6-membered heterocycloalkyl groups include, and are not limited to, piperidine, morpholine, piperazine, N-acetylpiperazine and N-acetylmorpholine. Other non-limiting examples of heterocyclyl groups are

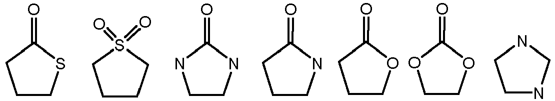

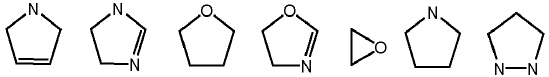

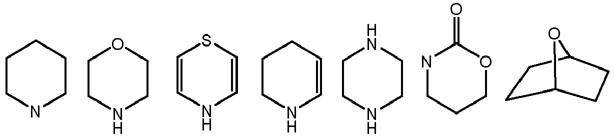

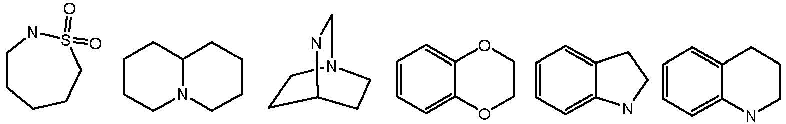

Examples of heterocycles include monocyclic groups such as aziridine, oxirane, thiirane, azetidine, oxetane, thietane, pyrrolidine, pyrroline, pyrazolidine, imidazoline, dioxolane, sulfolane, 2,3-dihydrofuran, 2,5-dihydrofuran, tetrahydrofuran, thiophane, piperidine, 1,2,3,6-tetrahydropyridine, 1,4-dihydropyridine, piperazine, morpholine, thiomorpholine, pyran, 2,3-dihydropyran, tetrahydropyran, 1,4-dioxane, 1,3-dioxane, 1,3-dioxolane, homopiperazine, homopiperidine, 1,3-dioxepane, 47-dihydro-1,3-dioxepin, and hexamethyleneoxide. The terms "C3-C7-heterocycloalkyl" includes but is not limited to tetrahydrofuran-2-yl, tetrahydrofuran-3-yl, 3-oxabicyclo[3.1.0]hexan-6-yl, 3-azabicyclo[3.1.0]hexan-6-yl, tetrahydropyran-4-yl, tetrahydropyran-3-yl, tetrahydropyran-2-yl, and azetidin-3-yl.

As used herein, the term "aromatic" refers to a carbocycle or heterocycle with one or more polyunsaturated rings and having aromatic character i.e. having (4n + 2) delocalized π(pi) electrons where n is an integer.

As used herein, the term "acyl", employed alone or in combination with other terms, means, unless otherwise stated, to mean to an alkyl, cycloalkyl, heterocycloalkyl, aryl or heteroaryl group linked via a carbonyl group.

As used herein, the terms "carbamoyl" and "substituted carbamoyl", employed alone or in combination with other terms, means, unless otherwise stated, to mean a carbonyl group linked to an amino group optionally mono or di-substituted by hydrogen, alkyl, cycloalkyl, heterocycloalkyl, aryl or heteroaryl. In some embodiments, the nitrogen substituents will be connected to form a heterocyclyl ring as defined above.

The term "prodrug" refers to a precursor of a drug that is a compound which upon administration to a patient, must undergo chemical conversion by metabolic processes before becoming an active pharmacological agent. Illustrative prodrugs of compounds in accordance with Formula I are esters and amides, preferably alkyl esters of fatty acid esters. Prodrug formulations here comprise all substances which are formed by simple transformation including hydrolysis, oxidation or reduction either enzymatically, metabolically or in any other way. A suitable prodrug contains e.g. a substance of general formula I bound via an enzymatically cleavable linker (e.g. carbamate, phosphate, N-glycoside or a disulfide group) to a dissolution-improving substance (e.g. tetraethylene glycol, saccharides, formic acids or glucuronic acid, etc.). Such a prodrug of a compound according to the invention can be applied to a patient, and this prodrug can be transformed into a substance of general formula I so as to obtain the desired pharmacological effect.

### Examples

The invention is now described with reference to the following Examples. These Examples are provided for the purpose of illustration only, and the invention is not limited to these Examples, but rather encompasses all variations that are evident as a result of the teachings provided herein.

The required substituted indole-2-carboxylic acids may be prepared in a number of ways; the main routes employed being outlined in Schemes 1-4. To the chemist skilled in the art it will be apparent that there are other methodologies that will also achieve the preparation of these intermediates.

Substituted indole-2-carboxylic acids can be prepared via the Hemetsberger-Knittel reaction (Organic Letters, 2011, 13(8) pp. 2012-2014, Journal of the American Chemical Society, 2007, pp. 7500-7501, and Monatshefte für Chemie, 103(1), pp. 194-204) (Scheme 1).

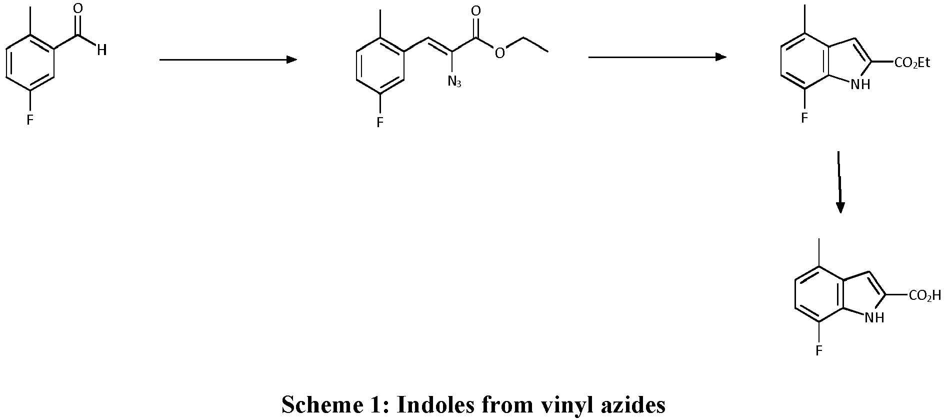

Substituted indoles may also be prepared using the Fischer method (Berichte der Deutschen Chemischen Gesellschaft. 17 (1): 559-568) (Scheme 2).

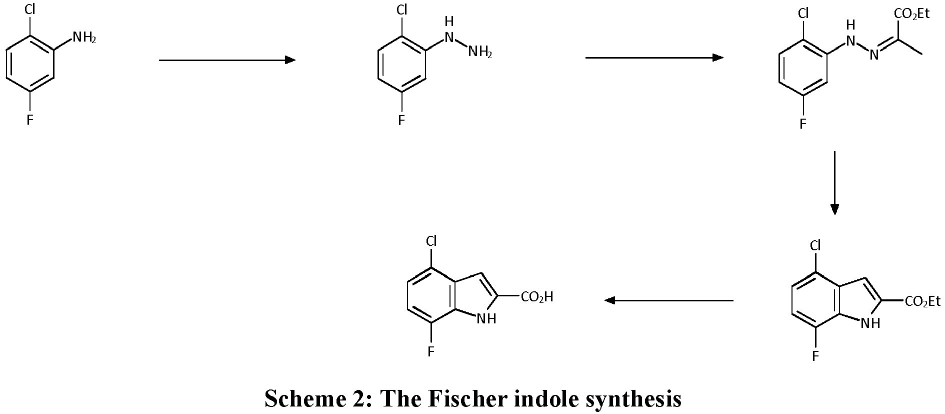

A further method for the preparation of substituted indoles is the palladium catalysed alkyne annulation reaction (Journal of the American Chemical Society, 1991, pp. 6690-6692) (Scheme 3).

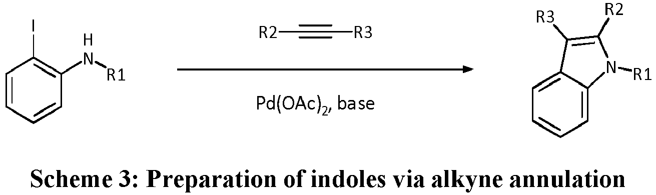

Additionally, indoles may be prepared from other suitably functionalized (halogenated) indoles (for example via palladium catalysed cross coupling or nucleophilic substitution reactions) as illustrated in Scheme 4.

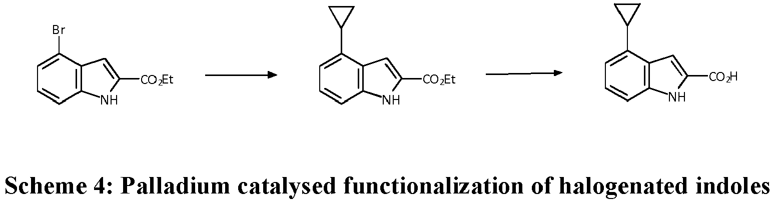

Chemists skilled in the art will appreciate that other methods are available for the synthesis of suitably functionalized indole-2-carboxylic acids and activated esters thereof.

The required substituted indolizine-2-carboxylic acids may be prepared in a number of ways, the main routes employed being outlined in Schemes 5-7. To the chemist skilled in the art it will be apparent that there are other methodologies that will also achieve the preparation of these intermediates.

Substituted indolizine-2-carboxylic acids may be prepared by the Morita-Bayliss-Hilmann reaction on suitably substituted pyridine-2-carbaldehydes, as shown in Scheme 5.

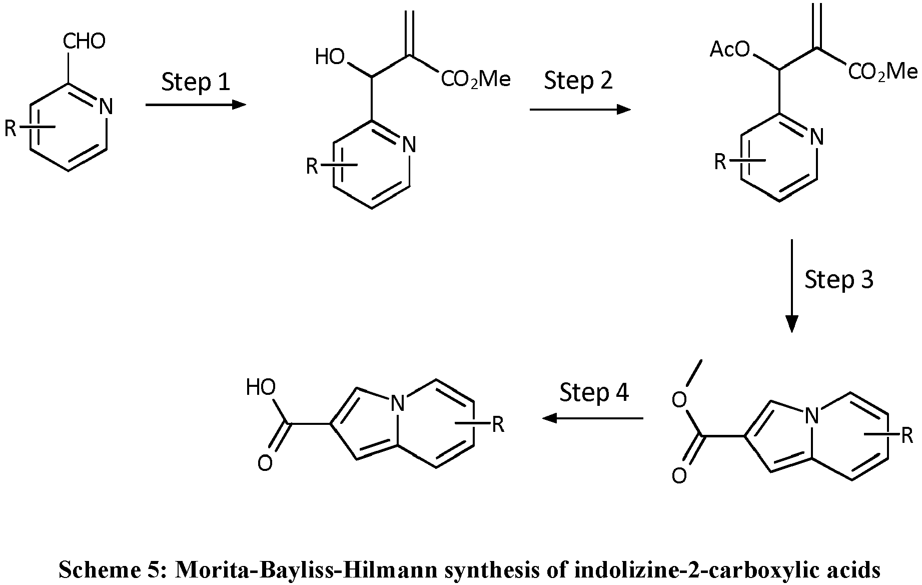

In Step 1 of Scheme 5, a suitably functionalized pyridine-2-carbaldehyde is reacted with methyl prop-2-enoate (methyl acrylate) and a tertiary amine e.g. 1,4-diazabicyclo[2.2.2]octane (DABCO) to give the Morita-Bayliss-Hillman adduct. In Step 2, this adduct is then acylated by, for example, acetic anhydride to give the ester, which is then cyclized under heating in Step 3 to give the indolizine-2-carboxylic acid ester. Hydrolysis of the ester in Step 4 with, for example, aqueous sodium hydroxide gives the desired indolizine-2-carboxylic acid.

Substituted indolizine-2-carboxylic acids may also be prepared by the Chichibabin reaction, using suitably functionalized 2-methyl-pyridines (2-picolines) as shown in Scheme 6.

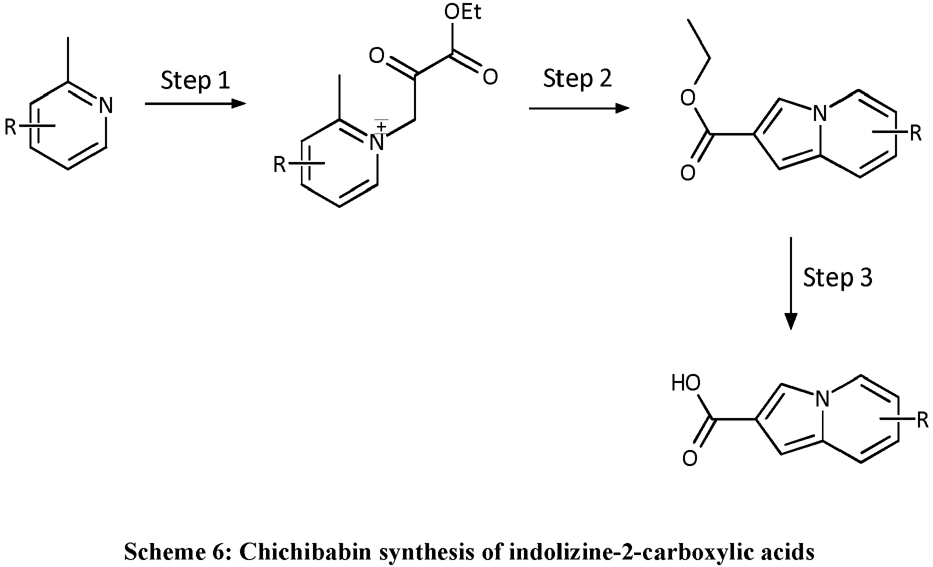

In Step 1 of Scheme 6, a suitably functionalized 2-methyl-pyridine (picoline) is reacted with an ester of bromopyruvic acid, for example ethyl bromopyruvate (as drawn) or tert-butyl 3-bromo-2-oxopropionate, to give the pyridinium salt. This adduct is then cyclized under basic conditions in Step 2 by, for example, caesium carbonate to give the indolizine ester. Hydrolysis of the carboxylic acid ester in Step 3 with, for example, aqueous sodium hydroxide gives the desired indolizine-2-carboxylic acid.

Substituted indolizine-2-carboxylic acids may also be prepared by the further functionalization of a substituted indolizine as shown in Scheme 7.

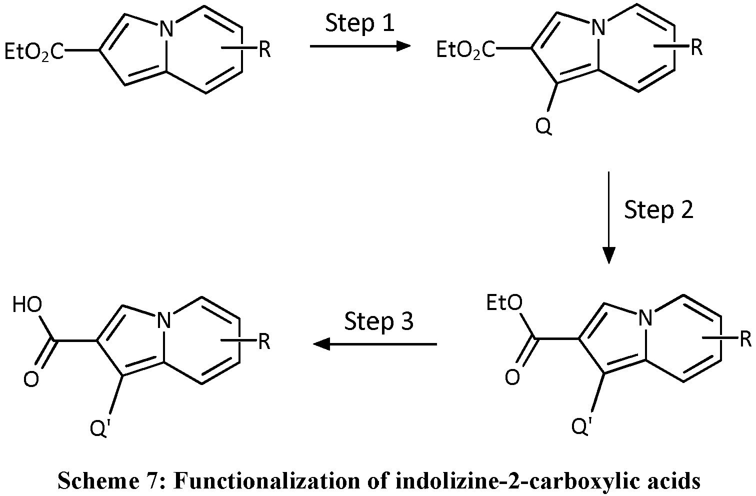

In Step 1 of Scheme 7, a suitably functionalized indolizine is reacted with a formylating or halogenating agent, for example N-bromo-succinimide or bromine, to give the substituted indolizine. This adduct can then be further functionalized by methods well known in the art in Step 2 by, for example, metalation-quenching, palladium catalysed cross-coupling reaction, or Wittig reaction. Hydrolysis of the carboxylic acid ester in Step 3 with, for example, aqueous sodium hydroxide gives an indolizine-2-carboxylic acid.

Chemists skilled in the art will appreciate that other methods are available for the synthesis of suitably functionalized indolizine-2-carboxylic acids and activated esters thereof.

In a preferred embodiment, compounds of Formula I can be prepared as shown in Scheme 8 below.

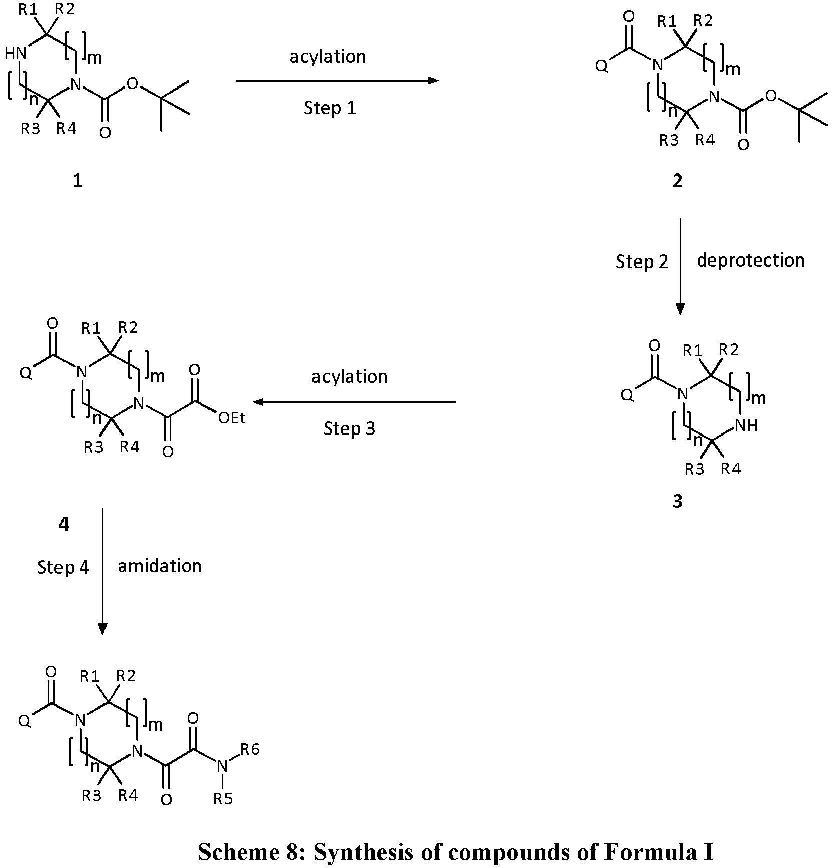

Compound 1 described in Scheme 8 is in step 1 acylated with methods known in literature (A. El-Faham, F. Albericio, Chem. Rev. 2011, 111, 6557-6602), e.g. with HATU results in compounds of structure 2. Deprotection of the nitrogen protective group (A. Isidro-Llobet et al., Chem. Rev., 2009, 109, 2455-2504), drawn as but not limited to Boc, e.g. with HCl gives amine 3. Acylation with ethyl chlorooxoacetate (J. Med. Chem. 60(16) pp. 6942-6990) in step 3 results in compounds of structure 4. Aminolysis of the ester, drawn as but not limited to the ethyl ester gives a compound of Formula I.

In a further embodiment, compounds of Formula I can be prepared as shown in Scheme 9 below.

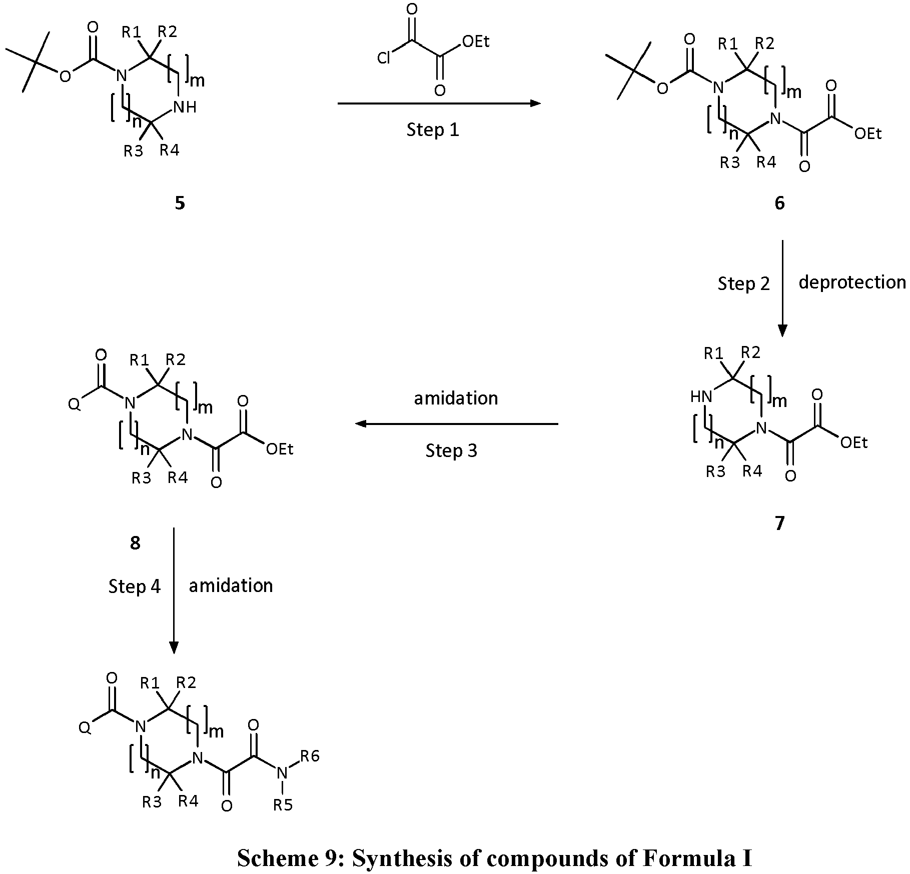

Compound 5 described in Scheme 9 is in step 1 acylated with ethyl chlorooxoacetate (J. Med. Chem. 60(16) pp. 6942-6990) to obtain compounds with the general structure 6. Deprotection of the nitrogen protective group (A. Isidro-Llobet et al., Chem. Rev., 2009, 109, 2455-2504), drawn as but not limited to Boc, e.g. with HCl gives amine 7. An amide coupling in step 3 with methods known in literature (A. El-Faham, F. Albericio, Chem. Rev. 2011, 111, 6557-6602), e.g. with HATU results in compounds of structure 8. Aminolysis of the ester, drawn as but not limited to the ethyl ester gives a compound of Formula I.

In a further embodiment, compounds of Formula I can be prepared as shown in Scheme 10 below.

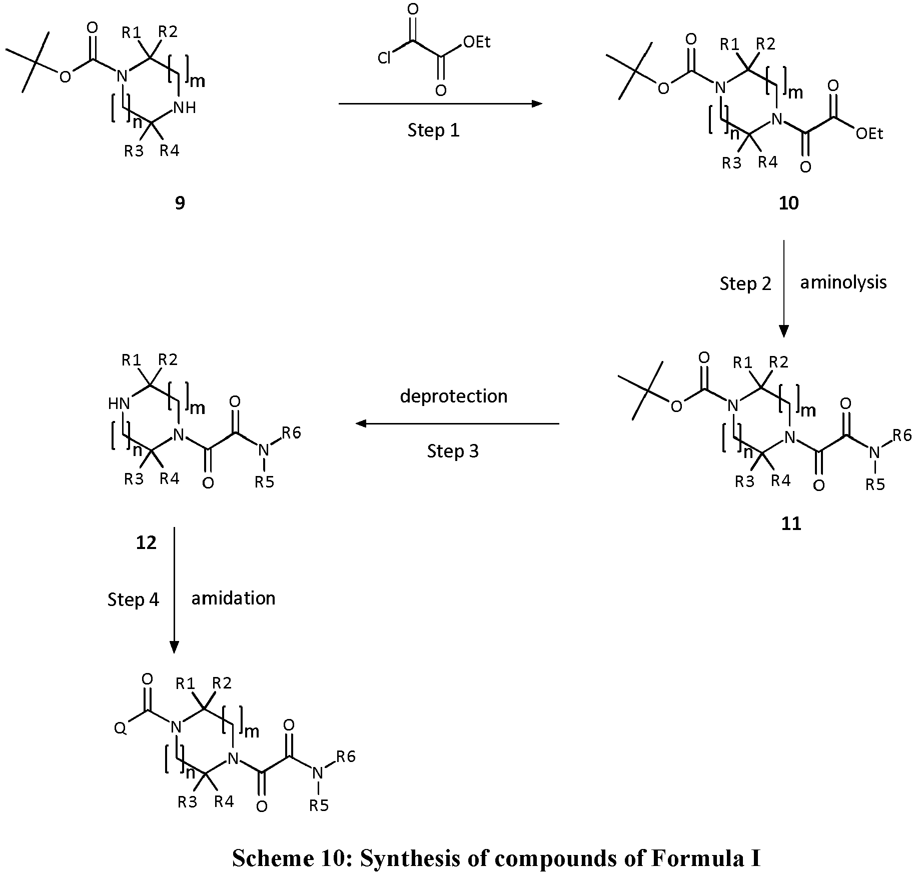

Compound 9 described in Scheme 10 is in step 1 acylated with ethyl chlorooxoacetate (J. Med. Chem. 60(16) pp. 6942-6990) to obtain compounds with the general structure 10. Aminolysis of the ester, drawn as but not limited to the ethyl ester, results in compounds of structure 11. Deprotection of the nitrogen protective group (A. Isidro-Llobet et al., Chem. Rev., 2009, 109, 2455-2504), drawn as but not limited to Boc, e.g. with HCl gives amine 12. An amide coupling in step 5 with methods known in literature (A. El-Faham, F. Albericio, Chem. Rev. 2011, 111, 6557-6602), e.g. with HATU results in compounds of Formula I.

In a further embodiment, compounds of Formula I can be prepared as shown in Scheme 11 below.

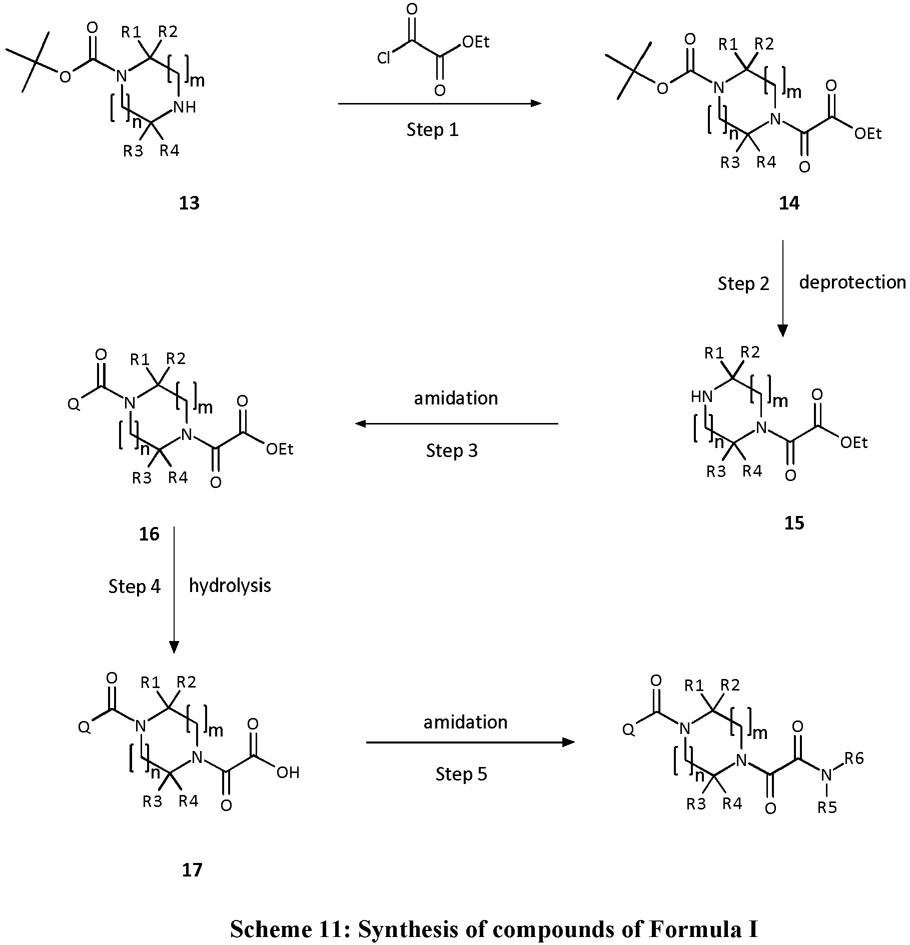

Compound 13 described in Scheme 11 is in step 1 acylated with ethyl chlorooxoacetate (J. Med. Chem. 60(16) pp. 6942-6990) to obtain compounds with the general structure 14. Deprotection of the nitrogen protective group (A. Isidro-Llobet et al., Chem. Rev., 2009, 109, 2455-2504), drawn as but not limited to Boc, e.g. with HCl gives amine 15. An amide coupling in step 3 with methods known in literature (A. El-Faham, F. Albericio, Chem. Rev. 2011, 111, 6557-6602), e.g. with HATU results in compounds of structure 16. Hydrolysis of the ester, drawn as but not limited to the ethyl ester with, for example aqueous sodium hydroxide gives a carboxylic acid of general structure 17. An amide coupling in step 5 with methods known in literature (A. El-Faham, F. Albericio, Chem. Rev. 2011, 111, 6557-6602), e.g. with HATU results in compounds of compound of Formula I.

In a further embodiment compounds of Formula II can be prepared as shown in Scheme 12 below.

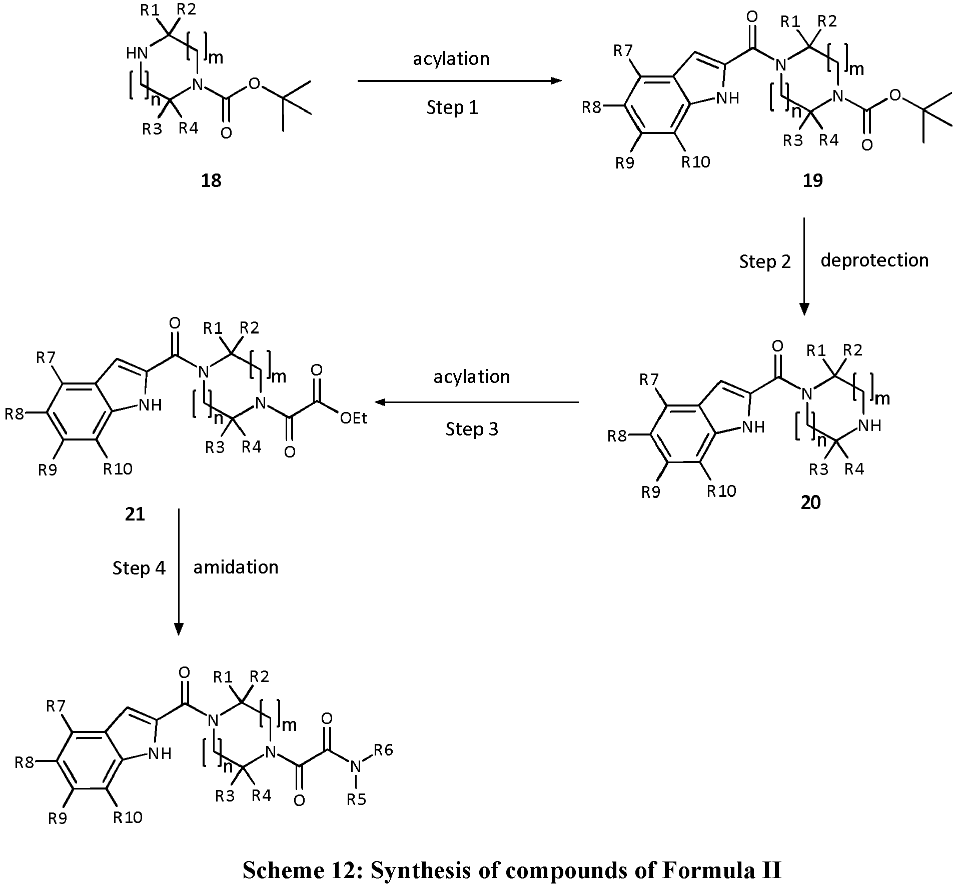

Compound 18 described in Scheme 12 is in step 1 acylated with methods known in literature (A. El-Faham, F. Albericio, Chem. Rev. 2011, 111, 6557-6602), e.g. with HATU results in compounds of structure 19. Deprotection of the nitrogen protective group (A. Isidro-Llobet et al., Chem. Rev., 2009, 109, 2455-2504), drawn as but not limited to Boc, e.g. with HCl gives amine 20. Acylation with ethyl chlorooxoacetate (J. Med. Chem. 60(16) pp. 6942-6990) in step 3 results in compounds of structure 21. Aminolysis of the ester, drawn as but not limited to the ethyl ester gives a compound of Formula II.

In a further embodiment, compounds of Formula II can be prepared as shown in Scheme 13 below.

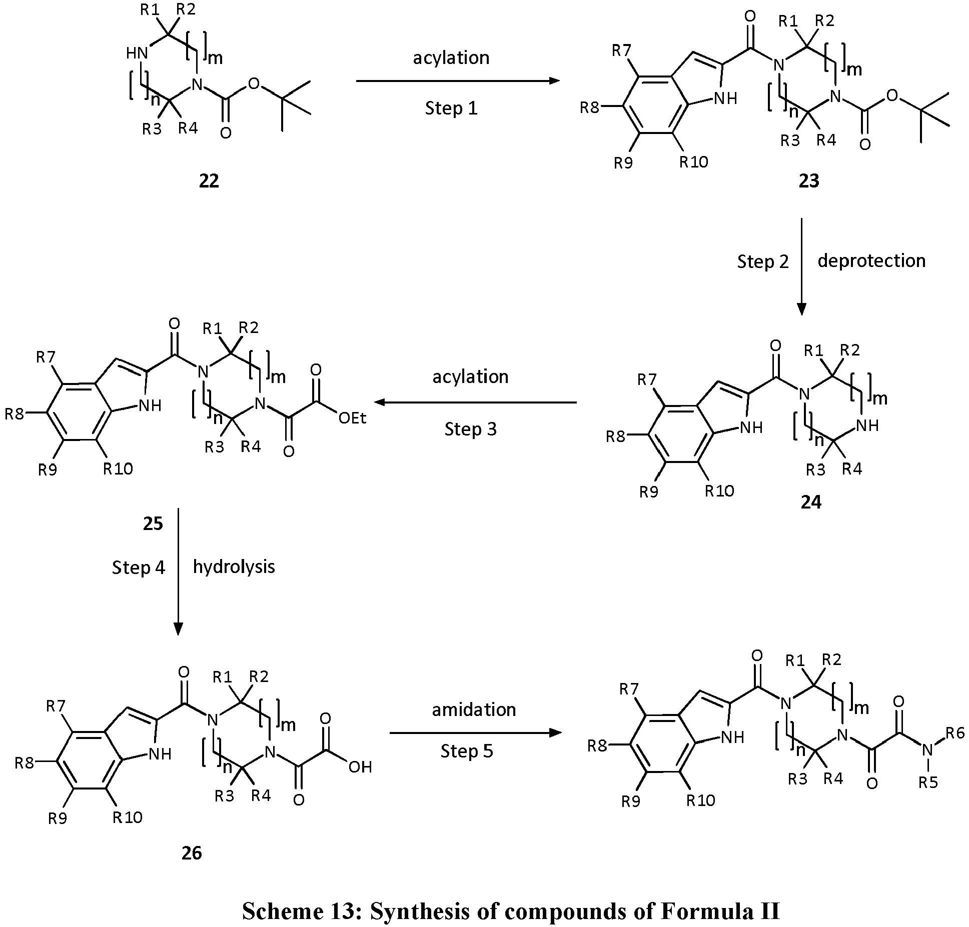

Compound 22 described in Scheme 13 is in step 1 acylated with methods known in literature (A. El-Faham, F. Albericio, Chem. Rev. 2011, 111, 6557-6602), e.g. with HATU results in compounds of structure 23. Deprotection of the nitrogen protective group (A. Isidro-Llobet et al., Chem. Rev., 2009, 109, 2455-2504), drawn as but not limited to Boc, e.g. with HCl gives amine 24. Acylation with ethyl chlorooxoacetate (J. Med. Chem. 60(16) pp. 6942-6990) in step 3 results in compounds of structure 25. Hydrolysis of the ester, drawn as but not limited to the ethyl ester with, for example aqueous sodium hydroxide, gives an acid of structure 26, which can then be amidated with methods known in literature (A. El-Faham, F. Albericio, Chem. Rev. 2011, 111, 6557-6602), e.g. with HATU to give compounds of Formula II.

In a further embodiment compounds of Formula III can be prepared as shown in Scheme 14 below.

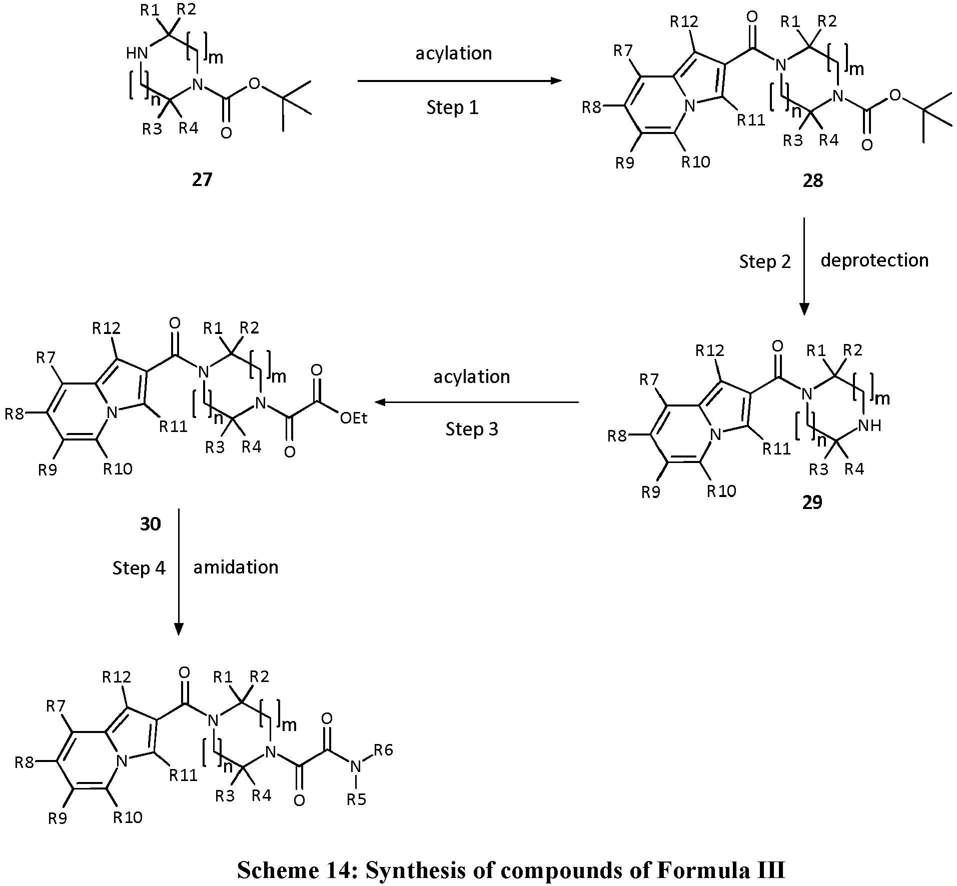

Compound 27 described in Scheme 14 is in step 1 acylated with methods known in literature (A. El-Faham, F. Albericio, Chem. Rev. 2011, 111, 6557-6602), e.g. with HATU results in compounds of structure 28. Deprotection of the nitrogen protective group (A. Isidro-Llobet et al., Chem. Rev., 2009, 109, 2455-2504), drawn as but not limited to Boc, e.g. with HCl gives amine 29. Acylation with ethyl chlorooxoacetate (J. Med. Chem. 60(16) pp. 6942-6990) in step 3 results in compounds of structure 30. Aminolysis of the ester, drawn as but not limited to the ethyl ester gives a compound of Formula III.

In a further embodiment, compounds of Formula III can be prepared as shown in Scheme 15 below.

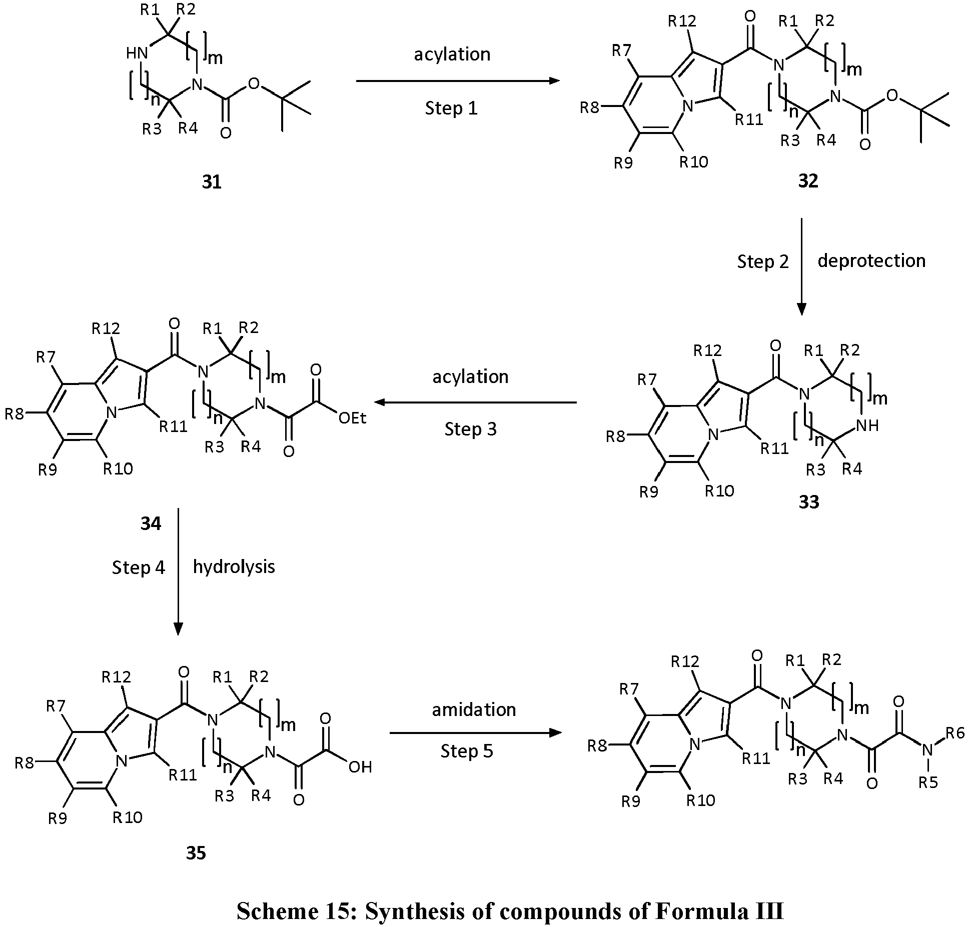

Compound 31 described in Scheme 15 is in step 1 acylated with methods known in literature (A. El-Faham, F. Albericio, Chem. Rev. 2011, 111, 6557-6602), e.g. with HATU results in compounds of structure 32. Deprotection of the nitrogen protective group (A. Isidro-Llobet et al., Chem. Rev., 2009, 109, 2455-2504), drawn as but not limited to Boc, e.g. with HCl gives amine 33. Acylation with ethyl chlorooxoacetate (J. Med. Chem. 60(16) pp. 6942-6990) in step 3 results in compounds of structure 34. Hydrolysis of the ester, drawn as but not limited to the ethyl ester with, for example aqueous sodium hydroxide, gives an acid of structure 35, which can then be amidated with methods known in literature (A. El-Faham, F. Albericio, Chem. Rev. 2011, 111, 6557-6602), e.g. with HATU to give compounds of Formula III.

The following abbreviations are used:
A - DNA nucleobase adenine
ACN - acetonitrile
Ar - argon
BODIPY-FL - 4,4-difluoro-5,7-dimethyl-4-bora-3a,4a-diaza-s-indacene-3-propionic acid (fluorescent dye)
Boc - tert-butoxycarbonyl
BnOH - benzyl alcohol
*n*-BuLi - n-butyl lithium
*t*-BuLi - t-butyl lithium
C - DNA nucleobase cytosine
CC₅₀ - half-maximal cytotoxic concentration
CO₂ - carbon dioxide
CuCN - copper (I) cyanide
DABCO - 1,4-diazabicyclo[2.2.2]octane
DCE - dichloroethane
DCM - dichloromethane
Dess-Martin periodinane - 1,1,1-triacetoxy-1,1-dihydro-1,2-benziodoxol-3(1H)-one
DIPEA - diisopropylethylamine
DIPE - di-isopropyl ether
DMAP - 4-dimethylaminopyridine
DMF - N,N-dimethylformamide
DMP - Dess-Martin periodinane
DMSO - dimethyl sulfoxide
DNA - deoxyribonucleic acid
DPPA - diphenylphosphoryl azide
DTT - dithiothreitol
EC₅₀ - half-maximal effective concentration
EDCI - N-(3-dimethylaminopropyl)-N'-ethylcarbodiimide hydrochloride
Et₂O - diethyl ether
EtOAc - ethyl acetate
EtOH - ethanol
FL- - five prime end labled with fluorescein
NEt₃ - triethylamine
ELS - Evaporative Light Scattering
g - gram(s)
G - DNA nucleobase guanine
HBV - hepatitis B virus
HATU - 2-(1H-7-azabenzotriazol-1-yl)-1,1,3,3-tetramethyl uronium hexafluorophosphate
HCl - hydrochloric acid
HEPES - 4-(2-hydroxyethyl)-1-piperazineethanesulfonic acid
HOAt - 1-hydroxy-7-azabenzotriazole
HOBt - 1-hydroxybenzotriazole
HPLC - high performance liquid chromatography
IC₅₀ - half-maximal inhibitory concentration
LC640- - 3 prime end modification with fluorescent dye LightCycler^{®} Red 640
LC/MS - liquid chromatography/mass spectrometry
LiAlH₄ - lithium aluminium hydride
LiOH - lithium hydroxide
Me - methyl
MeOH - methanol
MeCN - acetonitrile
MgSO₄ - magnesium sulfate
mg - milligram(s)
min - minutes
mol - moles
mmol - millimole(s)
mL - millilitre(s)
MTBE - methyl tert-butyl ether
N₂ - nitrogen
Na₂CO₃ - sodium carbonate
NaHCO₃ - sodium hydrogen carbonate
Na₂SO₄ - sodium sulfate
NdeI - restriction enzyme recognizes CA^TATG sites
NEt₃ - triethylamine
NaH - sodium hydride
NaOH - sodium hydroxide
NH₃ - ammonia
NH₄Cl - ammonium chloride
NMR - nuclear magnetic resonance
PAGE - polyacrylamide gel electrophoresis
PCR - polymerase chain reaction
qPCR - quantitative PCR
Pd/C - palladium on carbon
-PH - 3 prime end phosphate modification
pTSA - 4-toluene-sulfonic acid
*Rt* - retention time
r.t. - room temperature
sat. - saturated aqueous solution
SDS - sodium dodecyl sulfate
SI - selectivity index (= CC₅₀/EC₅₀)
STAB - sodium triacetoxyborohydride
T - DNA nucleobase thymine
TBAF - tetrabutylammonium fluoride
TEA - triethylamine
TFA - trifluoroacetic acid
THF - tetrahydrofuran
TLC - thin layer chromatography
Tris - tris(hydroxymethyl)-aminomethane
XhoI - restriction enzyme recognizes C^TCGAG sites

### Compound identification - NMR

For a number of compounds, NMR spectra were recorded either using a Bruker DPX400 spectrometer equipped with a 5 mm reverse triple-resonance probe head operating at 400 MHz for the proton and 100 MHz for carbon, a Bruker Advance spectrometer operating at 400 MHz, 300 MHz or 500 MHz (¹H) and 100 MHz ¹³C{¹H}), in CDCl3 or DMSO-d6 at room temperature, or using a Bruker DRX500 spectrometer equipped with a 5 mm reverse triple-resonance probe head operating at 500 MHz for the proton and 125 MHz for carbon. Deuterated solvents were chloroform-d (deuterated chloroform, CDCl₃) or d6-DMSO (deuterated DMSO, d6-dimethylsulfoxide). Chemical shifts are reported in parts per million (ppm) relative to tetramethylsilane (TMS) which was used as internal standard. Starting material and reagents were purchased from Sigma-Aldrich, Alfa Aesar, Acros and Fluorochem and were used as supplied unless stated otherwise. Reactions were monitored via thin layer chromatography (TLC) on pre-coated aluminium backed plates. Products were visualised by UV light (254 nm) and/or with Ehrlich's reagent stain.

Continuous flow experiments were performed using VapourTec R2+R4. HPLC analysis performed with HPLC Agilent. Flash chromatography purifications were carried out using 60 mesh silica gel and dry-packed columns.

### Compound identification - HPLC/MS

For a number of compounds, LC-MS spectra were recorded using the following analytical methods.

### Method A

Column - Reverse phase Waters Xselect CSH C18 (50x2.1mm, 3.5 micron)
Flow - 0.8 mL/min, 25 degrees Celsius
Eluent A - 95% acetonitrile + 5% 10mM ammonium carbonate in water (pH 9)
Eluent B - 10mM ammonium carbonate in water (pH 9)
Linear gradient t=0 min 5% A, t=3.5 min 98% A. t=6 min 98% A

### Method A2

Column - Reverse phase Waters Xselect CSH C18 (50x2.1mm, 3.5 micron)
Flow - 0.8 mL/min, 25 degrees Celsius
Eluent A - 95% acetonitrile + 5% 10mM ammonium carbonate in water (pH 9)
Eluent B - 10mM ammonium carbonate in water (pH 9)
Linear gradient t=0 min 5% A, t=4.5 min 98% A. t=6 min 98% A

### Method B

Column - Reverse phase Waters Xselect CSH C18 (50x2.1mm, 3.5 micron)
Flow - 0.8 mL/min, 35 degrees Celsius
Eluent A - 0.1% formic acid in acetonitrile
Eluent B - 0.1% formic acid in water
Linear gradient t=0 min 5% A, t=3.5 min 98% A. t=6 min 98% A

### Method B2

Column - Reverse phase Waters Xselect CSH C18 (50x2.1mm, 3.5 micron)
Flow - 0.8 mL/min, 40 degrees Celsius
Eluent A - 0.1% formic acid in acetonitrile
Eluent B - 0.1% formic acid in water
Linear gradient t=0 min 5% A, t=4.5 min 98% A. t=6 min 98% A

### Method C

Column - Reverse phase Waters Xselect CSH C18 (50x2.1mm, 3.5 micron)
Flow - 1 mL/min, 35 degrees Celsius
Eluent A - 0.1% formic acid in acetonitrile
Eluent B - 0.1% formic acid in water
Linear gradient t=0 min 5% A, t=1.6 min 98% A. t=3 min 98% A

### Method D

Column - Phenomenex Gemini NX C18 (50 × 2.0 mm, 3.0 micron)
Flow - 0.8 mL/min, 35 degrees Celsius
Eluent A - 95% acetonitrile + 5% 10mM ammonium bicarbonate in water
Eluent B - 10mM ammonium bicarbonate in water pH=9.0
Linear gradient t=0 min 5% A, t=3.5 min 98% A. t=6 min 98% A

### Method E

Column - Phenomenex Gemini NX C18 (50 × 2.0mm, 3.0 micron)
Flow - 0.8 mL/min, 25 degrees Celsius
Eluent A - 95% acetonitrile + 5% 10mM ammonium bicarbonate in water
Eluent B - 10mM ammonium bicarbonate in water (pH 9)
Linear gradient t=0 min 5% A, t=3.5 min 30% A. t=7 min 98% A, t=10 min 98% A

### Method F

Column - Waters XSelect HSS C18 (150 × 4.6mm, 3.5 micron)
Flow - 1.0 mL/min, 25 degrees Celsius
Eluent A - 0.1% TFA in acetonitrile
Eluent B - 0.1% TFA in water
Linear gradient t=0 min 2% A, t=1 min 2% A, t=15 min 60% A, t=20 min 60% A

### Method G

Column - Zorbax SB-C18 1.8 µm 4.6x15mm Rapid Resolution cartridge (PN 821975-932)
Flow - 3 mL/min
Eluent A - 0.1% formic acid in acetonitrile
Eluent B - 0.1% formic acid in water
Linear gradient t=0 min 0% A, t=1.8 min 100% A

### Method H

Column - Waters Xselect CSH C18 (50x2.1mm, 2.5 micron)
Flow - 0.6 mL/min
Eluent A - 0.1% formic acid in acetonitrile
Eluent B - 0.1% formic acid in water
Linear gradient t=0 min 5% A, t=2.0 min 98% A, t=2.7 min 98% A

### Method J

Column - Reverse phase Waters Xselect CSH C18 (50x2.1mm, 2.5 micron)
Flow - 0.6 mL/min
Eluent A - 100% acetonitrile
Eluent B - 10mM ammonium bicarbonate in water (pH 7.9)
Linear gradient t=0 min 5% A, t=2.0 min 98% A, t=2.7 min 98% A

### Method K

Column - Luna 3u 100A C18(2) 100x2.0 mm
Flow - 0.5 mL/min
Eluent A - H₂O
Eluent B - MeCN
Linear gradient - 0-3 min 95 to 50% water; 3-13 min 50 to 5% water

### Method L

Column - Zorbax Eclipse C18 100 × 4.6mm, 3.5 *µ* m
Flow - 1 mL/min
Eluent A - H₂O
Eluent B - MeOH
Linear gradient 50% MeOH up to 100% in 10', hold 5

### Synthesis of indole-2-carboxylic acids

### Preparation of 4-chloro-7-fluoro-1H-indole-2-carboxylic acid

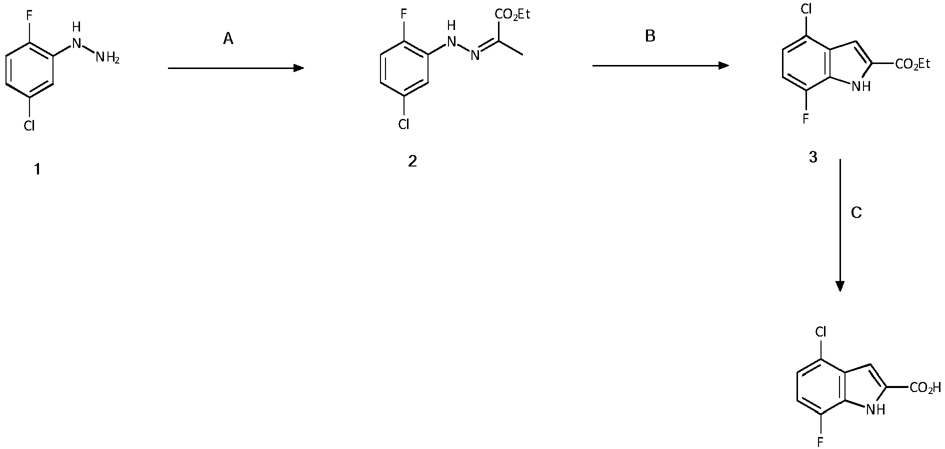

**Step A:** A mixture of compound 1·HCl (17.0 g, 86.2 mmol), sodium acetate (7.10 g, 86.6 mmol), and ethyl pyruvate (10.0 g, 86.1 mmol) in ethanol (100 mL) was refluxed for 1h, cooled to r.t., and diluted with water (100 mL). The precipitated solid was collected by filtration and dried to obtain 20.0 g (77.3 mmol, 90%) of compound 2 as a mixture of *cis-* and *trans-* isomers.

**Step B:** A mixture of compound 2 (20.0 g, 77.3 mmol), obtained in the previous step, and BF₃·Et₂O (50.0 g, 352 mmol) in acetic acid (125 mL) was refluxed for 18h and evaporated under reduced pressure. The residue was mixed with water (100 mL) and extracted with MTBE (2× 50 mL). The combined organic extracts were dried over Na₂SO₄ and evaporated under reduced pressure. The residue was purified by silica gel column chromatography to give 3.00 g (12.4 mmol, 16%) of compound 3.

**Step C:** A mixture of compound 3 (3.00 g, 12.4 mmol) and NaOH (0.500 g, 12.5 mmol) in ethanol (30 mL) was refluxed for 30 min and evaporated under reduced pressure. The residue was mixed with water (30 mL) and the insoluble material was filtered off. The filtrate was acidified with concentrated hydrochloric acid (5 mL). The precipitated solid was collected by filtration, washed with water (3 mL), and dried to obtain 2.41 g (11.3 mmol, 91%) of 4-chloro-7-fluoro-1H-indole-2-carboxylic acid.
*Rt* (Method G) 1.24 mins, *m*/*z* 212 [M-H]⁻

### Preparation of 7-fluoro-4-methyl-1H-indole-2-carboxylic acid

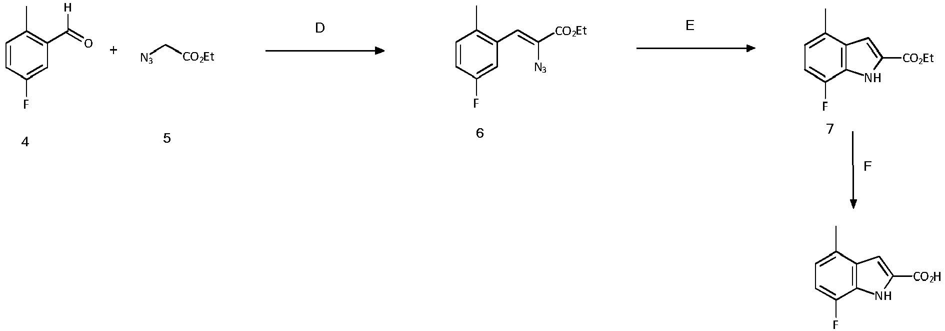

**Step D:** To a solution of sodium methoxide (21.6 g, 400 mmol) in methanol (300 mL) at at - 10°C was added dropwise a solution of compound 4 (26.4 g, 183 mmol) and compound 5 (59.0 g, 457 mmol) in methanol (100 mL). The reaction mass was stirred for 3 h maintaining temperature below 5°C and then quenched with ice water. The resulting mixture was stirred for 10 min, filtered, and washed with water to afford 35.0 g (156 mmol, 72%) of compound 6 as a white solid.

**Step E:** A solution of compound 6, obtained in the previous step, (35.0 g, 156 mmol) in xylene (250 mL) was refluxed for 1h under an argon atmosphere and then evaporated under reduced pressure. The residue was recrystallized form hexane-ethyl acetate mixture (60:40) to give 21.0 g (103 mmol, 60%) of compound 7.

**Step F:** To a solution of compound 7 (21.0 g, 101 mmol) in ethanol (200 mL) was added 2 N aqueous sodium hydroxide solution (47 mL). The mixture was stirred for 2h at 60°C. The solvent was evaporated and the residue was acidified with aqueous hydrochloric acid to pH 5-6. The resulting precipitate was filtered, washed with water, and dried to obtain 18.0 g (93.2 mmol, 92%) of 7-fluoro-4-methyl-1H-indole-2-carboxylic acid.
*Rt* (Method G) 1.12 mins, *m*/*z* 192 [M-H]⁻

### Preparation of 6,7-difluoro-1H-indole-2-carboxylic acid

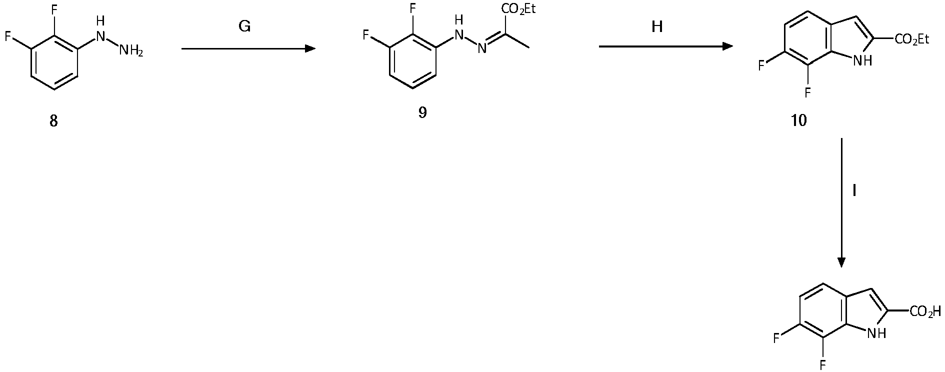

**Step G:** A mixture of compound 8 (5.00 g, 34.7 mmol), acetic acid (1 mL), and ethyl pyruvate (5.00 g, 43.1 mmol) in ethanol (20 mL) was refluxed for 1h, cooled to r.t., and diluted with water (20 mL). The precipitated solid was collected by filtration and dried to obtain 5.50 g (22.7 mmol, 66%) of compound 9 as a mixture of *cis-* and *trans-* isomers.

**Step H:** A mixture of compound 9 (5.50 g, 22.7 mmol), obtained in the previous step, and BF₃·Et₂O (10.0 g, 70.5 mmol) in acetic acid (25 mL) was refluxed for 18h and evaporated under reduced pressure. The residue was mixed with water (30 mL) and extracted with MTBE (2× 30 mL). The combined organic extracts were dried over Na₂SO₄ and evaporated under reduced pressure. The residue was purified by silica gel column chromatography to give 0.460 g (2.04 mmol, 9%) of compound 10.

**Step I:** A mixture of compound 10 (0.450 g, 2.00 mmol) and NaOH (0.100 g, 2.50 mmol) in ethanol (10 mL) was refluxed for 30 min and evaporated under reduced pressure. The residue was mixed with water (10 mL) and the insoluble material was filtered off. The filtrate was acidified with concentrated hydrochloric acid (1 mL). The precipitated solid was collected by filtration, washed with water (3 mL), and dried to obtain 0.38 g (1.93 mmol, 95%) of 6,7-difluoro-1H-indole-2-carboxylic acid.
*Rt* (Method G) 1.10 mins, *m*/*z* 196 [M-H]⁻

### Preparation of 4-cyano-1H-indole-2-carboxylic acid

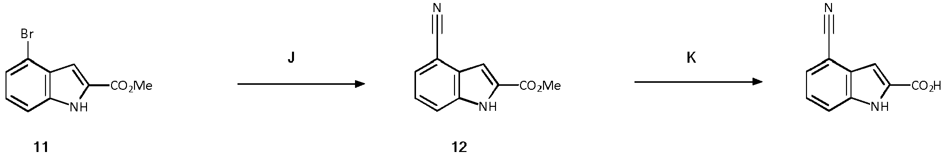

**Step J:** To a stirred solution of compound 11 (5.00 g, 19.7 mmol) in DMF (50 mL) was added CuCN (3.00 g, 33.5 mmol). The mixture was stirred for 4h at 150°C. The mixture was then cooled to r.t., and water (100 mL) added. The resulting mixture was extracted with ethyl acetate (4× 100 mL). The combined organic extracts were washed with water (50 mL) and brine (50 mL), dried over Na₂SO₄, and evaporated under reduced pressure to give 2.50 g (12.5 mmol, 63%) of compound 12, pure enough for the next step.

**Step K:** To a solution of compound 12 (2.50 g, 12.5 mmol) in ethanol (30 mL) was added LiOH·H₂O (0.600 g, 13.0 mmol). The mixture was refluxed for 10h. The solvent was evaporated under reduced pressure and the residue diluted with water (50 mL). The aqueous layer was acidified to pH 6 with 10% aq. hydrochloric acid and the precipitated solid was collected by filtration. The residue was washed with water and dried under vacuum to afford 1.20 g (6.45 mmol, 52%) of 4-cyano-1H-indole-2-carboxylic acid as a white solid.
*Rt* (Method G) 1.00 mins, *m*/*z* 197 [M+H]⁺

### Preparation of 4-cyano-7-fluoro-1H-indole-2-carboxylic acid

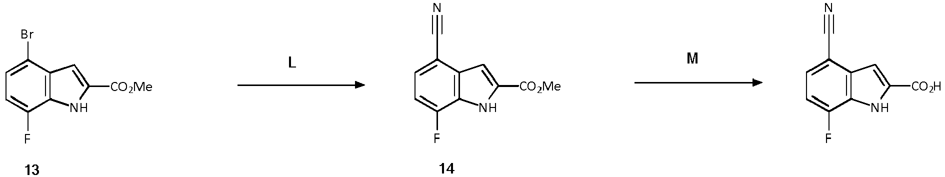

**Step L:** To a stirred solution of compound 13 (5.00 g, 18.4 mmol) in DMF (50 mL) was added CuCN (2.80 g, 31.2 mmol). The mixture was stirred for 4h at 150°C. The mixture was then cooled to r.t., and water (100 mL) added. The resulting mixture was extracted with ethyl acetate (4x 100 mL). The combined organic extracts were washed with water (50 mL) and brine (50 mL), dried over Na₂SO₄, and evaporated under reduced pressure to give 1.50 g (6.87 mmol, 37%) of compound 14, pure enough for the next step.

**Step M:** To a solution of compound 14 (1.50 g, 6.87 mmol) in ethanol (20 mL) was added LiOH·H₂O (0.400 g, 9.53 mmol). The mixture was refluxed for 10h. The solvent was evaporated under reduced pressure and the residue diluted with water (40 mL). The aqueous layer was acidified to pH 6.0 with 10% aq. hydrochloric acid and the precipitate was collected by filtration. The residue was washed with water and dried under vacuum to afford 0.400 g (1.95 mmol, 28%) of 4-cyano-7-fluoro-1H-indole-2-carboxylic acid as a white solid.
*Rt* (Method G) 1.02 mins, *m*/*z* 203 [M-H]⁻

### Preparation of 4-cyano-5-fluoro-1H-indole-2-carboxylic acid

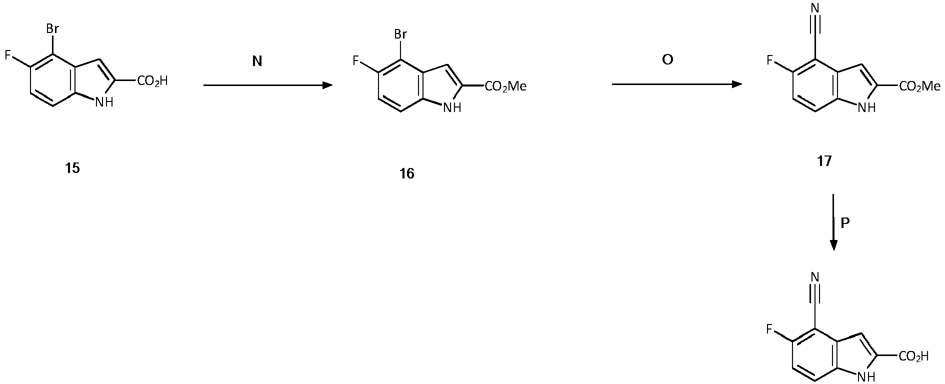

**Step N:** To a solution of compound 15 (5.00 g, 19.4 mmol) in DMF (50 mL) was added NaHCO₃ (1.59 g, 18.9 mmol) and iodomethane (3 mL). The resulting mixture was stirred overnight at r.t., then diluted with water (50 mL) and extracted with diethyl ether (3x 50 mL). The combined organic extracts were dried over Na₂SO₄, and evaporated under reduced pressure to obtain 4.90 g (18.0 mmol, 90%) of compound 16 as white solid.

**Step O:** To a stirred solution of compound 16 (4.80 g, 17.6 mmol) in DMF (50 mL) was added CuCN (2.70 g, 30.1 mmol). The mixture was stirred for 4h at 150°C. The mixture was then cooled to r.t., water (100 mL) added. The resulting mixture was extracted with ethyl acetate (4x 100 mL). The combined organic extracts were washed with water (50 mL) and brine (50 mL), dried over Na₂SO₄, and evaporated under reduced pressure to give 1.40 g (6.42 mmol, 36%) of compound 17, pure enough for the next step.

**Step P:** To a solution of compound 17 (1.40 g, 6.42 mmol) in ethanol (20 mL) was added LiOH·H₂O (0.350 g, 8.34 mmol). The mixture was refluxed for 10h. The solvent was evaporated under reduced pressure and the residue diluted with water (30 mL). The aqueous layer was acidified to pH 6.0 with 10% aq. hydrochloric acid and the precipitate collected by filtration. The residue was washed with water and dried under vacuum to afford 0.500 g (2.45 mmol, 38%) of 4-cyano-5-fluoro-1H-indole-2-carboxylic acid as a white solid.
*Rt* (Method G) 1.10 mins, *m*/*z* 203 [M-H]⁻

### Preparation of 4,5,6-trifluoro-1H-indole-2-carboxylic acid

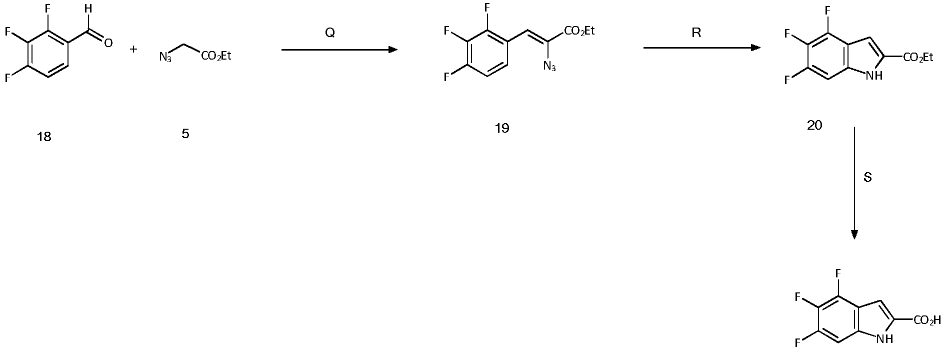

**Step Q:** To a solution of sodium methoxide (23.0 g, 426 mmol) in methanol (200 mL) at - 10°C was added dropwise a solution of compound 18 (15.0 g, 93.7 mmol) and compound 5 (26.0 g, 201 mmol) in methanol (100 mL). The reaction mixture was stirred for 3h, maintaining the temperature below 5°C and then quenched with ice water. The resulting mixture was stirred for 10 min,and the precipitate collected by filtration. The solid was washed with water and dried to afford 12.0 g (46.7 mmol, 72%) of compound 19 as a white solid.

**Step R:** A solution of compound 19, obtained in the previous step, (12.0 g, 46.7 mmol) in xylene (250 mL) was refluxed for 1h under an argon atmosphere and then evaporated under reduced pressure. The residue was recrystallized form hexane-ethyl acetate mixture (60:40) to give 7.00 g (30.5 mmol, 65%) of compound 20.

**Step S:** To a solution of compound 20 (7.00 g, 30.5 mmol) in ethanol (50 mL) was added 2 N aqueous sodium hydroxide solution (18 mL). The mixture was stirred for 2h at 60°C. The solvent was evaporated and the residue was acidified to pH 5-6 with aqueous hydrochloric acid. The resulting precipitate was collected by filtration, washed with water, and dried to obtain 5.00 g (23.2 mmol, 76%) 4,5,6-trifluoro-1H-indole-2-carboxylic acid.
¹H NMR (400 MHz, d6-dmso) 7.17 (1H, s), 7.22 (1H, dd), 12.3 (1H, br s), 13.3 (1H, br s)

### Preparation of 4,6,7-trifluoro-1H-indole-2-carboxylic acid

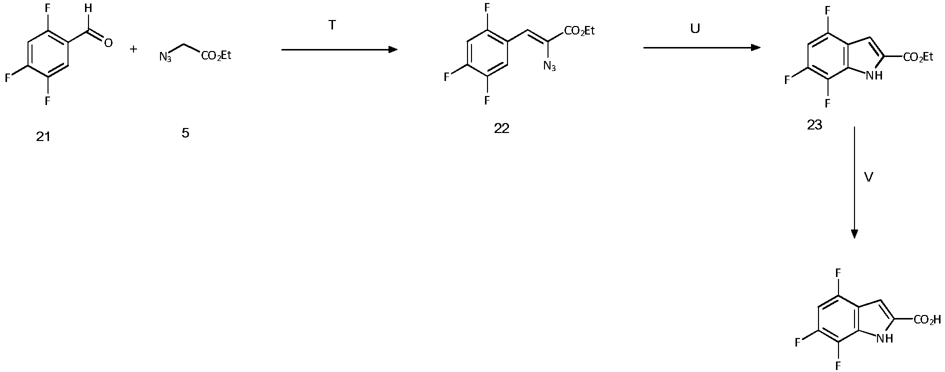

**Step T:** To a solution of sodium methoxide (23.0 g, 426 mmol) in methanol (200 mL) at - 10°C was added dropwise a solution of compound 21 (15.0 g, 90.3 mmol) and compound 5 (26.0 g, 201 mmol) in methanol (100 mL). The reaction mixture was stirred for 3h maintaining the temperature below 5°C and then quenched with ice water. The resulting mixture was stirred for 10 min. The precipitate was collected by filtration, washed with water and dried to afford 10.0 g (38.0 mmol, 42%) of compound 22 as a white solid.

**Step U:** A solution of compound 22, obtained in the previous step, (10.0 g, 38.0 mmol) in xylene (200 mL) was refluxed for 1h under an argon atmosphere and then concentrated under reduced pressure. The residue was recrystallized form hexane-ethyl acetate mixture (60:40) to give 6.00 g (26.2 mmol, 69%) of compound 23.

**Step V:** To a solution of compound 23 (7.00 g, 30.5 mmol) in ethanol (40 mL) was added 2 N aqueous sodium hydroxide solution (16 mL). The mixture was stirred for 2h at 60°C. The solvent was evaporated and the residue was acidified to pH 5-6 with aqueous hydrochloric acid. The resulting precipitate was collected by filtration, washed with water, and dried to obtain 4.10 g (19.1 mmol, 62%) of 4,6,7-trifluoro-1H-indole-2-carboxylic acid.
*Rt* (Method G) 1.16 mins, *m*/*z* 214 [M-H]⁻

### Preparation of 4-cyano-6-fluoro-1H-indole-2-carboxylic acid

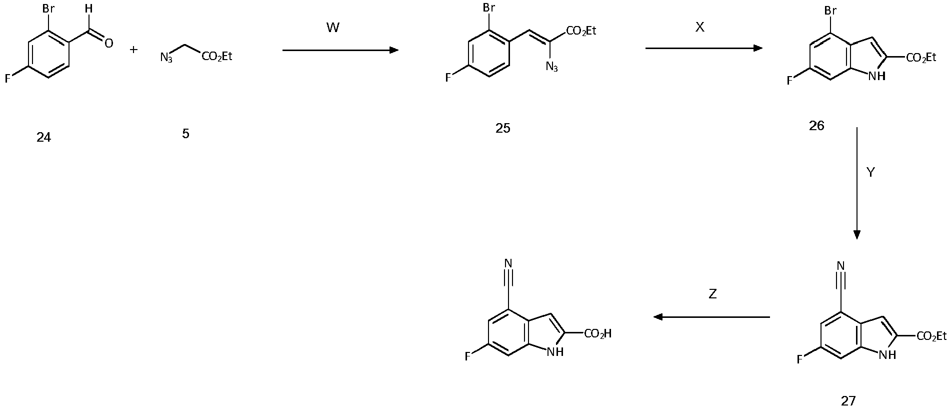

**Step W:** To a solution of sodium methoxide (65.0 g, 1203 mmol) in methanol (500 mL) at - 10°C was added dropwise a solution of compound 24 (60.0 g, 296 mmol) and compound 5 (85.0 g, 658 mmol) in methanol (200 mL). The reaction mixture was stirred for 3h maintaining the temperature below 5°C and then quenched with ice water. The resulting mixture was stirred for 10 min. The precipitate was collected by filtration, washed with water and dried to afford 45.0 g (143 mmol, 48%) of compound 25.

**Step X:** A solution of compound 25, obtained in the previous step, (35.0 g, 111 mmol) in xylene (250 mL) was refluxed for 1h under an argon atmosphere and then evaporated under reduced pressure. The residue was recrystallized form hexane-ethyl acetate mixture (60:40) to give 11.0 g (38.4 mmol, 35%) of compound 26.

**Step Y:** To a stirred solution of compound 26 (11.0 g, 38.4 mmol) in DMF (20 mL) was added CuCN (6.60 g, 73.7 mmol). The mixture was stirred for 4h at 150°C. The mixture was then cooled to r.t., and water (70 mL) added. The mixture was extracted with ethyl acetate (4x 50 mL). The combined organic extracts were washed with water (50 mL) and brine (50 mL), dried over Na₂SO₄, and evaporated under reduced pressure to give 2.40 g (10.3 mmol, 27%) of compound 27, pure enough for the next step.

**Step Z:** To a solution of compound 27 (2.40 g, 6.42 mmol) in ethanol (30 mL) was added LiOH·H₂O (0.600 g, 14.3 mmol). The mixture was refluxed for 10h. The mixture was concentrated under reduced pressure and the residue diluted with water (50 mL). The aqueous layer was acidified to pH 6 with 10% aq. hydrochloric acid and the precipitate was collected by filtration. The solid was washed with water and dried under vacuum to afford 1.20 g (5.88 mmol, 57%) of 4-cyano-6-fluoro-1H-indole-2-carboxylic acid as a white solid.
*Rt* (Method G) 1.06 mins, *m*/*z* 203 [M-H]⁻

### Preparation of 4-ethyl-1H-indole-2-carboxylic acid

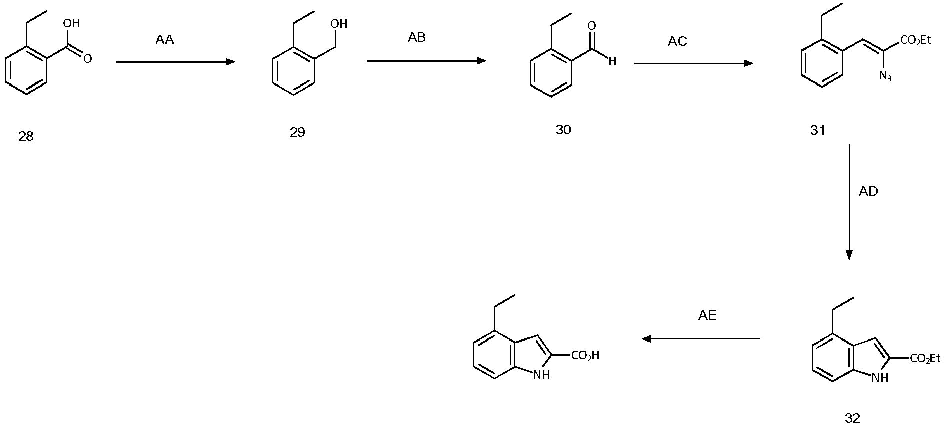

**Step AA:** A solution of compound 28 (70.0 g, 466 mmol) in dry THF (500 mL) was treated with 10 M solution of BH₃ in THF (53 mL, 53.0 mmol of BH₃) at 0°C. The reaction mass was stirred at r.t. for 24h before methanol (150 mL) was slowly added thereto. The resulting mixture was stirred for 45 min, and evaporated under reduced pressure to yield 55.0 g (404 mmol, 87%) of compound 29, pure enough for the next step.

**Step AB:** To a cooled (0°C) solution of compound 29 (55.0 g, 404 mmol) in CH₂Cl₂ (400 mL) was added Dess-Martin periodinane (177 g, 417 mmol) portionwise. After stirring for 1h at r.t., the reaction mixture was quenched with saturated aqueous Na₂S₂O₃ (300 mL) and saturated aqueous NaHCO₃ (500 mL). The mixture was extracted with CH₂Cl₂ (3x 300 mL). The combined organic extracts were washed with water and brine, dried over Na₂SO₄ and concentrated to yield 51.0 g of crude compound 30 as a yellow solid.

**Step AC:** To a solution of sodium methoxide (107 g, 1981 mmol) in methanol (600 mL) at -10°C was added dropwise a solution of compound 30, obtained in the previous step, (51.0 g) and compound 5 (126 g, 976 mmol) in methanol (300 mL). The reaction mixture was stirred for 4h maintaining temperature below 5°C, then quenched with ice water. The resulting mixture was stirred for 10 min, and the precipitate collected by filtration. The solid was washed with water and dried to afford 35.0 g (151 mmol, 37% over 2 steps) of compound 31.

**Step AD:** A solution of compound 31, obtained in the previous step, (35.0 g, 151 mmol) in xylene (500 mL) was refluxed for 1h under an argon atmosphere and then concentrated under reduced pressure. The residue was recrystallized form hexane-ethyl acetate mixture (60:40) to give 21.0 g (103 mmol, 68%) of compound 32.

**Step AE:** To a solution of compound 32 (21.0 g, 103 mmol) in ethanol (200 mL) was added 2 N aqueous sodium hydroxide solution (47 mL). The mixture was stirred for 2h at 60°C. The mixture was concentrated under reduced pressure, and the residue acidified to pH 5-6 with aqueous hydrochloric acid. The precipitate was collected by filtration, washed with water, and dried to obtain 19 g (100 mmol, 97%) of 4-ethyl-1H-indole-2-carboxylic acid.
*Rt* (Method G) 1.20 mins, *m*/*z* 188 [M-H]⁻
¹H NMR (400 MHz, d6-dmso) δ 1.25 (t, 3H), 2.88 (q, 2H), 6.86 (1H, d), 7.08-7.20 (2H, m), 7.26 (1H, d), 11.7 (1H, br s), 12.9 (1H, br s)

### Preparation of 4-cyclopropyl-1H-indole-2-carboxylic acid

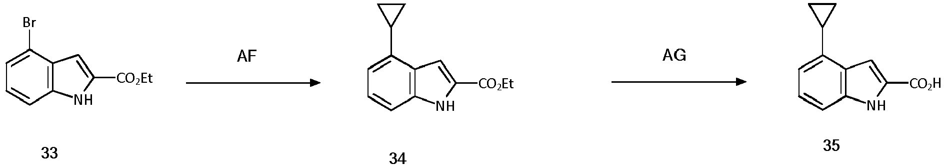

**Step AF:** To a degassed suspension of compound 33 (2.00 g, 7.80 mmol), cyclopropylboronic acid (0.754 g, 8.78 mmol), K₃PO₄ (5.02 g, 23.6 mmol), tricyclohexyl phosphine (0.189 g, 0.675 mmol), and water (2.0 mL) in toluene (60.0 mL) was added palladium (II) acetate (0.076 g, 0.340 mmol). The reaction mixture was stirred at 100°C for 4h. The reaction progress was monitored by diluting an aliquot of the reaction mixture with water and extracting with ethyl acetate. The organic layer was spotted over an analytical silica gel TLC plate and visualized using 254 nm UV light. The reaction progressed to completion with the formation of a polar spot. The R_{f} values of the starting material and product were 0.3 and 0.2, respectively. The reaction mixture was allowed to cool to r.t. and filtered through a pad of celite. The filtrate was concentrated under reduced pressure and the crude product was purified by flash column using 230-400 mesh silica gel and eluted with 10% ethyl acetate in petroleum ether to afford 1.10 g (5.11 mmol, 63%) of compound 34 as a brown liquid. TLC system: 5% ethyl acetate in petroleum ether.

**Step AG:** A mixture of compound 34 (1.10 g, 5.11 mmol) in ethanol (40 mL) and 2N aqueous sodium hydroxide (15 mL) was stirred for 2h at 60°C. The mixture was concentrated under reduced pressure, and the residue acidified to pH 5-6 with aqueous hydrochloric acid. The precipitate was collected by filtration, washed with water, and dried to yield 1.01 g (5.02 mmol, 92%) of 4-cyclopropyl-1H-indole-2-carboxylic acid.
*Rt* (Method G) 1.17 mins, *m*/*z* 200 [M-H]⁻

### Preparation of 4-chloro-5-fluoro-1H-indole-2-carboxylic acid

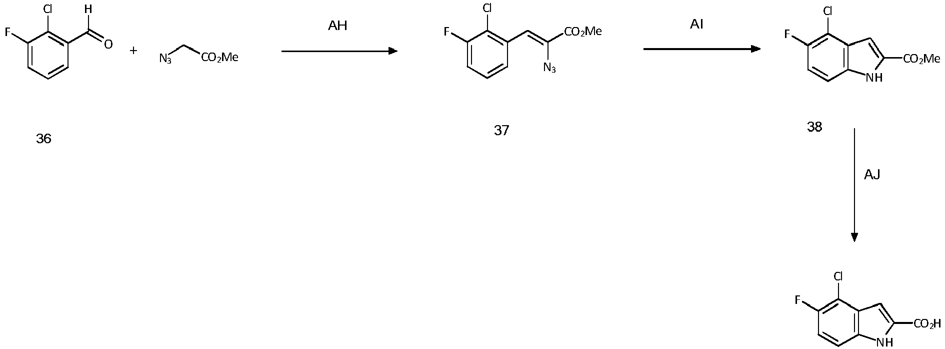

**Step AH:** To a solution of sodium methoxide (39.9 g, 738 mmol) in methanol (300 mL) at -10°C was added dropwise a solution of compound 36 (28.8 g, 182 mmol) and methyl azidoacetate (52.1 g, 404 mmol) in methanol (150 mL). The reaction mixture was stirred for 3h maintaining temperature below 5°C, then quenched with ice water. The resulting mixture was stirred for 10 min. The precipitate was collected by filtration, washed with water and dried to afford 20.0 g (78.2 mmol, 43%) of compound 37.

**Step AI:** A solution of compound 37 (19.4 g, 76.0 mmol) in xylene (250 mL) was refluxed for 1h under an argon atmosphere and then concentrated under reduced pressure. The residue was recrystallized from hexane-ethyl acetate (50:50) to give 9.00 g (39.5 mmol, 52%) of compound 38.

**Step AJ:** To a solution of compound 38 (8.98 g, 39.4 mmol) in ethanol (100 mL) was added 2 N aqueous sodium hydroxide solution (18 mL). The mixture was stirred for 2h at 60°C. The mixture was concentrated under reduced pressure, and the residue acidified to pH 5-6 with aqueous hydrochloric acid. The resulting precipitate was collected by filtration, washed with water, and dried to obtain 7.75 g (36.3 mmol, 92%) of 4-chloro-5-fluoro-1H-indole-2-carboxylic acid.
*Rt* (Method G) 1.15 mins, *m*/*z* 212 [M-H]⁻
¹H NMR (400 MHz, d6-dmso) 7.08 (1H, s), 7.28 (1H, dd) 7.42 (1H, dd), 12.2 (1H, br s), 13.2 (1H, br s)

### Preparation of 5-fluoro-4-(1-hydroxyethyl)-1H-indole-2-carboxylic acid

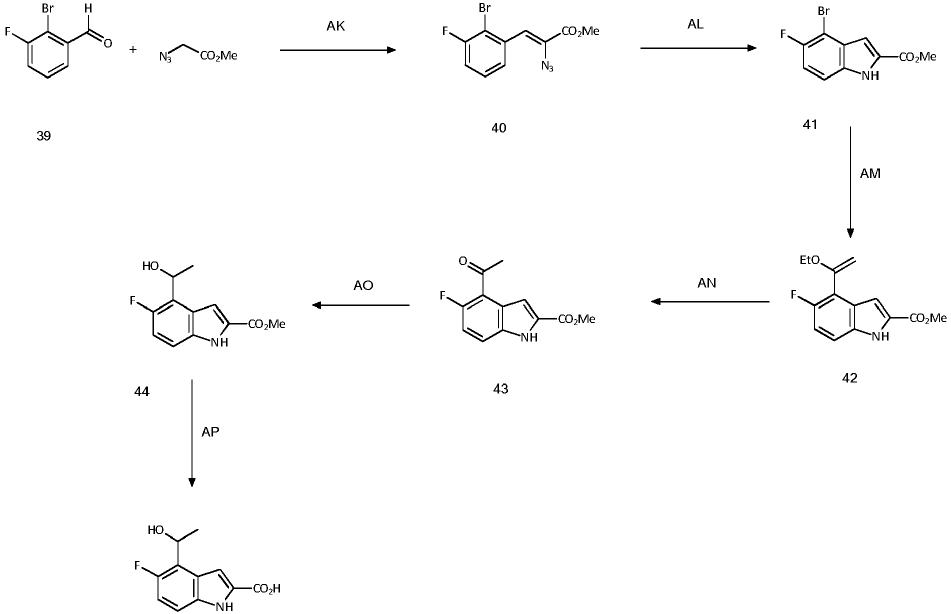

**Step AK:** To a solution of sodium methoxide (50.0 g, 926 mmol) in methanol (300 mL) at - 10°C was added dropwise a solution of compound 39 (45.0 g, 222 mmol) and methyl azidoacetate (59.0 g, 457 mmol) in methanol (100 mL). The reaction mixture was stirred for 3 h maintaining the temperature below 5°C, then quenched with ice water. The resulting mixture was stirred for 10 min. The precipitate was collected by filtration, washed with water and dried to afford 35.0 g (133 mmol, 60%) of compound 40 as a white solid.

**Step AL:** A solution of compound 40, obtained in the previous step, (35.0 g, 133 mmol) in xylene (250 mL) was refluxed for 1 h under an argon atmosphere and then evaporated under reduced pressure. The residue was recrystallized from hexane-ethyl acetate (60:40) to give 21.0 g (77.2 mmol, 58%) of compound 41.

**Step AM:** To a degassed solution of compound 41 (4.00 g, 14.7 mmol) and tributyl(1-ethoxyvinyl)stannane (5.50 g, 15.2 mmol) in toluene (50 mL) under nitrogen was added bis(triphenylphosphine) palladium(II) dichloride (1.16 g, 1.65 mmol). The reaction mixture was stirred at 60°C for 20 h. The reaction mixture was cooled to room temperature and filtered. The filtrate was concentrated under under reduced pressure and the residue purified by silica gel chromatography to afford 2.50 g (9.50 mmol, 65%) of compound 42 as a pale yellow solid.

**Step AN:** To a solution of compound 42 (2.40 g, 9.12 mmol) in 1,4-dioxane (30 mL) was added 2M hydrochloric acid (15 mL). The resulting mixture was stirred at room temperature for 30 min. The mixture was concentrated under vacuum and the residue partitioned between ethyl acetate and water. The organic extract was washed with water and brine, dried over sodium sulfate, filtered, and evaporated. The residue was triturated with 5% ether in isohexane and dried to afford 1.80 g (7.65 mmol, 84%) of compound 43 as a white solid.

**Step AO:** A suspension of compound 43 (1.70 g, 7.23 mmol) and NaBH₄ (2.50 g, 66.1 mmol) in ethanol (13 mL) was refluxed for 2 h, then cooled to room temperature, and filtered. The filtrate was concentrated under reduced pressure and the residue dissolved in ethyl acetate. The solution was washed with 1N hydrochloric acid and brine, dried over Na₂SO₄, and evaporated under reduced pressure to give 1.60 g (6.74 mmol, 93%) of compound 44 as a colourless oil.

**Step AP:** To a solution of compound 44 (1.50 g, 6.32 mmol) in methanol (40 mL) was added 2N aqueous NaOH (10 mL). The mixture was stirred for 2 h at 60°C. The mixture was concentrated under reduced pressure and the residue acidified to pH 5-6 with 10% hydrochloric acid. The precipitate was collected by filtration, washed with water (3 × 15 mL), and dried to obtain 1.30 g (5.82 mmol, 92%) of 5-fluoro-4-(1-hydroxyethyl)-1H-indole-2-carboxylic acid.
*Rt* (Method G) 1.00 mins, *m*/*z* 222 [M-H]⁻

### Preparation of 4-ethyl-5-fluoro-1H-indole-2-carboxylic acid

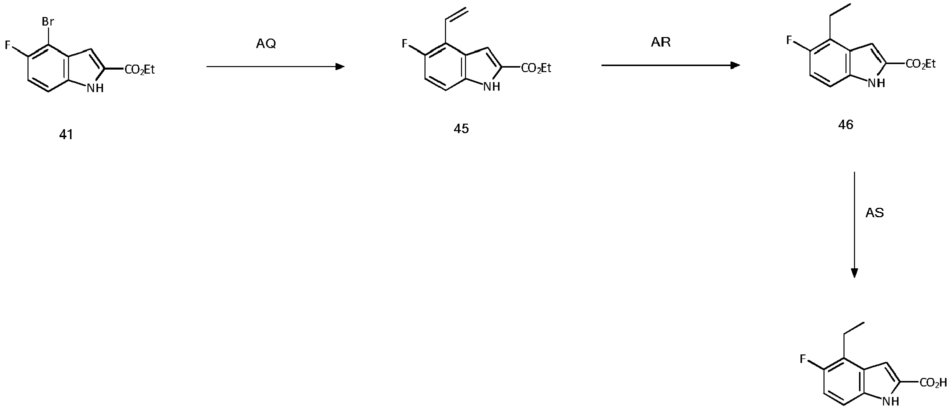

**Step AQ:** To a heated (90°C) solution of compound 41 (4.00 g, 14.7 mmol) in anhydrous DMF under nitrogen (10 mL) were added tri-n-butyl(vinyl)tin (3.60 g, 11.4 mmol) and Pd(PPh₃)₂Cl₂ (0.301 g, 0.757 mmol). The resulting mixture was stirred at 90°C for 1 h. The mixture was then cooled to room temperature and purified by silica gel column chromatography (60-80% ethyl acetate in hexane) to give 2.20 g (10.0 mmol, 68%) of compound 45 as yellow solid.

**Step AR:** A mixture of compound 45 (1.50 g, 6.84 mmol) and Pd/C (0.300 g, 10% wt.) in methanol (20 mL) was stirred under an atmosphere of hydrogen at room temperature for 16 h. The mixture was filtered, then concentrated under reduced pressure to give 1.45 g (6.55 mmol, 96%) of compound 46.

**Step AS:** To a solution of compound 46 (1.40 g, 6.33 mmol) in methanol (40 mL) was added 2N aqueous NaOH (10 mL). The mixture was stirred for 2 h at 60°C. The mixture was concentrated under vacuum, then the residue was acidified to pH 5-6 with 10% hydrochloric acid. The precipitate was collected by filtration, washed with water (3 × 15 mL), and dried to obtain 1.20 g (5.79 mmol, 91%) of target compound 4-ethyl-5-fluoro-1H-indole-2-carboxylic acid.
*Rt* (Method G) 1.33 mins, *m*/*z* 206 [M-H]⁻

### Preparation of 4-ethyl-6-fluoro-1H-indole-2-carboxylic acid

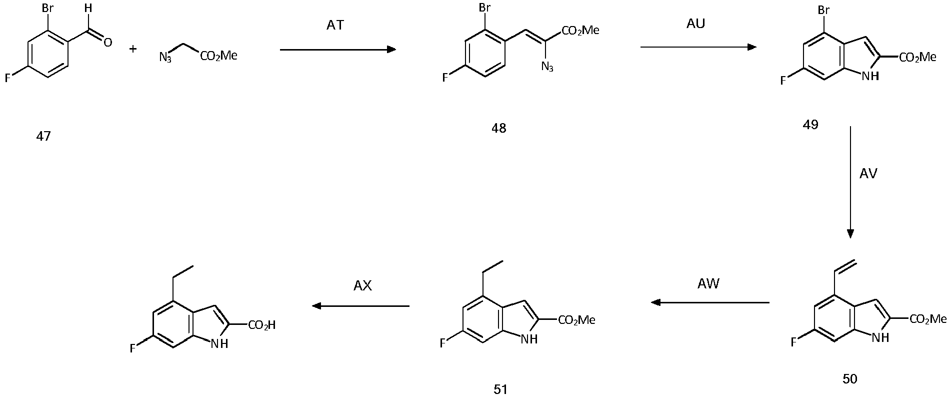

**Step AT:** To a solution of sodium methoxide (50.0 g, 926 mmol) in methanol (300 mL) at - 10°C was added dropwise a solution of compound 47 (45.0 g, 202 mmol) and methyl azidoacetate (59.0 g, 457 mmol) in methanol (100 mL). The reaction mixture was stirred for 3 h maintaining temperature below 5°C, then quenched with ice water. The resulting mixture was stirred for 10 min. The precipitate was collected by filtration, washed with water and dried to afford 38.5 g (128 mmol, 63%) of compound 48 as a white solid.

**Step AU:** A solution of compound 48, obtained in the previous step, (38.5 g, 128 mmol) in xylene (250 mL) was refluxed for 1 h under an argon atmosphere and then concentrated under reduced pressure. The residue was recrystallized hexane-ethyl acetate (60:40) to give 18.0 g (67.3 mmol, 53%) of compound 49.

**Step AV:** To a heated (90°C) solution of compound 49 (4.00 g, 14.7 mmol) in anhydrous DMF under nitrogen (10 mL) were added tri-n-butyl(vinyl)tin (3.60 g, 11.4 mmol) and Pd(PPh₃)₂Cl₂ (0.301 g, 0.757 mmol). The resulting mixture was stirred at 90°C for 1 h. The mixture was then cooled to room temperature and purified by silica gel column chromatography (60-80% ethyl acetate in hexane) to give 2.00 g (9.12 mmol, 62%) of compound 50 as yellow solid.

**Step AW:** A mixture of compound 50 (1.50 g, 6.84 mmol) and Pd/C (0.300 g, 10% wt.) in methanol (20 mL) was stirred under an atmosphere of hydrogen at room temperature for 16 h. The mixture was filtered and concentrated to give 1.40 g (6.33 mmol, 93%) of compound 51.

**Step AX:** To a solution of compound 51 (1.10 g, 4.97 mmol) in methanol (40 mL) was added 2N aqueous NaOH (10 mL). The mixture was stirred for 2 h at 60°C. The mixture was concentrated under reduced pressure, then acidified to pH 5-6 with 10% hydrochloric acid. The precipitate was collected by filtration, washed with water (3 × 15 mL), and dried to obtain 0.900 g (4.34 mmol, 87%) of target compound 4-ethyl-6-fluoro-1H-indole-2-carboxylic acid.
*Rt* (Method G) 1.29 mins, *m*/*z* 206 [M-H]⁻

### Preparation of 6-fluoro-4-(1-hydroxyethyl)-1H-indole-2-carboxylic acid

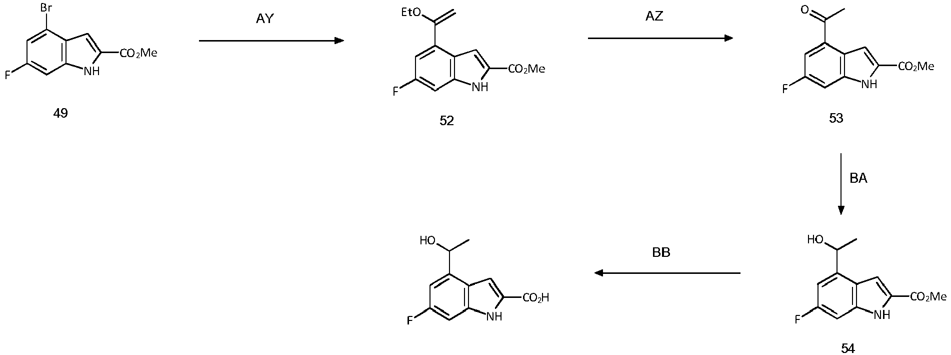

**Step AY:** To a degassed solution of compound 49 (4.00 g, 14.7 mmol) and tributyl(1-ethoxyvinyl)stannane (5.50 g, 15.2 mmol) in toluene (50 mL) under nitrogen were added bis(triphenylphosphine) palladium(II) dichloride (1.16 g, 1.65 mmol). The reaction mixture was stirred at 60°C for 20 h. The reaction mixture was cooled to room temperature and filtered. The filtrate was concentrated under reduced pressure and the residue purified by silica gel chromatography to give 2.10 g (7.98 mmol, 54%) of compound 52 as a pale yellow solid.

**Step AZ:** To a solution of compound 52 (2.10 g, 7.98 mmol) in 1,4-dioxane (30 mL) was added 2M hydrochloric acid (15 mL). The resulting mixture was stirred at room temperature for 30 min. The mixture was concentrated under reduced pressure, and residue partitioned between ethyl acetate and water. The organic extract was washed with water and brine, dried over sodium sulfate, filtered, and concentrated. The residue was triturated with 5% ether in isohexane and dried to afford 1.70 g (7.23 mmol, 91%) of compound 53 as a white solid.

**Step BA:** A suspension of compound 53 (1.70 g, 7.23 mmol) and NaBH₄ (2.50 g, 66.1 mmol) in ethanol (13 mL) was refluxed for 2 h, cooled to room temperature, and filtered. The filtrate was concentrated under reduced pressure and the residue was dissolved in ethyl acetate. The solution was washed with 1N hydrochloric acid and brine, dried over Na₂SO₄, and concentrated under reduced pressure to give 1.60 g (6.74 mmol, 93%) of compound 54 as a colourless oil.

**Step BB:** To a solution of compound 54 (1.40 g, 5.90 mmol) in methanol (40 mL) was added 2N aqueous NaOH (10 mL). The mixture was stirred for 2 h at 60°C. The mixture was concentrated and the residue acidified to pH 5-6 with 10% hydrochloric acid. The precipitate was collected by filtration, washed with water (3 × 15 mL), and dried to obtain 1.10 g (4.93 mmol, 48%) of target compound 6-fluoro-4-(1-hydroxyethyl)-1H-indole-2-carboxylic acid.
*Rt* (Method G) 1.00 mins, *m*/*z* 222 [M-H]⁻

### Preparation of 4-ethyl-7-fluoro-1H-indole-2-carboxylic acid

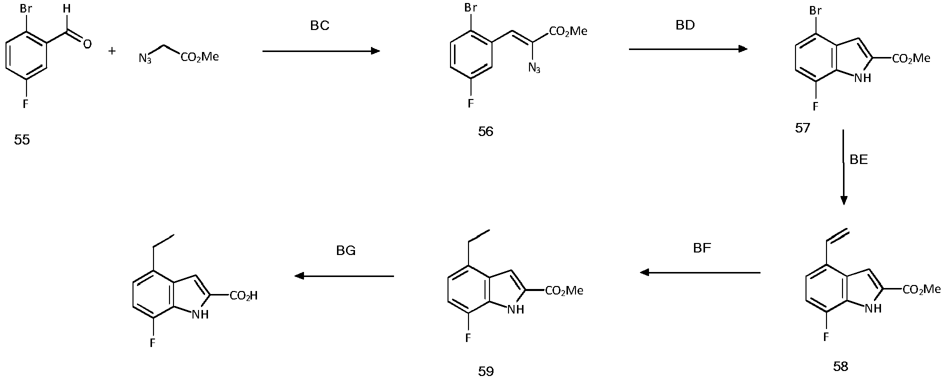

**Step BC:** To a solution of sodium methoxide (50.0 g, 926 mmol) in methanol (300 mL) - 10°C was added dropwise a solution of compound 55 (45.0 g, 222 mmol) and methyl azidoacetate (59.0 g, 457 mmol) in methanol (100 mL). The reaction mixture was stirred for 3 h maintaining temperature below 5°C, then quenched with ice water. The resulting mixture was stirred for 10 min. The precipitate was collected by filtration, washed with water and dried to afford 33.0 g (110 mmol, 50%) of compound 56 as a white solid.

**Step BD:** A solution of compound 56, obtained in the previous step, (33.0 g, 110 mmol) in xylene (250 mL) was refluxed for 1 h under an argon atmosphere and then concentrated under reduced pressure. The residue was recrystallized from hexane-ethyl acetate (60:40) to give 21.5 g (79.0 mmol, 72%) of compound 57.

**Step BE:** To a heated (90°C) solution of compound 57 (4.00 g, 14.7 mmol) in anhydrous DMF under nitrogen (10 mL) were added tri-n-butyl(vinyl)tin (3.60 g, 11.4 mmol) and Pd(PPh₃)₂Cl₂ (0.301 g, 0.757 mmol). The resulting mixture was stirred at 90°C for 1 h. The mixture was cooled to room temperature and purified by silica gel column chromatography (60-80% EtOAc in hexane). The combined product fractions of the product were concentrated, washed with water (3 × 100 mL), dried over Na₂SO₄, and concentrated to give 1.80 g (8.21 mmol, 56%) of compound 58 as yellow solid.

**Step BF:** A mixture of compound 58 (1.50 g, 6.84 mmol) and Pd/C (0.300 g, 10% wt.) in methanol (20 mL) was stirred under atmosphere of hydrogen at room temperature for 16 h. The mixture was filtered and concentrated to give 1.25 g (5.65 mmol, 83%) of compound 59.

**Step BG:** To a solution of compound 59 (1.40 g, 6.33 mmol) in methanol (40 mL) was added 2N aqueous NaOH (10 mL). The mixture was stirred for 2 h at 60°C. The mixture was concentrated under reduced pressure, and the residue acidified to pH 5-6 with 10% hydrochloric acid. The precipitate was collected by filtration, washed with water (3 × 15 mL), and dried to obtain 1.25 g (6.03 mmol, 95%) of target compound 4-ethyl-7-fluoro-1H-indole-2-carboxylic acid.
*Rt* (Method G) 1.27 mins, *m*/*z* 206 [M-H]⁻

### Preparation of 7-fluoro-4-(1-hydroxyethyl)-1H-indole-2-carboxylic acid

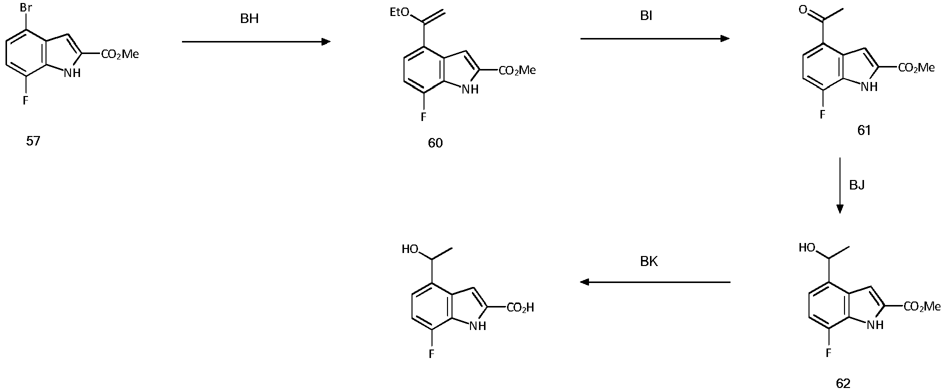

**Step BH:** To a degassed solution of compound 57 (4.00 g, 14.7 mmol) and tributyl(1-ethoxyvinyl)stannane (5.50 g, 15.2 mmol) in toluene (50 mL) under nitrogen was added bis(triphenylphosphine) palladium(II) dichloride (1.16 g, 1.65 mmol). The reaction mixture was stirred at 60°C for 20 h. The mixture was cooled to room temperature and filtered. The filtrate was concentrated under reduced pressure and the residue purified by silica gel chromatography to afford 2.70 g (10.3 mmol, 70%) of compound 60 as a pale yellow solid.

**Step BI:** To a solution of compound 60 (2.40 g, 9.12 mmol) in 1,4-dioxane (30 mL) was added 2M hydrochloric acid (15 mL). The mixture was stirred at room temperature for 30 min. The majority of the solvent was evaporated and the residue was partitioned between ethyl acetate and water. The combined organic extracts were washed with water and brine, dried over sodium sulfate, filtered, and evaporated. The residue was triturated with 5% ether in isohexane and dried to afford 1.90 g (8.08 mmol, 86%) of compound 61 as a white solid.

**Step BJ:** A suspension of compound 61 (1.70 g, 7.23 mmol) and NaBH₄ (2.50 g, 66.1 mmol) in ethanol (13 mL) was refluxed for 2 h, cooled to room temperature, and filtered. The filtrate was evaporated under reduced pressure and the residue was dissolved in ethyl acetate. The solution was washed with 1N hydrochloric acid and brine, dried over Na₂SO₄, and evaporated under reduced pressure to give 1.50 g (6.32 mmol, 87%) of compound 62 as a colourless oil.

**Step BK:** To a solution of compound 62 (1.50 g, 6.32 mmol) in methanol (40 mL) was added 2N aqueous NaOH (10 mL). The mixture was stirred for 2 h at 60°C. The mixture was concentrated under reduced pressure and the residue acidified to pH 5-6 with 10% hydrochloric acid. The precipitate was collected by filtration, washed with water (3 × 15 mL), and dried to obtain 1.35 g (6.05 mmol, 96%) of target compound 7-fluoro-4-(1-hydroxyethyl)-1H-indole-2-carboxylic acid.
*Rt* (Method G) 0.90 mins, *m*/*z* 222 [M-H]⁻

### Preparation of 4-(hydroxymethyl)-1H-indole-2-carboxylic acid

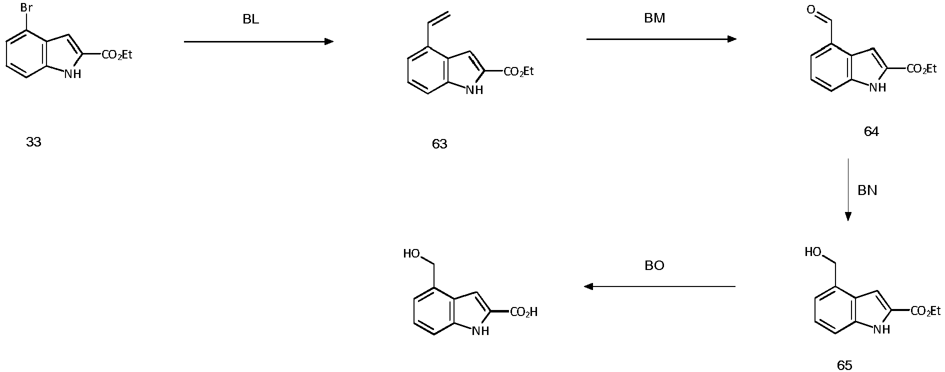

**Step BL:** To a solution of compound 33 (10.0 g, 39.4 mmol) in a mixture of dioxane (200 mL) and water (50 mL) were added potassium vinyltrifluoroborate (11.0 g, 82.1 mmol), triethylamine (30 mL, 248 mmol) and Pd(dppf)Cl₂ (1.0 g, 1.37 mmol). The mixture was stirred at 80°C for 48h. The mixture was concentrated under vacuum, and the residue was dissolved in ethyl acetate. The solution was washed with water and concentrated under reduced pressure. The obtained material was purified by silica gel column chromatography to give 2.50 g (12.4 mmol, 38%) of compound 63.

**Step BM:** To a mixture of compound 63 (2.50 g, 12.4 mmol), acetone (200 mL), and water (40 mL) were added OsO₄ (0.100 g, 0.393 mmol) and NaIO₄ (13.4 g, 62.6 mmol). The reaction was stirred for 10 h at room temperature. The acetone was distilled off and the remaining aqueous solution extracted with dichloromethane. The organic layer was washed with saturated NaHCO₃ solution (2 × 50 mL) and brine (2 × 50 mL), dried over Na₂SO₄, and concentrated under reduced pressure to obtain 1.50 g (7.40 mmol, 60%) of compound 64.

**Step BN:** To a cooled (0°C) solution of compound 64 (1.50 g, 7.38 mmol) in THF/methanol mixture (100 mL) was added NaBH₄ (0.491 g, 13.0 mmol). The reaction mixture was stirred for 12 h at room temperature. Then the mixture was cooled to 0°C, treated with 2N hydrochloric acid (40 mL), and concentrated. The residue was extracted with ethyl acetate. The organic extract was washed with water, dried over Na₂SO₄, and concentrated under reduced pressure to obtain 1.00 g (4.87 mmol, 65%) of compound 65, pure enough for the next step.

**Step BO:** To a solution of compound 65, obtained in the previous step, (1.00 g, 4.87 mmol) in THF (50 mL), was added 1N aqueous LiOH (9 mL). The resulting mixture was stirred for 48 h at room temperature, then concentrated and diluted with 1N aqueous NaHSO₄ (9 mL). The mixture was extracted with ethyl acetate. The organic extract was dried over Na₂SO₄, and concentrated under reduced pressure. The residue was recrystallized from MTBE to obtain 0.250 g (1.30 mmol, 27%) of target compound 4-(hydroxymethyl)-1H-indole-2-carboxylic acid.
*Rt* (Method G) 0.98 mins, *m*/*z* 190 [M-H]⁻

### Preparation of 4-(2-hydroxypropan-2-yl)-1H-indole-2-carboxylic acid

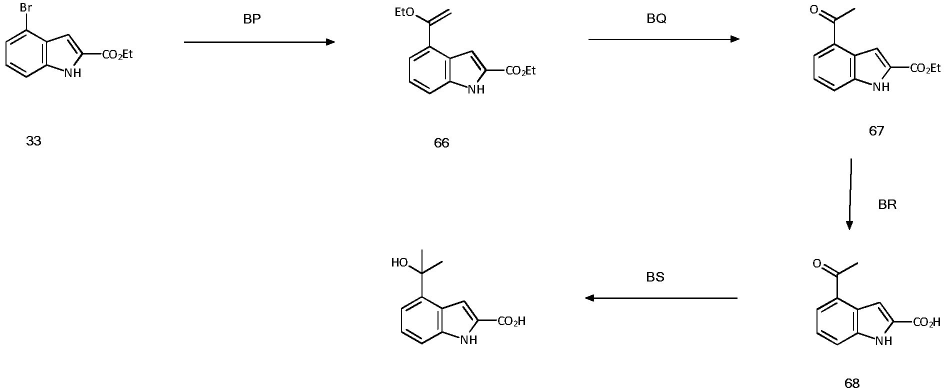

**Steps BP and BQ:** To a degassed solution of compound 33 (1.00 g, 3.94 mmol) and tributyl-(1-ethoxyvinyl)stannane (1.58 g, 4.37 mmol) in DMF (25 mL) under argon was added bis(triphenylphosphine)palladium(II) dichloride (0.100 g, 0.142 mmol). The reaction mixture was stirred at room temperature until TLC revealed completion of the reaction (approx. 7 days). The mixture was concentrated under reduced pressure and the residue partitioned between ethyl acetate and water. The organic layer was filtered through a plug of silica gel, dried over MgSO4, and concentrated under reduced pressure. The resulting black oil was dissolved in methanol (100 mL), treated with 5N hydrochloric acid (100 mL), and stirred at room temperature overnight. The mixture was concentrated and the residue dissolved in ethyl acetate. The solution was washed with water, dried over Na₂SO₄, and concentrated under reduced pressure. The crude product was purified by silica gel column chromatography to give 0.500 g (2.30 mmol, 58%) of compound 67.

**Step BR:** To a solution of compound 67 (1.00 g, 4.60 mmol) in THF (50 mL), was added 1N aqueous LiOH (7 mL). The resulting mixture was stirred for 48 h at room temperature, then concentrated under reduced pressure and diluted with 1N aqueous NaHSO4 (7 mL). The mixture was extracted with ethyl acetate. The organic extract was dried over MgSO₄, and concentrated under reduced pressure. The residue was recrystallized from MTBE to obtain 0.900 g (4.43 mmol, 96%) of compound 68.

**Step BS:** To a cooled (0°C) solution of compound 68 (0.900 g, 4.43 mmol) in THF (50 mL) under argon was added a 1N solution of MeMgCl (16 mL) in hexane. The resulting mixture was stirred for 48 h at room temperature. The mixture was carefully quenched with 1N NaHSO₄ and extracted with ethyl acetate. The organic extract was dried over Na₂SO₄, and concentrated under reduced pressure. The residue was recrystallized from MTBE to obtain 0.250 g (1.14 mmol, 26%) of target compound 4-(2-hydroxypropan-2-yl)-1H-indole-2-carboxylic acid.
*Rt* (Method G) 0.99 mins, *m*/*z* 202 [M-H]⁻

### Preparation of 4-(1-hydroxyethyl)-1H-indole-2-carboxylic acid

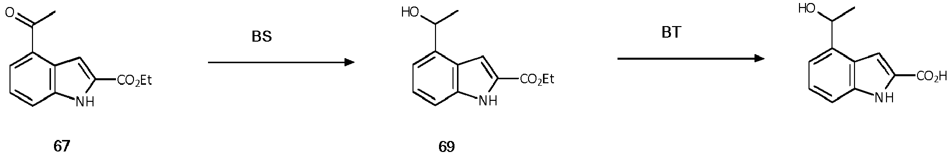

**Step BS:** To a cooled (0°C) solution of compound 67 (1.00 g, 4.60 mmol) in THF/methanol mixture (50 mL) was added NaBH₄ (0.385 g, 10.2 mmol). The reaction mixture was stirred for 12h at room temperature. The mixture was cooled to 0°C, treated with 2N hydrochloric acid (20 mL), and concentrated. The residue was extracted with ethyl acetate. The organic extract was washed with water, dried over Na₂SO₄, and evaporated under reduced pressure to obtain 0.800 g (3.65 mmol, 79%) of compound 69, pure enough for the next step.

**Step BT:** To a solution of compound 69, obtained in the previous step, (0.800 g, 3.65 mmol) in THF (50 mL), was added 1N aqueous LiOH (6 mL). The resulting mixture was stirred for 48 h at room temperature, then concentrated and diluted with 1N aqueous NaHSO₄ (6 mL). The mixture was extracted with ethyl acetate. The organic extract was dried over MgSO₄, and concentrated under reduced pressure. The residue was recrystallized from MTBE to obtain 0.300 g (1.46 mmol, 40%) of target compound 4-(1-hydroxyethyl)-1H-indole-2-carboxylic acid.
*Rt* (Method G) 0.82 mins, *m*/*z* 204 [M-H]⁻

### Preparation of 4-(propan-2-yl)-1H-indole-2-carboxylic acid

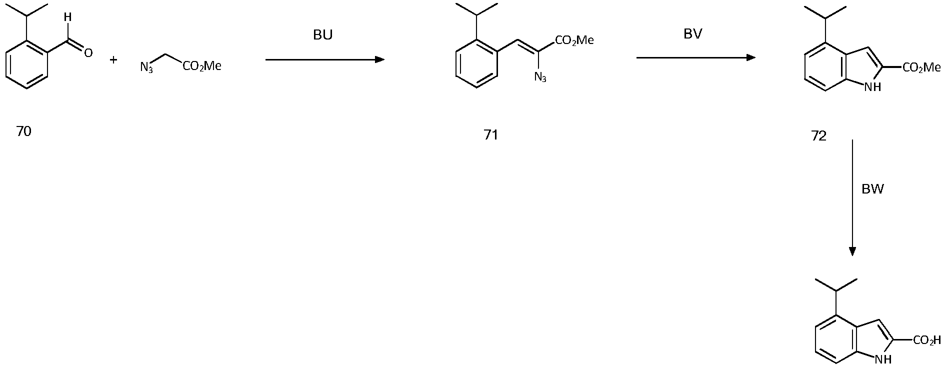

**Step BU:** To a solution of sodium methoxide (10.0 g, 185 mmol) in methanol (150 mL) at - 10°C was added dropwise a solution of compound 70 (15.0 g, 101 mmol) and methyl azidoacetate (12.0 g, 104 mmol) in methanol (100 mL). The reaction mixture was stirred for 3 h maintaining the temperature below 5°C, then quenched with ice water. The resulting mixture was stirred for 10 min. The precipitate was then collected by filtration, washed with water and dried to afford 7.00 g (23.3 mmol, 23%) of compound 71 as a white solid.

**Step BV:** A solution of compound 71, obtained in the previous step, (7.00 g, 23.3 mmol) in xylene (200 mL) was refluxed for 1h under an argon atmosphere and then concentrated under reduced pressure. The residue was recrystallized from hexane-ethyl acetate (60:40) to give 3.50 g (16.1 mmol, 69%) of compound 72.

**Step BW:** To a solution of compound 72 (3.50 g, 16.1 mmol) in methanol (100 mL) was added 2N aqueous NaOH (40 mL). The mixture was stirred for 2 h at 60°C. The mixture was concentrated under reduced pressure, and then residue acidified to pH 5-6 with 10% hydrochloric acid. The precipitate was collected by filtration, washed with water (3 × 50 mL), and dried to obtain 2.70 g (13.3 mmol, 83%) of target compound 4-(propan-2-yl)-1H-indole-2-carboxylic acid.
*Rt* (Method G) 1.32 mins, *m*/*z* 202 [M-H]⁻

### Preparation of 4-ethenyl-1H-indole-2-carboxylic acid

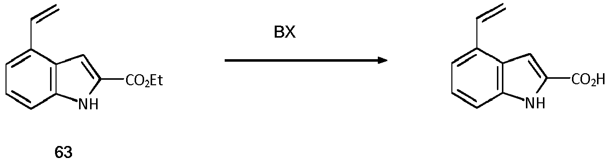

**Step BX:** To a solution of compound 63 (0.900 g, 4.47 mmol) in THF (50 mL), was added 1N aqueous LiOH (8 mL). The resulting mixture was stirred for 48 h at room temperature, then concentrated under reduced pressure and diluted with 1N aqueous NaHSO₄ (8 mL). The mixture was extracted with ethyl acetate. The organic extract was dried over MgSO₄ and concentrated under reduced pressure. The residue was recrystallized from MTBE to obtain 0.500 g (2.67 mmol, 59%) of target compound 4-ethenyl-1H-indole-2-carboxylic acid.
*Rt* (Method G) 1.14 mins, *m*/*z* 186 [M-H]⁻

### Preparation of 4-ethynyl-1H-indole-2-carboxylic acid

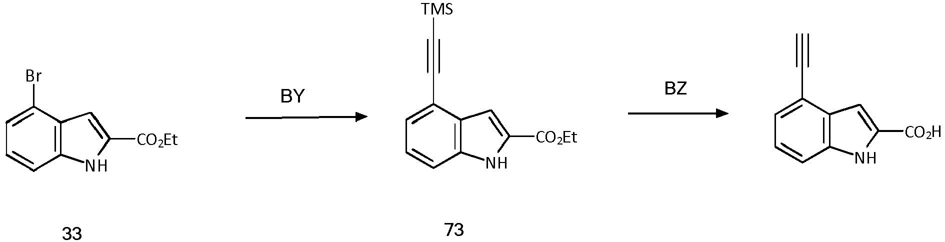

**Step BY:** To a solution of compound 33 (1.00 g, 3.94 mmol) in THF (50 mL) under argon were added TMS-acetylene (0.68 mL, 4.80 mmol), CuI (0.076 g, 0.399 mmol), triethylamine (2.80 mL, 20.0 mmol), and Pd(dppf)Cl₂ (0.100 g, 0.137 mmol). The mixture was stirred at 60°C until TLC revealed completion of the reaction (approx. 5 days). The mixture was concentrated under reduced pressure, and the residue dissolved in ethyl acetate. The solution was washed with water, dried over Na₂SO₄, and concentrated under reduced pressure. The residue was purified by silica gel column chromatography to give 0.600 g (2.14 mmol, 56%) of compound 73.

**Step BZ:** To a solution of compound 73 (0.840 g, 3.10 mmol) in THF (50 mL), was added 1N aqueous LiOH (7 mL). The resulting mixture was stirred for 48 h at room temperature, then concentrated under reduced pressure and diluted with 1N aqueous NaHSO₄ (7 mL). The mixture was extracted with ethyl acetate. The organic extract was dried over MgSO₄ and concentrated under reduced pressure. The residue was recrystallized from MTBE to obtain 0.400 g (2.17 mmol, 70%) of target compound 4-ethynyl-1H-indole-2-carboxylic acid.
*Rt* (Method G) 1.12 mins, *m*/*z* 184 [M-H]⁻

### Preparation of 4-(1,1-difluoroethyl)-1H-indole-2-carboxylic acid

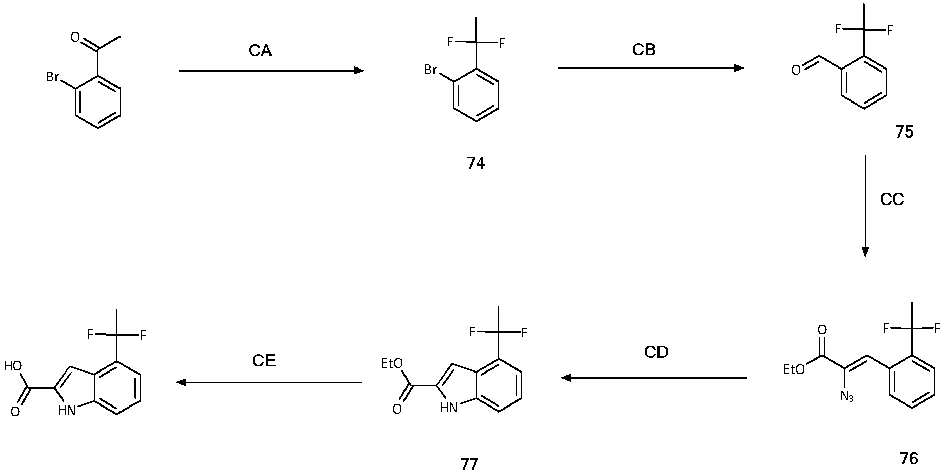

**Step CA:** To a mixture of 2-bromoacetophenone (63.0 g, 317 mmol), water (0.5 mL), and dichloromethane (100 mL) was added Morph-DAST (121 mL, 992 mmol). The resulting mixture was stirred for 28 days at room temperature. The reaction mixture was then poured into saturated aqueous NaHCO₃ (1000 mL) and extracted with ethyl acetate (2 × 500 mL). The organic layer was dried over Na₂SO₄ and concentrated under reduced pressure. The residue was purified by silica gel column chromatography to give 16.8 g (76.0 mmol, 12%) of compound 74.

**Step CB:** To a cooled (-85°C) solution of compound 74 (16.8 g, 76.0 mmol) in THF (300 mL) under Ar was added 2.5M solution of n-BuLi in hexanes (36.5 mL, 91.5 mmol) over 30 min. The resulting mixture was stirred for 1 h at -85°C. DMF (8.80 mL, 114 mmol) was then added (maintaining temperature below -80°C) and the reaction stirred for a further 45 min. The reaction was quenched with saturated aqueous NH₄Cl (100 mL) and diluted with water (600 mL). The obtained mixture was extracted with ethyl acetate (2 × 500 mL). The combined organic extracts were dried over Na₂SO₄, and concentrated under reduced pressure to obtain 12.5 g (73.6 mmol, 97%) of compound 75 (sufficiently pure for the next step).

**Step CC:** To a cooled (-30°C) mixture of compound 75 (12.5 g, 73.5 mmol), ethanol (500 mL), and ethyl azidoacetate (28.5 g, 221 mmol) was added a freshly prepared solution of sodium methoxide (prepared by mixing Na (5.00 g, 217 mmol) and methanol (100 mL)) portionwise under Ar (maintaining the temperature below -25°C). The reaction mixture was warmed to 15°C and stirred for 12 h. The obtained mixture was poured into saturated aqueous NH₄Cl (2500 mL) and stirred for 20 min. The precipitate was collected by filtration, washed with water, and dried to obtain 10.0 g (35.6 mmol, 51%) of compound 76.

**Step CD:** A solution of compound 76 (10.0 g, 35.6 mmol) in xylene (500 mL) was refluxed until gas evolution ceased (approx. 2 h) and then concentrated under reduced pressure. The orange oil obtained was triturated with hexane/ethyl acetate (5:1), collected by filtration, and dried to obtain 1.53 g (6.04 mmol, 17%) of compound 77.

**Step CE:** To a solution of compound 77 (1.53 g, 6.04 mmol) in THF/water 9:1 mixture (100 mL) was added LiOH·H₂O (0.590 g, 14.1 mmol). The resulting mixture was stirred overnight at r.t. The volatiles were evaporated and the residue mixed with water (50 mL) and 1N hydrochloric acid (10 mL). The mixture was extracted with ethyl acetate (2 × 100 mL). The combined organic extracts were dried over Na₂SO₄, and concentrated under reduced pressure. The crude product was purified by silica gel column chromatography to give 0.340 g (1.33 mmol, 24%) of 4-(1,1-difluoroethyl)-1H-indole-2-carboxylic acid.
*Rt* (Method G) 1.16 mins, *m*/*z* 224 [M-H]⁻

### Preparation of 4-(trimethylsilyl)-1H-indole-2-carboxylic acid

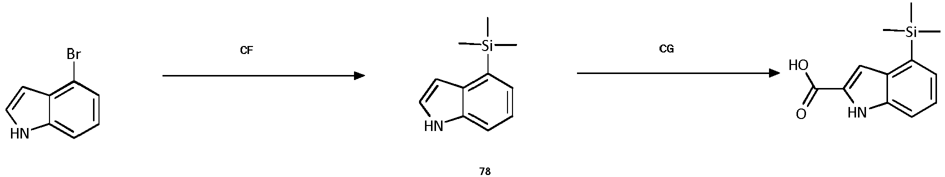

**Step CF:** To a cooled (-78°C) solution of 4-bromo-1H-indole (5.00 g, 25.5 mmol) in THF (100 mL) under Ar was added a 2.5M solution of n-BuLi in hexanes (23 mL, 57.5 mmol). The resulting mixture was stirred for 30 min. TMSCl (16 mL, 126 mmol) was added and the reaction mixture warmed to room temperature. After 1h the mixture was diluted with MTBE (250 mL), washed with water (2 × 200 mL) and brine (200 mL), then dried over Na₂SO₄, and concentrated under reduced pressure. The residue was refluxed in methanol (100 mL) for 1 h. The solvent was then distilled off to obtain 3.60 g (19.0 mmol, 74%) of compound 78.

**Step CG:** To a cooled (-78°C) solution of compound 78 (1.50 g, 7.92 mmol) in THF (50 mL) under Ar was added a 2.5M solution of n-BuLi in hexanes (3.8 mL, 9.5 mmol). The resulting mixture was stirred for 20 min. CO₂ (2 L) was then bubbled through the mixture for 10 min, and the reaction mixture warmed to room temperature. The volatiles were evaporated and the residue dissolved in THF (50 mL). The solution was cooled to -78°C, and a 1.7M solution of t-BuLi (5.6 mL, 9.50 mmol) was added. The mixture was warmed to -30°C, then again cooled to -78°C. CO₂ (2 L) was bubbled through the solution for 10 min. The obtained solution was allowed to slowly warm to r.t. then concentrated under reduced pressure. The residue was dissolved in water (50 mL), washed with MTBE (2 × 50 mL), then acidified to pH 4, and extracted with ethyl acetate (2x 50 mL). The organic extract was washed with water (2 × 50 mL), and brine (50 mL), dried over Na₂SO₄, and evaporated under reduced pressure. The crude product was washed with hexane and dried to obtain 1.24 g (5.31 mmol, 67%) of target compound 4-(trimethylsilyl)-1H-indole-2-carboxylic acid.
*Rt* (Method G) 1.47 mins, *m*/*z* 232 [M-H]⁻

### Preparation of 6-chloro-5-fluoro-1H-indole-2-carboxylic acid

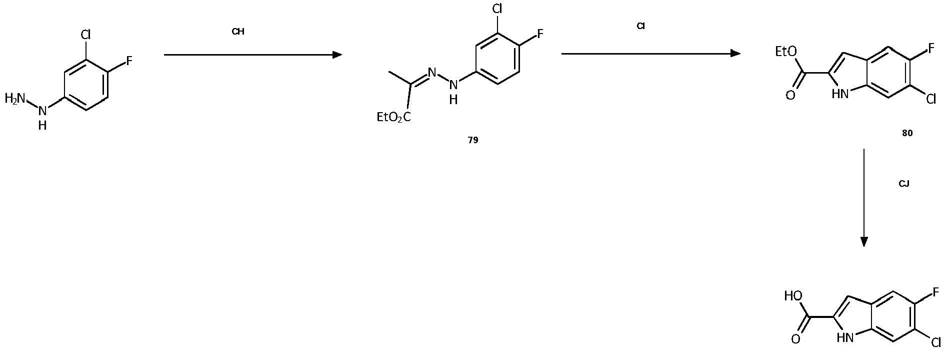

**Step CH:** To a solution of (3-chloro-4-fluorophenyl)hydrazine (80.0 g, 498 mmol) in ethanol (200 mL) was added ethyl pyruvate (58.0 g, 499 mmol). The mixture was refluxed for 1 h, then concentrated under reduced pressure, and diluted with water (300 mL). The solid was collected by filtration then dried to obtain 122 g (472 mmol, 95%) of compound 79.

**Step CI:** A suspension of compound 79 (122 g, 472 mmol) and pTSA (81.5 g, 473 mmol) in toluene (500 mL) was refluxed for 48 h, then cooled to room temperature. The precipitate was collected by filtration and purified by fractional crystallization from toluene to obtain 4.00 g (16.6 mmol, 4%) of compound 80.

**Step CJ:** To a refluxing solution of compound 80 (4.00 g, 16.6 mmol) in ethanol (30 mL) was added NaOH (0.660 g, 16.5 mmol). The mixture was refluxed for 1 h, then concentrated under reduced pressure. The residue was triturated with warm water (80°C, 50 mL) and the solution acidified (pH 2) with concentrated hydrochloric acid. The precipitate was collected by filtration, washed with water (2 × 10 mL), and dried to obtain 3.18 g (14.9 mmol, 90%) of target compound 6-chloro-5-fluoro-1H-indole-2-carboxylic acid.
*Rt* (Method G) 1.23 mins, *m*/*z* 212 [M-H]⁻

### Preparation of 4-(difluoromethyl)-6-fluoro-1H-indole-2-carboxylic acid

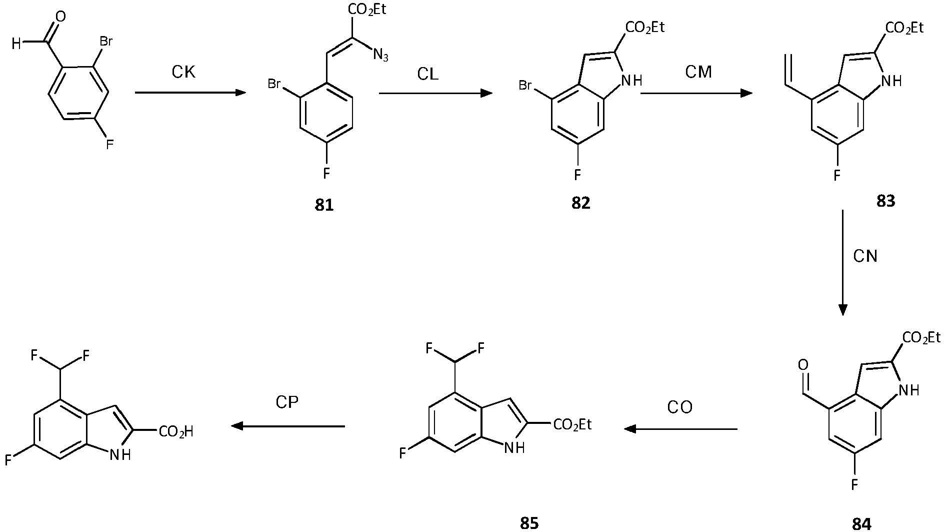

**Step CK:** To a solution of sodium methoxide (50.0 g, 926 mmol) in methanol (300 mL) at -10°C was added dropwise a solution of 2-bromo-4-fluorobenzaldehyde (222 mmol) and methyl azidoacetate (59.0 g, 457 mmol) in methanol (100 mL). The reaction mixture was stirred for 3h, maintaining the temperature below 5°C, then quenched with ice water. The resulting mixture was stirred for 10 min and the solid collected by filtration. The solid was washed with water to afford compound 81 as a white solid (62% yield).

**Step CL:** A solution of compound 81 (133 mmol) in xylene (250 mL) was refluxed for 1h under an argon atmosphere and then concentrated under reduced pressure. The residue was recrystallized form hexane-ethyl acetate mixture (60:40) to give compound 82 (58% yield).

**Step CM**: To a heated (90 °C) solution of compound 82 (14.7 mmol) in anhydrous DMF (10 mL) tri-n-butyl(vinyl)tin (3.60 g, 11.4 mmol) and Pd(PPh₃)₂Cl₂ (0.301 g, 0.757 mmol) were added under nitrogen and the resulting mixture was stirred at 90°C for 1 h. The mixture was cooled to room temperature and purified by silica gel column chromatography (60-80% ethyl acetate in hexane). The combined product fractions were concentrated, washed with water (3 × 100 mL), dried over Na₂SO₄, and concentrated under reduced pressure to afford compound 83 as a yellow solid (60% yield).

**Step CN:** To a mixture of compound 83 (12.4 mmol), acetone (200 mL), and water (40 mL) OsO₄ (0.100 g, 0.393 mmol) and NaIO₄ (13.4 g, 62.6 mmol) were added and the reaction was stirred for 10 h at room temperature. Acetone was distilled off and the aqueous solution was extracted with dichloromethane. The combined organic layer was washed with saturated NaHCO₃ solution (2 × 50 mL) and brine (2 × 50 mL), dried over Na₂SO₄, and concentrated under reduced pressure to afford compound 84 (33% yield).

**Step CO:** To a solution of compound 84 (11.0 mmol) in dichloromethane (50 mL) was added Morph-DAST (4.10 mL, 33.6 mmol). The resulting mixture was stirred until NMR of an aliquot revealed completion of the reaction (2-5 days). The reaction mixture was added dropwise to a cold saturated NaHCO₃ solution (1000 mL). The mixture obtained was extracted with ethyl acetate. The organic layer was dried over MgSO₄ and concentrated. The residue was purified by column chromatography to give compound 85 as yellow solid (48% yield).

**Step CP:** To a solution of compound 85 (4.50 mmol) in THF (50 mL), was added 1N aqueous LiOH (8 mL). The resulting mixture was stirred for 48 h at room temperature then concentrated under reduced pressure and diluted with 1N aqueous NaHSO₄ (8 mL). The obtained mixture was extracted with ethyl acetate. The organic extract was dried over MgSO₄ and concentrated under reduced pressure. The residue was recrystallized from MTBE to obtain 4-(difluoromethyl)-6-fluoro-1H-indole-2-carboxylic acid (87%).
*Rt* (Method G) 1.22 mins, *m*/*z* 228 [M-H]⁻

### Preparation of 4-(difluoromethyl)-7-fluoro-1H-indole-2-carboxylic acid

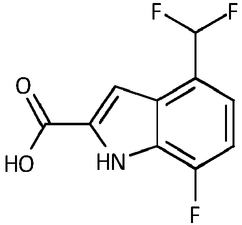

Prepared as described for 4-(difluoromethyl)-6-fluoro-1H-indole-2-carboxylic acid, starting from 2-bromo-5-fluorobenzaldehyde (2.5% overall yield).
*Rt* (Method G) 1.13 mins, *m*/*z* 228 [M-H]⁻

### Preparation of 4-(difluoromethyl)-1H-indole-2-carboxylic acid

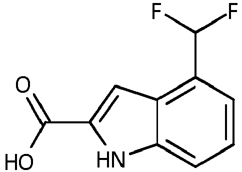

Prepared as described for 4-(difluoromethyl)-6-fluoro-1H-indole-2-carboxylic acid, starting from 4-bromo-1H-indole-2-carboxylic acid (11% overall yield).
*Rt* (Method G) 1.17 mins, *m*/*z* 210 [M-H]⁻

### Preparation of 4-(1,1-difluoroethyl)-6-fluoro-1H-indole-2-carboxylic acid

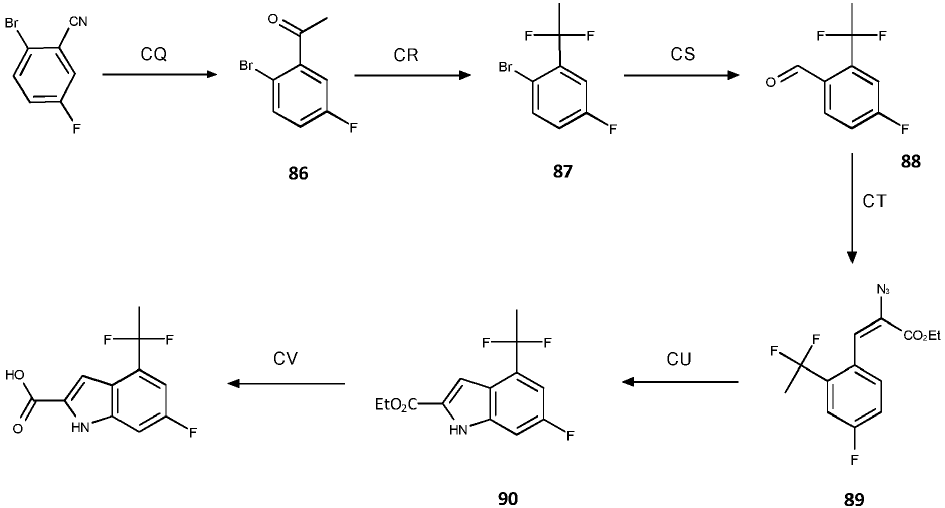

**Step CQ**: To a solution of 2-bromo-5-fluorobenzonitrile (10.0 g, 48.5 mmol) in anhydrous tetrahydrofuran (100 mL) under nitrogen was added methylmagnesium bromide (3.2M in ether, 19 mL, 60.0 mmol). The resulting mixture was heated to reflux for 4 h. The reaction mixture was then cooled, poured into 2N hydrochloric acid (100 mL), and diluted with methanol (100 mL). The organic solvents were removed and the crude product precipitated out. The reaction mixture was extracted with ethyl acetate, dried over MgSO₄, and concentrated. The residue was purified by column chromatography (heptane/dichloromethane) to give 4.88 g (21.9 mmol, 45%) of compound 86 as a pink oil.

**Step CR:** To a solution of compound 86 (110 mmol) in dichloromethane (50mL) at room temperature was added Morph-DAST (41 mL, 336 mmol) and a few drops of water. The resulting mixture was stirred for 48 days at room temperature; every 7 days an additional portion of Morph-DAST (41 mL, 336 mmol) was added. After the reaction was complete, the mixture was carefully added dropwise to cold saturated aqueous NaHCO₃. The product was extracted with ethyl acetate and the organic extract dried over MgSO₄ and concentrated. The residue was purified by column chromatography to give 87 as a colorless liquid (37% yield).

**Step CS:** To a cooled (-80 °C) solution of compound 87 (21.0 mmol) in THF (150 mL) was added slowly a 2.5M solution of n-BuLi in hexanes (10.0 mL, 25.0 mmol of n-BuLi). The mixture was stirred for 1h, then DMF (2.62 mL, 33.8 mmol) was added and the mixture stirred for a further 1h. The reaction was quenched with saturated aqueous NH₄Cl (250 mL) and extracted with Et₂O (3 × 150 mL). The organic layer was dried over Na₂SO₄ and concentrated under reduced pressure. The residue was purified by silica gel chromatography (ethyl acetate/hexane 1:9) to give compound 88 (52% yield).

**Step CT**: To a solution of sodium methoxide (50.0 g, 926 mmol) in methanol (300 mL) at -10 °C was added dropwise a solution of compound 88 (222 mmol) and methyl azidoacetate (59.0 g, 457 mmol) in methanol (100 mL). The reaction mixture was stirred for 3h, maintaining the temperature below 5°C, then quenched with ice water. The resulting mixture was stirred for 10 min. The solid obtained was collected by filtration, and washed with water to afford compound 89 as a white solid (66% yield).

**Step CU:** A solution of compound 89 (120 mmol) in xylene (250 mL) was refluxed for 1 h under an argon atmosphere and then concentrated under reduced pressure. The residue was recrystallized from hexane-ethyl acetate to give compound 90 (70% yield).

**Step CV:** To a solution of compound 90 (4.40 mmol) in THF (50 mL) was added 1N aqueous LiOH (8 mL). The resulting mixture was stirred for 48 h at room temperature, then concentrated under reduced pressure and diluted with 1N aqueous NaHSO₄ (8 mL). The residue obtained was extracted with ethyl acetate. The organic extract was dried over MgSO₄ and concentrated under reduced pressure. The residue was recrystallized from MTBE to obtain target compound 4-(1,1-difluoroethyl)-6-fluoro-1H-indole-2-carboxylic acid (95% yield).
*Rt* (Method G) 1.26 mins, *m*/*z* 242 [M-H]⁻

### Preparation of 4-(1,1-difluoroethyl)-7-fluoro-1H-indole-2-carboxylic acid

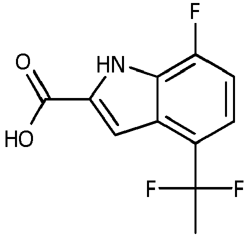

Prepared as described for 4-(1,1-difluoroethyl)-6-fluoro-1H-indole-2-carboxylic acid, starting from 2-bromo-4-fluoroacetophenone (3.6% overall yield).
*Rt* (Method G) 1.23 mins, *m*/*z* 242 [M-H]⁻

### Synthesis of indolizine-2-carboxylic acids

### Synthesis of 1-cyanoindolizine-2-carboxylic acid

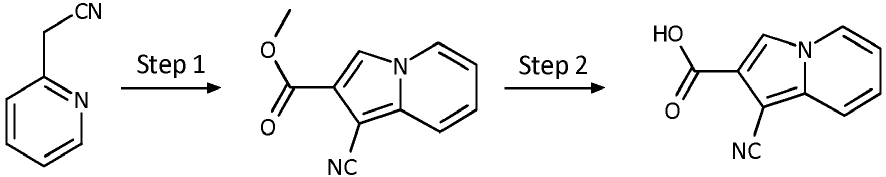

### Step 1: ethyl 1-cyanoindolizine-2-carboxylate

2-(Pyridin-2-yl)acetonitrile (2.42 g, 20.51 mmol) and ethyl 3-bromo-2-oxopropanoate (2.0 g, 10.26 mmol) were mixed in acetone (50 mL) and refluxed for 5h. The mixture was cooled, the precipitated solid was removed, and the filtrate was concentrated. The residue was triturated with water (50ml), stirred for 1h, and the product collected by filtration to give ethyl 1-cyanoindolizine-2-carboxylate (1.9 g, 8.87 mmol, 86.5% yield) as brown solid.

### Step 2: 1-cyanoindolizine-2-carboxylic acid

To a suspension of ethyl 1-cyanoindolizine-2-carboxylate (400.44 mg, 1.87 mmol) in THF/H₂O (3 mL/ 3mL) was added lithium hydroxide monohydrate (313.77 mg, 7.48 mmol). The mixture was stirred at r.t. for 10h. The mixture was concentrated; the residue was dissolved in water (10ml) and acidified with 10% aq. HCl to pH 3. The precipitated solid was collected by filtration and dried to afford 1-cyanoindolizine-2-carboxylic acid (237.0 mg, 1.27 mmol, 68.1% yield) as brown solid.
*Rt* (Method G) 1.29 mins, *m*/*z* 215 [M+H]⁺
¹H NMR (400 MHz, DMSO) δ 6.98 (t, *J* = 6.8 Hz, 1H), 7.25 (dd, *J* = 9.1, 6.7 Hz, 1H), 7.64 (d, *J* = 9.1 Hz, 1H), 8.21 (s, 1H), 8.51 (d, *J* = 7.0 Hz, 1H), 13.10 (br.s, 1H).

### Synthesis of 8-(trifluoromethyl)indolizine-2-carboxylic acid

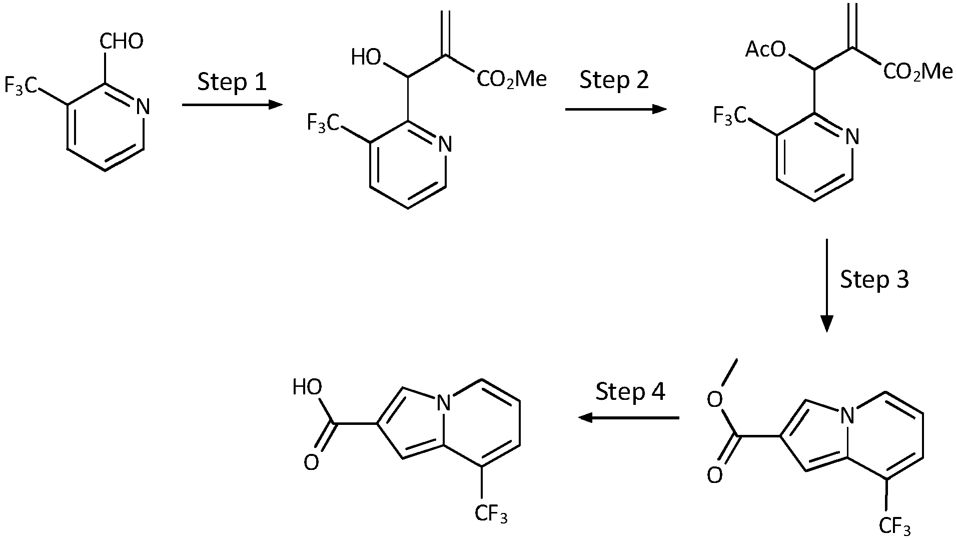

### Step 1: Methyl 2-{hydroxy[3-(trifluoromethyl)pyridin-2-yl]methyl }prop-2-enoate

To a solution of 3-(trifluoromethyl)pyridine-2-carbaldehyde (5.1 g, 29.12 mmol) and methyl prop-2-enoate (7.52 g, 87.37 mmol, 7.92 mL) in dioxane/ H₂O (1/1 v/v, 150 mL), was added 1,4-diazabicyclo[2.2.2]octane (3.27 g, 29.12 mmol). The resulting mixture was stirred at r.t. overnight. The reaction mixture was then diluted with 500 mL of H₂O and extracted with MTBE (300 mL). The organic phase was washed with brine, dried over Na₂SO₄ and concentrated under reduced pressure to give methyl 2-hydroxy[3-(trifluoromethyl)pyridin-2-yl]methylprop-2-enoate (6.1 g, 23.35 mmol, 80.2% yield) as brown oil.

### Step 2: Methyl 2-[(acetyloxy)[3-(trifluoromethyl)pyridin-2-yl]methyl]prop-2-enoate

Methyl 2-hydroxy[3-(trifluoromethyl)pyridin-2-yl]methylprop-2-enoate (5.9 g, 22.59 mmol) was dissolved in acetic anhydride (57.65 g, 564.75 mmol, 53.38 mL) and heated at 100°C for 2h. The reaction mixture was concentrated under reduced pressure, the residue was triturated with MTBE (80 mL) and the solution was quenched with NaHCO₃ sat. aq. 50mL. The organic phase was separated, washed with brine, dried over Na₂SO₄ and concentrated under reduced pressure to give 6 g of a brown liquid, which was ~1/1 mixture of methyl 2-[(acetyloxy)[3-(trifluoromethyl)pyridin-2-yl]methyl]prop-2-enoate (6.0 g, 50.0% purity, 9.89 mmol, 43.8% yield) and cyclized indolizine as shown by ¹H NMR. This mixture was used in the next step without further purification.

### Step 3: Methyl 8-(trifluoromethyl)indolizine-2-carboxylate

The solution of methyl 2-[(acetyloxy)[3-(trifluoromethyl)pyridin-2-yl]methyl]prop-2-enoate (6.0 g, 19.79 mmol) in 100 mL of xylene was heated under reflux overnight. After cooling to r.t. the reaction mixture was diluted with MTBE (200 mL) and washed with Na₂CO₃, brine, dried over Na₂SO₄ and concentrated under reduced pressure to give methyl 8-(trifluoromethyl)indolizine-2-carboxylate (4.67 g, 19.2 mmol, 97% yield) an off-white crystalline solid.

### Step 4: 8-(trifluoromethyl)indolizine-2-carboxylic acid

To a solution of methyl 8-(trifluoromethyl)indolizine-2-carboxylate (230.0 mg, 945.79 µmol) in methanol (15 mL) was added a solution of sodium hydroxide (113.63 mg, 2.84 mmol) in H₂O (5 mL). The resulting mixture was stirred overnight at r.t. The reaction mixture was concentrated under reduced pressure and the residue was triturated with H₂O (50 mL). The resulting solution was acidified with HCl 5N to pH~2 and extracted with MTBE (30 mL). The combined organic extract was dried over Na₂SO₄ and concentrated under vacuum to give 8-(trifluoromethyl)indolizine-2-carboxylic acid (180.0 mg, 785.49 µmol, 82.9% yield) as pale brown solid.
*Rt* (Method G) 1.19 mins, *m*/*z* 228 [M-H]⁻, *m*/*z* 230 [M+H]⁺
¹H NMR (400 MHz, DMSO-d6) δ 12.61 (s, 1H), 8.55 (d, J = 7.1 Hz, 1H), 8.24 (d, J = 1.6 Hz, 1H), 7.29 (d, J = 6.9 Hz, 1H), 6.79 (t, J = 7.9 Hz, 1H), 6.76 (s, 1H).

### Synthesis of 8-fluoroindolizine-2-carboxylic acid

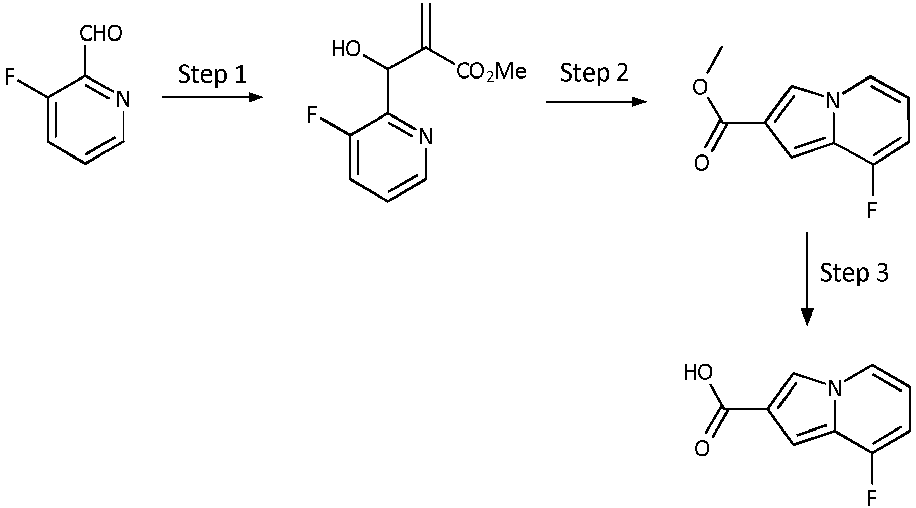

### Step 1: Methyl 2-[(3-fluoropyridin-2-yl)(hydroxy)methyl]prop-2-enoate

To a solution of 3-fluoropyridine-2-carbaldehyde (500.0 mg, 4.0 mmol) in dioxane (10 mL) and H₂O (2mL), was added methyl prop-2-enoate (412.89 mg, 4.8 mmol, 430.0 µl) and 1,4-diazabicyclo[2.2.2]octane (224.16 mg, 2.0 mmol). The mixture was stirred for 24 hours at r.t. and the volatiles were removed under reduced pressure. The crude residue was partitioned between CHCl₃ (15mL) and 3% aq. H₃PO₄ (20mL), and product extracted with CHCl₃ (2*10 mL). The combined organic extracts were dried over Na₂SO₄ and concentrated under reduced pressure to give methyl 2-[(3-fluoropyridin-2-yl)(hydroxy)methyl]prop-2-enoate (430.0 mg, 2.04 mmol, 54.6% yield) as yellow oil.

### Step 2: Methyl 8-fluoroindolizine-2-carboxylate

A mixture of methyl 2-[(3-fluoropyridin-2-yl)(hydroxy)methyl]prop-2-enoate (430.34 mg, 2.04 mmol) and acetic anhydride (5.4 g, 52.9 mmol, 5.0 mL) was heated at 100°C for 1h, then 140°C for 4h, then cooled and concentrated. The residue was dissolved in EtOAc, and the mixture washed with sat. aq NaHCO₃, dried over Na₂SO₄ and concentrated. The residue was purified by flash column chromatography (hexane-EtOAc 3:2) to afford methyl 8-fluoroindolizine-2-carboxylate (260.0 mg, 1.35 mmol, 50.4% yield) as yellow solid.

### Step 3: 8-fluoroindolizine-2-carboxylic acid

To a solution of methyl 8-fluoroindolizine-2-carboxylate (259.87 mg, 1.35 mmol) in MeOH-THF (5ml/5mL) was added 10% aq. NaOH (107.61 mg, 2.69 mmol,). The mixture was stirred at 65°C for 5h. The reaction mixture was concentrated, and the residue dissolved in H₂O (10mL) and acidified with 10% aq. HCl to pH 4. The precipitate was collected by filtration and dried to afford 8-fluoroindolizine-2-carboxylic acid (200.0 mg, 1.12 mmol, 83% yield) as beige solid.
*Rt* (Method G) 1.11 mins, *m*/*z* 178 [M-H]⁻, *m*/*z* 180 [M+H]⁺
¹H NMR (500 MHz, DMSO-d6) δ 12.53 (s, 1H), 8.23 - 7.99 (m, 2H), 6.79 (s, 1H), 6.72 - 6.52 (m, 2H).

### Synthesis of 6-fluoroindolizine-2-carboxylic acid

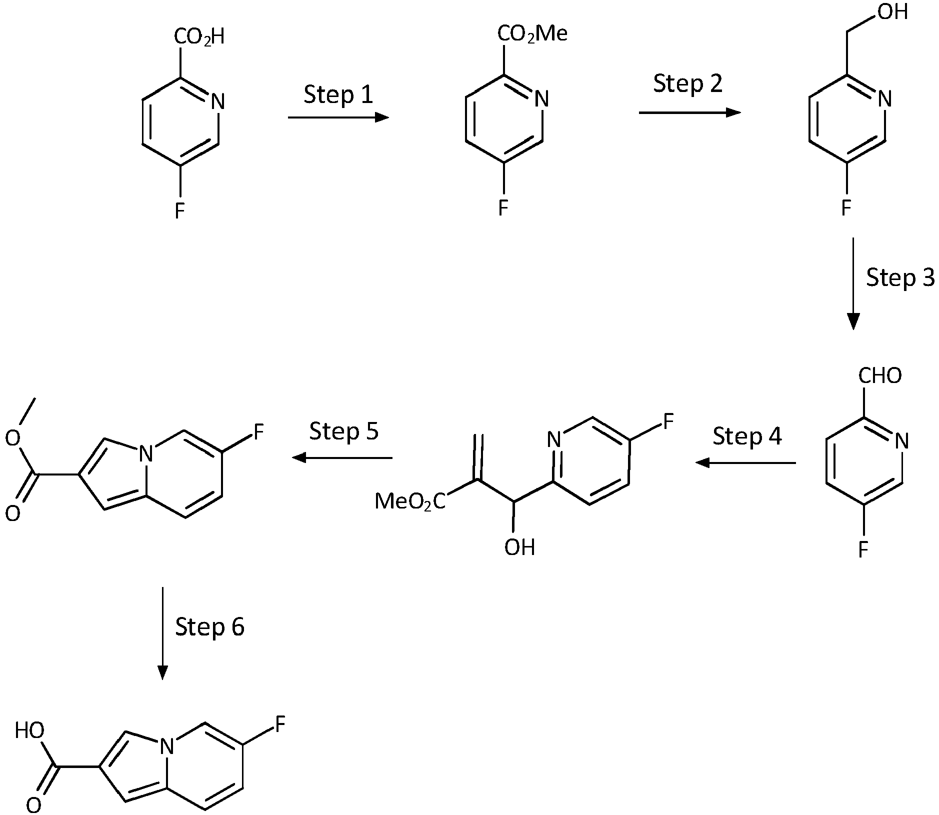

### Step 1: Methyl 5-fluoropyridine-2-carboxylate

To a cooled (0° C) solution of dry MeOH (25mL) was added dropwise thionyl chloride (2.53 g, 21.26 mmol). 5-fluoropyridine-2-carboxylic acid (2.0 g, 14.18 mmol) was added and the reaction mixture was heated at 55°C for 5h. The reaction mixture was cooled to r.t. and concentrated. The residue was triturated with NaHCO₃ (20ml sat. aq.) and the H₂O phase was extracted with EtOAc (3*15mL). The combined organic phases was dried over Na₂SO₄, filtered and concentrated to afford methyl 5-fluoropyridine-2-carboxylate (1.8 g, 11.6 mmol, 81.9% yield) as white solid.

### Step 2: (5-fluoropyridin-2-yl)methanol

To a stirred, cooled (-60°C) solution of methyl 5-fluoropyridine-2-carboxylate (1.8 g, 11.6 mmol) in dry DCM (50mL) under Ar was added dropwise diisobutylaluminum hydride (4.13 g, 29.01 mmol, 5.29 mL). The reaction mixture was warmed to r.t. and stirred overnight. The mixture was cooled to -10°C and HCl 1M was added dropwise. The mixture was stirred for 1h at r.t. and organic phase was separated. The H₂O phase was extracted with DCM (20mL). The combined organic phases were dried over Na₂SO₄, filtered and concentrated to afford (5-fluoropyridin-2-yl)methanol (850.0 mg, 6.69 mmol, 57.6% yield) as yellow oil.

### Step 3: 5-fluoropyridine-2-carbaldehyde

To a stirred solution of (5-fluoropyridin-2-yl)methanol (850.0 mg, 6.69 mmol) in dry DCM (15mL) at r.t. was added portionwise 1,1,1-tris(acetoxy)-1,1-dihydro-1,2-benziodoxol-3(1H)-one (2.84 g, 6.69 mmol). The mixture was stirred for 2h and cooled to 0°C, then NaOH 20% aq. (1.2 g, 30.09 mmol) was added dropwise with stirring. The organic phase was separated, dried over Na₂SO₄ and concentrated to afford 5-fluoropyridine-2-carbaldehyde (300.0 mg, 2.4 mmol, 35.9% yield) as yellow solid.

### Step 4: Methyl 2-[(5-fluoropyridin-2-yl)(hydroxy)methyl]prop-2-enoate

To a solution of 5-fluoropyridine-2-carbaldehyde (299.08 mg, 2.39 mmol) in dioxane (10mL) and H₂O (2 mL) was added methyl prop-2-enoate (247.0 mg, 2.87 mmol, 260.0 µl) and 1,4-diazabicyclo[2.2.2]octane (134.09 mg, 1.2 mmol). After 24 hours the volatiles were removed under reduced pressure and the crude residue was partitioned between CHCl₃ (15mL) and H₂O (25mL). Product was extracted with CHCl₃ (2*10 mL). The combined organic extracts were dried over Na₂SO₄ and concentrated under reduced pressure to give methyl 2-[(5-fluoropyridin-2-yl)(hydroxy)methyl]prop-2-enoate (410.0 mg, 1.94 mmol, 81.2% yield) as brown oil.

### Step 5: Methyl 6-fluoroindolizine-2-carboxylate

A mixture of methyl 2-[(5-fluoropyridin-2-yl)(hydroxy)methyl]prop-2-enoate (410.0 mg, 1.94 mmol) and acetic anhydride (5.4 g, 52.9 mmol, 5.0 mL) was heated at 100°C for 2h (the formation of O-acetyl intermediate was checked by LCMS). Than the mixture was heated at 140°C for 15h, cooled and concentrated in vacuum. The residue was dissolved in EtOAc (30mL) and washed with NaHCO₃ sat. aq (40 mL) for 1h at r.t. The organic phase was separated, dried over Na₂SO₄, filtered and concentrated. The residue was purified by column chromatography on silica (hexane-EtOAc 10:1) to afford methyl 6-fluoroindolizine-2-carboxylate (140.0 mg, 724.74 µmol, 37.3% yield) as white solid.

### Step 6: 6-fluoroindolizine-2-carboxylic acid

To a solution of methyl 6-fluoroindolizine-2-carboxylate (140.18 mg, 725.66 µmol) in MeOH/THF/ H₂O (4/4/1) was added 20% aq. sodium hydroxide (58.05 mg, 1.45 mmol). The mixture was heated at 65 °C overnight. The solvent was removed under reduced pressure, and the resulting residue was dissolved in H₂O. The solution was acidified to pH 3-4 with 1M HCl. The precipitated solid was collected by filtration, washed with H₂O and dissolved in EtOAc-THF (2:1). The solution was dried over Na₂SO₄, filtered and concentrated to afford 6-fluoroindolizine-2-carboxylic acid (105.0 mg, 586.11 µmol, 80.8% yield) as beige solid.
*Rt* (Method G) 1.07 mins, *m*/*z* 178 [M-H]⁻, *m*/*z* 180 [M+H]⁺
¹H NMR (500 MHz, DMSO-d6) δ 12.57 - 12.02 (m, 1H), 8.47 (s, 1H), 8.06 - 7.91 (m, 1H), 7.61 - 7.44 (m, 1H), 6.91 - 6.73 (m, 2H).

### Synthesis of 7-chloroindolizine-2-carboxylic acid

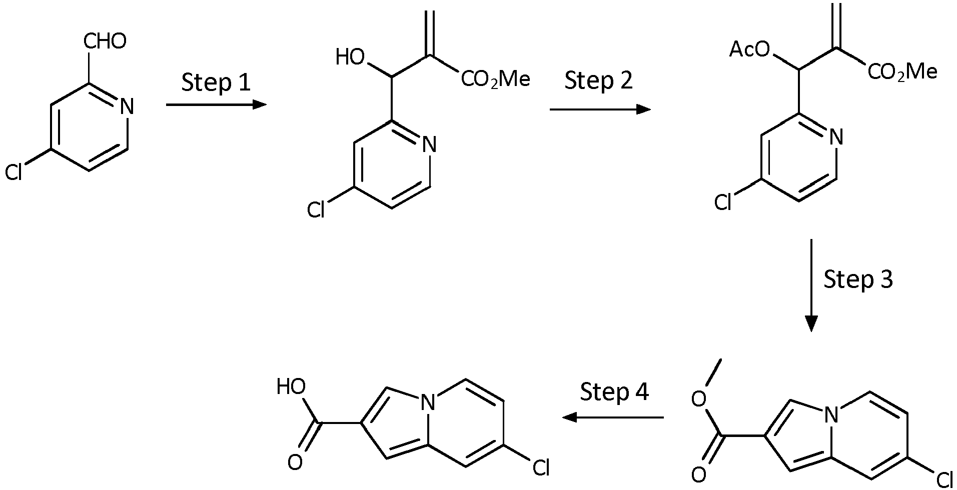

### Step 1: Methyl 2-[(4-chloropyridin-2-yl)(hydroxy)methyl]prop-2-enoate

To a solution of 4-chloropyridine-2-carbaldehyde (500.0 mg, 3.53 mmol) in dioxane (10 mL) and H₂O was added 20mL methyl prop-2-enoate (364.9 mg, 4.24 mmol, 380.0 µl) and 1,4-diazabicyclo[2.2.2]octane (198.11 mg, 1.77 mmol). The mixture was stirred at r.t. for 24 hours. The volatiles were removed under reduced pressure and the crude residue was partitioned between CHCl₃ and aqueous diluted phosphoric acid. Product was extracted with CHCl₃ (2*10 mL). The combined organic extracts were dried over Na₂SO₄ and concentrated under reduced pressure to give methyl 2-[(4-chloropyridin-2-yl)(hydroxy)methyl]prop-2-enoate (430.0 mg, 1.89 mmol, 53.5% yield) as yellow oil.

### Step 2: Methyl 2-[(acetyloxy)(4-chloropyridin-2-yl)methyl]prop-2-enoate

A mixture of methyl 2-[(4-chloropyridin-2-yl)(hydroxy)methyl]prop-2-enoate (430.0 mg, 1.89 mmol) and acetic anhydride (5.4 g, 52.9 mmol, 5.0 mL) was heated at 100°C for 1h. The mixture was cooled to r.t., concentrated in vacuo and the residue was partitioned between CHCl₃ (20mL) and sat. aq NaHCO₃ (30mL). The organic phase was separated; the H₂O phase was additionally extracted with CHCl₃ (2*5mL). The combined organic phases were dried over Na₂SO₄, filtered and concentrated to afford methyl 2-[(acetyloxy)(4-chloropyridin-2-yl)methyl]prop-2-enoate (490.0 mg, 1.82 mmol, 96.2% yield) as brown oil, that was used in the next step.

### Step 3: Methyl 7-chloroindolizine-2-carboxylate

A mixture of methyl 2-[(acetyloxy)(4-chloropyridin-2-yl)methyl]prop-2-enoate (490.02 mg, 1.82 mmol) and acetic anhydride (2.16 g, 21.16 mmol, 2.0 mL) was heated at reflux for 3h. The reaction mixture was cooled, concentrated under vacuum and the residue dissolved in EtOAc (15mL). The solution was washed with sat. aq. NaHCO₃ then dried over Na₂SO₄ and concentrated. The residue was purified by flash column chromatography (EtOAC-hexane 2:3) to afford methyl 7-chloroindolizine-2-carboxylate (215.0 mg, 1.03 mmol, 56.4% yield) as an orange solid.

### Step 4: 7-chloroindolizine-2-carboxylic acid

To a solution of methyl 7-chloroindolizine-2-carboxylate (215.0 mg, 1.03 mmol) in MeOH/THF/ H₂O (4/4/1) was added 20% aq. NaOH (82.04 mg, 2.05 mmol). The mixture was refluxed at 80 °C overnight. The organic solvent was removed under reduced pressure. The remaining solution was cooled (ice bath, 0-5 °C) and adjusted to pH 3-4 with 1M HCl. The suspension was stirred for 30 minutes, then product was collected by filtration and dried to afforded 7-chloroindolizine-2-carboxylic acid (160.0 mg, 817.99 µmol, 79.8% yield) as yellow solid.
*Rt* (Method G) 1.17 mins, *m*/*z* 194/196 [M-H]⁻, *m*/*z* 196/198 [M+H]⁺
¹H NMR (400 MHz, DMSO-d6) δ 12.47 (s, 1H), 8.30 (d, J = 7.5 Hz, 1H), 8.04 (s, 1H), 7.59 (s, 1H), 6.75 - 6.57 (m, 2H).

### Synthesis of 6-chloroindolizine-2-carboxylic acid

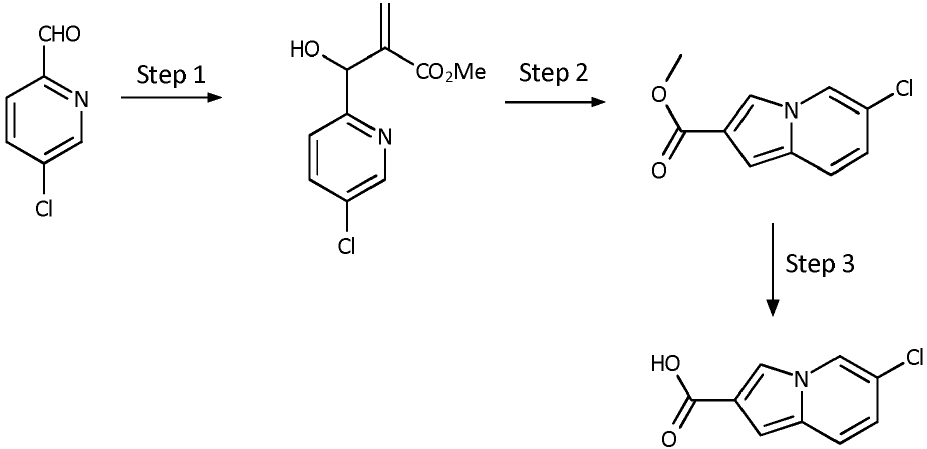

### Step 1: Methyl 2-[(5-chloropyridin-2-yl)(hydroxy)methyl]prop-2-enoate

To a solution of 5-chloropyridine-2-carboxaldehyde (1.0 g, 7.08 mmol) in 20 ml of dioxane and H₂O (4mL) was added methyl prop-2-enoate (731.5 mg, 8.5 mmol, 770.0 µl) and 1,4-diazabicyclo[2.2.2]octane (397.16 mg, 3.54 mmol). After 24 hours the volatiles were removed under reduced pressure and the crude mixture was partitioned between CHCl₃ and H₂O. Product was extracted with CHCl₃ (2*10 mL). The combined organic extracts were dried over Na₂SO₄ and concentrated under reduced pressure to give methyl 2-[(5-chloropyridin-2-yl)(hydroxy)methyl]prop-2-enoate (1.48 g, 6.5 mmol, 91.8% yield) as yellow oil.

### Step 2: Methyl 6-chloroindolizine-2-carboxylate

A mixture of methyl 2-[(5-chloropyridin-2-yl)(hydroxy)methyl]prop-2-enoate (1.48 g, 6.5 mmol) and acetic anhydride (16.2 g, 158.69 mmol, 15.0 mL) was heated at 100°C for 2h (the formation of O-acetyl intermediate was checked by LCMS). The mixture was heated at 140°C for 10h, then cooled and concentrated in vacuum. The residue was dissolved in EtOAc, sat. aq. NaHCO₃ was added and the mixture was stirred for 1h at r.t. The organic phase was separated, dried over Na₂SO₄, filtered and concentrated. The residue was purified with column chromatography on silica (hexane-EtOAc from 10:1 to 4:1 gradient) to afford methyl 6-chloroindolizine-2-carboxylate (400.0 mg, 1.91 mmol, 29.3% yield) as white solid.

### Step 3: 6-chloroindolizine-2-carboxylic acid

To a solution of methyl 6-chloroindolizine-2-carboxylate (399.75 mg, 1.91 mmol) in MeOH/THF/ H₂O (4/4/1) was added 20% aqueous sodium hydroxide (152.54 mg, 3.81 mmol) . The mixture was heated at 65 °C overnight. The solvent was removed under reduced pressure. The residue was dissolved in H₂O and the solution adjusted to pH 3-4 with 1M HCl. The suspension was stirred for 30 minutes, and product was collected by filtration and dried over Na₂SO₄ to afford 6-chloroindolizine-2-carboxylic acid (305.0 mg, 1.56 mmol, 81.8% yield) as beige solid.
Rt (Method G) 1.05 mins, *m*/*z* 194/196 [M-H]⁻, *m*/*z* 196/198 [M+H]⁺
¹H NMR (400 MHz, DMSO-d6) δ 12.46 (s, 1H), 8.55 (s, 1H), 8.01 (s, 1H), 7.51 (d, J = 9.6 Hz, 1H), 6.86 - 6.70 (m, 2H).

### Synthesis of 7-chloro-6-fluoroindolizine-2-carboxylic acid

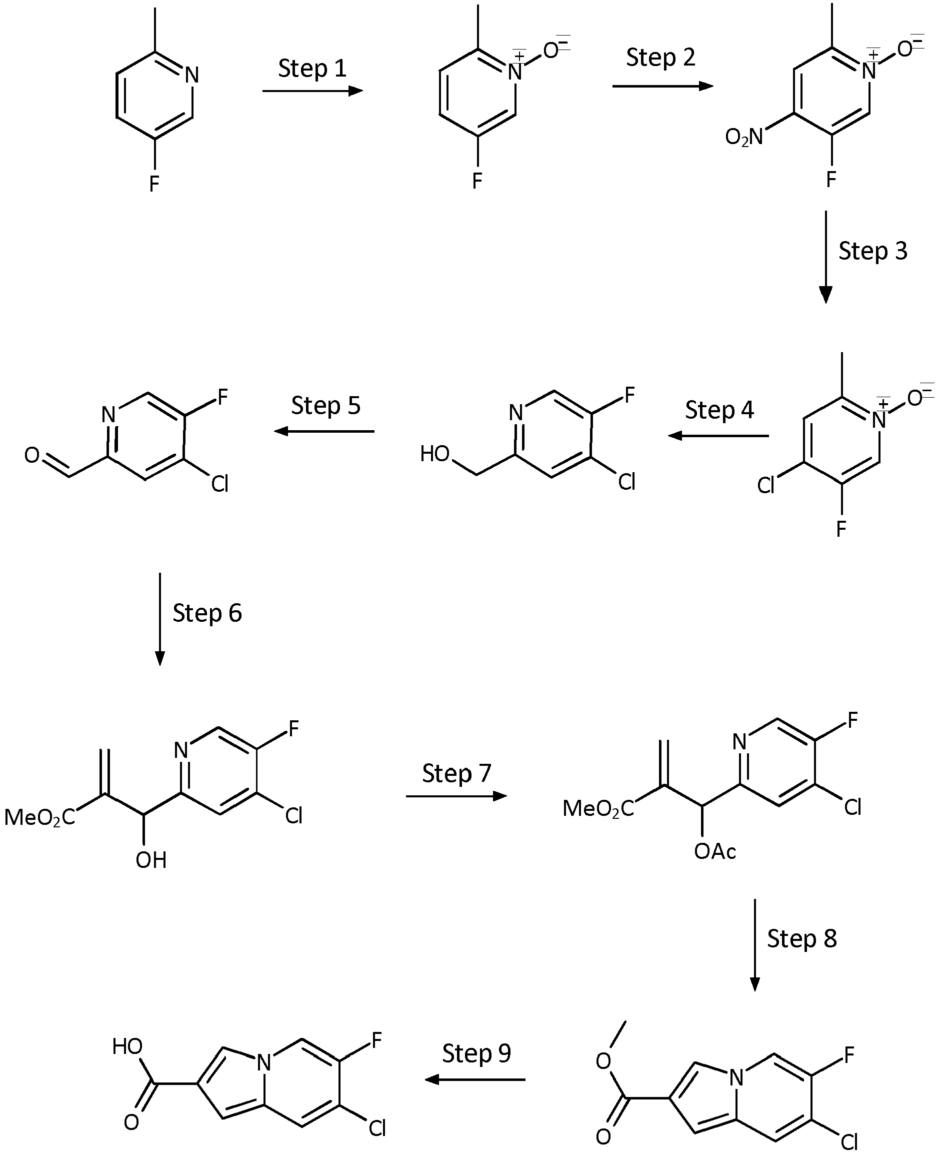

### Step 1: 5-fluoro-2-methylpyridin-1-ium-1-olate

To a cooled (5°C) solution of 5-fluoro-2-methylpyridine (15.0 g, 134.99 mmol) (1.0 eq) in CH₂Cl₂ (300 mL) was added portionwise 3-chlorobenzene-1-carboperoxoic acid (34.94 g, 202.49 mmol) (1.5 eq). The resulting solution was stirred at room temperature overnight. After stirring for 16 hours the solution was washed with aqueous sodium bicarbonate and the aqueous re-extracted with dichloromethane (3 × 200 mL). The combined organic fractions were dried and concentrated to give crude 5-fluoro-2-methylpyridin-1-ium-1-olate (11.0 g, 93.0% purity, 80.48 mmol, 59.6% yield).

### Step 2: 5-fluoro-2-methyl-4-nitropyridin-1-ium-1-olate

A mixture of H₂SO₄ (50 mL conc.) and fuming nitric acid (81 mL) was added dropwise over 10 min with ice-cooling (5°C) and stirring to a solution of 5-fluoro-2-methylpyridin-1-ium-1-olate (11.0 g, 86.53 mmol) in concentrated sulfuric acid (40mL). The mixture was allowed to warm to room temperature over 1h and then heated on a steam bath for 2 h. After cooling, the solution was poured onto ice and neutralized by addition of solid ammonium carbonate. The mixture was extracted with CHCl₃ (3x35 mL), dried (Na₂SO₄), and concentrated in vacuo to a solid which was triturated with petroleum ether (60/80), to give 5-fluoro-2-methyl-4-nitropyridin-1-ium-1-olate (8.37 g, 90.0% purity, 43.77 mmol, 50.6% yield) as yellow solid.

### Step 3: 4-chloro-5-fluoro-2-methylpyridin-1-ium-1-olate

Phosphoryl trichloride (22.37 g, 145.91 mmol, 13.6 mL) (3eq.) in dichloromethane (170mL) was added dropwise under Ar to a cooled (5-10°C), stirred solution of 5-fluoro-2-methyl-4-nitropyridine-1-oxide (8.37 g, 48.64 mmol) (1eq) in dichloromethane (170 mL). After standing for 16 h at room temperature, the solution was refluxed for 4 h, cooled, and poured onto ice (350g). The mixture was stirred for 10 min and then adjusted to pH13 with cooling, using 40% sodium hydroxide. The aqueous phase was separated and then extracted with dichloromethane. The combined extracts were dried (Na₂SO₄) and concentrated in vacuo. The resulting solid was triturated with petroleum ether (60/80), collected by filtration and dried, to give 4-chloro-5-fluoro-2-methylpyridin-1-ium-1-olate (6.72 g, 97.0% purity, 40.35 mmol, 83% yield).

### Step 4: (4-chloro-5-fluoropyridin-2-yl)methanol

Trifluoroacetyl 2,2,2-trifluoroacetate (1.76 g, 8.36 mmol, 1.17 mL) (3eq.) was added dropwise over 1 min to a stirred, cooled (10-15°C) solution of 4-chloro-5-fluoro-2-methylpyridin-1-ium-1-olate (450.0 mg, 2.79 mmol) (1eq.) in dichloromethane (10mL). The solution was warmed to room temperature and left for 7 days. It was poured onto ice, the pH was adjusted to 13 by addition K₂CO₃ aq sat. and 40% aq. NaOH. The aqueous layer was separated and further extracted with dichloromethane (15 mL), and the combined organic layers were dried over K₂CO₃ and concentrated to give crude product as red oil. Pure (4-chloro-5-fluoropyridin-2-yl)methanol (92.0 mg, 97.0% purity, 552.36 µmol, 19.8% yield) was obtained after purification by HPLC.

### Step 5: 4-chloro-5-fluoropyridine-2-carbaldehyde

To a cooled (0°C) solution of (4-chloro-5-fluoropyridin-2-yl)methanol (500.0 mg, 3.09 mmol) in DCM (30 mL) was added 1,1,1-tris(acetoxy)-1,1-dihydro-1,2-benziodoxol-3(1H)-one (1.44 g, 3.41 mmol) in one portion. After reaction was complete (monitored by ¹H NMR,) the mixture was poured into a stirred aqueous solution of NaHCO₃ and Na₂S₂O₃ and stirred until the organic phase became transparent (about 1h.). The layers were separated and the aqueous layer extracted with DCM (3x50 mL). The combined organic extracts were washed with brine, dried over Na₂SO₄ and concentrated under reduced pressure to give 4-chloro-5-fluoropyridine-2-carbaldehyde (400.0 mg, 90.0% purity, 2.26 mmol, 72.9% yield).

### Step 6: Methyl 2-[(4-chloro-5-fluoropyridin-2-yl)(hydroxy)methyl]prop-2-enoate

To a solution of 4-chloro-5-fluoropyridine-2-carbaldehyde (1.21 g, 7.6 mmol) in 18 ml of dioxane and H₂O (6mL) was added methyl prop-2-enoate (850.0 mg, 9.87 mmol, 890.0 µl) and 1,4-diazabicyclo[2.2.2]octane (76.69 mg, 683.7 µmol). After 24 hours the volatiles were removed under reduced pressure and the residue was partitioned between CHCl₃ (100mL) and H₂O (30mL). The H₂O layer was extracted with CHCl₃ (2*30 mL). The combined organic extracts were dried over Na₂SO₄ and concentrated under reduced pressure to give methyl 2-[(4-chloro-5-fluoropyridin-2-yl)(hydroxy)methyl]prop-2-enoate (1.5 g, 95.0% purity, 5.8 mmol, 76.4% yield) as yellow oil.

### Step 7: Methyl 2-[(acetyloxy)(4-chloro-5-fluoropyridin-2-yl)methyl]prop-2-enoate

A single-neck round bottomed flask equipped with a magnetic stirrer and a reflux condenser was charged with acetic anhydride (43.65 g, 427.54 mmol) and methyl 2-[(4-chloro-5-fluoropyridin-2-yl)(hydroxy)methyl]prop-2-enoate (1.5 g, 6.11 mmol). The reaction mixture was stirred at 100 °C for 3 hours to give methyl 2-[(acetyloxy)(4-chloro-5-fluoropyridin-2-yl)methyl]prop-2-enoate (1.5 g, 95.0% purity, 4.95 mmol, 81.1% yield) as solution in AczO.

### Step 8: Methyl 7-chloro-6-fluoroindolizine-2-carboxylate

The solution of methyl 2-[(acetyloxy)(4-chloro-5-fluoropyridin-2-yl)methyl]prop-2-enoate (1.5 g, 5.21 mmol) in Ac₂O was heated under reflux under N₂ for 96 hours. The reaction mixture was cooled to room temperature, then poured into a mixture of ice and sat. aq NaHCO₃, and stirred for 1 hour. The mixture was extracted with ethyl acetate (3×25mL). The combined organic extracts were dried over anhydrous Na₂SO₄, filtered and concentrated. The crude product was purified by silica gel column chromatography eluting with hexane/ethyl acetate (3/1) to afford methyl 7-chloro-6-fluoroindolizine-2-carboxylate (770.0 mg, 98.0% purity, 3.32 mmol, 63.6% yield) as white solid.

### Step 9: 7-chloro-6-fluoroindolizine-2-carboxylic acid

To a solution of methyl 7-chloro-6-fluoroindolizine-2-carboxylate (600.0 mg, 2.64 mmol) in MeOH/THF/ H₂O (4/4/1) (20mL) was added sodium hydroxide (527.13 mg, 13.18 mmol). The mixture was refluxed at 80°C for 6h. Volatiles were removed under reduced pressure. The remaining solution was cooled (0~5°C) and adjusted to pH 3~4 with 1M HCl. The suspension was stirred for 30 minutes and product was collected by filtration. The filter cake was dried to give 7-chloro-6-fluoroindolizine-2-carboxylic acid (440.0 mg, 98.0% purity, 2.02 mmol, 76.6% yield) as yellow solid.
Rt (Method G) 1.24 mins, *mlz* 212/214 [M-H]⁻, *m*/*z* 214/216 [M+H]⁺
¹H NMR (400 MHz, DMSO-d6) δ 12.56 (s, 1H), 8.69 (d, J = 5.6 Hz, 1H), 8.03 (s, 1H), 7.84 (d, J = 7.6 Hz, 1H), 6.79 (s, 1H).

### Synthesis of 6,8-dichloroindolizine-2-carboxylic acid

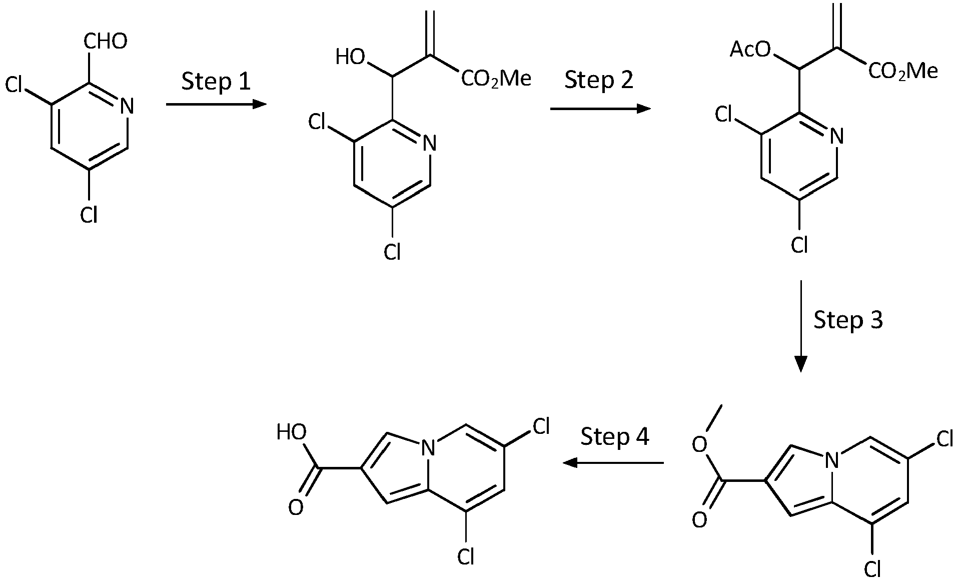

### Step 1: Methyl 2-[(3,5-dichloropyridin-2-yl)(hydroxy)methyl]prop-2-enoate

To a solution of 3,5-dichloropyridine-2-carbaldehyde (462.0 mg, 2.62 mmol) and methyl prop-2-enoate (677.97 mg, 7.88 mmol, 710.0 µl) in dioxane/ H₂O (1/1 v/v) (15 mL) was added 1,4-diazabicyclo[2.2.2]octane (294.46 mg, 2.63 mmol). The resulting mixture was stirred at r.t. overnight. The reaction mixture was diluted with H₂O (200 mL) and extracted with 50 mL of MTBE. The organic phase was washed with brine, dried over Na₂SO₄ and concentrated under reduced pressure to give methyl 2-[(3,5-dichloropyridin-2-yl)(hydroxy)methyl]prop-2-enoate (570.0 mg, 2.17 mmol, 82.8% yield) as brown oil.

### Step 2: Methyl 2-[(acetyloxy)(3,5-dichloropyridin-2-yl)methyl]prop-2-enoate

Methyl 2-[(3,5-dichloropyridin-2-yl)(hydroxy)methyl]prop-2-enoate (570.0 mg, 2.17 mmol) was dissolved in acetic anhydride (5.55 g, 54.34 mmol, 5.14 mL) and heated at 100°C for 2h. The reaction mixture was concentrated under reduced pressure, the residue was taken up in 20 mL of MTBE and the resulting mixture was quenched with sat. aq NaHCO₃. The organic phase was separated, washed with brine, dried over Na₂SO₄ and concentrated under reduced pressure to give methyl 2-[(acetyloxy)(3,5-dichloropyridin-2-yl)methyl]prop-2-enoate (450.0 mg, 1.48 mmol, 68.1% yield) as brown liquid.

### Step 3: Methyl 6,8-dichloroindolizine-2-carboxylate

Methyl 2-[(acetyloxy)(3,5-dichloropyridin-2-yl)methyl]prop-2-enoate (440.0 mg, 1.45 mmol) was dissolved in 15 mL of xylene and refluxed overnight. The reaction mixture was cooled to r.t., diluted with MTBE (50 mL), quenched with NaHCO₃ aq (30mL), washed with brine, dried over Na₂SO₄ and concentrated under reduced pressure to give methyl 6,8-dichloroindolizine-2-carboxylate (360.0 mg, 1.47 mmol, 98.9% yield) as light yellow crystals.

### Step 4: 6,8-dichloroindolizine-2-carboxylic acid

To a solution of methyl 6,8-dichloroindolizine-2-carboxylate (360.0 mg, 1.47 mmol) in methanol 50 (mL) was added a solution of sodium hydroxide (589.92 mg, 14.75 mmol) in H₂O (10 mL). The resulting mixture was stirred overnight at r.t. The reaction mixture was concentrated under reduced pressure and the residue was taken up in H₂O (100 mL). The resulting solution was acidified with 5N HCl to pH~2 and extracted with MTBE (2 × 100 mL). The combined organic extracts were dried over Na₂SO₄ and concentrated in vacuum to give 6,8-dichloroindolizine-2-carboxylic acid (220.0 mg, 956.32 µmol, 64.8% yield) as yellow powder.
Rt (Method G) 1.29 mins, *mlz* 228/230 [M-H]⁻, *m*/*z* 230/232 [M+H]⁺
¹H NMR (400 MHz, DMSO-d6) δ 12.62 (s, 1H), 8.62 (s, 1H), 8.14 (d, J = 1.7 Hz, 1H), 7.15 (d, J = 1.5 Hz, 1H), 6.85 (s, 1H).

### Synthesis of 5-methylindolizine-2-carboxylic acid

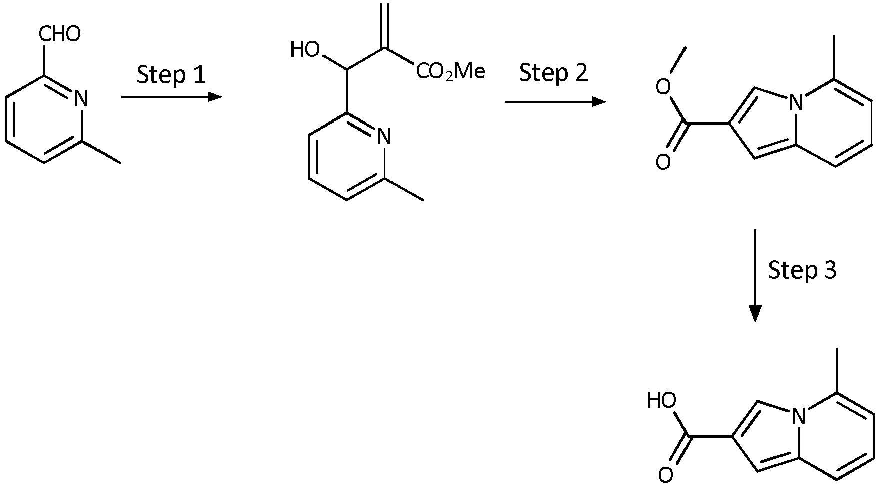

### Step 1: Methyl 2-[hydroxy(6-methylpyridin-2-yl)methyl]prop-2-enoate

To a solution of 6-methylpyridine-2-carbaldehyde (500.0 mg, 4.13 mmol) in dioxane (10 mL) and H₂O (2mL) was added methyl prop-2-enoate (426.24 mg, 4.95 mmol, 450.0 µl) and 1,4-diazabicyclo[2.2.2]octane (231.41 mg, 2.06 mmol). The mixture was stirred for 24 hours, the volatiles were removed under reduced pressure and the crude mixture was partitioned between CHCl₃ and H₂O. Product was extracted with CHCl₃ (2*10 mL). The combined organic extracts were dried over Na₂SO₄ and concentrated under reduced pressure to give methyl 2-[hydroxy(6-methylpyridin-2-yl)methyl]prop-2-enoate (580.0 mg, 2.8 mmol, 67.8% yield) as white solid.

### Step 2: Methyl 5-methylindolizine-2-carboxylate

A mixture of methyl 2-[hydroxy(6-methylpyridin-2-yl)methyl]prop-2-enoate (580.46 mg, 2.8 mmol) and acetic anhydride (5.4 g, 52.9 mmol, 5.0 mL) was heated at 100°C for 3h. The mixture was cooled and concentrated under reduced pressure. The residue was dissolved in EtOAc (20mL) and washed with sat. aq NaHCO₃ The organic phase was dried over Na₂SO₄, filtered and concentrated. The residue was purified by flash column chromatography (hexane-EtOAc 3:1) to afford methyl 5-methylindolizine-2-carboxylate (350.0 mg, 1.85 mmol, 66% yield) as beige solid.

### Step 3: 5-methylindolizine-2-carboxylic acid

To a solution of methyl 5-methylindolizine-2-carboxylate (350.05 mg, 1.85 mmol) in MeOH (5mL) was added 20% H₂O solution of sodium hydroxide (147.99 mg, 3.7 mmol). The reaction mixture was heated at 65°C for 3h. The mixture was cooled, and concentrated; the residue was dissolved in H₂O and acidified with 2M HCl 2M to pH 4. The precipitated solid was collected by filtration, and dried to afford 5-methylindolizine-2-carboxylic acid (250.0 mg, 1.43 mmol, 77.1% yield) as grey solid.
Rt (Method G) 1.16 mins, *m*/*z* 176 [M+H]⁺
¹H NMR (500 MHz, DMSO-d6) δ 12.35 (s, 1H), 7.79 (s, 1H), 7.40 (d, J = 9.1 Hz, 1H), 6.80 (s, 1H), 6.76 (dd, J = 9.3, 6.8 Hz, 1H), 6.56 (d, J = 6.6 Hz, 1H), 2.52 (s, 3H).

### Synthesis of 1-propylindolizine-2-carboxylic acid

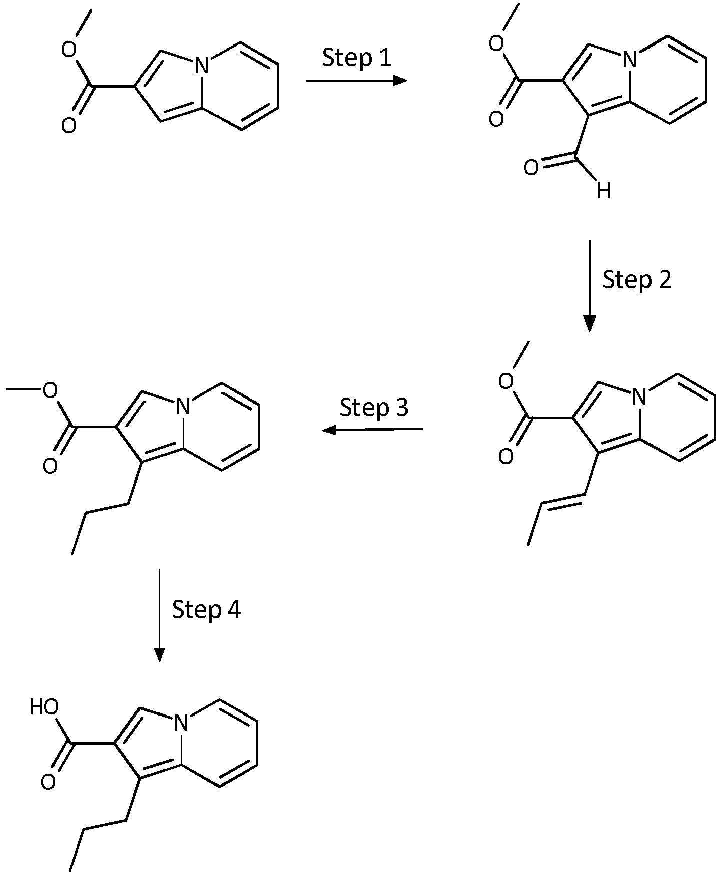

### Step 1: Methyl 1-formylindolizine-2-carboxylate

A solution of phosphoryl trichloride (14.89 g, 97.09 mmol, 9.05 mL) (1.7eq) in DMF (300mL) was stirred at 0° C. for 1 h. To a cooled (0° C) stirred solution of methyl indolizine-2-carboxylate (10.01 g, 57.11 mmol) in dry CH₂Cl₂ (1100 mL), was added 2/3 of the previously prepared POCl₃ solution in DMF (200ml, 1.1 eq.). After being stirred at r.t. for 2 h, the reaction mixture was quenched with aqueous sat. NaHCO₃. The organic layer was washed with H₂O (0.5 L), dried over Na₂SO₄ and concentrated under reduced pressure to give methyl 3-formylindolizine-2-carboxylate (8.0 g, 95.0% purity, 37.4 mmol, 65.5% yield) which was used in the next step without further purification.

### Step 2: Methyl 1-[(1E)-prop-1-en-1-yl]indolizine-2-carboxylate

To a cooled (-15°C ) solution of ethyl(trisphenyl)phosphonium bromide (13.7 g, 36.91 mmol) in anhydrous THF (200 mL) under Ar was slowly added n-BuLi (16mL, 2.5 M in n-hexane). The mixture was warmed to r.t. and stirred for 1.5h. Then a solution of methyl 3-formylindolizine-2-carboxylate (2.5 g, 12.3 mmol) in anhydrous THF (50 mL) was added dropwise to the solution, and the reaction stirred at r.t. for another 24h. The reaction mixture was cooled and quenched by addition of H₂O (200 mL). MTBE (150mL) was added and the resulting mixture was stirred at r.t. for 15 min. The organic layer was separated, dried over Na₂SO₄, filtered and the filtrate concentrated under reduced pressure. The crude product was purified by HPLC to give methyl 3-[(1E)-prop-1-en-1-yl]indolizine-2-carboxylate (500.0 mg, 95.0% purity, 2.21 mmol, 17.9% yield).

### Step 3: Methyl 1-propylindolizine-2-carboxylate

To a solution of methyl 3-[(1E)-prop-1-en-1-yl]indolizine-2-carboxylate (150.0 mg, 696.85 µmol) in THF (5 mL) was added 10% Pd on carbon (5% mass). The mixture was hydrogenated at 1 bar and then allowed to stir at r.t for 1h. (¹H NMR monitoring). The reaction mixture was filtered. The filtrate was concentrated under reduced pressure to give crude methyl 3-propylindolizine-2-carboxylate (150.0 mg, 91.0% purity, 628.27 µmol, 90.2% yield), which was used in the next step without further purification

### Step 4: 1-propylindolizine-2-carboxylic acid

Methyl 3-propylindolizine-2-carboxylate (399.81 mg, 1.84 mmol) and lithium hydroxide monohydrate (108.11 mg, 2.58 mmol) were stirred in a mixture of THF : H₂O : CH3OH (v/v 3 : 1 : 1, 50 mL) at 50°C for 18h.The reaction mixture was then concentrated under reduced pressure and acidified to pH 4 with saturated solution of citric acid. The product was collected by filtration, washed with H₂O (3×50 mL), and then dried in vacuo at 45°C to give 3-propylindolizine-2-carboxylic acid (244.0 mg, 94.0% purity, 1.13 mmol, 61.3% yield) as a yellow solid.
Rt (Method G) 1.32 mins, *m*/*z* 204 [M+H]⁺
¹H NMR (500 MHz, DMSO-d6) δ 12.17 (s, 1H), 8.11 (d, J = 7.1 Hz, 1H), 7.41 (d, J = 9.0 Hz, 1H), 6.75 - 6.67 (m, 2H), 6.63 (t, J = 6.4 Hz, 1H), 3.22 (t, J = 7.7 Hz, 2H), 1.56 (h, J = 7.4 Hz, 2H), 0.89 (t, J = 7.4 Hz, 3H).

### Synthesis of 5-chloroindolizine-2-carboxylic acid

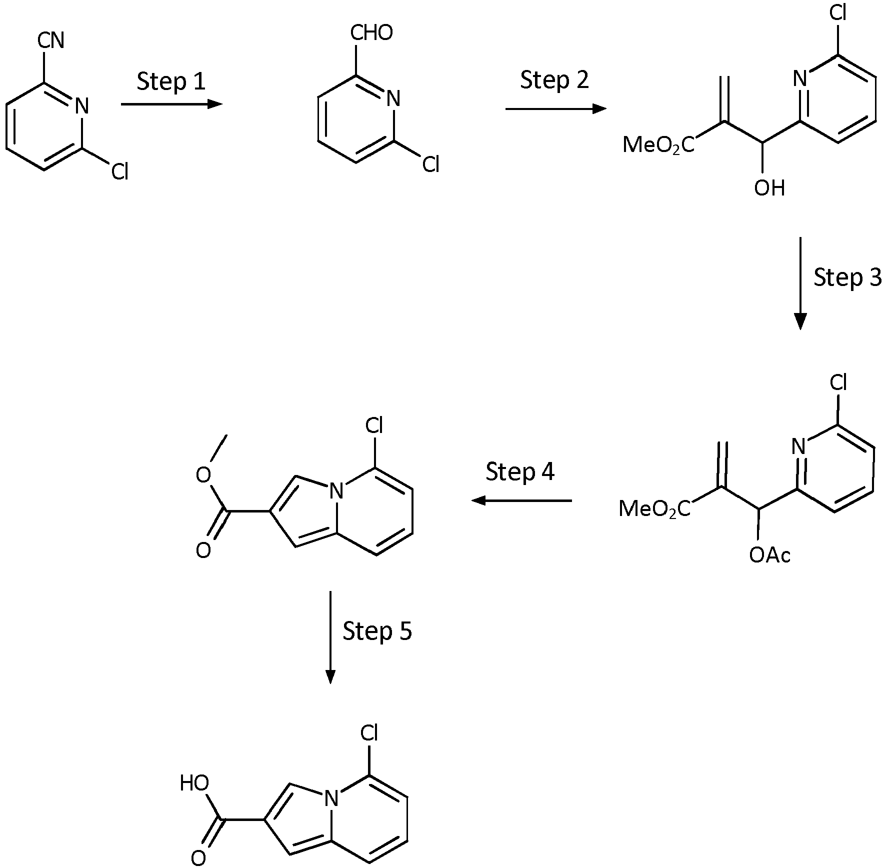

### Step 1: 6-chloropicolinaldehyde.

To a cooled (-78°C), stirred solution of 6-chloropicolinonitrile (15.0 g, 108 mmol) in dichloromethane (500 mL) under flow of argon was added DIBAL-H (23 mL). The reaction mixture was stirred for 3 h maintaining the temperature below -60°C. Then, the mixture was cooled to -78°C and the reaction was quenched with H₂O (46 mL). The suspension obtained was warmed to r.t. and acidified to pH 4 with hydrochloric acid (approx. 50 mL). The organic layer was separated, washed with brine, dried over Na₂SO₄, and concentrated under reduced pressure. The residue was purified by silica gel column chromatography (hexane/ethyl acetate 8:2) to give 4.15 g (29.3 mmol, 27%) of 6-chloropicolinaldehyde.

### Step 2: methyl 2-((6-chloropyridin-2-yl)(hydroxy)methyl)acrylate

To a mixture of 6-chloropicolinaldehyde (3.15 g, 22.3 mmol), dioxane (27 mL), and H₂O (9 mL) was added methyl methacrylate (2.30 g, 23.0 mmol) and DABCO (0.250 g, 2.23 mmol). The mixture was stirred at r.t. overnight. The mixture was diluted with H₂O (50 mL) and extracted with ethyl acetate (2 × 50 mL). The combined organic extracts were washed with H₂O and brine, then dried over Na₂SO₄, and evaporated under reduced pressure to obtain 5.00 g (22.0 mmol, 99%) of methyl 2-((6-chloropyridin-2-yl)(hydroxy)methyl)acrylate.

### Step 3: methyl 2-[(acetyloxy)(6-chloropyridin-2-yl)methyl]prop-2-enoate

A mixture of methyl 2-((6-chloropyridin-2-yl)(hydroxy)methyl)acrylate (5.00 g, 22.0 mmol) and acetic anhydride (40 mL) was stirred at 100°C overnight, cooled to r.t., and used in the next step without further purification.

### Step 4: methyl 5-chloroindolizine-2-carboxylate

A solution of methyl 2-(acetoxy(6-chloropyridin-2-yl)methyl)acrylate, obtained in the previous step, was poured into H₂O (250 mL) and extracted with MTBE (2 × 70 mL). The organic extract was washed with H₂O (3 × 100 mL) and NaHCO₃ solution (3 × 100 mL), dried over Na₂SO₄, and concentrated under reduced pressure. The obtained solid was purified by silica gel column chromatography (hexane/ethyl acetate 8:2) to give 2.00 g (9.54 mmol, 44%) of compound methyl 5-chloroindolizine-2-carboxylate.

### Step 5: 5-chloroindolizine-2-carboxylic acid

To a mixture of methyl 5-chloroindolizine-2-carboxylate (1.20 g, 5.72 mmol), THF (8 mL), methanol (8 mL), and H₂O (4 mL) was added a solution of NaOH (0.275 g, 6.88 mmol) in H₂O (3 mL). The mixture was stirred at r.t. for 1h. Volatiles were evaporated and the residue was mixed with H₂O (10 mL). The obtained solution was acidified with NaHSO₄ (3.00 g). The precipitated solid was collected by filtration, washed with H₂O, and dried to obtain 1.10 g (5.62 mmol, 98%) of 5-chloroindolizine-2-carboxylic acid.
Rt (Method G) 1.18 mins, *m*/*z* 196 [M+H]⁺
¹H NMR (400 MHz, DMSO-d6) δ 12.63 (s, 1H), 7.97 (s, 1H), 7.57 (d, J = 9.0 Hz, 1H), 7.04 - 6.90 (m, 2H), 6.84 (t, J = 8.0 Hz, 1H).

### Synthesis of 8-chloroindolizine-2-carboxylic acid

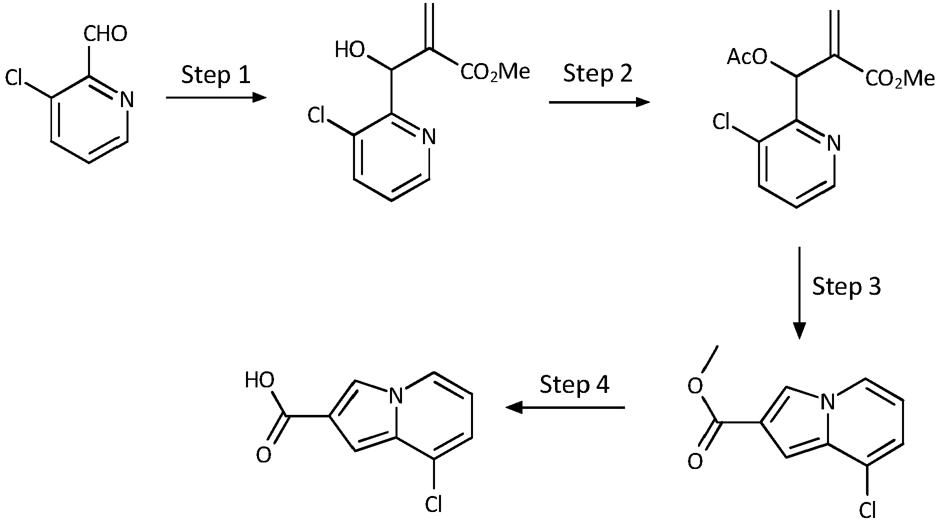

### Step 1: methyl 2-((3-chloropyridin-2-yl)(hydroxy)methyl)acrylate

To a mixture of 3-chloropicolinaldehyde (7.10 g, 50.2 mmol), dioxane (60 mL), and H₂O (20 mL) were added methyl acrylate (5.40 mL, 59.6 mmol) and DABCO (0.340 g, 3.03 mmol. The reaction mixture was stirred at r.t. overnight. The mixture was diluted with ethyl acetate (100 mL) and H₂O (50 mL). The aqueous layer was separated and extracted with ethyl acetate (2 × 50 mL). The combined organic extracts were washed with H₂O and brine, dried over Na₂SO₄, and concentrated under reduced pressure to obtain 8.00 g (35.1 mmol, 70%) of methyl 2-((3-chloropyridin-2-yl)(hydroxy)methyl)acrylate.

### Step 2: 2-[(acetyloxy)(3-chloropyridin-2-yl)methyl]prop-2-enoic acid

A mixture of methyl 2-((3-chloropyridin-2-yl)(hydroxy)methyl)acrylate (8.00 g, 35.1 mmol) and acetic anhydride (100 mL) was stirred at 100°C for 3 h, then concentrated under reduced pressure (80°C). The residue was used in the next step without further purification.

### Step 3: methyl 8-chloroindolizine-2-carboxylate

Methyl 2-(acetoxy(3-chloropyridin-2-yl)methyl)acrylate was mixed with H₂O and extracted with MTBE. The organic extract was washed with sat. aq. NaHCO₃, dried over Na₂SO₄, and concentrated under reduced pressure to give 5.90 g (28.1 mmol, 80% over 2 steps) of methyl 8-chloroindolizine-2-carboxylate.

### Step 4: 8-chloroindolizine-2-carboxylic acid

To a mixture of methyl 8-chloroindolizine-2-carboxylate (2.50 g, 11.9 mmol), THF (8 mL), methanol (8 mL), and H₂O (2 mL) was added a solution of NaOH (1.43 g, 35.7 mmol) in H₂O (7 mL). The mixture was stirred at r.t. overnight, then the volatiles were evaporated and the residue was mixed with H₂O. The obtained slurry was washed with ethyl acetate and then acidified with hydrochloric acid. The precipitate was collected by filtration, then dried to obtain 2.00 g (10.2 mmol, 86%) of 8-chloroindolizine-2-carboxylic acid.
Rt (Method G) 1.08 mins, *m*/*z* 194 [M-H]⁻
¹H NMR (400 MHz, DMSO-d6) δ 12.55 (s, 1H), 8.31 (d, J = 7.0 Hz, 1H), 8.17 (d, J = 1.8 Hz, 1H), 6.97 (d, J = 7.1 Hz, 1H), 6.78 (s, 1H), 6.67 (t, J = 7.1 Hz, 1H).

### Synthesis of 5-(trifluoromethyl)indolizine-2-carboxylic acid

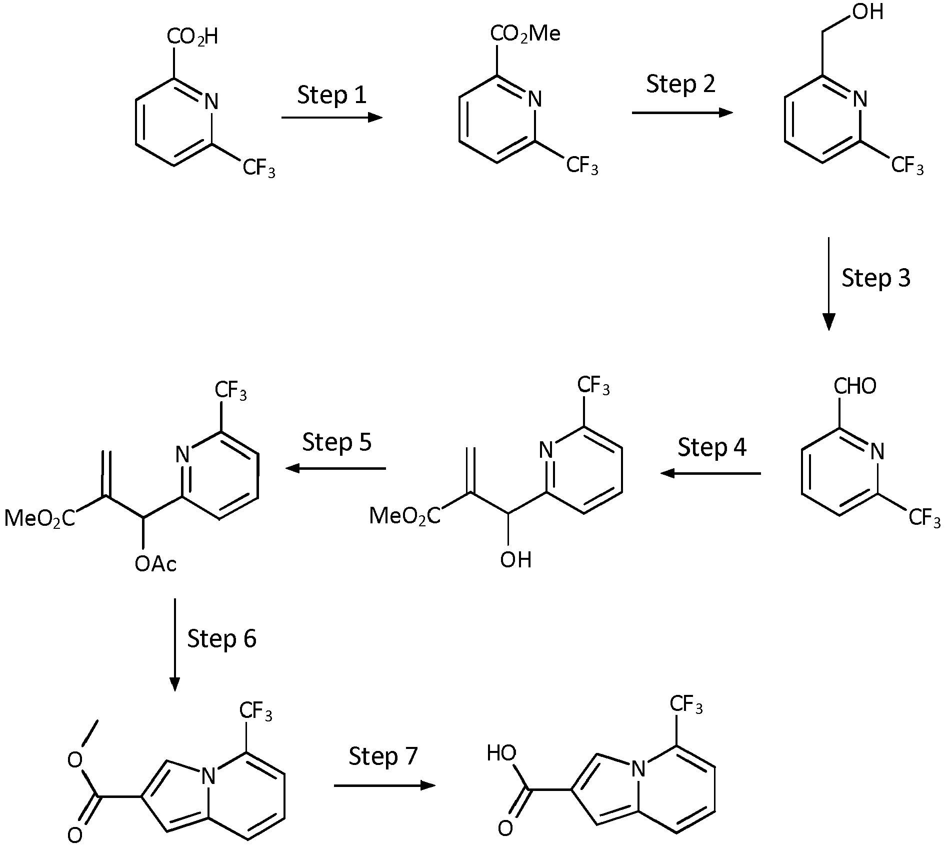

### Step 1: Methyl 6-(trifluoromethyl)pyridine-2-carboxylate

To a stirred solution of 6-(trifluoromethyl)pyridine-2-carboxylic acid (8.8 g, 46.05 mmol) in dry MeOH (150 mL) was added carefully sulfuric acid (6.77 g, 69.07 mmol, 3.76 mL). The resulting mixture was refluxed overnight. The reaction mixture was concentrated under reduced pressure and the residue partitioned between MTBE (200 mL) and sat. aq NaHCO₃ (200 mL). The organic phase was washed with brine, dried over Na₂SO₄ and concentrated under reduced pressure to give methyl 6-(trifluoromethyl)pyridine-2-carboxylate (8.0 g, 39.0 mmol, 84.7% yield) as light yellow crystalline solid.

### Step 2: [6-(trifluoromethyl)pyridin-2-yl]methanol

To a stirred solution of methyl 6-(trifluoromethyl)picolinate (8.0 g, 39.0 mmol) in dry toluene (200 mL) at r.t. was added dropwise diisobutylaluminum hydride (16.64 g, 117.0 mmol, 112.5 mL) was added dropwise. The resulting mixture was stirred at r.t. overnight. The reaction mixture was quenched with HCl (1M, 50 mL) solution, then basified with 10% aq. NaOH aq until the precipitate dissolved. The organic phase was washed with brine, dried over Na₂SO₄ and concentrated over reduced pressure to give [6-(trifluoromethyl)pyridin-2-yl]methanol (6.0 g, 96.0% purity, 32.52 mmol, 83.4% yield) as yellow liquid.

### Step 3: 6-(trifluoromethyl)pyridine-2-carbaldehyde

To a solution of (6-(trifluoromethyl)pyridin-2-yl)methanol (6.0 g, 33.87 mmol) in 100 mL of DCM was added 1,1,1-tris(acetoxy)-1,1-dihydro-1,2-benziodoxol-3(1H)-one (17.24 g, 40.65 mmol) in a few portions, maintaining temperature below 30°C (H₂O cooling bath). After reaction was complete (monitored by ¹H NMR), the mixture was poured into a stirred aqueous solution of Na₂CO₃ and Na₂S₂O₃ and stirred until the organic phase became transparent (about 15 min). The layers were separated and the aqueous layer was extracted with DCM (50 mL). The combined organic extracts were washed with brine, dried over Na₂SO₄ and concentrated under reduced pressure to give 6-(trifluoromethyl)pyridine-2-carbaldehyde (9.0 g, 33.0% purity, 16.96 mmol, 50.1% yield) as light brown liquid.

### Step 4: Methyl 2-{hydroxy[6-(trifluoromethyl)pyridin-2-yl]methyl }prop-2-enoate

To a solution of 6-(trifluoromethyl)pyridine-2-carbaldehyde (3.3 g, 18.85 mmol) and methyl prop-2-enoate (4.87 g, 56.53 mmol, 5.12 mL) in dioxane/ H₂O (1/1 v/v) (100 mL) was added 1,4-diazabicyclo[2.2.2]octane (2.11 g, 18.84 mmol). The mixture was stirred at r.t. overnight. The mixture was then diluted with H₂O (300 mL) and extracted with MTBE (3x100 mL). The combined organic extracts were washed with brine, dried over Na₂SO₄ and concentrated under reduced pressure to give methyl 2-hydroxy[6-(trifluoromethyl)pyridin-2-yl]methylprop-2-enoate (2.7 g, 10.34 mmol, 54.9% yield) as brown oil.

### Step 5: Methyl 2-[(acetyloxy)[6-(trifluoromethyl)pyridin-2-yl]methyl]prop-2-enoate

Methyl 2-hydroxy[6-(trifluoromethyl)pyridin-2-yl]methylprop-2-enoate (2.7 g, 10.34 mmol) was dissolved in acetic anhydride (26.39 g, 258.46 mmol, 24.43 mL) and heated at 100°C for 2h. The reaction mixture was concentrated under reduced pressure, the residue was triturated with 100 mL of MTBE and the resulting mixture was quenched with sat. aq NaHCO₃. The organic phase was separated, washed with brine, dried over Na₂SO₄ and concentrated under reduced pressure to give methyl 2-[(acetyloxy)[6-(trifluoromethyl)pyridin-2-yl]methyl]prop-2-enoate (3.0 g, 9.89 mmol, 95.7% yield) as brown liquid.

### Step 6: Methyl 5-(trifluoromethyl)indolizine-2-carboxylate

Methyl 2-[(acetyloxy)[6-(trifluoromethyl)pyridin-2-yl]methyl]prop-2-enoate (3.0 g, 9.89 mmol) was dissolved in xylene (70 mL) and refluxed for a week. The reaction mixture was cooled to r.t., diluted with MTBE (50 mL), quenched with NaHCO₃ aq, washed with brine, dried over Na₂SO₄ and concentrated under reduced pressure to give dark brown oil, which was purified by flash chromatography (Companion comb flash; SiO2 (120g), petroleum ether/MTBE with MTBE from 0-12%, flow rate = 85 mL/min, Rv = 7 CV) to give methyl 5-(trifluoromethyl)indolizine-2-carboxylate (67.0 mg, 275.51 µmol, 2.8% yield) as light yellow crystals.

### Step 7: 5-(trifluoromethyl)indolizine-2-carboxylic acid

To a solution of methyl 5-(trifluoromethyl)indolizine-2-carboxylate (67.0 mg, 275.51 µmol) in MeOH (4 mL) was added a solution of lithium hydroxide monohydrate (12.71 mg, 302.9 µmol) in 1 mL of H₂O. The resulting mixture was stirred at r.t. overnight. The reaction mixture was concentrated under reduced pressure and the residue was triturated with H₂O (15mL). The resulting solution was acidified with 2N HCl to pH~2 and extracted with MTBE (4x20 mL). The combined organic extracts were dried over Na₂SO₄ and concentrated in vacuo to give 5-(trifluoromethyl)indolizine-2-carboxylic acid (47.0 mg, 205.1 µmol, 74.5% yield) as pale yellow solid.
*Rt* (Method G) 1.10 mins, *m*/*z* 230 [M+H]⁺

### Synthesis of indolizine-2-carboxylic acid

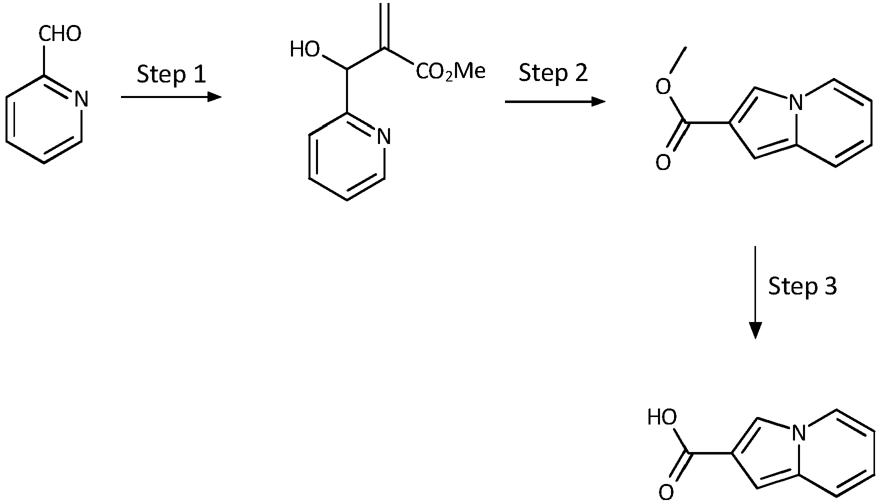

### Step 1: Methyl 2-(hydroxy(pyridin-2-yl)methyl)acrylate

Pyridine-2-carbaldehyde (0.85 g, 1 eq), methyl acrylate (3 eq) were dissolved in mixture of dioxane/ H₂O (1/1) and stirred at room temperature in the presence of DABCO (1 eq). After the reaction was completed (as monitored by TLC), the mixture was diluted with MTBE and extracted twice. The combined organic layers were washed with brine, dried over Na₂SO₄ and solvents were removed under reduced pressure. The product was purified by column chromatography to give methyl 2-(hydroxy(pyridin-2-yl)methyl)acrylate (1g, 65% yield).

### Step 2: Methyl indolizine-2-carboxylate

The reaction vessel was charged with methyl 2-(hydroxy(pyridin-2-yl)methyl)acrylate (0.74 g) and acetic anhydride. Then the reaction mixture was charged with Ar and heated under reflux for 4 hours. The cooled solution was poured onto ice with saturated NaHCO₃ aq solution and stirred for 1 hour, than resulted mixture was extracted with DCM (3×25mL). The combined organic layers were dried over Na₂SO₄ and concentrated under reduced pressure. The product was purified by HPLC to give methyl indolizine-2-carboxylate. (0.2 g, 30% yield).

### Step 3: indolizine-2-carboxylic acid

Methyl ester (0.164 g) was dissolved in MeOH/THF/ H₂O (4/4/1) and NaOH (20%aq, 1.5 eq) was added. The obtained mixture was refluxed at 80 °C for 12 hours. Than the mixture was concentrated 1/2 under reduced pressure and resulting solution was acidified to pH = 3-4 (with HCl 1N) at 0-5°C. The precipitate was filtered and dried to give indolizine-2-carboxylic acid (0.13g, 86% yield)
Rt (Method G) 0.91 mins, *m*/*z* 160 [M-H]⁻
¹H NMR (400 MHz, DMSO-d6) δ 12.28 (s, 1H), 8.26 (d, J = 7.1 Hz, 1H), 8.01 (d, J = 1.6 Hz, 1H), 7.43 (d, J = 9.1 Hz, 1H), 6.74 (dd, J = 9.1, 6.5 Hz, 1H), 6.69 (s, 1H), 6.62 (t, J = 6.7 Hz, 1H).

### Synthesis of 8-methylindolizine-2-carboxylic acid

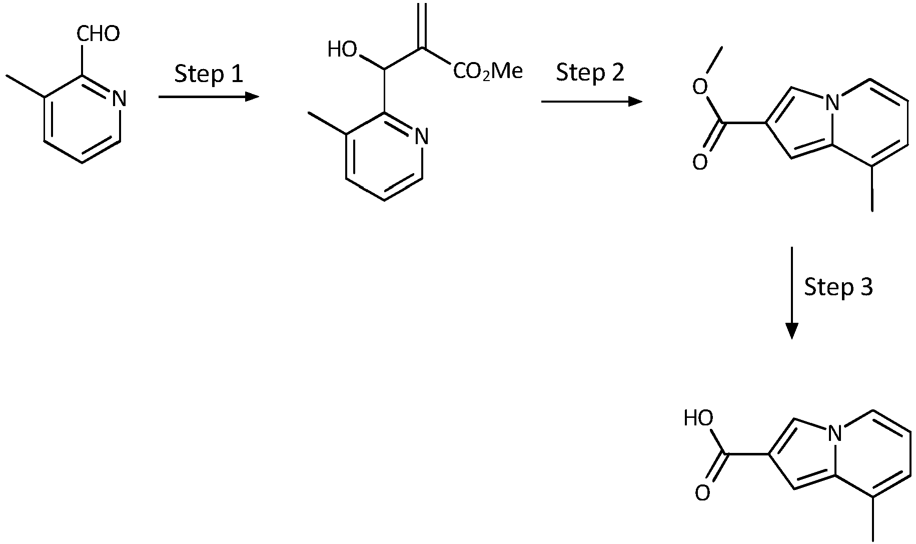

### Step 1: Methyl 2-[hydroxy(3-methylpyridin-2-yl)methyl]prop-2-enoate

Performed as described for indolizine-2-carboxylic acid, starting from 3-methylpyridine-2-carbaldehyde (58% yield).

### Step 2: methyl 2-[(acetyloxy)(3-methylpyridin-2-yl)methyl]prop-2-enoate

Performed as described for indolizine-2-carboxylic acid (42% yield)

### Step 3: 8-methylindolizine-2-carboxylic acid

Performed as described for indolizine-2-carboxylic acid (83% yield)
*Rt* (Method G) 1.11 mins, *m*/*z* 174 [M-H]⁻
¹H NMR (400 MHz, DMSO-d6) δ 12.27 (s, 1H), 8.21 - 8.10 (m, 1H), 8.01 (s, 1H), 6.69 (s, 1H), 6.63 - 6.49 (m, 2H), 2.33 (s, 3H).

### Synthesis of 1-nitro-lindolizine-2-carboxylic acid

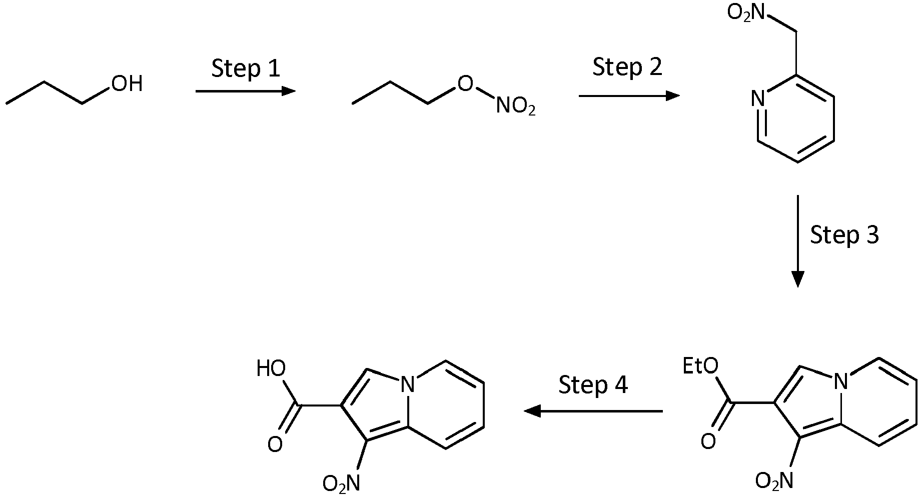

### Step 1: propyl nitrate

Nitric acid (22.15 g, 351.46 mmol, 14.67 mL) (2.2 eq) was slowly added (over 2-3 min) to cooled (ice bath) sulfuric acid (34.47 g, 351.45 mmol, 19.15 mL) (2.2eq). The resulting mixture was stirred for 10 min. Propan-1-ol (9.6 g, 159.75 mmol) (1eq) in CH₂Cl₂ (150mL) was then added dropwise to the reaction mixture (at 0° C). The temperature of the reaction mixture was kept below 5° C. After stirring for 18h at RT, the reaction mixture was diluted with ice-water (200mL) and CH₂Cl₂ (300 mL) was added for extraction. The organic layer was separated, washed with H₂O (2 × 50 mL), dried over Na₂SO₄ and concentrated under reduced pressure (cold water bath -10° C) to give propyl nitrate (13.0 g, 98.0% purity, 121.23 mmol, 75.9% yield) which was used in the next step without additional purification.

### Step 2: 2-(nitromethyl)pyridine

To a cooled (-40° C) solution of LDA (2.1 M in THF, 41 mL, 1.7eq) in dry THF (250 mL) was added 2-methylpyridine (4.72 g, 50.63 mmol, 5.0 mL). After stirring for 5 min, propyl nitrate (15.96 g, 151.89 mmol) (2.3 eq) in 50 mL of THF was added as rapidly as possible, while the temperature was kept below -40° C. The mixture was stirred at -40° C for 1 h and then at room temperature for 4 h. Then the reaction mixture was concentrated in the cooled bath (25-30° C) and Et₂O was added to the residue obtained. Filtration of the precipitate gave the crude lithium nitronate. This was taken up in water (50 mL) and the resulting solution was acidified with glacial acetic acid (10 mL) at room temperature. The solution was extracted with chloroform, dried over Na₂SO₄, then the extract was concentrated in vacuo. The crude product was purified by column chromatography to give 2-(nitromethyl)pyridine (700.0 mg, 95.0% purity, 4.81 mmol, 9.5% yield).

### Step 3: ethyl 1-nitroindolizine-2-carboxylate

To the stirred solution of 2-(nitromethyl)pyridine (220.57 mg, 1.6 mmol) in acetone (5 mL) was added ethyl 3-bromo-2-oxopropanoate (155.7 mg, 0.8 mmol). The resulting mixture was refluxed for 2h. The acetone was evaporated and the residue was partitioned between H₂O (20 mL) and CHCl₃ (50 mL). The organic layer was dried over Na₂SO₄ and evaporated to give ethyl 1-nitroindolizine-2-carboxylate (150.0 mg, 91.0% purity, 608.43 µmol, 76.2% yield).

### Step 4: 1-nitroindolizine-2-carboxylic acid

To a stirred solution of ethyl-1-nitroindolizine-2-carboxylate (220.0 mg, 939.34 µmol) in MeOH/THF/H₂O (4/4/1) (18 mL) was added lithium hydroxide monohydrate (110.84 mg, 2.64 mmol). The resulting mixture was stirred for 18h at 50° C. The solvent was removed under reduced pressure. The remaining solution was cooled to 0~5 °C and adjusted to pH 3-4 with NaHSO₄ (aq). The resulting suspension was stirred for 30 minutes and product was collected by filtration. The filter cake was dried under reduced pressure to afford 1-nitroindolizine-2-carboxylic acid (50.0 mg, 98.0% purity, 237.69 µmol, 29.7% yield) as yellow solid.

1H NMR (400 MHz, DMSO) δ 13.17 (br.s, 1H), 8.61 (d, J = 6.9 Hz, 1H), 8.20 (d, J = 9.2 Hz, 1H), 8.04 (s, 1H), 7.61 (dd, J = 9.2, 6.9 Hz, 1H), 7.19 (t, J = 6.9 Hz, 1H).

The following examples illustrate the preparation and properties of some specific compounds of the invention.

### Example 1

### N-(3-bromophenyl)-2-[4-(1H-indole-2-carbonyl)piperazin-1-yl]-2-oxoacetamide

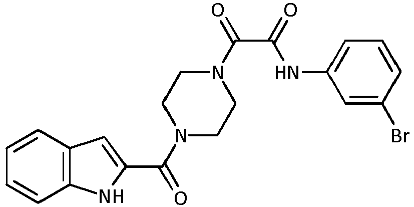

### Example 5

### 2-[4-(7-bromo-1H-indole-2-carbonyl)piperazin-1-yl]-N-butyl-2-oxoacetamide

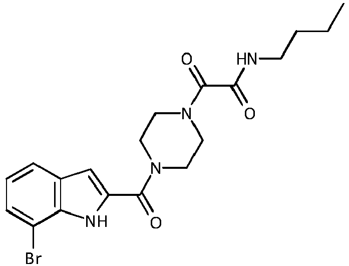

### Example 6

### N-butyl-2-[4-(1H-indole-2-carbonyl)piperazin-1-yl]-2-oxoacetamide

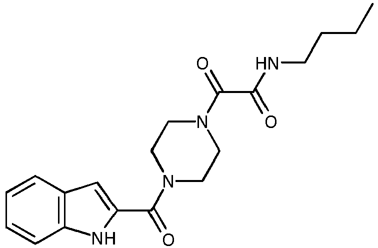

### Example 7

### 2-[4-(7-bromo-1H-indole-2-carbonyl)piperazin-1-yl]-N- cyclopentyl-2-oxoacetamide

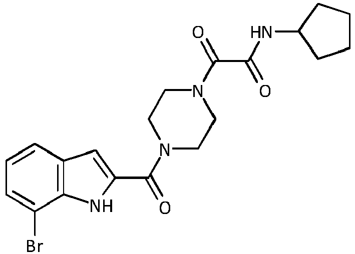

### Synthesis method 1

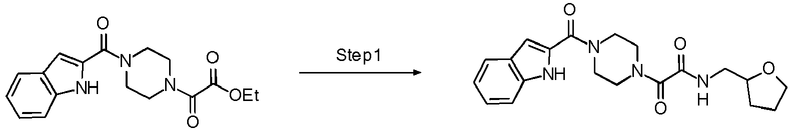

Step 1: To a solution of ethyl 2-(4-(1H-indole-2-carbonyl)piperazin-1-yl)-2-oxoacetate (50 mg, 0.15 mmol) in EtOH (1 mL) was added (tetrahydrofuran-3-yl)methanamine (38 uL, 0.37 mmol). The mixture was warmed up to 80°C for 15h. Then, the reaction mixture was evaporated and purified by column chromatography to give the desired product (50.6 mg, 88 % yield).

### The following examples were prepared following synthesis method 1.

### Example 4

### 2-(4-(1H-indole-2-carbonyl)piperazin-1-yl)-2-oxo-N-((tetrahydrofuran-3-yl)methyl)acetamide

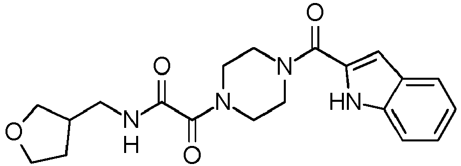

¹H NMR (500 MHz, Chloroform-d) δ 9.33 (s, 1H), 7.66 (m, 1H), 7.44 (m, 1H), 7.31 (m, 1H), 7.16 (m, 1H), 6.80 (m, 1H), 4.36 - 4.29 (m, 2H), 4.03 (s, 4H), 3.94 - 3.87 (m, 1H), 3.85-3.72 (m, 4H), 3.56 (m, 1H), 3.37 - 3.30 (m, 2H), 2.52 (m, 1H), 2.07 (m, 1H), 1.68 - 1.58 (m, 1H).

GC analysis: retention time = 18.054 min, peak area: 99%, Method L; mass *(m*/*z):* 384.1.

### Example 10

### 2-(4-(1H-indole-2-carbonyl)piperazin-1-yl)-N-(furan-3-ylmethyl)-2-oxoacetamide

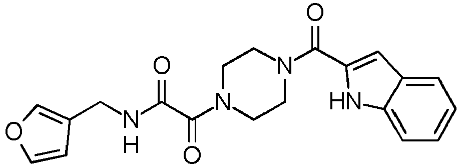

¹H NMR (500 MHz, Chloroform-d) δ 9.19 (s, 1H), 7.69 - 7.62 (m, 2H), 7.43 (m, 1H), 7.38 (m, 1H), 7.31 (ddd, J = 8.3, 7.0, 1.1 Hz, 1H), 7.16 (ddd, J = 8.1, 7.0, 1.0 Hz, 1H), 6.79 (dd, J = 2.2, 0.9 Hz, 1H), 6.34 (m, J = 3.2, 1.9 Hz, 1H), 6.28 (m, 1H), 4.49 (d, J = 5.9 Hz, 2H), 4.37-4.30 (m, 2H), 4.02 (s, 4H), 3.82 - 3.74 (m, 2H).

GC analysis: retention time = 16.553 min, peak area: 99%, Method L; mass *(m*/*z):* 380.1.

### Example 11

### 2-(4-(1H-indole-2-carbonyl)piperazin-1-yl)-N-hexyl-2-oxoacetamide

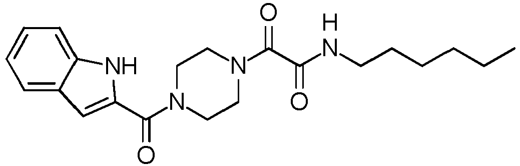

¹H NMR (400 MHz, Chloroform-d) δ 9.70 (s, 1H), 7.65 (dt, J = 8.1, 1.0 Hz, 1H), 7.48 - 7.40 (m, 2H), 7.29 (ddd, J = 8.3, 7.0, 1.2 Hz, 1H), 7.14 (ddd, J = 8.0, 7.0, 1.0 Hz, 1H), 6.78 (dd, J = 2.2, 0.9 Hz, 1H), 4.35 - 4.27 (m, 2H), 4.02 (s, 4H), 3.82 - 3.74 (m, 2H), 3.30 (m, 2H), 1.60 - 1.48 (m, 2H), 1.30 (m, 6H), 0.92 - 0.86 (m, 3H).

GC analysis: retention time = 16.335 min, peak area: 97%, Method L; mass *(m*/*z):* 384.2.

### Synthesis method 2

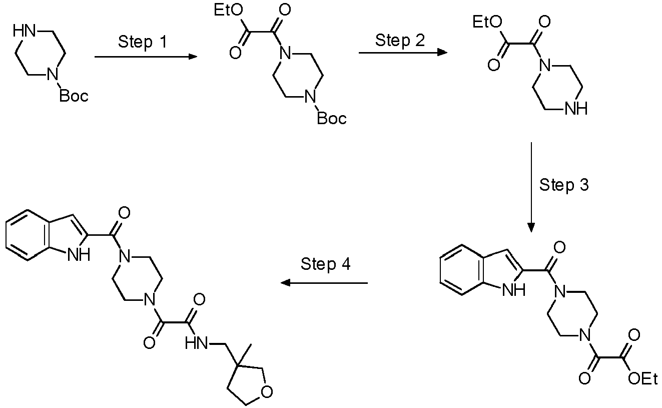

**Step 1:** To a solution of *tert*-butyl piperazine-1-carboxylate (4.0 g, 21.48 mmol) in CH₂Cl₂ (45 ml), NEt₃ (4.49 ml, 32.2 mmol) was added at room temperature. Then, ethyl 2-chloro-2-oxoacetate (2.64 ml, 23.62 mmol) in CH₂Cl₂ (72 ml) was added at 0 °C and the resulting mixture was stirred at room temperature overnight. The solvent was removed under reduced pressure and the crude mixture was diluted in EtOAc. Water was added and it was extracted with EtOAc (x3). The combined organic phases were washed with saturated NH₄Cl solution, saturated NaHCO₃ solution, brine, dried over Na₂SO₄ and solvent was removed under reduced pressure to afford *tert*-butyl 4-(2-ethoxy-2-oxoacetyl)piperazine-1-carboxylate (5.9 g, 96% yield).

**Step 2:** To a solution of *tert*-butyl 4-(1*H*-indole-2-carbonyl)piperazine-1-carboxylate (2.95 g, 10.3 mmol) in CH₂Cl₂ (47 mL) was slowly added trifluoroacetic acid (15.78 mL, 206 mmol). After stirring for 2h, the solvent was removed under reduced pressure. The crude mixture concentrated in vacuo to provide (1*H*-indol-2-yl)(piperazin-1-yl)methanone (1.8 g, 94% yield).

**Step 3:** To a solution of 1*H*-indole-2-carboxylic acid (1.7 g, 10.55 mmol) in dry THF (65 mL) was added CDI) (1.42 g, 8.76 mmol). The mixture stirred under an inert atmosphere for 1h at 50 °C. Then, *tert*-Butyl piperazine-1-carboxylate (1.8 g, 9.70 mmol) was added and the resulting mixture stirred overnight at 50°C under inert atmosphere. The solvent was removed under reduced pressure and diluted in EtOAc and saturated NaHCO₃ solution. The aqueous layer was extracted with EtOAc (x3). The combined organic phases were washed with water, brine, dried over Na₂SO₄ and solvent was removed under reduced pressure. Then, crystallization was performed diluting the crude with the minimum amount of EtOH and adding water to get the precipitation of the product as white solid, *tert*-butyl 4-(1*H*-indole-2-carbonyl)piperazine-1-carboxylate (2.70 g, 73% yield).

**Step 4:** A sealed tube was charged with ethyl 2-(4-(1*H*-indole-2-carbonyl)piperazin-1-yl)-2-oxoacetate (50 mg, 0.152 mmol) and (3-methyltetrahydrofuran-3-yl)methanamine (26.2 mg, 0.228 mmol) in EtOH (0.5 mL), and the mixture was stirred overnight at 80°C. Then, the solvent was removed under reduced pressure and the resulting residue was purified by flash column chromatography on silica gel (0 to 10% MeOH in CH₂Cl₂, gradient) to afford 2-(4-(1*H*-indole-2-carbonyl)piperazin-1-yl)-*N*-((3-methyltetrahydrofuran-3-yl)methyl)-2-oxoacetamide (40 mg, 66% yield), as a solid (92% purity, based on HPLC).

^{∗}In case of using the corresponding amine hydrochloride, additional base is required and it is specified in every case.

### The following examples were prepared following synthesis method 2

### Example 12

### 2-[4-(1H-indole-2-carbonyl)piperazin-1-yl]-N-[(3-methyloxolan-3-yl)methyl]-2-oxoacetamide

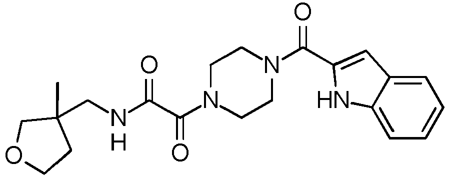

### Rt (Method K) 4.73 mins, m/z 399 [M+H]+

¹H NMR (400 MHz, CDCl₃) δ 9.18 (br s, 1H), 7.67 (dq, J = 8.1, 1.0 Hz, 1H), 7.57 (s, 1H), 7.44 (dq, J = 8.3, 0.9 Hz, 1H), 7.31 (ddd, J = 8.3, 7.0, 1.1 Hz, 1H), 7.16 (ddd, J = 8.0, 7.0, 1.0 Hz, 1H), 6.80 (dd, J = 2.1, 0.9 Hz, 1H), 4.36 - 4.29 (m, 2H), 4.03 (br s, 4H), 3.95 (td, J = 8.5, 5.9 Hz, 1H), 3.87 (td, J = 8.5, 6.6 Hz, 1H), 3.82 - 3.76 (m, 2H), 3.65 (d, J = 8.7 Hz, 1H), 3.42 (d, J = 8.7 Hz, 1H), 3.33 (dd, J = 6.5, 1.4 Hz, 2H), 1.84 (ddd, J = 12.5, 8.3, 6.6 Hz, 1H), 1.70 (ddd, J= 12.5, 8.3, 5.9 Hz, 1H), 1.15 (s, 3H).

### Example 13

### 2-(4-(1H-indole-2-carbonyl)piperazin-1-yl)-N-((1-methoxycyclopropyl)methyl)-2-oxoacetamide

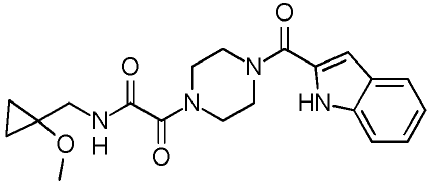

From ethyl 2-(4-(1*H*-indole-2-carbonyl)piperazin-1-yl)-2-oxoacetate (50 mg, 0.152 mmol), (1-methoxycyclopropyl)methanamine hydrochloride (31.3 mg, 0.228 mmol) and triethylamine (32 µL, 0.228 mmol), following method 2 described above, 2-(4-(1*H*-indole-2-carbonyl)piperazin-1-yl)-*N*-((1-methoxycyclopropyl)methyl)-2-oxoacetamide (24.8 mg, 43% yield) was obtained as a solid (93% purity, based on HPLC).
Rt (Method K) 4.79 mins, *m*/*z* 385 [M+H]+

¹H NMR (500 MHz, CDCl₃) δ 9.24 (br s, 1H), 7.66 (dd, J = 8.1, 1.0 Hz, 1H), 7.64 - 7.54 (m, 1H), 7.44 (dd, J = 8.3, 1.0 Hz, 1H), 7.31 (ddd, J = 8.3, 7.0, 1.2 Hz, 1H), 7.16 (ddd, J = 8.0, 7.0, 1.0 Hz, 1H), 6.80 (dd, J = 2.1, 0.9 Hz, 1H), 4.32 - 4.24 (m, 2H), 4.02 (br s, 4H), 3.86-3.74 (m, 2H), 3.46 (d, J = 5.7 Hz, 2H), 3.31 (s, 3H), 0.90 - 0.83 (m, 2H), 0.64 - 0.56 (m, 2H).

### Example 14

### 2-[4-(1H-indole-2-carbonyl)piperazin-1-yl]-2-oxo-N-[(5-oxopyrrolidin-2-yl)methyl]acetamide

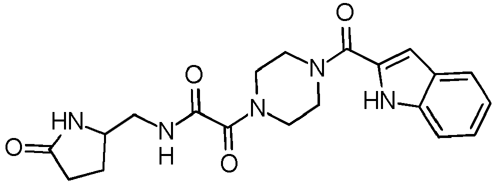

From ethyl 2-(4-(1*H*-indole-2-carbonyl)piperazin-1-yl)-2-oxoacetate (50 mg, 0.152 mmol) and 5-(aminomethyl)pyrrolidin-2-one (26 mg, 0.228 mmol), following method 2 described above, 2-(4-(1*H*-indole-2-carbonyl)piperazin-1-yl)-*N*-((1-methoxycyclopropyl)methyl)-2-oxoacetamide (35.8 mg, 59% yield) was obtained as a solid (96% purity, based on HPLC).
Rt (Method K) 4.28 mins, *m*/*z* 398 [M+H]+
¹H NMR (500 MHz, CDCl₃) δ 8.83 (t, J = 5.9 Hz, 1H), 7.68 - 7.51 (m, 2H), 7.43 (dq, J = 8.3, 0.9 Hz, 1H), 7.19 (ddd, J = 8.2, 6.9, 1.2 Hz, 1H), 7.05 (ddd, J = 8.0, 6.9, 1.0 Hz, 1H), 6.86 (dd, J = 2.3, 0.9 Hz, 1H), 3.90 - 3.72 (m, 4H), 3.68 - 3.52 (m, 5H), 3.26 - 3.10 (m, 2H), 2.24 - 1.99 (m, 3H), 1.80 - 1.61 (m, 1H).

### Example 15

### 2-(4-(1H-indole-2-carbonyl)piperazin-1-yl)-2-oxo-N (prop-2-yn-1-yl)acetamide

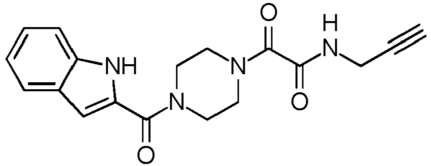

From ethyl 2-(4-(1*H*-indole-2-carbonyl)piperazin-1-yl)-2-oxoacetate (50 mg, 0.152 mmol) and prop-2-yn-1-amine (12.54 mg, 0.228 mmol), following method 2 described above, 2-(4-(1*H*-indole-2-carbonyl)piperazin-1-yl)-2-oxo-*N*-(prop-2-yn-1-yl)acetamide (15.2 mg, 30% yield) was obtained as a solid (96% purity, based on HPLC).

### Rt (Method K) 4.79 mins, m/z 339 [M+H]+

¹H NMR (500 MHz, CDCl₃) δ 9.19 (br s, 1H), 7.68 - 7.64 (m, 1H), 7.60 - 7.52 (m, 1H), 7.47 - 7.41 (m, 1H), 7.31 (ddd, J = 8.2, 7.0, 1.1 Hz, 1H), 7.16 (ddd, J = 8.0, 7.0, 1.0 Hz, 1H), 6.80 (dd, J = 2.2, 0.9 Hz, 1H), 4.35 - 4.30 (m, 2H), 4.10 (dd, J = 5.6, 2.6 Hz, 2H), 4.02 (br s, 4H), 3.84 - 3.76 (m, 2H), 2.28 (t, J = 2.6 Hz, 1H).

### Example 16

### 2-(4-(1H-indole-2-carbonyl)piperazin-1-yl)-N-((1s,4s)-4-hydroxycyclohexyl)-2-oxoacetamide

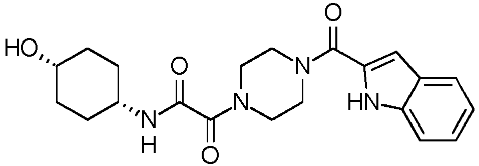

From ethyl 2-(4-(1*H*-indole-2-carbonyl)piperazin-1-yl)-2-oxoacetate (40 mg, 0.121 mmol), (1*s*,4*s*)-4-aminocyclohexan-1-ol hydrochloride (27.6 mg, 0.182 mmol) and *N*-ethyl-*N-*isopropylpropan-2-amine (32 µL, 0.182 mmol), following method 2 described above 2-(4-(1*H*-indole-2-carbonyl)piperazin-*1*-yl)-*N*-((1*s*,4*s*)-4-hydroxycyclohexyl)-2-oxoacetamide (3.6 mg, 7% yield) was obtained as a solid (91% purity, based on HPLC).

### Rt (Method K) 4.42 mins, m/z 399 [M+H]+

¹H NMR (300 MHz, CDCl₃) δ 9.11 (br s, 1H), 7.69 (d, J = 8.1 Hz, 1H), 7.46 (d, J = 8.3 Hz, 1H), 7.41 - 7.28 (m, 2H), 7.18 (t, J = 7.5 Hz, 1H), 6.83 - 6.80 (m, 1H), 4.45 - 4.28 (m, 2H), 4.14 - 3.92 (m, 5H), 3.92 - 3.71 (m, 3H), 1.97 - 1.65 (m, 8H).

### Example 17

### 2-(4-(1H-indole-2-carbonyl)piperazin-1-yl)-N-((1r,3r)-3-hydroxycyclobutyl)-2-oxoacetamide

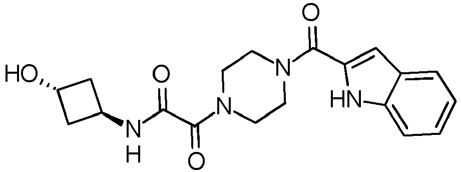

From ethyl 2-(4-(1*H*-indole-2-carbonyl)piperazin-1-yl)-2-oxoacetate (100 mg, 0.304 mmol), (1*r*,3*r*)-3-aminocyclobutan-1-ol hydrochloride (56 mg, 0.455 mmol) and *N*-ethyl-*N-*isopropylpropan-2-amine (79 µL, 0.455 mmol), following method 2 described above 2-(4-(1*H*-indole-2-carbonyl)piperazin-1-yl)-*N*-((1*r*,3*r*)-3-hydroxycyclobutyl)-2-oxoacetamide (30 mg, 27% yield) was obtained as a solid (98% purity, based on HPLC).

### Rt (Method K) 4.20 mins, m/z 371 [M+H]+

¹H NMR (400 MHz, CD₃OD) δ 7.62 (dd, J = 8.0, 1.1 Hz, 1H), 7.44 (dd, J = 8.3, 0.9 Hz, 1H), 7.23 (ddd, J = 8.3, 7.0, 1.2 Hz, 1H), 7.11 - 7.00 (m, 1H), 6.87 (d, J = 0.9 Hz, 1H), 4.47 - 4.36 (m, 2H), 3.94 (br s, 4H), 3.77 - 3.68 (m, 4H), 2.37 - 2.28 (m, 4H).

### Example 18

### 2-(4-(1H-indole-2-carbonyl)piperazin-1-yl)-N-((1-hydroxycyclopropyl)methyl)-2-oxoacetamide

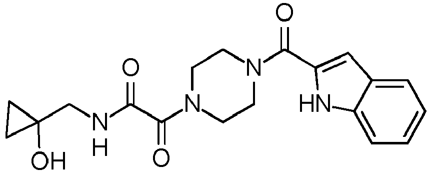

From ethyl 2-(4-(1*H*-indole-2-carbonyl)piperazin-1-yl)-2-oxoacetate (100 mg, 0.304 mmol), 1-(aminomethyl)cyclopropan-1-ol hydrochloride (56.3 mg, 0.455 mmol) and *N*-ethyl-*N-*isopropylpropan-2-amine (79 µL, 0.455 mmol), following method 2 described above 2-(4-(1*H*-indole-2-carbonyl)piperazin-1-yl)-*N*-((1-hydroxycyclopropyl)methyl)-2-oxoacetamide (67.7 mg, 70% yield) was obtained as a solid (100% purity, based on HPLC).
Rt (Method K) 4.42 mins, *m*/*z* 371 [M+H]+
¹H NMR (500 MHz, CDCl₃) δ 9.17 (br s, 1H), 7.75 (br s, 1H), 7.67 (dd, J = 8.1, 1.0 Hz, 1H), 7.44 (dd, J = 8.3, 1.0 Hz, 1H), 7.34 - 7.28 (m, 1H), 7.16 (ddd, J = 8.0, 7.0, 1.0 Hz, 1H), 6.80 (dd, J = 2.2, 0.9 Hz, 1H), 4.31 (dd, J = 6.5, 4.1 Hz, 2H), 4.03 (br s, 4H), 3.80 (dd, J = 6.6, 4.2 Hz, 2H), 3.46 (d, J = 6.0 Hz, 2H), 0.90 - 0.83 (m, 2H), 0.68 - 0.59 (m, 2H).

### Example 19

### 2-(4-(1H-indole-2-carbonyl)piperazin-1-yl)-2-oxo-N-((2-oxo-1-azaspiro[3.3]heptan-6-yl)methyl)acetamide

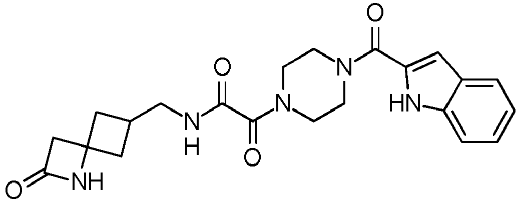

From ethyl 2-(4-(1*H*-indole-2-carbonyl)piperazin-1-yl)-2-oxoacetate (40 mg, 0.121 mmol) and 6-(aminomethyl)-1-azaspiro[3.3]heptan-2-one (25.5 mg, 0.182 mmol), following method 2 described above 2-(4-(1*H*-indole-2-carbonyl)piperazin-1-yl)-2-oxo-*N*-((2-oxo-1-azaspiro[3.3]heptan-6-yl)methyl)acetamide (31.5 mg, 61% yield) was obtained as a solid (99% purity, based on HPLC).
Rt (Method K) 4.42 mins, *m*/*z* 424 [M+H]+
¹H NMR (500 MHz, CDCl₃) δ 9.20 (br s, 1H), 7.66 (dd, J = 8.1, 1.0 Hz, 1H), 7.49 - 7.42 (m, 2H), 7.31 (ddd, J = 8.3, 7.0, 1.1 Hz, 1H), 7.16 (ddd, J = 8.0, 7.0, 0.9 Hz, 1H), 6.80 (dd, J = 2.2, 0.9 Hz, 1H), 6.04 (br s, 1H), 4.36 - 4.32 (m, 2H), 4.03 (br s, 4H), 3.81 - 3.77 (m, 2H), 3.40 (dd, J = 7.7, 6.3 Hz, 2H), 3.03 (d, J = 1.7 Hz, 2H), 2.58 - 2.48 (m, 2H), 2.48 - 2.36 (m, 1H), 2.23 - 2.15 (m, 2H).

### Example 20

### 1-((2-(4-(1H-indole-2-carbonyl)piperazin-1-yl)-2-oxoacetamido)methyl)cyclobutane-1-carboxamide

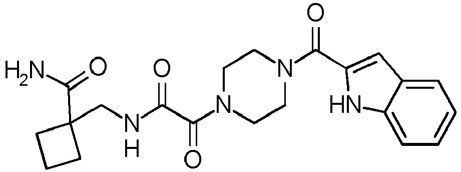

From ethyl 2-(4-(1*H*-indole-2-carbonyl)piperazin-1-yl)-2-oxoacetate (60 mg, 0.182 mmol), 1-(aminomethyl)cyclobutane-1-carboxamide hydrochloride (45 mg, 0.273 mmol) and *N*-ethyl-N isopropylpropan-2-amine (48 µL, 0.273 mmol), following method 2 described above 1-((2-(4-(1*H*-indole-2-carbonyl)piperazin-1-yl)-2-oxoacetamido)methyl)cyclobutane-1-carboxamide (22.9 mg, 31% yield) was obtained as a solid (95% purity, based on HPLC).
Rt (Method K) 4.41 mins, *m*/*z* 412 [M+H]+
¹H NMR (500 MHz, DMSO-d6) δ 11.60 (s, 1H), 8.62 (t, J = 6.1 Hz, 1H), 7.61 (d, J = 8.0 Hz, 1H), 7.43 (dd, J = 8.2, 1.0 Hz, 1H), 7.23 - 7.15 (m, 2H), 7.05 (ddd, J = 8.0, 6.9, 1.0 Hz, 1H), 6.95 (s, 1H), 6.85 (d, J = 1.2 Hz, 1H), 3.80 (br s, 4H), 3.61 - 3.54 (m, 4H), 3.51 (d, J = 6.2 Hz, 2H), 2.22 (ddd, J = 11.8, 9.3, 7.0 Hz, 2H), 1.97 - 1.64 (m, 4H).

### Example 21

### 1-((2-(4-(1H-indole-2-carbonyl)piperazin-1-yl)-2-oxoacetamido)methyl)-3,3-difluorocyclobutane-1-carboxamide

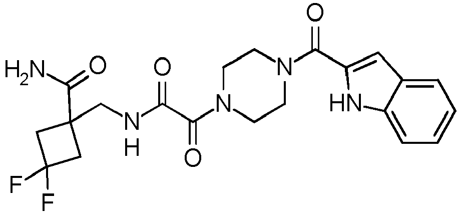

From ethyl 2-(4-(1*H*-indole-2-carbonyl)piperazin-1-yl)-2-oxoacetate (30 mg, 0.091 mmol), 1-(aminomethyl)-3,3-difluorocyclobutane-1-carboxamide hydrochloride (27.4 mg, 0.137 mmol) and *N*-ethyl-*N*-isopropylpropan-2-amine (24 µL, 0.137 mmol), following method 2 described above 1-((2-(4-(1*H*-indole-2-carbonyl)piperazin-1-yl)-2-oxoacetamido)methyl)-3,3-difluorocyclobutane-1-carboxamide (15.3 mg, 38% yield) was obtained as a solid (93% purity, based on HPLC).
Rt (Method K) 4.68 mins, *m*/*z* 448 [M+H]+
¹H NMR (500 MHz, DMSO-d6) δ 11.61 (s, 1H), 8.87 (t, J = 6.2 Hz, 1H), 7.58 (dd, J = 8.0, 1.0 Hz, 1H), 7.46 (s, 1H), 7.43 - 7.37 (m, 1H), 7.22 - 7.13 (m, 2H), 7.02 (ddd, J = 8.0, 7.0, 1.0 Hz, 1H), 6.82 (dd, J = 2.2, 0.9 Hz, 1H), 3.76 (br s, 4H), 3.56 (d, J = 5.9 Hz, 4H), 3.53-3.48 (m, 2H), 2.82 (q, J = 13.5 Hz, 2H), 2.63 (q, J = 12.8 Hz, 2H).

### Example 22

### 1-((2-(4-(1H-indole-2-carbonyl)piperazin-1-yl)-2-oxoacetamido)methyl)-N-methylcyclobutane-1-carboxamide

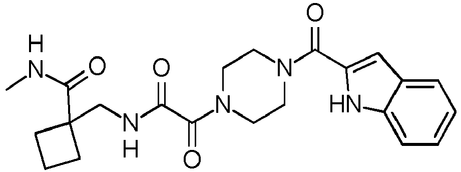

From ethyl 2-(4-(1*H*-indole-2-carbonyl)piperazin-1-yl)-2-oxoacetate (100 mg, 0.304 mmol), 1-(aminomethyl)-3,3-difluorocyclobutane-1-carboxamide hydrochloride (81 mg, 0.455 mmol) and *N*-ethyl-*N*-isopropylpropan-2-amine (79 µL, 0.455 mmol), following method 2 described above 1-((2-(4-(1H-indole-2-carbonyl)piperazin-1-yl)-2-oxoacetamido)methyl)-*N-*methylcyclobutane-1-carboxamide (41.9 mg, 32% yield) was obtained as a solid (100% purity, based on HPLC).
Rt (Method K) 4.52 mins, *m*/*z* 426 [M+H]+
¹H NMR (500 MHz, CDCl₃) δ 9.43 (br s, 1H), 7.72 (t, J = 6.2 Hz, 1H), 7.66 (dd, J = 8.0, 1.1 Hz, 1H), 7.44 (dd, J = 8.3, 1.0 Hz, 1H), 7.30 (ddd, J = 8.2, 6.9, 1.1 Hz, 1H), 7.15 (ddd, J = 8.0, 6.9, 1.0 Hz, 1H), 6.79 - 6.76 (m, 1H), 5.84 (br s, 1H), 4.22 - 4.15 (m, 2H), 4.00 (br s, 4H), 3.78 - 3.73 (m, 2H), 3.67 (d, J = 6.2 Hz, 2H), 2.83 (d, J = 4.8 Hz, 3H), 2.38 - 2.26 (m, 2H), 2.13 - 1.86 (m, 4H).

### Synthesis method 3

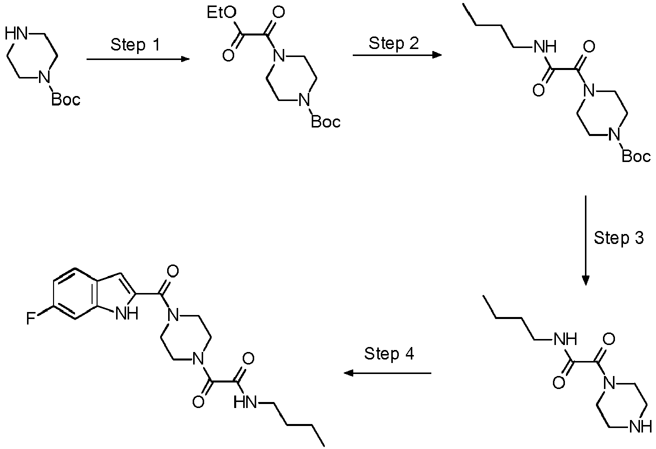

**Step 1:** To a cooled (0°C), stirred solution of tert-butyl piperazine-1-carboxylate (2 g, 10.74 mmol) in MeCN (20 mL) was added a mixture of triethylamine (2.24 mL, 16 mmol) and ethyl 2-chloro-2-oxoacetate (1.32 mL, 12 mmol) in MeCN (33 mL). The mixture was warmed to room temperature and stirred overnight. The reaction mixture was concentrated, mixed with water (15 mL), and extracted with EtOAc (3 × 15 mL). The combined organic extracts were washed with sat. aq. NaHCO₃, then washed with sat. aq. NaCl (15 mL), dried over MgSO₄, filtered and concentrated. The desired product was obtained as an orange/brown oil that crystallized spontaneously upon standing (2.75 g, 89% yield).

**Step 2:** To a solution of tert-butyl 4-(2-ethoxy-2-oxoacetyl)piperazine-1-carboxylate (746 mg, 2.61 mmol) in EtOH (3 mL) in a sealed tube, was added butan-1-amine (2.58 mL, 26 mmol). The mixture was heated at 80°C for 15h. Excess ethanol and excess of butan-1-amine were removed by evaporation to give the desired product as a colourless oil (703 mg, 86 % yield).

**Step 3:** Trifluoroacetic acid was added to a solution of tert-butyl 4-(2-ethoxy-2-oxoacetyl)piperazine-1-carboxylate (817 mg, 2.85 mmol) in DCM and stirred at room temperature until completion (TLC) (DCM:MeOH:TEA/90:10:1). The reaction was quenched by slowly addition of ice. The mixture was concentrated and purified by alumina column chromatography to give the desired product (382 mg, 63% yield)

**Step 4:** To a solution of 6-fluoro-1H-indole-2-carboxylic acid (86.6mg, 0.48 mmol) in dry Me-THF (1 mL) was added a solution of CDI (86 mg, 0.53 mmol) in Me-THF (1 mL) the resulting mixture was stirred at 50°C for 1h. N-butyl-2-oxo-2-(piperazin-1-yl)acetamide (119 mg, 0.56 mmol) in Me-THF (1 mL) was added and the mixture was stirred at 80 °C for 12h. The crude mixture was concentrated and purified by flash column chromatography to give N-butyl-2-[4-(6-fluoro-1H-indole-2-carbonyl)piperazin-1-yl]-2-oxoacetamide (120 mg 66 % yield)

### The following examples were prepared following synthesis method 3.

### Example 23

### N-butyl-2-(4-(6-fluoro-1H-indole-2-carbonyl)piperazin-1-yl)-2-oxoacetamide

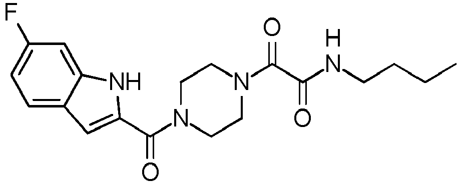

GC analysis: retention time = 15.165 min, peak area: 100%, Method L; mass *(m*/*z):* 374.1.

¹H NMR (400 MHz, Chloroform-d) δ 9.21 (s, 1H), 7.42 - 7.32 (m, 2H), 7.29 (m, J = 9.2, 2.5, 0.7 Hz, 1H), 7.07 (td, J = 9.1, 2.5 Hz, 1H), 6.75 (dd, J = 2.2, 0.9 Hz, 1H), 4.40 - 4.25 (m, 2H), 4.01 (s, 4H), 3.84 - 3.74 (m, 2H), 3.31 (td, J = 7.1, 6.1 Hz, 2H), 1.56 (m, 2H), 1.43 - 1.32 (m, 2H), 0.95 (t, J = 7.3 Hz, 3H).

### Example 24

### N-butyl-2-(4-(7-methyl-1H-indole-2-carbonyl)piperazin-1-yl)-2-oxoacetamide

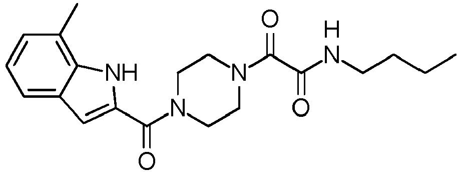

GC analysis: retention time = 15.567 min, peak area: 100%, Method L; mass *(m*/*z):* 370.3.

¹H NMR (500 MHz, Chloroform-d) δ 9.11 (s, 1H), 7.28 (m, 1H), 7.21 (m, 1H), 6.95 (m, 1H), 6.80 (dd, 1H), 4.37 - 4.32 (m, 2H), 4.03 (s, 4H), 3.82 - 3.78 (m, 2H), 3.31 (m, 2H), 2.56 (s, 3H), 1.57 - 1.52 (m, 2H), 1.38 (m, 2H), 0.95 (t, J = 7.3 Hz, 3H). ).

### Example 9

### N-butyl-2-(4-(4,6-difluoro-1H-indole-2-carbonyl)piperazin-1-yl)-2-oxoacetamide

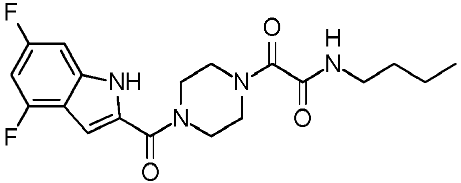

GC analysis: retention time = 14.772 min, peak area: 100%, Method L; mass *(m*/*z):* 392.1.

¹H NMR (500 MHz, Chloroform-d) δ 9.38 (s, 1H), 6.92 (ddd, J = 8.8, 2.4, 0.9 Hz, 1H), 6.83 (dd, J = 2.3, 0.9 Hz, 1H), 6.65 (td, J = 10.0, 2.0 Hz, 1H), 4.38 - 4.32 (m, 2H), 4.01 (s, 4H), 3.82 - 3.77 (m, 2H), 3.31 (td, J = 7.2, 6.1 Hz, 2H), 1.56 (tt, J = 7.8, 6.8 Hz, 2H), 1.39 (m, 2H), 0.95 (t, J = 7.4 Hz, 3H). ).

### Example 25

### N-butyl-2-(4-(7-fluoro-1H-indole-2-carbonyl)piperazin-1-yl)-2-oxoacetamide

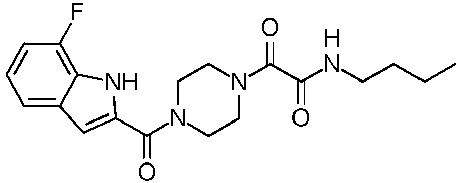

GC analysis: retention time = 14.979 min, peak area: 100%, Method L; mass *(m*/*z):* 374.1.

¹H NMR (500 MHz, Chloroform-d) δ 9.29 (s, 1H), 7.34 (s, 1H), 7.24 - 7.19 (m, 2H), 6.87 (dd, J = 2.3, 0.7 Hz, 1H), 6.85 - 6.77 (m, 1H), 4.38 - 4.32 (m, 2H), 4.02 (s, 4H), 3.82 - 3.77 (m, 2H), 3.31 (td, J = 7.1, 6.1 Hz, 2H), 1.59 - 1.51 (m, 2H), 1.43 - 1.34 (m, 2H), 0.95 (t, J = 7.4 Hz, 3H).

### Example 26

### N-butyl-2-(4-(4-fluoro-1H-indole-2-carbonyl)piperazin-1-yl)-2-oxoacetamide

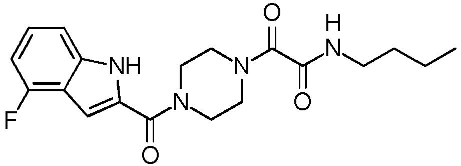

GC analysis: retention time = 14.741 min, peak area: 100%, Method L; mass *(m*/*z):* 374.1.

¹H NMR (400 MHz, Chloroform-d) δ 9.44 (s, 1H), 7.42 (dt, J = 7.9, 0.9 Hz, 1H), 7.35 (s, 1H), 7.06 (td, J = 7.9, 4.8 Hz, 1H), 7.00 (ddd, J = 10.8, 7.8, 1.0 Hz, 1H), 6.81 (dd, J = 3.2, 2.2 Hz, 1H), 4.38 - 4.30 (m, 2H), 4.01 (s, 4H), 3.83 - 3.75 (m, 2H), 3.31 (td, J = 7.1, 6.1 Hz, 2H), 1.58 - 1.50 (m, 2H), 1.43 - 1.31 (m, 2H), 0.99 - 0.91 (m, 3H).

### Example 27

### N-butyl-2-(4-(5-fluoro-1H-indole-2-carbonyl)piperazin-1-yl)-2-oxoacetamide

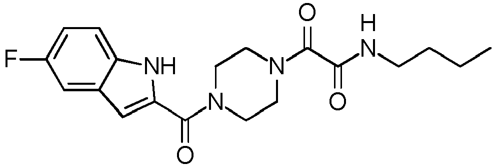

From -fluoro-1*H*-indole-2-carboxylic acid (50 mg, 0.279 mmol) and *N*-butyl-2-oxo-2-(piperazin-1-yl)acetamide (54.8 mg, 0.257 mmol), following method 3 described above, N-butyl-2-(4-(5-fluoro-1*H*-indole-2-carbonyl)piperazin-1-yl)-2-oxoacetamide (26.6 mg, 25% yield) was obtained as a solid (95% purity, based on HPLC).
Rt (Method K) 5.32 mins, *m*/*z* 375 [M+H]+
¹H NMR (400 MHz, CDCl₃) δ 9.41 (br s, 1H), 7.36 (dd, J = 8.9, 4.4 Hz, 2H), 7.29 (dd, J = 9.1, 2.5 Hz, 1H), 7.06 (td, J = 9.1, 2.5 Hz, 1H), 6.74 (dd, J = 2.2, 0.9 Hz, 1H), 4.37 - 4.27 (m, 2H), 4.01 (br s, 4H), 3.86 - 3.72 (m, 2H), 3.41 - 3.23 (m, 2H), 1.61 - 1.48 (m, 2H), 1.46 - 1.32 (m, 2H), 0.94 (t, J = 7.3 Hz, 3H).

### Synthesis method 4

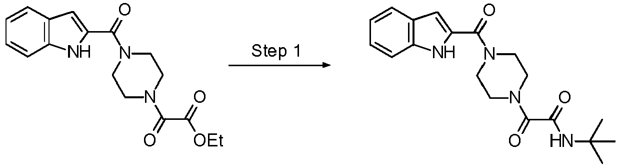

To a solution of ethyl 2-[4-(1H-indole-2-carbonyl)piperazin-1-yl]-2-oxoacetate (129 mg, 0.4 mmol) was added 2-methylpropan-2-amine in EtOH. The resulting mixture was refluxed overnight to provide the hydrolysed product. The reaction mixture was then evaporated and the residue was dissolved in Me-THF (2 mL) together with 2-methylpropan-2-amine (45.0 µl, 0.42 mmol), N-ethyl-N-isopropylpropan-amine (224 µl, 1.28 mmol) and cooled on ice under N₂. HATU (179 mg, 0.47 mmol) was added, the ice bath was removed and the mixture was stirred at room temperature for 12 hours. The reaction mixture was diluted with EtOAc (15 mL) and washed with 1N HCl, NaHCO₃ solution, and brine. The organic layer was dried over Na₂SO₄, filtered and concentrated under reduced pressure. The residue was purified by flash column chromatography to give the product as a white solid (129 mg, 85% yield).

### The following examples were prepared following synthesis method 4.

### Example 52

### N-tert-butyl-2-[4-(1H-indole-2-carbonyl)piperazin-1-yl]-2-oxoacetamide

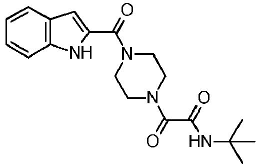

GC analysis: retention time = 14.338 min, peak area: 100%, Method L; mass *(m*/*z):* 356.1.

¹H NMR (400 MHz, Chloroform-d) δ 9.23 (s, 1H), 7.66 (dt, J = 8.1, 1.0 Hz, 1H), 7.44 (dq, J = 8.3, 1.0 Hz, 1H), 7.31 (ddd, J = 8.3, 7.0, 1.2 Hz, 1H), 7.15 (ddd, J = 8.0, 7.0, 1.0 Hz, 1H), 6.79 (dd, J = 2.2, 0.9 Hz, 1H), 4.31 â€" 4.27 (m, 2H), 4.02 (s, 4H), 3.79 â€" 3.74 (m, 2H), 1.40 (s, 9H).

### Synthesis method 5

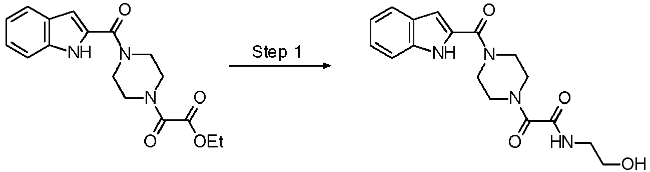

To a solution of 1,3-bis(2-isopropylphenyl)-4,5-dihydro-1H-imidazol-3-ium-2-ide (1.86 mg, 6.0 µmol) (IMes) in dry Me-THF was added ethyl 2-(4-(1H-indole-2-carbonyl)piperazin-1-yl)-2-oxoacetate (0.04 g, 0.12 mmol) and 2-aminoethan-1-ol (11 µL, 0.18 mmol) sequentially under N₂. Monitoring the reaction mixture by TLC and HPLC-MS analysis indicated complete conversion after 5h at 50°C. The volatiles were removed under reduced pressure on a rotary evaporator, and the resulting residue was purified by flash column chromatography to afford the desired product as a white solid (18 mg, 38% yield)

### The following examples were prepared following synthesis method 5.

### Example 3

### 2-(4-(1H-indole-2-carbonyl)piperazin-1-yl)-N-(2-hydroxyethyl)-2-oxoacetamide

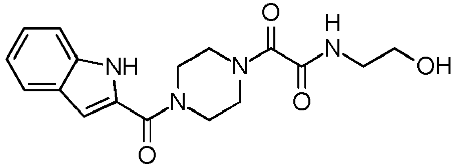

HPLC analysis: retention time = 6.585 min, peak area: 98%, Method L; mass *(m*/*z):* 345.1 [M+H]⁺ 343.0 [M-H]⁻.

¹H NMR (400 MHz, Methanol-d4) δ 7.63 (m, 1H), 7.44 (m, 1H), 7.23 (m, 1H), 7.10 - 7.05 (m, 1H), 6.88 (m, 1H), 3.95 (s, 4H), 3.83 - 3.78 (m, 2H), 3.77 - 3.72 (m, 2H), 3.66 (t, 2H), 3.41 (t, 2H).

### Example 2

### (R)-2-(4-(1H-indole-2-carbonyl)piperazin-1-yl)-N-(2-hydroxypropyl)-2-oxoacetamide

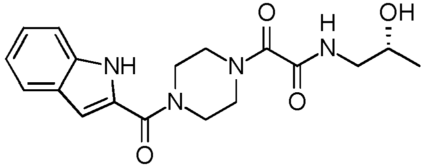

HPLC analysis: retention time = 7.007 min, peak area: 99%, Method L; mass *(m*/*z):* 359.1 [M+H]⁺ 357.1 [M-H]⁻.

¹H NMR (400 MHz, Methanol-d4) δ 7.63 (m, J = 8.0 Hz, 1H), 7.45 (m, J = 8.2 Hz, 1H), 7.26 - 7.21 (m, 1H), 7.08 (m, J = 7.0 Hz, 1H), 6.88 (m, 1H), 3.95 (s, 4H), 3.91 - 3.87 (m, 1H), 3.83 - 3.78 (m, 2H), 3.77 - 3.72 (m, 2H), 3.22 (dd, J = 13.5, 7.2 Hz, 2H), 1.19 (d, J = 6.3 Hz, 3H).

### Example 28

### 2-(4-(1H-indole-2-carbonyl)piperazin-1-yl)-N-(2-hydroxypropyl)-2-oxoacetamide

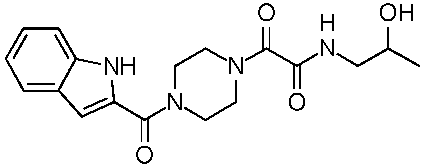

HPLC analysis: retention time = 6.845 min, peak area: 100%, Method L; mass *(m*/*z):* 359.1 [M+H]⁺ 357.1 [M-H]⁻.

¹H NMR (500 MHz, Methanol-d4) δ 7.63 (dd, J = 8.1, 1.2 Hz, 1H), 7.47 - 7.41 (m, 1H), 7.23 (ddd, J = 8.2, 7.0, 1.2 Hz, 1H), 7.08 (ddd, J = 8.0, 6.9, 1.0 Hz, 1H), 6.90 - 6.86 (m, 1H), 3.94 (s, 4H), 3.89 (dd, J = 6.6, 4.5 Hz, 1H), 3.83 - 3.78 (m, 2H), 3.78 - 3.72 (m, 2H), 3.35 (d, J = 2.9 Hz, 1H), 3.26 - 3.19 (m, 1H), 1.19 (d, J = 6.3 Hz, 3H).

### Example 29

### 2-(4-(1H-indole-2-carbonyl)piperazin-1-yl)-N-(4-hydroxybutyl)-2-oxoacetamide

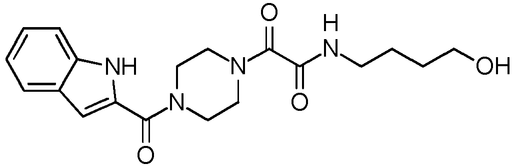

HPLC analysis: retention time = 7.133 min, peak area: 95%, Method L; mass *(m*/*z):* 373.0 [M+H]⁺ 407.0 [M+Cl]⁻.

¹H NMR (300 MHz, Methanol-d4) δ 7.62 (d, J = 8.1 Hz, 1H), 7.44 (d, J = 8.3 Hz, 1H), 7.27 - 7.17 (m, 2H), 7.07 (t, J = 7.5 Hz, 1H), 6.87 (s, 1H), 3.94 (s, 4H), 3.74 (q, J = 6.4, 4.5 Hz, 4H), 3.58 (q, J = 5.7 Hz, 2H), 3.21-3.31(m,2H), 1.74 - 1.48 (m, 4H).

### Example 30

### 2-(4-(1H-indole-2-carbonyl)piperazin-1-yl)-N-((1-hydroxycyclobutyl)methyl)-2-oxoacetamide

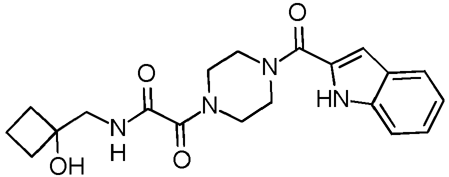

From ethyl 2-(4-(1*H*-indole-2-carbonyl)piperazin-1-yl)-2-oxoacetate (50 mg, 0.152 mmol) and and 1-(aminomethyl)cyclobutan-1-ol (23 mg, 0.228 mmol), following method 5 described above, 2-(4-(1*H*-indole-2-carbonyl)piperazin-1-yl)-*N*-((1-hydroxycyclobutyl)methyl)-2-oxoacetamide (50 mg, 86% yield) was obtained as a solid (100% purity, based on HPLC).
Rt (Method K) 4.57 mins, *m*/*z* 385 [M+H]+
¹H NMR (400 MHz, CDCl₃) δ 9.14 (br s, 1H), 7.67 (d, J = 8.0 Hz, 2H), 7.44 (dd, J = 8.3, 1.0 Hz, 1H), 7.31 (ddd, J = 8.3, 7.0, 1.1 Hz, 1H), 7.16 (ddd, J = 8.0, 7.0, 1.0 Hz, 1H), 6.80 (dd, J = 2.2, 0.9 Hz, 1H), 4.37 - 4.22 (m, 2H), 4.02 (br s, 4H), 3.87 - 3.71 (m, 2H), 3.51 (d, J = 6.1 Hz, 2H), 2.13 - 1.99 (m, 4H), 1.85 - 1.70 (m, 1H), 1.67 - 1.52 (m, 1H).

### Example 31

### 2-(4-(1H-indole-2-carbonyl)piperazin-1-yl)-N-((1-(hydroxymethyl)cyclopropyl)methyl)-2-oxoacetamide

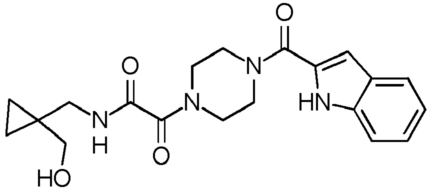

From ethyl 2-(4-(1*H*-indole-2-carbonyl)piperazin-1-yl)-2-oxoacetate (50 mg, 0.152 mmol) and (1-(aminomethyl)cyclopropyl)methanol (23 mg, 0.228 mmol), following method 5 described above, 2-(4-(1*H*-indole-2-carbonyl)piperazin-1-yl)-*N*-((1-(hydroxymethyl)cyclopropyl)methyl)-2-oxoacetamide (37 mg, 64% yield) was obtained as a solid (99% purity, based on HPLC).
Rt (Method K) 4.47 mins, *m*/*z* 385 [M+H]+
¹H NMR (400 MHz, CDCl₃) δ 9.30 (br s, 1H), 7.87 (br s, 1H), 7.66 (dd, J = 8.0, 1.0 Hz, 1H), 7.44 (dd, J = 8.3, 0.9 Hz, 1H), 7.31 (ddd, J = 8.3, 7.0, 1.2 Hz, 1H), 7.16 (ddd, J = 8.0, 7.0, 1.0 Hz, 1H), 6.80 - 6.71 (m, 1H), 4.32 - 4.27 (m, 2H), 4.03 (br s, 4H), 3.82 - 3.77 (m, 2H), 3.43 (s, 2H), 3.32 (d, J = 6.3 Hz, 2H), 0.56 - 0.49 (m, 4H).

### Example 32

### 2-(4-(1H-indole-2-carbonyl)piperazin-1-yl)-N-(2-hydroxy-2-methylpropyl)-2-oxoacetamide

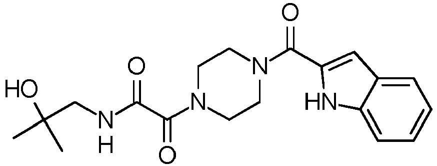

From ethyl 2-(4-(1*H*-indole-2-carbonyl)piperazin-1-yl)-2-oxoacetate (50 mg, 0.152 mmol) and 1-amino-2-methylpropan-2-ol (20.3 mg, 0.228 mmol), following method 5 described above, 2-(4-(1*H*-indole-2-carbonyl)piperazin-1-yl)-*N*-(2-hydroxy-2-methylpropyl)-2-oxoacetamide (56.5 mg, 44% yield) was obtained as a solid (100% purity, based on HPLC).
Rt (Method K) 4.41 mins, *m*/*z* 373 [M+H]+
¹H NMR (300 MHz, CDCl₃) δ 9.19 (br s, 1H), 7.71 - 7.58 (m, 2H), 7.44 (dd, J = 8.3, 1.0 Hz, 1H), 7.31 (ddd, J = 8.2, 7.0, 1.2 Hz, 1H), 7.16 (ddd, J = 8.0, 7.0, 1.0 Hz, 1H), 6.80 (dd, J = 2.1, 0.9 Hz, 1H), 4.35 - 4.24 (m, 2H), 4.03 (br s, 4H), 3.86 - 3.76 (m, 2H), 3.33 (d, J = 6.4 Hz, 2H), 1.27 (s, 6H).

### Example 33

### 2-(4-(1H-indole-2-carbonyl)piperazin-1-yl)-N-((1r,4r)-4-hydroxycyclohexyl)-2-oxoacetamide

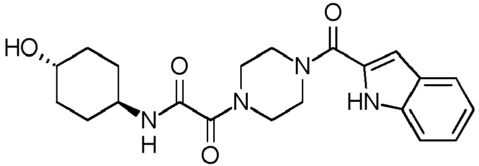

From ethyl 2-(4-(1*H*-indole-2-carbonyl)piperazin-1-yl)-2-oxoacetate (50 mg, 0.152 mmol) and (1*r*,4*r*)-4-aminocyclohexan-1-ol (26 mg, 0.228 mmol), following method 5 described above, 2-(4-(1*H*-indole-2-carbonyl)piperazin-1-yl)-*N*-((1*r*,4*r*)-4-hydroxycyclohexyl)-2-oxoacetamide (13 mg, 22% yield) was obtained as a solid (95% purity, based on HPLC).
Rt (Method K) 4.34 mins, *m*/*z* 399 [M+H]+
¹H NMR (300 MHz, CDCl₃) δ 9.32 (br s, 1H), 7.68 (dd, J = 8.1, 1.1 Hz, 1H), 7.45 (dd, J = 8.3, 1.0 Hz, 1H), 7.32 (ddd, J = 8.3, 7.0, 1.2 Hz, 1H), 7.26 - 7.21 (m, 1H), 7.17 (ddd, J = 8.0, 6.9, 1.0 Hz, 1H), 6.81 (d, J = 2.3 Hz, 1H), 4.42 - 4.27 (m, 2H), 4.04 (br s, 4H), 3.86 - 3.77 (m, 2H), 3.76 - 3.60 (m, 2H), 2.04 (d, J = 11.6 Hz, 4H), 1.57 - 1.19 (m, 4H).

### Synthesis method 6

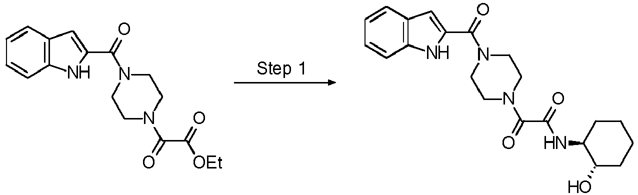

**Step 4:** To a solution of 1,3-bis(2-isopropylphenyl)-4,5-dihydro-1*H*-imidazol-3-ium-2-ide (IMes) (2.33 mg, 7.59 µmol) in dry 2-Me-THF (1.2 mL) was added ethyl 2-(4-(1*H*-indole-2-carbonyl)piperazin-1-yl)-2-oxoacetate (50 mg, 0.152 mmol) (see Synthesis method 2) and (1*R*,2*R*)-2-aminocyclohexan-1-ol (26.2 mg, 0.228 mmol). The mixture was stirred overnight at 50°C. The solvent was removed under reduced pressure and the resulting residue was purified by flash column chromatography on silica gel (0 to 10% MeOH in CH₂Cl₂, gradient) to give 2-(4-(1*H*-indole-2-carbonyl)piperazin-1-yl)-*N-*((1*R*,2*R*)-2-hydroxycyclohexyl)-2-oxoacetamide (35.6 mg, 59% yield), as a solid (100% purity, based on HPLC).

### The following examples were prepared following synthesis method 6.

### Example 34

### N-[(1S,2S)-2-hydroxycyclohexyl]-2-[4-(1H-indole-2-carbonyl)piperazin-1-yl]-2-oxoacetamide

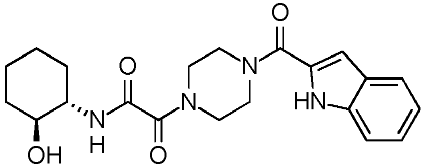

Rt (Method K) 4.68 mins, *m*/*z* 399 [M+H]+
¹H NMR (500 MHz, , CDCl₃) δ 9.17 (br s, 1H), 7.67 (dd, J = 8.1, 1.1 Hz, 1H), 7.44 (dd, J = 8.3, 1.0 Hz, 1H), 7.40 - 7.34 (m, 1H), 7.31 (ddd, J = 8.2, 7.0, 1.2 Hz, 1H), 7.16 (ddd, J = 8.0, 7.0, 1.0 Hz, 1H), 6.80 (dd, J = 2.2, 0.9 Hz, 1H), 4.32 (dd, J = 6.4, 4.1 Hz, 2H), 4.02 (br s, 4H), 3.83 - 3.73 (m, 2H), 3.70 - 3.60 (m, 1H), 3.43 (td, J = 10.0, 4.5 Hz, 1H), 2.12 - 1.97 (m, 2H), 1.84 - 1.57 (m, 2H), 1.48 - 1.16 (m, 4H).

### Example 35

### 2-(4-(1H-indole-2-carbonyl)piperazin-1-yl)-2-oxo-N-(tetrahydrofuran-3-yl)acetamide

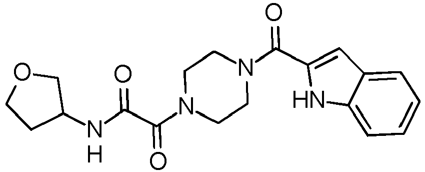

From 2-(4-(1*H*-indole-2-carbonyl)piperazin-1-yl)-2-oxoacetate (40 mg, 0.121 mmol) and tetrahydrofuran-3-amine (15.87 mg, 0.182 mmol), following method 6 described above, 2-(4-(1*H*-indole-2-carbonyl)piperazin-1-yl)-2-oxo-*N*-(tetrahydrofuran-3-yl)acetamide (16 mg, 36% yield) was obtained as a solid (95% purity, based on HPLC).
Rt (Method K) 4.46 mins, *m*/*z* 371 [M+H]+
¹H NMR (500 MHz, , CDCl₃) δ 9.33 (br s, 1H), 7.66 (dq, J = 8.0, 1.0 Hz, 1H), 7.58 (d, J = 7.9 Hz, 1H), 7.48 - 7.40 (m, 1H), 7.31 (ddd, J = 8.2, 7.0, 1.2 Hz, 1H), 7.16 (ddd, J = 8.0, 7.0, 1.0 Hz, 1H), 6.79 (dd, J = 2.2, 0.9 Hz, 1H), 4.53 - 4.44 (m, 1H), 4.32 (td, J = 4.6, 1.7 Hz, 2H), 4.02 (br s, 4H), 3.96 (dt, J = 8.9, 7.4 Hz, 1H), 3.89 (dd, J = 9.5, 5.6 Hz, 1H), 3.83 (td, J = 8.6, 5.7 Hz, 1H), 3.80 - 3.76 (m, 2H), 3.72 (dd, J = 9.5, 3.0 Hz, 1H), 2.39 - 2.25 (m, 1H), 1.96-1.83 (m, 1H).

### Example 36

### (S)-2-(4-(1H-indole-2-carbonyl)piperazin-1-yl)-N-(2-hydroxypropyl)-2-oxoacetamide

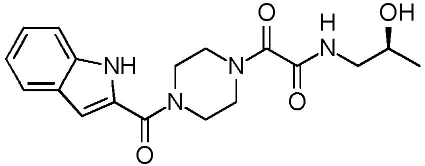

From 2-(4-(1*H*-indole-2-carbonyl)piperazin-1-yl)-2-oxoacetate (50 mg, 0.152 mmol) and (S)-1-aminopropan-2-ol (17.1 mg, 0.228 mmol), following method 6 described above, (*S*)-2-(4-(1*H*-indole-2-carbonyl)piperazin-1-yl)-*N*-(2-hydroxypropyl)-2-oxoacetamide (19.7 mg, 20% yield) was obtained as a solid (95% purity, based on HPLC).
Rt (Method K) 4.80 mins, *m*/*z* 359 [M+H]+
¹H NMR (500 MHz, CD₃OD) δ 8.04 (s, 1H), 7.62 (dt, J = 8.0, 1.0 Hz, 1H), 7.44 (dd, J = 8.3, 1.0 Hz, 1H), 7.23 (ddd, J = 8.3, 7.0, 1.1 Hz, 1H), 7.19 (s, 1H), 7.07 (ddd, J = 8.0, 7.0, 1.0 Hz, 1H), 6.87 (d, J = 0.9 Hz, 1H), 3.94 (br s, 4H), 3.89 (ddd, J = 7.2, 6.3, 4.5 Hz, 1H), 3.82 - 3.78 (m, 2H), 3.75 - 3.70 (m, 2H), 3.36 - 3.30 (m, 1H), 3.21 (dd, J = 13.5, 7.2 Hz, 1H), 1.18 (d, J = 6.3 Hz, 3H).

### Synthesis method 7

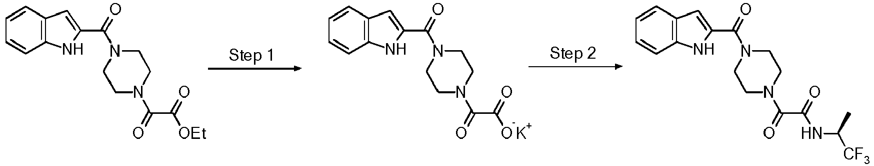

**Step** 1: A solution of KOH (0.5M in dry MeOH) (7.68 mL, 3.84 mmol) was added to a solution of *tert*-butyl 4-(2-ethoxy-2-oxoacetyl)piperazine-1-carboxylate (1 g, 3.49 mmol) in dry MeOH (2 mL). The resulting solution was stirred 2 hours at room temperature. The reaction mixture was evaporated under vacuum affording a white solid. Et₂O was added and the reaction mixture was sonicated until a suspension was obtained. The latter was filtered, filter cake was washed with Et₂O and dried under vacuum to afford potassium 2-(4-(*tert-*butoxycarbonyl)piperazin-1-yl)-2-oxoacetate (898 mg, 87% yield).

**Step 2:** Potassium 2-(4-(tert-butoxycarbonyl)piperazin-1-yl)-2-oxoacetate (55 mg, 0.162 mmol), (*S*)-1,1,1-trifluoropropan-2-amine hydrochloride (24.23 mg, 0.162 mmol) , and *N-*ethyl-N-isopropylpropan-2-amine (85 µL, 0.486 mmol) were dissolved in dry 2-Me-THF (1.5 mL) and cooled on ice under inert atmoshere. HATU (67.6 mg, 0.178 mmol) was added, the ice bath was removed and the mixture was stirred overnight at room temperature. The reaction mixture was diluted with EtOAc and washed with 1M HCl, saturated NaHCO₃ solution, and brine. The organic layer was dried over Na₂SO₄, filtered and concentrated under reduced pressure. The residue was purified by column chromatography (0 to 5% MeOH in CH₂Cl₂, gradient) to afford (*S*)-2-(4-(1*H*-indole-2-carbonyl)piperazin-1-yl)-2-oxo-*N*-(1,1,1-trifluoropropan-2-yl)acetamide (42.2 mg, 66% yield), as a solid (100% purity, based on HPLC).

**The following examples were prepared following synthesis method 7.**

### Example 37

### 2-[4-(1H-indole-2-carbonyl)piperazin-1-yl]-2-oxo-N-[(2S)-1,1,1-trifluoropropan-2-yl]acetamide

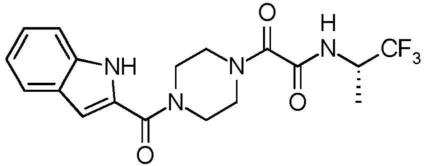

Rt (Method K) 5.40 mins, *m*/*z* 397 [M+H]+
¹H NMR (500 MHz, , CDCl₃) δ 9.32 (br s, 1H), 7.66 (dq, J = 8.1, 0.9 Hz, 1H), 7.57 (d, J = 9.8 Hz, 1H), 7.44 (dd, J = 8.3, 1.0 Hz, 1H), 7.31 (ddd, J = 8.2, 7.0, 1.1 Hz, 1H), 7.16 (ddd, J = 8.0, 7.0, 1.0 Hz, 1H), 6.80 (dd, J = 2.2, 0.9 Hz, 1H), 4.68 - 4.56 (m, 1H), 4.39 - 4.24 (m, 2H), 4.04 (br s, 4H), 3.87 - 3.75 (m, 2H), 1.40 (d, J = 7.0 Hz, 3H).

### Example 38

### (R)-2-(4-(1H-indole-2-carbonyl)piperazin-1-yl)-2-oxo-N (1,1,1-trifluoropropan-2-yl)acetamide

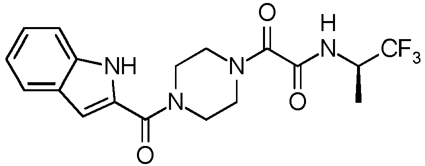

From potassium 2-(4-(tert-butoxycarbonyl)piperazin-1-yl)-2-oxoacetate (48.3 mg, 0.142 mmol) and (R)-1,1,1-trifluoropropan-2-amine hydrochloride (21.28 mg, 0.142 mmol), following method 7 described above, (*R*)-2-(4-(1*H*-indole-2-carbonyl)piperazin-1-yl)-2-oxo-*N*-(1,1,1-trifluoropropan-2-yl)acetamide (15 mg, 27% yield) was obtained as a solid (91% purity, based on HPLC).
Rt (Method K) 5.32 mins, *m*/*z* 397 [M+H]+
¹H NMR (400 MHz, , CDCl₃) δ 9.26 (br s, 1H), 7.67 (d, J = 8.0 Hz, 1H), 7.55 (d, J = 9.7 Hz, 1H), 7.44 (dt, J = 8.4, 1.0 Hz, 1H), 7.31 (ddd, J = 8.3, 7.0, 1.1 Hz, 1H), 7.16 (ddd, J = 8.0, 7.0, 1.0 Hz, 1H), 6.80 (dd, J = 2.1, 0.9 Hz, 1H), 4.70 - 4.55 (m, 1H), 4.41 - 4.23 (m, 2H), 4.04 (br s, 4H), 3.84 - 3.68 (m, 2H), 1.40 (d, J = 7.0 Hz, 3H).

### Example 39

### 2-(4-(1H-indole-2-carbonyl)piperazin-1-yl)-N-(4-methyltetrahydro-2H-pyran-4-yl)-2-oxoacetamide

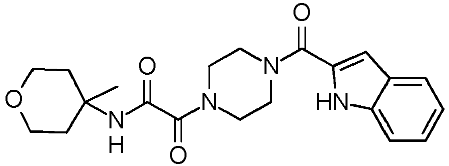

From potassium 2-(4-(*tert*-butoxycarbonyl)piperazin-1-yl)-2-oxoacetate (50 mg, 0.147 mmol) and 4-methyltetrahydro-2*H*-pyran-4-amine hydrochloride (22.34 mg, 0.147 mmol), following method 7 described above, 2-(4-(1*H*-indole-2-carbonyl)piperazin-1-yl)-*N*-(4-methyltetrahydro-2H-pyran-4-yl)-2-oxoacetamide (9.5 mg, 16% yield) was obtained as a solid (95% purity, based on HPLC).
Rt (Method K) 4.75 mins, *m*/*z* 399 [M+H]+
¹H NMR (500 MHz, , CDCl₃) δ 9.39 (br s, 1H), 7.66 (dd, J = 8.1, 1.0 Hz, 1H), 7.44 (dd, J = 8.3, 0.9 Hz, 1H), 7.30 (ddd, J = 8.3, 7.0, 1.1 Hz, 1H), 7.28 (br s, 1H), 7.15 (ddd, J = 8.0, 7.0, 1.0 Hz, 1H), 6.79 (dd, J = 2.2, 0.9 Hz, 1H), 4.33 - 4.28 (m, 2H), 4.03 (br s, 4H), 3.81 - 3.77 (m, 2H), 3.76 - 3.72 (m, 2H), 3.64 (ddd, J = 12.2, 9.6, 2.8 Hz, 2H), 2.12 - 2.04 (m, 2H), 1.73 (ddd, J= 13.9, 9.7, 4.2 Hz, 2H), 1.47 (s, 3H).

### Example 40

### 2-(4-(1H-indole-2-carbonyl)piperazin-1-yl)-2-oxo-N-(tetrahydro-2H-pyran-4-yl)acetamide

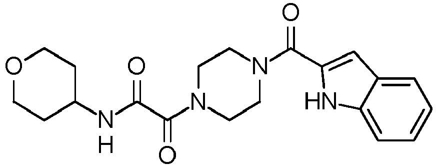

From potassium 2-(4-(*tert*-butoxycarbonyl)piperazin-1-yl)-2-oxoacetate (100 mg, 0.295 mmol) and tetrahydro-2H-pyran-4-amine hydrochloride (40.5 mg, 0.295 mmol), following method 7 described above, 2-(4-(1*H*-indole-2-carbonyl)piperazin-1-yl)-2-oxo-*N*-(tetrahydro-2H-pyran-4-yl)acetamide (51 mg, 45% yield) was obtained as a solid (97% purity, based on HPLC).
Rt (Method K) 4.53 mins, *m*/*z* 385 [M+H]+
¹H NMR (400 MHz, , CDCl₃) δ 9.46 (br s, 1H), 7.66 (dd, J = 8.0, 1.1 Hz, 1H), 7.44 (dd, J = 8.3, 1.0 Hz, 1H), 7.38 (s, 1H), 7.30 (ddd, J = 8.6, 6.8, 1.0 Hz, 1H), 7.15 (td, J = 7.4, 6.9, 0.8 Hz, 1H), 6.84 - 6.75 (m, 1H), 4.33 (t, J = 5.3 Hz, 2H), 4.08 - 3.94 (m, 7H), 3.83 - 3.76 (m, 2H), 3.55 - 3.45 (m, 2H), 1.90 (d, J = 12.9 Hz, 2H), 1.62 - 1.50 (m, 2H).

### Example 41

### 2-(4-(1H-indole-2-carbonyl)piperazin-1-yl)-N-(1-(methoxymethyl)cyclobutyl)-2-oxoacetamide

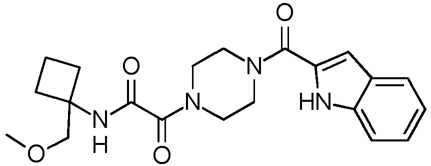

From potassium 2-(4-(*tert*-butoxycarbonyl)piperazin-1-yl)-2-oxoacetate (100 mg, 0.295 mmol) and 1-(methoxymethyl)cyclobutan-1-amine hydrochloride (44.7 mg, 0.295 mmol), following method 7 described above, 2-(4-(1*H*-indole-2-carbonyl)piperazin-1-yl)-*N*-(1-(methoxymethyl)cyclobutyl)-2-oxoacetamide (42 mg, 36% yield) was obtained as a solid (98% purity, based on HPLC).
Rt (Method K) 5.04 mins, *m*/*z* 399 [M+H]+
¹H NMR (400 MHz, , CDCl₃) δ 9.41 (br s, 1H), 7.66 (dd, J = 8.1, 1.1 Hz, 1H), 7.52 (br s, 1H), 7.44 (dd, J = 8.3, 1.0 Hz, 1H), 7.34 - 7.27 (m, 1H), 7.15 (ddd, J = 8.0, 6.9, 1.0 Hz, 1H), 6.78 (d, J = 1.5 Hz, 1H), 4.31 - 4.23 (m, 2H), 4.01 (br s, 4H), 3.82 - 3.73 (m, 2H), 3.60 (s, 2H), 3.40 (s, 3H), 2.61 - 2.41 (m, 2H), 2.20 - 2.08 (m, 2H), 2.02 - 1.77 (m, 2H).

### Synthesis method 8

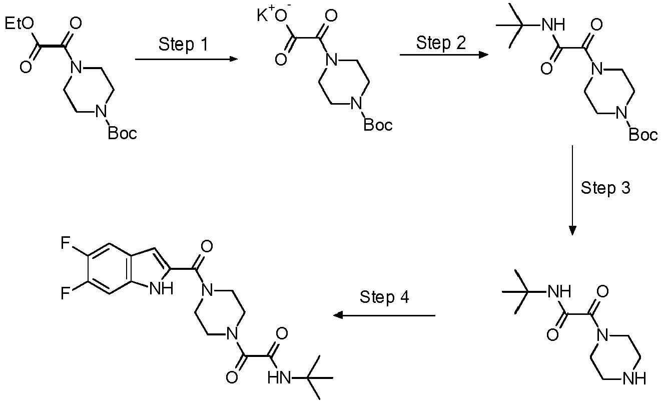

**Step 1:** A solution of KOH 0.5 M in dry MeOH (7 mL, 3.5 mmol) was added to a solution of *tert*-butyl 4-(2-ethoxy-2-oxoacetyl)piperazine-1-carboxylate (911.5 mg, 3.18 mmol) in dry MeOH (1.82 mL). The resulting solution was stirred 2 hours at room temperature. The reaction mixture was evaporated under vacuum affording a white solid. Et₂O was added and the reaction mixture was sonicated until a suspension was obtained. The latter was filtered, filter cake was washed with Et₂O and dried under vacuum to afford potassium 2-(4-(*tert-*butoxycarbonyl)piperazin-1-yl)-2-oxoacetate (841.4 mg, 89% yield).

**Step 2:** Potassium 2-(4-(*tert*-butoxycarbonyl)piperazin-1-yl)-2-oxoacetate (841.4 mg, 2.84 mmol), 2-methylpropan-2-amine (0.448 mL, 4.26 mmol), and DIPEA (0.989 mL, 5.68 mmol) were dissolved in dry 2-Me-THF (10 mL) and cooled on ice under inert atmosphere. HATU (1.184 g, 3.12 mmol) was added, the ice bath was removed, and the mixture was stirred overnight at room temperature. The reaction mixture was diluted with EtOAc and washed with 1M HCl, saturated NaHCO₃ solution, and brine. The solvent was removed under reduced pressure to afford tert-butyl 4-(2-(*tert*-butylamino)-2-oxoacetyl)piperazine-1-carboxylate (297 mg, 33% yield).

**Step 3:** To a solution of *tert*-butyl 4-(2-(*tert*-butylamino)-2-oxoacetyl)piperazine-1-carboxylate (295 mg, 0.94 mmol) in CH₂Cl₂ (4.38 mL) was slowly added trifluoroacetic acid (1.44 mL, 18.83 mmol). After stirring for 2h, the solvent was removed under reduced pressure. The crude mixture concentrated in vacuo to provide *N-*(*tert*-butyl)-2-oxo-2-(piperazin-1-yl)acetamide (183 mg, 91% yield).

**Step 4:** To a solution of 5,6-difluoro-1*H*-indole-2-carboxylic acid (25 mg, 0.127 mmol) in dry THF (0.7 mL) was added CDI) (17.07 g, 0.105 mmol). The mixture stirred under intert atmosphere for 1h at 50 °C. Then, *N*-(*tert*-butyl)-2-oxo-2-(piperazin-1-yl)acetamide (24.88 mg, 0.117 mmol) was added and the resulting mixture stirred overnight at 50°C under inert atmosphere. The solvent was removed under reduced pressure and diluted in EtOAc and saturated NaHCO₃ solution. The aqueous layer was extracted with EtOAc (x3). The combined organic phases were washed with water, brine, dried over Na₂SO₄ and solvent was removed under reduced pressure. The residue was purified by column chromatography (0 to 10% MeOH in CH₂Cl₂, gradient) to afford *N*-(*tert*-butyl)-2-(4-(5,6-difluoro-1*H*-indole-2-carbonyl)piperazin-1-yl)-2-oxoacetamide (16 mg, 32% yield), as a solid (97% purity, based on HPLC).

### The following examples were prepared following synthesis method 8.

### Example 42

### N-tert-butyl-2-[4-(5,6-difluoro-1H-indole-2-carbonyl)piperazin-1-yl]-2-oxoacetamide

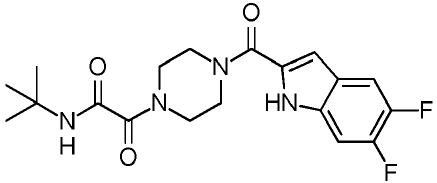

Rt (Method K) 5.34 mins, *m*/*z* 393 [M+H]+
¹H NMR (500 MHz, , CDCl₃) δ 9.35 (br s, 1H), 7.38 (dd, J = 10.3, 7.7 Hz, 1H), 7.23 - 7.15 (m, 2H), 6.73 (s, 1H), 4.34 - 4.27 (m, 2H), 4.00 (br s, 4H), 3.82 - 3.71 (m, 2H), 1.40 (s, 9H).

### Example 43

### N-(tert-butyl)-2-(4-(4,6-difluoro-1H-indole-2-carbonyl)piperazin-1-yl)-2-oxoacetamide

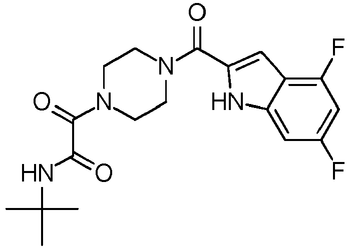

From 4,6-difluoro-1*H*-indole-2-carboxylic acid (40 mg, 0.203 mmol) and *N*-(tert-butyl)-2-oxo-2-(piperazin-1-yl)acetamide (39.8 mg, 0.187 mmol), following method 8 described above, *N*-(tert-butyl)-2-(4-(4,6-difluoro-1*H*-indole-2-carbonyl)piperazin-1-yl)-2-oxoacetamide (24.3 mg, 31% yield) was obtained as a solid (94% purity, based on HPLC).
Rt (Method K) 5.51 mins, *m*/*z* 393 [M+H]+
¹H NMR (400 MHz, , CDCl₃) δ 9.59 (br s, 1H), 7.18 (br s, 1H), 7.00 - 6.88 (m, 1H), 6.83 (dd, J = 2.2, 0.9 Hz, 1H), 6.65 (td, J = 10.0, 2.0 Hz, 1H), 4.35 - 4.24 (m, 2H), 4.01 (br s, 4H), 3.81 - 3.72 (m, 2H), 1.40 (s, 9H).

### Example 44

### N-(tert-butyl)-2-(4-(4-methyl-1H-indole-2-carbonyl)piperazin-1-yl)-2-oxoacetamide

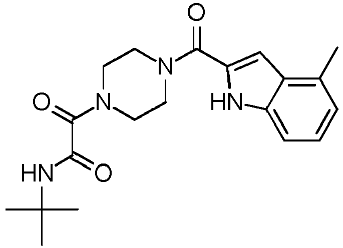

From 4-methyl-1*H*-indole-2-carboxylic acid (40 mg, 0.228 mmol) and *N-*(*tert*-butyl)-2-oxo-2-(piperazin-1-yl)acetamide (44.8 mg, 0.1210 mmol), following method 8 described above, *N-*(*tert*-butyl)-2-(4-(4-methyl-1*H*-indole-2-carbonyl)piperazin-1-yl)-2-oxoacetamide (10.2 mg, 12% yield) was obtained as a solid (96% purity, based on HPLC).
Rt (Method K) 5.41 mins, *m*/*z* 371 [M+H]+
¹H NMR (400 MHz, , CDCl₃) δ 9.38 (br s, 1H), 7.28 (br s, 1H), 7.24 - 7.14 (m, 2H), 6.94 (dt, J = 7.0, 1.0 Hz, 1H), 6.79 (dd, J = 2.2, 1.0 Hz, 1H), 4.31 - 4.25 (m, 2H), 4.04 (br s, 4H), 3.81 - 3.72 (m, 2H), 2.56 (s, 3H), 1.41 (s, 9H).

### Example 45

### N-(tert-butyl)-2-(4-(6-chloro-5-fluoro-1H-indole-2-carbonyl)piperazin-1-yl)-2-oxoacetamide

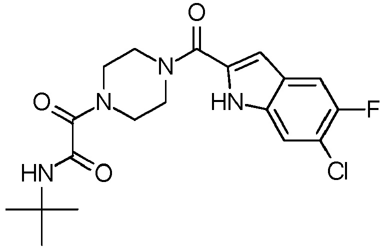

From 6-chloro-5-fluoro-1*H*-indole-2-carboxylic acid (25 mg, 0.117 mmol) and *N*-(*tert*-butyl)-2-oxo-2-(piperazin-1-yl)acetamide (22.97 mg, 0.108 mmol), following method 8 described above, N-(*tert*-butyl)-2-(4-(6-chloro-5-fluoro-1*H*-indole-2-carbonyl)piperazin-1-yl)-2-oxoacetamide (22 mg, 46% yield) was obtained as a solid (91% purity, based on HPLC).
Rt (Method K) 5.64 mins, *m*/*z* 409 [M+H]+
¹H NMR (500 MHz, , CDCl₃) δ 9.52 (br s, 1H), 7.47 (dd, J = 6.0, 0.9 Hz, 1H), 7.37 (d, J = 9.2 Hz, 1H), 7.18 (s, 1H), 6.73 (dd, J = 2.2, 0.9 Hz, 1H), 4.33 - 4.26 (m, 2H), 4.00 (br s, 4H), 3.79 - 3.73 (m, 2H), 1.40 (s, 9H).

### Example 46

### N-(tert-butyl)-2-(4-(5-fluoro-4-methyl-1H-indole-2-carbonyl)piperazin-1-yl)-2-oxoacetamide

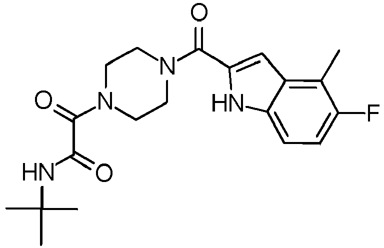

From 5-fluoro-4-methyl-1*H*-indole-2-carboxylic acid (25 mg, 0.129 mmol) and *N*-(*tert-*butyl)-2-oxo-2-(piperazin-1-yl)acetamide (25.4 mg, 0.119 mmol), following method 8 described above, *N*-(*tert*-butyl)-2-(4-(5-fluoro-4-methyl-1*H*-indole-2-carbonyl)piperazin-1-yl)-2-oxoacetamide (14 mg, 28% yield) was obtained as a solid (91% purity, based on HPLC).
Rt (Method K) 5.55 mins, *m*/*z* 389 [M+H]+
¹H NMR (300 MHz, , CDCl₃) δ 9.33 (br s, 1H), 7.25 - 7.14 (m, 2H), 7.08 - 6.97 (m, 1H), 6.79 - 6.72 (m, 1H), 4.36 - 4.23 (m, 2H), 4.03 (br s, 4H), 3.81 - 3.67 (m, 2H), 2.45 (d, J = 1.9 Hz, 3H), 1.40 (s, 9H).

### Example 47

### N-(tert-butyl)-2-(4-(5-chloro-1H-indole-2-carbonyl)piperazin-1-yl)-2-oxoacetamide

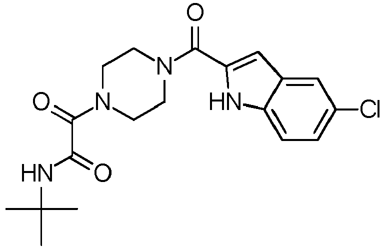

From 5-chloro-1*H*-indole-2-carboxylic acid (25 mg, 0.128 mmol) and *N*-(*tert*-butyl)-2-oxo-2-(piperazin-1-yl)acetamide (25.1 mg, 0.118 mmol), following method 8 described above, *N-*(*tert*-butyl)-2-(4-(5-chloro-1*H*-indole-2-carbonyl)piperazin-1-yl)-2-oxoacetamide (17,7 mg, 35% yield) was obtained as a solid (93% purity, based on HPLC).
Rt (Method K) 5.68 mins, *m*/*z* 391 [M+H]+
¹H NMR (500 MHz, , CDCl₃) δ 9.29 (s, 1H), 7.62 (d, J = 2.0 Hz, 1H), 7.36 (d, J = 8.8 Hz, 1H), 7.29 - 7.22 (m, 1H), 7.17 (br s, 1H), 6.72 (dd, J = 2.2, 1.0 Hz, 1H), 4.33 - 4.26 (m, 2H), 4.00 (br s, 4H), 3.81 - 3.73 (m, 2H), 1.40 (s, 9H).

### Synthesis method 9

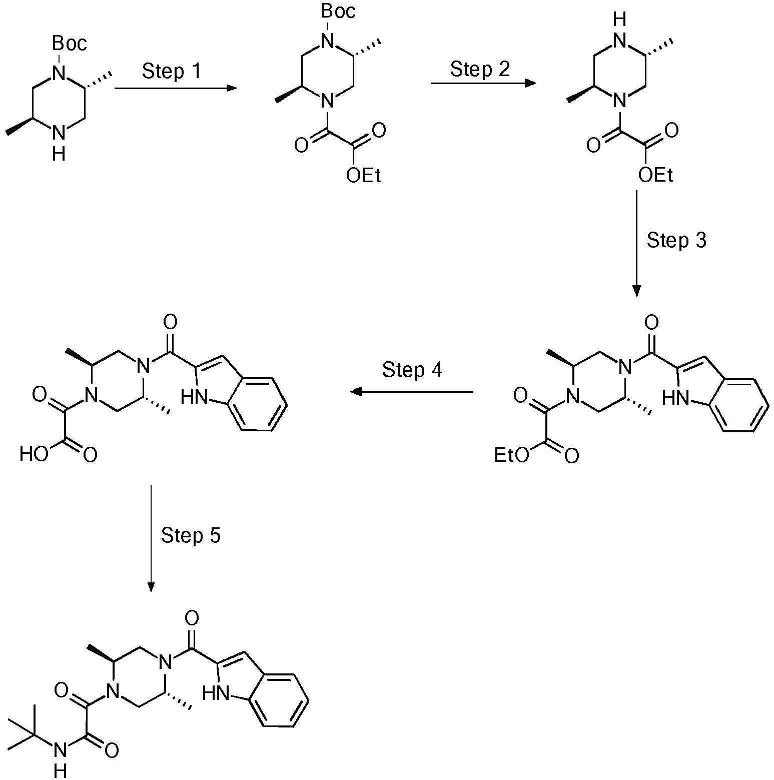

**Step 1**: Feed solution 1: A solution of *tert*-butyl (2*R*,5*S*)-2,5-dimethylpiperazine-1-carboxylate (321 mg, 1.5 mmol) in CH₂Cl₂ (7.5 mL) was mixed with NEt₃ (314 µL, 2.25 mmol) at room temperature and the resulting the mixture was pumped at 0.5 ml/min. Feed solution 2: A solution of ethyl 2-chloro-2-oxoacetate (184 µL, 1.65 mmol) in CH₂Cl₂ (7.5 mL) was pumped at 0.5 ml/min. The reaction mixture flowed through PTF 1/16" tubing coil at room temperature, with 5 min residence time. Once the resulting crude mixture is collected, solvent was removed and the white crystalline product was mixed with water. The product was extracted with EtOAc (x3) and washed with saturated NH₄Cl solution, saturated NaHCOs solution and brine. The combined organic phases were dried over Na₂SO₄ and concentrated under reduced pressure to afford *tert*-butyl (2*R*,5*S*)-4-(2-ethoxy-2-oxoacetyl)-2,5-dimethylpiperazine-1-carboxylate (360.8 mg, 77% yield).

**Step 2:** To a solution of *tert*-butyl (2*R*,5*S*)-4-(2-ethoxy-2-oxoacetyl)-2,5-dimethylpiperazine-1-carboxylate (360.8 mg, 1.15 mmol) in CH₂Cl₂ (5.3 mL) was slowly added trifluoroacetic acid (1.76 mL, 23.0 mmol). After stirring for 2h, the solvent was removed under reduced pressure. The crude mixture concentrated in vacuo to provide ethyl 2-((2*S*,5*R*)-2,5-dimethylpiperazin-1-yl)-2-oxoacetate (240 mg, 98% yield).

**Step 3:** To a solution of 1*H*-indole-2-carboxylic acid (195 mg, 1.21 mmol) in dry THF (7.5 mL) was added CDI (163 mg, 1.00 mmol). The mixture was stirred under an inert atmosphere for 1h at 50 °C. Then, ethyl 2-((2*S*,5*R*)-2,5-dimethylpiperazin-1-yl)-2-oxoacetate (239 mg, 1.11 mmol) was added and the resulting mixture stirred overnight at 50°C under an inert atmosphere. The solvent was removed under reduced pressure and partitioned between EtOAc and saturated NaHCOs solution. The aqueous layer was extracted with EtOAc (x3). The combined organic phases were washed with water, brine, dried over Na₂SO₄ and solvent was removed under reduced pressure. The residue was dissolved in the minimum volume of EtOH and product precipitated by addition of water to give the product as white solid 2-((2*S*,5*R*)-4-(1*H*-indole-2-carbonyl)-2,5-dimethylpiperazin-1-yl)-2-oxoacetate (254.5 mg, 59% yield).

**Step 4:** A solution of KOH 0.5 M in dry MeOH (0.5 mL, 0.251 mmol) was added to a solution of ethyl 2-((2*S*,5*R*)-4-(1*H*-indole-2-carbonyl)-2,5-dimethylpiperazin-1-yl)-2-oxoacetate (81.4 mg, 0.228 mmol) in dry MeOH (0.15 mL). The resulting solution was stirred 2 hours at room temperature. The reaction mixture was evaporated under vacuum affording a white solid. Et₂O was added and the reaction mixture was sonicated until a suspension was obtained. The latter was filtered, filter cake was washed with Et₂O and dried under vacuum to afford potassium 2-((2*S*,5*R*)-4-(1*H*-indole-2-carbonyl)-2,5-dimethylpiperazin-1-yl)-2-oxoacetate (60 mg, 72% yield).

**Step 5:** Potassium 2-((2*S*,5*R*)-4-(1*H*-indole-2-carbonyl)-2,5-dimethylpiperazin-1-yl)-2-oxoacetate (50 mg, 0.14 mmol), 2-methylpropan-2-amine (29 µL, 0.272 mmol) , and *N*-ethyl-*N*-isopropylpropan-2-amine (71 µL, 0.408 mmol) were dissolved in dry 2-Me-THF (1.3 mL) and cooled on ice under inert atmoshere. HATU (56.8 mg, 0.15 mmol) was added, the ice bath was removed and the mixture was stirred overnight at room temperature. The reaction mixture was diluted with EtOAc and washed with 1M HCl, saturated NaHCOs solution, and brine. The organic layer was dried over Na₂SO₄, filtered and concentrated under reduced pressure. The residue was purified by column chromatography (0 to 5% MeOH in CH₂Cl₂, gradient) to afford 2-((2*S*,5*R*)-4-(1*H*-indole-2-carbonyl)-2,5-dimethylpiperazin-1-yl)-*N*-(*tert-*butyl)-2-oxoacetamide (29.6 mg, 57% yield), as a solid (98% purity, based on HPLC).

### The following examples were prepared following synthesis method 9.

### Example 48

### N-tert-butyl-2-[(2S,SR)-4-(1H-indole-2-carbonyl)-2, 5-dimethylpiperazin-1-yl]-2-oxoacetamide

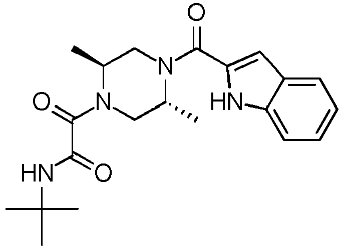

Rt (Method K) 5.35 mins, *m*/*z* 385 [M+H]+
¹H NMR (300 MHz, , CDCl₃) δ 9.49 (br s, 1H), 7.66 (d, J = 8.0 Hz, 1H), 7.44 (dq, J = 8.3, 0.9 Hz, 1H), 7.29 (ddd, J = 8.2, 6.9, 1.1 Hz, 1H), 7.15 (ddd, J = 8.0, 6.9, 1.1 Hz, 2H), 6.77 (s, 1H), 5.43 - 5.31 (m, 1H), 5.16 - 5.01 (m, 1H), 5.01 - 4.71 (m, 2H), 4.54 - 4.20 (m, 2H), 1.45 - 1.33 (m, 15H).

### Example 49

### (S)-2-(4-(1H-indole-2-carbonyl)-3-methylpiperazin-1-yl)-N-(tert-butyl)-2-oxoacetamide

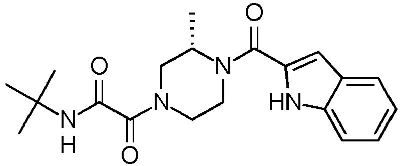

From potassium (*S*)-2-(4-(1*H*-indole-2-carbonyl)-3-methylpiperazin-1-yl)-2-oxoacetate (50 mg, 0.141 mmol) and 2-methylpropan-2-amine (30 µL, 0.283 mmol), following method 9 described above, (*S*)-2-(4-(1*H*-indole-2-carbonyl)-3-methylpiperazin-1-yl)-*N*-(*tert*-butyl)-2-oxoacetamide (24.4 mg, 47% yield) was obtained as a solid (96% purity, based on HPLC).
Rt (Method K) 5.26 mins, *m*/*z* 371 [M+H]+
¹H NMR (300 MHz, , CDCl₃) δ 9.34 (br s, 1H), 7.66 (dt, J = 8.0, 1.0 Hz, 1H), 7.43 (dd, J = 8.3, 0.9 Hz, 1H), 7.30 (ddd, J = 8.3, 7.0, 1.2 Hz, 1H), 7.21 - 7.09 (m, 2H), 6.82 - 6.75 (m, 1H), 5.24 - 4.90 (m, 2H), 4.67 - 4.32 (m, 2H), 3.57 - 3.24 (m, 2H), 3.12 - 2.90 (m, 1H), 1.40 - 1.40 (m, 12H).

### Example 50

### (R)-2-(4-(1H-indole-2-carbonyl)-3-methylpiperazin-1-yl)-N-(tert-butyl)-2-oxoacetamide

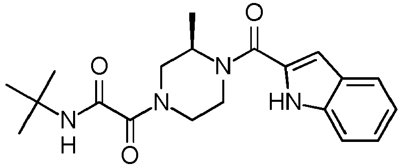

From potassium (*R*)-2-(4-(1*H*-indole-2-carbonyl)-3-methylpiperazin-1-yl)-2-oxoacetate (35 mg, 0.099 mmol) and 2-methylpropan-2-amine (21 µL, 0.198 mmol), following method 9 described above, (*R*)-2-(4-(1*H*-indole-2-carbonyl)-3-methylpiperazin-1-yl)-*N*-(*tert*-butyl)-2-oxoacetamide (17.4 mg, 47% yield) was obtained as a solid (97% purity, based on HPLC).
Rt (Method K) 5.24 mins, *m*/*z* 371 [M+H]+
¹H NMR (300 MHz, , CDCl₃) δ 9.31 (br s, 1H), 7.66 (d, J = 8.1 Hz, 1H), 7.43 (d, J = 8.2 Hz, 1H), 7.34 - 7.27 (m, 1H), 7.22 - 7.09 (m, 2H), 6.79 (s, 1H), 5.27 - 4.91 (m, 2H), 4.71 - 4.33 (m, 2H), 3.61 - 3.44 (m, 1H), 3.38 - 3.21 (m, 1H), 3.13 - 2.91 (m, 1H), 1.47 - 1.36 (m, 12H).

### Example 51

### 2-(4-(1H-indole-2-carbonyl)-1,4-diazepan-1-yl)-N-(tert-butyl)-2-oxoacetamide

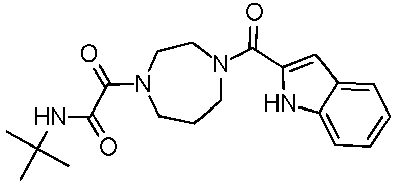

From potassium 2-(4-(1*H*-indole-2-carbonyl)-1,4-diazepan-1-yl)-2-oxoacetate (50 mg, 0.141 mmol) and 2-methylpropan-2-amine (30 µL, 0.283 mmol), following method 9 described above, 2-(4-(1*H*-indole-2-carbonyl)-1,4-diazepan-1-yl)-*N*-(tert-butyl)-2-oxoacetamide (39.4 mg, 75% yield) was obtained as a solid (99% purity, based on HPLC).
Rt (Method K) 5.13 mins, *m*/*z* 371 [M+H]+
¹H NMR (500 MHz, , CDCl₃) δ 9.61 (br s, 1H), 7.70 - 7.60 (m, 1H), 7.43 (ddd, J = 8.3, 1.9, 0.9 Hz, 1H), 7.28 (ddd, J = 8.3, 7.0, 1.1 Hz, 1H), 7.13 (ddd, J = 8.1, 7.0, 1.0 Hz, 2H), 6.83 (s, 1H), 4.33 - 3.62 (m, 8H), 2.34 - 1.81 (m, 2H), 1.37 (s, 12H).

### Synthesis method 10

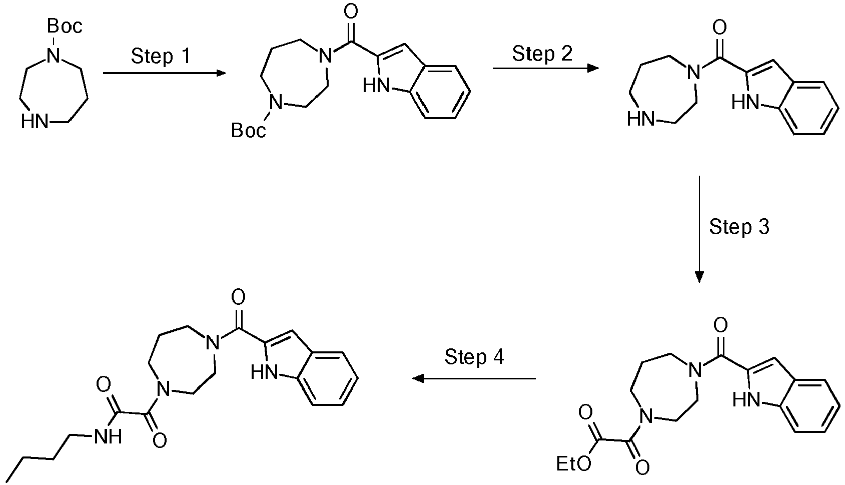

**Step 1:** A solution of 1H-indole-2-carboxylic acid (0.4M, 3.2 mmol) in dry THF (0.4M, 8mL, flowrate 0.33 mL/min) and a solution of CDI in dry THF (0.4M, 8mL, flowrate 0.33 mL/min) were combined in a Y-piece and reacted in a 10 mL (1/8" o.d) PFA reactor at 70°C (15 min residence time). The exiting stream was then immediately delivered in a Y-piece together with an incoming solution of tert-butyl 1,4-diazepane-1-carboxylate in dry THF (0.42M, 8mL, 0.33 mL/min) and reacted in a 20 mL VapourTec rapid mixer (3.2 mm o.d) bore reactor at 70°C. A 40 psi back pressure regulator was placed after the reactor. The output stream was collected after 40 min. The solvent was removed under reduced pressure and the resulting oil partitioned between ethyl acetate and saturated NaHCO₃ aqueous solution. The aqueous layer is extracted 3 times with EtOAc. The ethyl acetate layer was then washed with water and brine and dried over Na₂SO₄. The solvent was evaporated to give the desired product (563 mg, 51 % yield) as a white solid.

**Step** 2: TFA (1 mL, 12.9 mmol) was added to a solution tert-butyl 4-(1H-indole-2-carbonyl)-1,4-diazepane-1-carboxylate (280 mg, 0.81 mmol) in DCM (2 mL). After 2h, the reaction mixture was evaporated and used as such in the next step.

**Step** 3: To a cooled (0-5°C), stirred solution of (1,4-diazepan-1-yl)(1H-indol-2-yl)methanone (198 mg, 0.81 mmol, TFA salt) and triethylamine (0.28 mL, 2.0 mmol) in dry DCM (2 mL) was added ethyl-2-chloro-2-oxoacetate (0.091 mL, 0.81 mmol) in dry DCM (1.5 mL) . The reaction mixture was stirred for 1h, then warmed to room temperature and stirred overnight. The solvent was removed under reduced pressure. The residue was taken up inwater (15mL), extracted with EtOAc (3 x 15 mL) then washed with sat. aq. NH₄Cl (15 mL), sat. aq. NaHCOs (15mL) and sat. aq. NaCl (15 mL), dried over MgSO₄, filtered and concentrated. The desired product was obtained as an off white solid (53 mg, 19 % yield).

**Step 4:** Ethyl 2-(4-(1H-indole-2-carbonyl)-1,4-diazepan-1-yl)-2-oxoacetate in solution in EtOH:DCM(8:2) (0.3M, 1.3 mL, flowrate 0.15 mL/min) and n-butylamine in solution in EtOH:DCM(8:2) (3M, 1.3 mL, flowrate 0.15 mL/min) are mixed in a Y-piece and reacted in a 10 mL (1/8" o.d) PFA reactor at 100°C (35 min residence time) with a back pressure regulator of 175 psi. The output stream was collected after 50 min, evaporated and purified via flash column chromatography to yield the final product as a white solid (32.1 mg, 36 % yield)

### The following examples were prepared following synthesis method 10.

### Example 8

### N-butyl-2-[4-(1H-indole-2-carbonyl)-1,4-diazepan-1-yl]-2-oxoacetamide

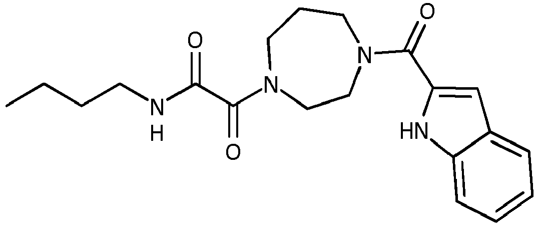

GC analysis: retention time = 15.287min, peak area: 100%, Method L; mass (*m*/*z*): 370.2.

¹H NMR (500 MHz, Chloroform-d) δ 9.28 (d, J = 17.2 Hz, 1H), 7.65 (ddd, J = 8.0, 1.0 Hz, 1H), 7.41 (ddd, J = 8.2, 1.0 Hz, 1H), 7.28 (dddd, J= 8.2, 7.0, 1.1 Hz, 1H), 7.13 (dddd, J = 8.0, 7.0, 1.0 Hz, 1H), 6.83 (s, 1H), 4.34 - 3.73 (m, 7H), 3.68 (t, J = 6.2 Hz, 1H), 3.27 (m, 2H), 2.35 - 1.97 (m, 2H), 1.54 - 1.44 (m, 2H), 1.39 - 1.30 (m, 2H), 0.91 (t, J = 7.4 Hz, 3H).

### Synthesis method 11

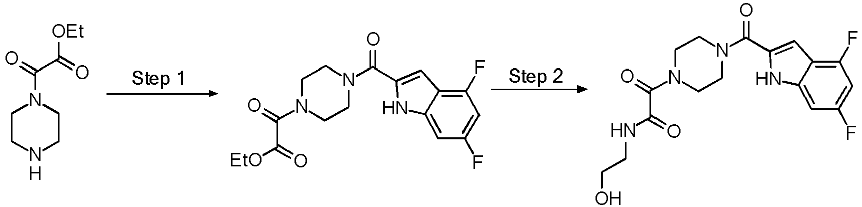

**Step 1:** To a solution of 4,6-difluoro-1H-indole-2-carboxylic acid (1.9 g, 10 mmol) in dry 2-Me-THF (100 mL) was added CDI (g, 10 mmol). The mixture was stirred under nitrogen for 1h at 50 °C. Then, ethyl 2-oxo-2-(piperazin-1-yl)acetate (2.0 g, 11 mmol) was added and the resulting mixture was stirred at 70°C for 15h. The solvent was removed under reduced pressure and the resulting oil partitioned between ethyl acetate and saturated NaHCOs solution. The aqueous layer was extracted thrice with EtOAc. The combined organic extracts were then washed with water and brine and dried over Na₂SO₄. The solvent was evaporated to afford the desired product (3.5 g, 91 % yield) as yellow solid.

**Step 2:** To a solution of 1,3-bis(2-isopropylphenyl)-4,5-dihydro-1H-imidazol-3-ium-2-ide (2.52 mg, 8.1 µmol) (IMes) in dry 2-Me-THF was added ethyl 2-(4-(4,6-difluoro-1H-indole-2-carbonyl)piperazin-1-yl)-2-oxoacetate (60 mg g, 0.16 mmol) and 2-aminoethan-1-ol (11.00 µL, 0.18 mmol) sequentially under N₂. After 6h at 50°C the volatiles were removed under reduced pressure on a rotary evaporator, and the resulting residue was purified by flash column chromatography to afford the desired product as a white solid (62.5 mg, 51 % yield)

### The following examples were prepared following synthesis method 11.

### Example 53

### 2-(4-(4,6-difluoro-1H-indole-2-carbonyl)piperazin-1-yl)-N-((1-hydroxycyclopropyl)methyl)-2-oxoacetamide

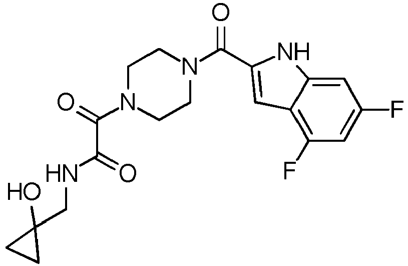

HPLC analysis: retention time = 8.338 min, peak area: 93%, Method L; mass *(m*/*z):* 407.1 [M+H]⁺ 405.0 [M-H]⁻.

¹H NMR (400 MHz, DMSO-d6) δ 8.73 (t, J = 5.8 Hz, 1H), 7.07 - 7.02 (m, 1H), 6.95 (dd, J = 2.3, 0.9 Hz, 1H), 6.91 (td, J = 10.4, 2.1 Hz, 1H), 5.37 (s, 1H), 3.81 (s, 4H), 3.65 - 3.57 (m, 4H), 3.29 (d, J = 5.8 Hz, 2H), 0.58 - 0.49 (m, 4H).

### Example 54

### 2-(4-(4,6-difluoro-1H-indole-2-carbonyl)piperazin-1-yl)-N-(3-hy droxypropyl)-2-oxoacetamide

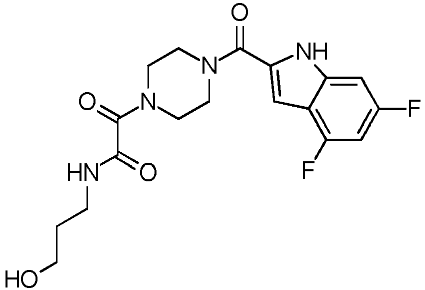

HPLC analysis: retention time = 7.911 min, peak area: 95%, Method L; mass *(m*/*z):* 395.1 [M+H]⁺ 393.0 [M-H]⁻.

¹H NMR (500 MHz, DMSO-d6) δ 8.69 (t, J = 5.7 Hz, 1H), 7.04 (ddd, J = 9.4, 2.1, 0.8 Hz, 1H), 6.95 (d, J = 1.5 Hz, 1H), 6.90 (td, J = 10.4, 2.1 Hz, 1H), 4.46 (t, J = 5.1 Hz, 1H), 3.80 (s, 4H), 3.65 - 3.55 (m, 4H), 3.43 (td, J = 6.3, 5.0 Hz, 2H), 3.24 - 3.16 (m, 2H), 1.61 (p, J = 6.6 Hz, 2H).

### Example 55

### 2-(4-(4,6-difluoro-1H-indole-2-carbonyl)piperazin-1-yl)-N-((1-(hydroxymethyl)cyclopropyl)methyl)-2-oxoacetamide

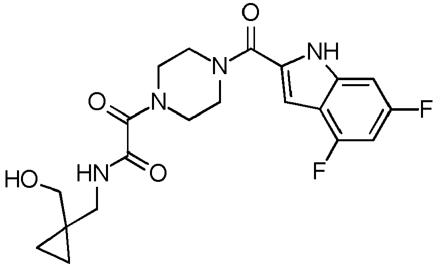

¹H NMR (500 MHz, DMSO-d6) δ 8.68 (t, J = 5.8 Hz, 1H), 7.04 (ddd, J = 9.4, 2.1, 0.9 Hz, 1H), 6.95 (d, J = 0.9 Hz, 1H), 6.90 (td, J = 10.4, 2.1 Hz, 1H), 4.52 (t, J = 5.6 Hz, 1H), 3.80 (s, 3H), 3.59 (dt, J = 7.8, 4.4 Hz, 4H), 3.28 (d, J = 5.6 Hz, 2H), 3.19 (d, J = 5.8 Hz, 2H), 0.47 - 0.32 (m, 4H). HPLC analysis: retention time = 8.430 min, peak area: 95%, Method L; mass *(m*/*z):* 421.1 [M+H]⁺ 419.0 [M-H]⁻.

### Example 56

### 2-(4-(4,6-difluoro-1H-indole-2-carbonyl)piperazin-1-yl)-N-(6-hydroxyhexyl)-2-oxoacetamide

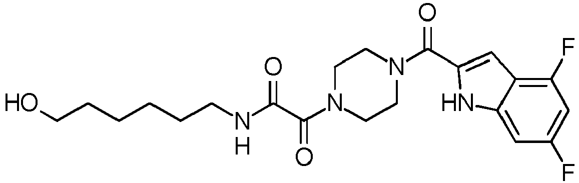

HPLC analysis: retention time = 8.720 min, peak area: 97%, Method L; mass *(m*/*z):* 437.1 [M+H]⁺ 435.0 [M-H]⁻.

¹H NMR (500 MHz, DMSO-d6) δ 8.71 (t, J = 5.7 Hz, 1H), 7.04 (ddd, J = 9.3, 2.1, 0.9 Hz, 1H), 6.95 (dd, J = 2.3, 0.9 Hz, 1H), 6.90 (td, J = 10.4, 2.1 Hz, 1H), 4.32 (t, J = 5.2 Hz, 1H), 3.79 (s, 4H), 3.59 (q, J = 5.8 Hz, 4H), 3.38 (td, J = 6.5, 5.1 Hz, 2H), 3.12 (q, J = 6.7 Hz, 2H), 1.42 (m, 3H), 1.31 - 1.24 (m, 5H).

### Example 57

### 2-(4-(4,6-difluoro-1H-indole-2-carbonyl)piperazin-1-yl)-N-(5-hydroxypentyl)-2-oxoacetamide

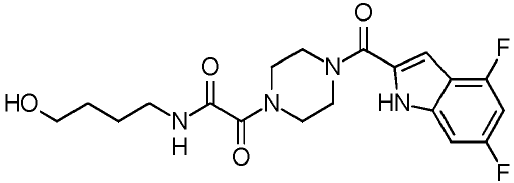

HPLC analysis: retention time = 8.389 min, peak area: 99%, Method L; mass *(m*/*z):* 423.1 [M+H]⁺ 421.0 [M-H]⁻
¹H NMR (500 MHz, DMSO-d6) δ 8.71 (t, J = 5.8 Hz, 1H), 7.04 (ddd, J = 9.4, 2.2, 0.8 Hz, 1H), 6.95 (d, J = 0.9 Hz, 1H), 6.89 (td, J = 10.4, 2.1 Hz, 1H), 4.35 (t, J = 5.1 Hz, 1H), 3.90 - 3.70 (m, 4H), 3.63 - 3.56 (m, 4H), 3.38 (td, J = 6.5, 5.1 Hz, 2H), 3.13 (td, J = 7.0, 5.7 Hz, 2H), 1.50 - 1.38 (m, 4H), 1.34 - 1.25 (m, 2H).

### Example 58

### (S)-2-(4-(4,6-difluoro-1H-indole-2-carbonyl)piperazin-1-yl)-N-(2-hydroxypropyl)-2-oxoacetamide

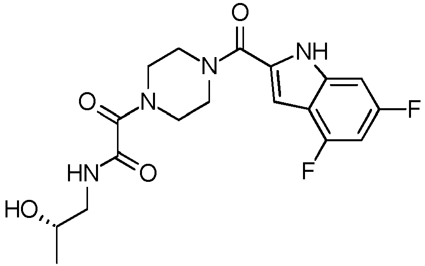

¹H NMR (500 MHz, DMSO-d6) δ 8.64 (t, J = 5.9 Hz, 1H), 7.04 (ddd, J = 9.4, 2.1, 0.8 Hz, 1H), 6.95 (s, 1H), 6.90 (td, J = 10.4, 2.1 Hz, 1H), 4.73 (d, J = 4.9 Hz, 1H), 3.80 (s, 4H), 3.71 (qd, J = 6.2, 4.9 Hz, 1H), 3.60 (dt, J = 7.2, 3.4 Hz, 4H), 3.09 (t, J = 6.0 Hz, 2H), 1.04 (d, J = 6.2 Hz, 3H).

HPLC analysis: retention time = 8.098 min, peak area: 97%, Method L; mass *(m*/*z):* 395.1 [M+H]⁺ 393.0 [M-H]⁻.

### Example 59

### 2-(4-(4,6-difluoro-1H-indole-2-carbonyl)piperazin-1-yl)-N-(2-ethyl-4-hydroxybutyl)-2-oxoacetamide

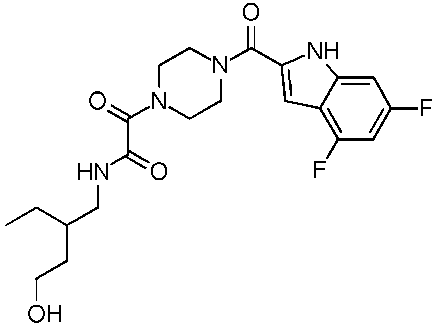

HPLC analysis: retention time = 8.923 min, peak area: 99%, Method L; mass *(m*/*z):* 437.1 [M+H]⁺ 435.0 [M-H]⁻.

¹H NMR (400 MHz, DMSO-d6) δ 8.65 (t, J = 5.9 Hz, 1H), 7.04 (ddd, J = 9.4, 2.1, 0.8 Hz, 1H), 6.97 - 6.94 (m, 1H), 6.90 (td, J = 10.4, 2.1 Hz, 1H), 4.38 (t, J = 5.0 Hz, 1H), 3.80 (s, 4H), 3.63 - 3.54 (m, 4H), 3.43 (tdt, J = 7.0, 5.1, 3.4 Hz, 2H), 3.17 - 3.05 (m, 2H), 1.59 (p, J = 6.3 Hz, 1H), 1.45 - 1.22 (m, 4H), 0.85 (t, J = 7.4 Hz, 3H).

### Example 60

### 2-(4-(4,6-difluoro-1H-indole-2-carbonyl)piperazin-1-yl)-N-(4-hydroxypentyl)-2-oxoacetamide

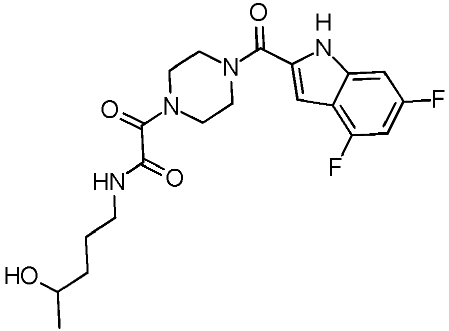

HPLC analysis: retention time = 8.455 min, peak area: 99%, Method L; mass *(m*/*z):* 423.1 [M+H]⁺ 421.0 [M-H]⁻.

¹H NMR (400 MHz, DMSO-d6) δ 8.72 (t, J = 5.8 Hz, 1H), 7.05 (ddd, J = 9.4, 2.1, 0.8 Hz, 1H), 6.95 (d, J = 0.9 Hz, 1H), 6.91 (td, J = 10.4, 2.1 Hz, 1H), 4.38 (d, J = 4.7 Hz, 1H), 3.80 (s, 4H), 3.66 - 3.53 (m, 5H), 3.13 (q, J = 6.9 Hz, 2H), 1.61 - 1.37 (m, 2H), 1.36 - 1.27 (m, 2H), 1.04 (d, J = 6.1 Hz, 3H).

### Example 61

### 2-(4-(4,6-difluoro-1H-indole-2-carbonyl)piperazin-1-yl)-N-(4-hydroxy-2,2-dimethylbutyl)-2-oxoacetamide

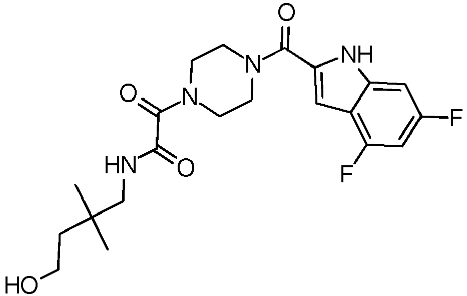

HPLC analysis: retention time = 8.878 min, peak area: 99%, Method L; mass *(m*/*z):* 437.1 [M+H]⁺ 435.0 [M-H]⁻.

¹H NMR (500 MHz, DMSO-d6) δ 8.58 (t, J = 6.3 Hz, 1H), 7.04 (ddd, J = 9.3, 2.1, 0.8 Hz, 1H), 6.95 (d, J = 0.9 Hz, 1H), 6.90 (td, J = 10.4, 2.1 Hz, 1H), 4.35 (t, J = 4.9 Hz, 1H), 3.81 (s, 4H), 3.66 - 3.52 (m, 4H), 3.47 (td, J = 7.2, 4.8 Hz, 2H), 3.01 (d, J = 6.4 Hz, 2H), 1.39 (t, J = 7.2 Hz, 2H), 0.86 (s, 6H).

### Example 62

### 2-(4-(4,6-difluoro-1H-indole-2-carbonyl)piperazin-1-yl)-N-(4-hydroxybutyl)-2-oxoacetamide

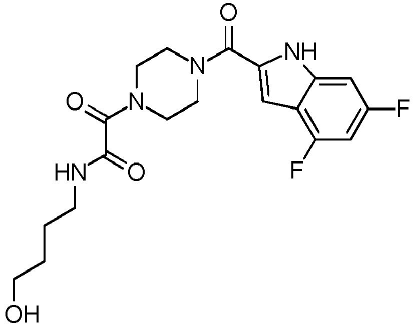

HPLC analysis: retention time = 8.134 min, peak area: 100%, Method L; mass *(m*/*z):* 395.1 [M+H]⁺ 393.0 [M-H]⁻.

¹H NMR (500 MHz, DMSO-d6) δ 4.40 (t, J = 5.1 Hz, 1H), 3.80 (s, 4H), 3.65 - 3.55 (m, 4H), 3.40 (td, J= 6.3, 5.0 Hz, 2H), 3.14 (q, J = 6.8 Hz, 2H), 1.51 - 1.38 (m, 4H).

### Example 63

### 2-(4-(4,6-difluoro-1H-indole-2-carbonyl)piperazin-1-yl)-N-(2-methoxypropyl)-2-oxoacetamide

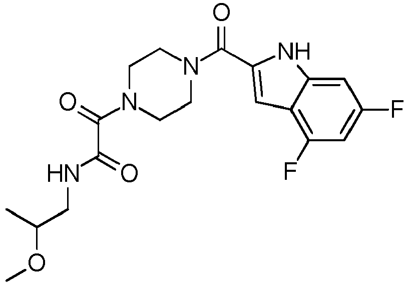

1H NMR (400 MHz, Chloroform-d) δ 9.54 (s, 1H), 7.57 (s, 1H), 6.95 - 6.89 (m, 1H), 6.83 (dd, J = 2.3, 0.9 Hz, 1H), 6.65 (td, J = 10.0, 2.0 Hz, 1H), 4.34 - 4.26 (m, 2H), 4.01 (s, 4H), 3.84 - 3.76 (m, 2H), 3.62 - 3.44 (m, 2H), 3.36 (s, 3H), 3.18 (ddd, J = 13.4, 6.9, 5.1 Hz, 1H), 1.17 (d, J = 6.1 Hz, 3H).HPLC analysis: retention time = 8.725 min, peak area: 97%, Method L; mass (*m*/*z*): 409.1 [M+H]⁺ 407.0 [M-H]⁻.

### Example 64

### (R)-2-(4-(4,6-difluoro-1H-indole-2-carbonyl)piperazin-1-yl)-N-(2-hydroxypropyl)-2-oxoacetamide

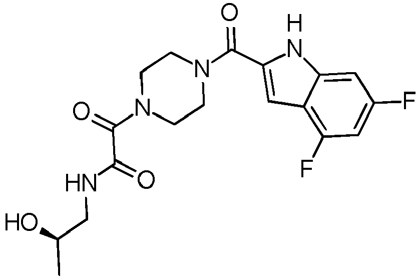

HPLC analysis: retention time = 8.134 min, peak area: 95%, Method L; mass (*m*/*z*): 395.1 [M+H]⁺ 393.0 [M-H]⁻.

¹H NMR (400 MHz, DMSO-d6) δ 8.65 (t, J = 5.9 Hz, 1H), 7.04 (ddd, J = 9.4, 2.1, 0.8 Hz, 1H), 6.95 (d, J = 0.9 Hz, 1H), 6.90 (td, J = 10.4, 2.1 Hz, 1H), 4.74 (d, J = 4.8 Hz, 1H), 3.80 (s, 4H), 3.71 (qd, J = 6.1, 4.9 Hz, 1H), 3.65 - 3.53 (m, 4H), 3.09 (t, J = 6.0 Hz, 2H), 1.04 (d, J = 6.2 Hz, 3H).

### Example 65

### 2-(4-(4,6-difluoro-1H-indole-2-carbonyl)piperazin-1-yl)-N-((1-methoxycyclopropyl)methyl)-2-oxoacetamide

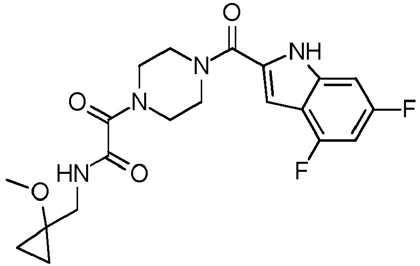

HPLC analysis: retention time = 8.923 min, peak area: 97%, Method L; mass (*m*/*z*): 421.1 [M+H]⁺ 419.0 [M-H]⁻.

¹H NMR (500 MHz, DMSO) δ 8.85 (t, J = 6.0 Hz, 1H), 7.04 (ddd, J = 9.3, 2.1, 0.8 Hz, 1H), 6.95 (dd, J = 2.2, 0.9 Hz, 1H), 6.89 (td, J = 10.4, 2.1 Hz, 1H), 3.80 (s, 4H), 3.60 (dt, J = 7.2, 3.7 Hz, 4H), 3.37 (d, J = 6.0 Hz, 2H), 3.22 (d, J = 1.0 Hz, 3H), 1.53 - 1.44 (m, 1H), 0.71 - 0.65 (m, 2H), 0.60 - 0.55 (m, 2H).

### Example 66

### 2-[4-(1H-indole-2-carbonyl)piperazin-1-yl]-N-(1-methylcycloheptyl)-2-oxoacetamide

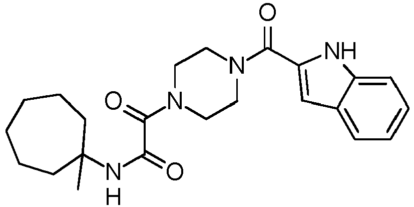

LCMS (ESI): [M+H]⁺ *m*/*z:* calcd. 411.27; found 411.2; Rt = 2.662 min.

### Example 67

### 2-[4-(1H-indole-2-carbonyl)piperazin-1-yl]-N-(2-methylbutan-2-yl)-2-oxoacetamide

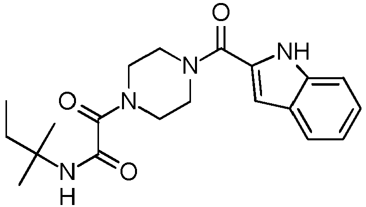

LCMS (ESI): [M+H]⁺ *m*/*z:* calcd. 371.23; found 371.2; Rt = 2.539 min.

### Example 68

### N-(5-hydroxy-2-methylpentan-2-yl)-2-[4-(1H-indole-2-carbonyl)piperazin-1-yl]-2-oxoacetamide

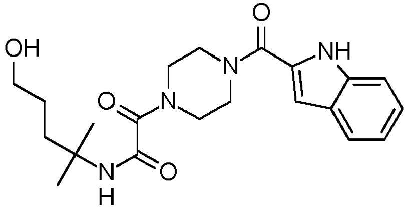

LCMS (ESI): [M+H]⁺ *m*/*z:* calcd. 401.24; found 401.0; Rt = 2.216 min.

### Example 69

### 2-[4-(1H-indole-2-carbonyl)piperazin-1-yl]-2-oxo-N-(4,4,4-trifluoro-2-methylbutan-2-yl)acetamide

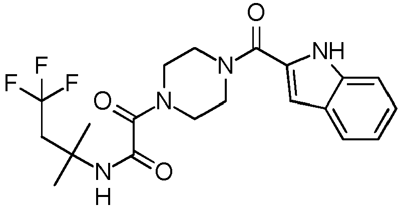

LCMS (ESI): [M+H]⁺ *m*/*z:* calcd. 425.2; found 425.0; Rt = 2.925 min.

### Example 70

### N-cycloheptyl-2-[4-(1H-indole-2-carbonyl)piperazin-1-yl]-2-oxoacetamide

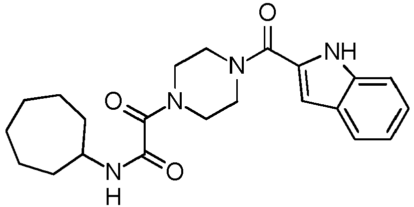

LCMS (ESI): [M+H]⁺ *m*/*z:* calcd. 397.25; found 397.4; Rt = 3.129 min.

Selected compounds of the invention were assayed in capsid assembly and HBV replication assays, as described below and a representative group of these active compounds is shown in Table 1 (capsid assembly assay) and Table 2 (HBV replication assay).

### Biochemical capsid assembly assay

The screening for assembly effector activity was done based on a fluorescence quenching assay published by Zlotnick et al. (2007). The C-terminal truncated core protein containing 149 amino acids of the N-terminal assembly domain fused to a unique cysteine residue at position 150 and was expressed in *E. coli* using the pET expression system (Merck Chemicals, Darmstadt). Purification of core dimer protein was performed using a sequence of size exclusion chromatography steps. In brief, the cell pellet from 1 L BL21 (DE3) Rosetta2 culture expressing the coding sequence of core protein cloned NdeI/ XhoI into expression plasmid pET21b was treated for 1 h on ice with a native lysis buffer (Qproteome Bacterial Protein Prep Kit; Qiagen, Hilden). After a centrifugation step the supernatant was precipitated during 2 h stirring on ice with 0.23 g/ml of solid ammonium sulfate. Following further centrifugation the resulting pellet was resolved in buffer A (100mM Tris, pH 7.5; 100mM NaCl; 2mM DTT) and was subsequently loaded onto a buffer A equilibrated CaptoCore 700 column (GE HealthCare, Frankfurt). The column flow through containing the assembled HBV capsid was dialyzed against buffer N (50mM NaHCO3 pH 9.6; 5mM DTT) before urea was added to a final concentration of 3M to dissociate the capsid into core dimers for 1.5 h on ice. The protein solution was then loaded onto a 1L Sephacryl S300 column. After elution with buffer N core dimer containing fractions were identified by SDS-PAGE and subsequently pooled and dialyzed against 50mM HEPES pH 7.5; 5mM DTT. To improve the assembly capacity of the purified core dimers a second round of assembly and disassembly starting with the addition of 5 M NaCl and including the size exclusion chromatography steps described above was performed. From the last chromatography step core dimer containing fractions were pooled and stored in aliquots at concentrations between 1.5 to 2.0 mg/ml at -80°C.

Immediately before labelling the core protein was reduced by adding freshly prepared DTT in a final concentration of 20 mM. After 40 min incubation on ice storage buffer and DTT was removed using a Sephadex G-25 column (GE HealthCare, Frankfurt) and 50 mM HEPES, pH 7.5. For labelling 1.6 mg/ml core protein was incubated at 4°C and darkness overnight with BODIPY-FL maleimide (Invitrogen, Karlsruhe) in a final concentration of 1 mM. After labelling the free dye was removed by an additional desalting step using a Sephadex G-25 column. Labelled core dimers were stored in aliquots at 4°C. In the dimeric state the fluorescence signal of the labelled core protein is high and is quenched during the assembly of the core dimers to high molecular capsid structures. The screening assay was performed in black 384 well microtiter plates in a total assay volume of 10 µl using 50 mM HEPES pH 7.5 and 1.0 to 2.0 µM labelled core protein. Each screening compound was added in 8 different concentrations using a 0.5 log-unit serial dilution starting at a final concentration of 100 µM, 31.6 µM or 10 µM, In any case the DMSO concentration over the entire microtiter plate was 0.5%. The assembly reaction was started by the injection of NaCl to a final concentration of 300 µM which induces the assembly process to approximately 25% of the maximal quenched signal. 6 min after starting the reaction the fluorescence signal was measured using a Clariostar plate reader (BMG Labtech, Ortenberg) with an excitation of 477 nm and an emission of 525 nm. As 100% and 0% assembly control HEPES buffer containing 2.5 M and 0 M NaCl was used. Experiments were performed thrice in triplicates. EC₅₀ values were calculated by non-linear regression analysis using the Graph Pad Prism 6 software (GraphPad Software, La Jolla, USA).

### Determination of HBV DNA from the supernatants of HepAD38 cells

The anti-HBV activity was analysed in the stable transfected cell line HepAD38, which has been described to secrete high levels of HBV virion particles (Ladner et al., 1997). In brief, HepAD38 cells were cultured at 37°C at 5% CO₂ and 95% humidity in 200 µl maintenance medium, which was Dulbecco's modified Eagle's medium/ Nutrient Mixture F-12 (Gibco, Karlsruhe), 10% fetal bovine serum (PAN Biotech Aidenbach) supplemented with 50 µg/ml penicillin/streptomycin (Gibco, Karlsruhe), 2 mM L-glutamine (PAN Biotech, Aidenbach), 400 µg/ml G418 (AppliChem, Darmstadt) and 0.3 µg/ml tetracycline. Cells were subcultured once a week in a 1:5 ratio, but were usually not passaged more than ten times. For the assay 60,000 cells were seeded in maintenance medium without any tetracycline into each well of a 96-well plate and treated with serial half-log dilutions of test compound. To minimize edge effects the outer 36 wells of the plate were not used but were filled with assay medium. On each assay plate six wells for the virus control (untreated HepAD38 cells) and six wells for the cell control (HepAD38 cells treated with 0.3 µg/ml tetracycline) were allocated, respectively. In addition, one plate set with reference inhibitors like BAY 41-4109, entecavir, and lamivudine instead of screening compounds were prepared in each experiment. In general, experiments were performed thrice in triplicates. At day 6 HBV DNA from 100 µl filtrated cell culture supernatant (AcroPrep Advance 96 Filter Plate, 0.45 µM Supor membran, PALL GmbH, Dreieich) was automatically purified on the MagNa Pure LC instrument using the MagNA Pure 96 DNA and Viral NA Small Volume Kit (Roche Diagnostics, Mannheim) according to the instructions of the manufacturer. EC50 values were calculated from relative copy numbers of HBV DNA In brief, 5 µl of the 100 µl eluate containing HBV DNA were subjected to PCR LC480 Probes Master Kit (Roche) together with 1 µM antisense primer tgcagaggtgaagcgaagtgcaca, 0.5 µM sense primer gacgtcctttgtttacgtcccgtc, 0.3 µM hybprobes acggggcgcacctctctttacgcgg-FL and LC640-ctccccgtctgtgccttctcatctgc-PH (TIBMolBiol, Berlin) to a final volume of 12.5 µl. The PCR was performed on the Light Cycler 480 real time system (Roche Diagnostics, Mannheim) using the following protocol: Pre-incubation for 1 min at 95°C, amplification: 40 cycles x (10 sec at 95°C, 50 sec at 60°C, 1 sec at 70°C), cooling for 10 sec at 40°C. Viral load was quantitated against known standards using HBV plasmid DNA of pCH-9/3091 (Nassal et al., 1990, Cell 63: 1357-1363) and the LightCycler 480 SW 1.5 software (Roche Diagnostics, Mannheim) and EC₅₀ values were calculated using non-linear regression with GraphPad Prism 6 (GraphPad Software Inc., La Jolla, USA).

### Cell Viability Assay

Using the AlamarBlue viability assay cytotoxicity was evaluated in HepAD38 cells in the presence of 0.3 µg/ml tetracycline, which blocks the expression of the HBV genome. Assay condition and plate layout were in analogy to the anti-HBV assay, however other controls were used. On each assay plate six wells containing untreated HepAD38 cells were used as the 100% viability control, and six wells filled with assay medium only were used as 0% viability control. In addition, a geometric concentration series of cycloheximide starting at 60 µM final assay concentration was used as positive control in each experiment. After six days incubation period Alamar Blue Presto cell viability reagent (ThermoFisher, Dreieich) was added in 1/11 dilution to each well of the assay plate. After an incubation for 30 to 45 min at 37°C the fluorescence signal, which is proportional to the number of living cells, was read using a Tecan Spectrafluor Plus plate reader with an excitation filter 550 nm and emission filter 595 nm, respectively. Data were normalized into percentages of the untreated control (100% viability) and assay medium (0% viability) before CC50 values were calculated using non-linear regression and the GraphPad Prism 6.0 (GraphPad Software, La Jolla, USA). Mean EC₅₀ and CC₅₀ values were used to calculate the selectivity index (SI = CC₅₀/EC₅₀) for each test compound.

### In vivo efficacy models

HBV research and preclinical testing of antiviral agents are limited by the narrow species- and tissue-tropism of the virus, the paucity of infection models available and the restrictions imposed by the use of chimpanzees, the only animals fully susceptible to HBV infection. Alternative animal models are based on the use of HBV-related hepadnaviruses and various antiviral compounds have been tested in woodchuck hepatitis virus (WHV) infected woodchucks or in duck hepatitis B virus (DHBV) infected ducks or in woolly monkey HBV (WM-HBV) infected tupaia (overview in Dandri et al., 2017, Best Pract Res Clin Gastroenterol 31, 273-279). However, the use of surrogate viruses has several limitations. For example is the sequence homology between the most distantly related DHBV and HBV is only about 40% and that is why core protein assembly modifiers of the HAP family appeared inactive on DHBV and WHV but efficiently suppressed HBV (Campagna et al., 2013, J. Virol. 87, 6931-6942). Mice are not HBV permissive but major efforts have focused on the development of mouse models of HBV replication and infection, such as the generation of mice transgenic for the human HBV (HBV tg mice), the hydrodynamic injection (HDI) of HBV genomes in mice or the generation of mice having humanized livers and/ or humanized immune systems and the intravenous injection of viral vectors based on adenoviruses containing HBV genomes (Ad-HBV) or the adenoassociated virus (AAV-HBV) into immune competent mice (overview in Dandri et al., 2017, Best Pract Res Clin Gastroenterol 31, 273-279).. Using mice transgenic for the full HBV genome the ability of murine hepatocytes to produce infectious HBV virions could be demonstrated (Guidotti et al., 1995, J. Virol., 69: 6158-6169). Since transgenic mice are immunological tolerant to viral proteins and no liver injury was observed in HBV-producing mice, these studies demonstrated that HBV itself is not cytopathic. HBV transgenic mice have been employed to test the efficacy of several anti-HBV agents like the polymerase inhibitors and core protein assembly modifiers (Weber et al., 2002, Antiviral Research 54 69-78; Julander et al., 2003, Antivir. Res., 59: 155-161), thus proving that HBV transgenic mice are well suitable for many type of preclinical antiviral testing *in vivo.*

As described in Paulsen et al., 2015, PLOSone, 10: e0144383 HBV-transgenic mice (Tg [HBV1.3 fsX⁻3'5']) carrying a frameshift mutation (GC) at position 2916/2917 could be used to demonstrate antiviral activity of core protein assembly modifiers *in vivo.* In brief, The HBV-transgenic mice were checked for HBV-specific DNA in the serum by qPCR prior to the experiments (see section "Determination of HBV DNA from the supernatants of HepAD38 cells"). Each treatment group consisted of five male and five female animals approximately 10 weeks age with a titer of 10⁷-10⁸ virions per ml serum. Compounds were formulated as a suspension in a suitable vehicle such as 2% DMSO / 98% tylose (0.5% Methylcellulose / 99.5% PBS) or 50% PEG400 and administered per os to the animals one to three times/day for a 10 day period. The vehicle served as negative control, whereas 1 µg/kg entecavir in a suitable vehicle was the positive control. Blood was obtained by retro bulbar blood sampling using an Isoflurane Vaporizer. For collection of terminal heart puncture six hours after the last treatment blood or organs, mice were anaesthetized with isoflurane and subsequently sacrificed by CO₂ exposure. Retro bulbar (100-150 µl) and heart puncture (400-500 µl) blood samples were collected into a Microvette 300 LH or Microvette 500 LH, respectively, followed by separation of plasma via centrifugation (10 min, 2000g, 4°C). Liver tissue was taken and snap frozen in liquid N₂. All samples were stored at -80°C until further use. Viral DNA was extracted from 50 µl plasma or 25 mg liver tissue and eluted in 50 µl AE buffer (plasma) using the DNeasy 96 Blood & Tissue Kit (Qiagen, Hilden) or 320 µl AE buffer (liver tissue) using the DNeasy Tissue Kit (Qiagen, Hilden) according to the manufacturer's instructions. Eluted viral DNA was subjected to qPCR using the LightCycler 480 Probes Master PCR kit (Roche, Mannheim) according to the manufacturer's instructions to determine the HBV copy number. HBV specific primers used included the forward primer 5'-CTG TAC CAA ACC TTC GGA CGG-3', the reverse primer 5'-AGG AGA AAC GGG CTG AGG C-3' and the FAM labelled probe FAM-CCA TCA TCC TGG GCT TTC GGA AAA TT-BBQ. One PCR reaction sample with a total volume of 20 µl contained 5 µl DNA eluate and 15 µl master mix (comprising 0.3µM of the forward primer, 0.3µM of the reverse primer, 0.15µM of the FAM labelled probe). qPCR was carried out on the Roche LightCycler1480 using the following protocol: Pre-incubation for 1 min at 95°C, amplification: (10 sec at 95°C, 50 sec at 60°C, 1 sec at 70°C) x 45 cycles, cooling for 10 sec at 40°C. Standard curves were generated as described above. All samples were tested in duplicate. The detection limit of the assay is ~50 HBV DNA copies (using standards ranging from 250-2.5 x 107 copy numbers). Results are expressed as HBV DNA copies / 10µl plasma or HBV DNA copies / 100ng total liver DNA (normalized to negative control).

It has been shown in multiple studies that not only transgenic mice are a suitable model to prove the antiviral activity of new chemical entities *in vivo* the use of hydrodynamic injection of HBV genomes in mice as well as the use of immune deficient human liver chimeric mice infected with HBV positive patient serum have also frequently used to profile drugs targeting HBV (Li et al., 2016, Hepat. Mon. 16: e34420; Qiu et al., 2016, J. Med. Chem. 59: 7651-7666; Lutgehetmann et al., 2011, Gastroenterology, 140: 2074-2083). In addition chronic HBV infection has also been successfully established in immunecompetent mice by inoculating low doses of adenovirus- (Huang et al., 2012, Gastroenterology 142: 1447-1450) or adeno-associated virus (AAV) vectors containing the HBV genome (Dion et al., 2013, J Virol. 87: 5554-5563). This model could also be used to demonstrate the in vivo antiviral activity of novel anti-HBV agents.

**Table 1: Capsid assembly assay**

| In Table 1, "A" represents an IC₅₀ < 5 µM; "B" represents 5 µM < IC₅₀ < 10 µM; "C" represents IC₅₀ < 100 µM | |
|---|---|
| **Example** | **Assembly Activity** |
| Example 2 | B |
| Example 3 | C |
| Example 4 | C |
| Example 5 | A |
| Example 6 | A |
| Example 7 | B |
| Example 8 | C |
| Example 9 | A |
| Example 10 | A |
| Example 11 | A |
| Example 12 | A |
| Example 13 | B |
| Example 14 | C |
| Example 15 | A |
| Example 16 | B |
| Example 17 | B |
| Example 18 | C |
| Example 19 | C |
| Example 20 | C |
| Example 21 | B |
| Example 22 | C |
| Example 23 | A |
| Example 24 | A |
| Example 25 | A |
| Example 26 | A |
| Example 27 | A |
| Example 28 | A |
| Example 29 | A |
| Example 30 | A |
| Example 31 | B |
| Example 32 | A |
| Example 33 | C |
| Example 24 | C |
| Example 35 | B |
| Example 36 | A |
| Example 37 | A |
| Example 38 | A |
| Example 39 | A |
| Example 40 | B |
| Example 41 | A |
| Example 42 | B |
| Example 43 | A |
| Example 44 | A |
| Example 45 | B |
| Example 46 | B |
| Example 48 | A |
| Example 49 | C |
| Example 50 | A |
| Example 51 | C |
| Example 52 | A |
| Example 53 | A |
| Example 54 | A |
| Example 55 | A |
| Example 56 | A |
| Example 57 | A |
| Example 58 | A |
| Example 59 | A |
| Example 60 | A |
| Example 61 | A |
| Example 62 | A |
| Example 63 | A |
| Example 64 | A |
| Example 65 | A |
| Example 66 | B |
| Example 67 | A |
| Example 68 | A |
| Example 69 | A |
| Example 70 | B |

**Table 2: HBV Replication assay**

| In Table 1, "+++" represents an EC₅₀ < 1 µM; "++" represents 1 µM < EC₅₀ < 10 µM; "+" represents EC₅₀ < 100 µM | | |
|---|---|---|
| **Example** | **CC₅₀ (µM)** | **Cell Activity** |
| Example 1 | | ++ |
| Example 2 | > 100 | ++ |
| Example 3 | > 100 | ++ |
| Example 4 | > 100 | ++ |
| Example 5 | > 100 | +++ |
| Example 6 | > 100 | +++ |
| Example 7 | > 100 | +++ |
| Example 8 | > 100 | +++ |
| Example 9 | > 100 | +++ |
| Example 10 | > 100 | +++ |
| Example 11 | > 100 | ++ |
| Example 12 | > 100 | +++ |
| Example 13 | > 100 | +++ |
| Example 23 | > 100 | +++ |
| Example 24 | > 100 | +++ |
| Example 25 | > 100 | +++ |
| Example 26 | > 100 | +++ |
| Example 28 | > 100 | +++ |
| Example 29 | > 100 | ++ |
| Example 30 | > 100 | +++ |
| Example 31 | > 100 | +++ |
| Example 32 | > 100 | +++ |
| Example 24 | > 100 | +++ |
| Example 35 | > 100 | +++ |
| Example 52 | > 100 | +++ |
| Example 53 | > 100 | +++ |
| Example 54 | > 100 | +++ |
| Example 55 | > 100 | +++ |
| Example 56 | > 100 | ++ |
| Example 57 | > 100 | ++ |
| Example 58 | > 100 | +++ |
| Example 59 | > 100 | +++ |
| Example 60 | > 100 | +++ |
| Example 61 | > 100 | +++ |
| Example 62 | > 100 | +++ |
| Example 63 | > 100 | +++ |
| Example 64 | > 100 | +++ |
| Example 65 | > 100 | +++ |

The project leading to this application has received funding from the European Union's Horizon 2020 research and innovation programme under the Marie Sklodowska-Curie grant agreement No [766058].

## Claims

1. Compound of Formula I 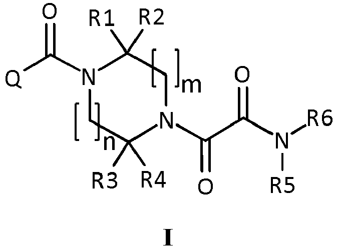 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-C5-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C 1-C6-alkyl-O-C 1-C6-alkyl, C(=O)NH₂, C(=O)N(H)CH₃ and carboxy
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
wherein heteroaryl and heterocycloalkyl each has 1 or 2 heteroatoms each independently selected from N, O and S,
or a pharmaceutically acceptable salt thereof or a solvate of a compound of Formula I or the pharmaceutically acceptable salt thereof or a prodrug of a compound of Formula I or a pharmaceutically acceptable salt or a solvate thereof for use in the prevention or treatment of an HBV infection in a subject,
wherein the prodrug is selected from the group consisting of esters and amides.

2. A compound of Formula I for use in the prevention or treatment of an HBV infection in a subject according to claim 1 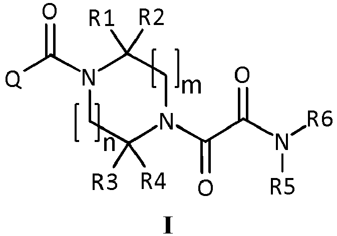 in which
- R1, R2, R3, and R4 are for each position independently selected from the group comprising H, D, and C1-C6-alkyl
- R5 and R6 are independently selected from the group comprising H, C6-aryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1 -C2-alkyl-C3 -C5-cycloalkyl, C1-C2-alkyl-C3-CS-heteroaryl, and C1-C2-alkyl-C3-C5-heterocycloalkyl optionally substituted with 1, 2, or 3 groups each independently selected from OH, C3-C7-heterocycloalkyl, C1-C6-alkyl, C1-C6-hydroxyalkyl, C1-C4-alkoxy, C1-C6-alkyl-O-C1-C6-alkyl, C(=O)NH₂, and C(=O)N(H)CH₃
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
wherein heteroaryl and heterocycloalkyl each has 1 or 2 heteroatoms each independently selected from N, O and S,
or a pharmaceutically acceptable salt thereof or a solvate of a compound of Formula I or the pharmaceutically acceptable salt thereof or a prodrug of a compound of Formula I or a pharmaceutically acceptable salt or a solvate thereof,
wherein the prodrug is selected from the group consisting of esters and amides.

3. A compound of Formula I for use in the prevention or treatment of an HBV infection in a subject according to claim 1 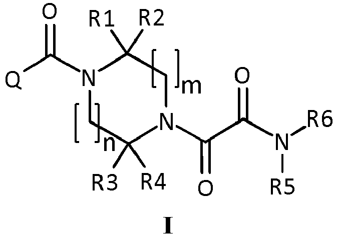 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R5 and R6 are optionally connected to form a C4-C8-heterocyclyl ring
- n is 0, 1, or 2
- m is 0, 1, or 2
- Q is indol-2-yl, optionally substituted with 1, 2, 3, or 4 groups independently selected from H, D, F, Cl, Br, I, CF₃, CF₂H, C1-C4-alkyl, CF₂CH₃, cyclopropyl, cyano, and nitro; or
indolizin-2-yl, optionally substituted with 1, 2, 3, 4, 5 or 6 groups independently selected from H, D, F, Cl, Br, I, CF₃, CF₂H, C1-C4-alkyl, CF₂CH₃, cyclopropyl, cyano, C2-C5-alkenyl, and nitro,
wherein heteroaryl and heterocycloalkyl each has 1 or 2 heteroatoms each independently selected from N, O and S,
or a pharmaceutically acceptable salt thereof or a solvate of a compound of Formula I or the pharmaceutically acceptable salt thereof or a prodrug of a compound of Formula I or a pharmaceutically acceptable salt or a solvate thereof,
wherein the prodrug is selected from the group consisting of esters and amides.

4. A compound of Formula I that is a compound of Formula II for use in the prevention or treatment of an HBV infection in a subject according to claim 1 or 3 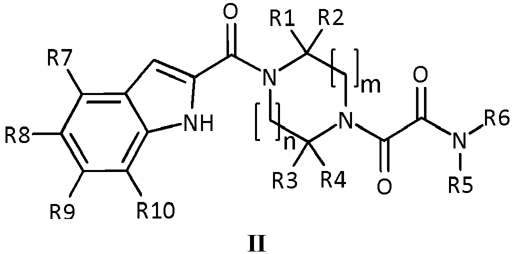 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C 1-C2-alkyl-C3-C5-heteroaryl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C 1-C6-alkyl-O-C 1-C6-alkyl, C(=O)NH₂, C(=O)N(H)CH3 and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula II or the pharmaceutically acceptable salt thereof or a prodrug of a compound of Formula II or a pharmaceutically acceptable salt or a solvate thereof,
wherein the prodrug is selected from the group consisting of esters and amides.

5. A compound of Formula I that is a compound of Formula II for use in the prevention or treatment of an HBV infection in a subject according to any of claims 1, 3 or 4 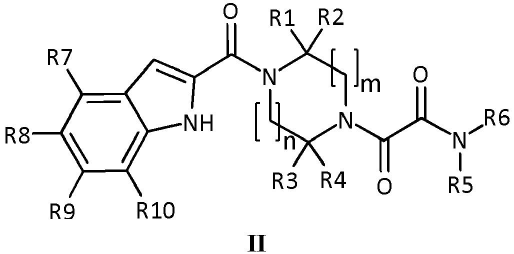 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-C5-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH3 and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula II or the pharmaceutically acceptable salt thereof or a prodrug of a compound of Formula II or a pharmaceutically acceptable salt or a solvate thereof,
wherein the prodrug is selected from the group consisting of esters and amides.

6. A compound of Formula I that is a compound of Formula IIa for use in the prevention or treatment of an HBV infection in a subject according to any of claims 1 or 3 to 5 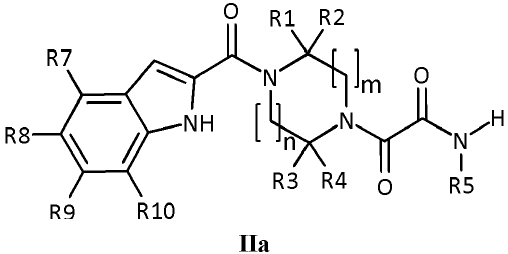 in which
- R1, R2, R3, and R4 are for each position independently selected from the group comprising H, D, and C1-C6-alkyl
- R5 is selected from the group comprising H, C6-aryl, C3-C5-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-C5-heteroaryl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula IIa or the pharmaceutically acceptable salt thereof or a prodrug of a compound of Formula IIa or a pharmaceutically acceptable salt or a solvate thereof,
wherein the prodrug is selected from the group consisting of esters and amides.

7. A compound of Formula I that is a compound of Formula IIa for use in the prevention or treatment of an HBV infection in a subject according to any of claims 1 or 3 to 6 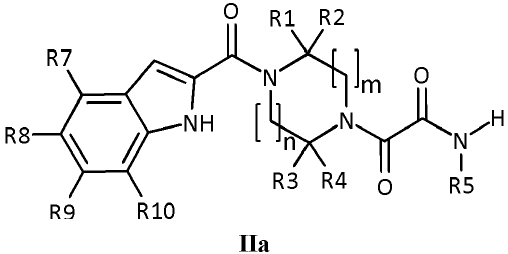 in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula IIa or the pharmaceutically acceptable salt thereof or a prodrug of a compound of Formula IIa or a pharmaceutically acceptable salt or a solvate thereof,
wherein the prodrug is selected from the group consisting of esters and amides.

8. A compound of Formula I that is a compound of Formula IIb for use in the prevention or treatment of an HBV infection in a subject according to any of claims 1 or 3 to 7 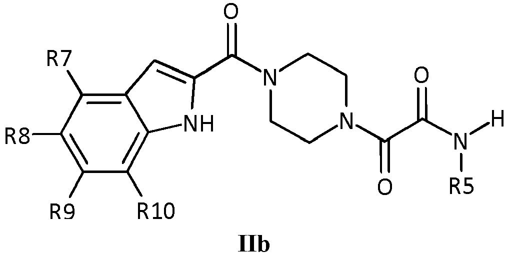 in which
- R5 is selected from the group comprising H, C6-aryl, C3-CS-heteroaryl, C1-C6-alkyl, C2-C6-alkynyl, C1-C6-haloalkyl, C3-C7-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, C1-C2-alkyl-C3-CS-heteroaryl and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C1-C4-alkoxy, C1-C6-alkyl-O-C1-C6-alkyl, C(=O)NH₂, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula IIb or the pharmaceutically acceptable salt thereof or a prodrug of a compound of Formula IIb or a pharmaceutically acceptable salt or a solvate thereof,
wherein the prodrug is selected from the group consisting of esters and amides.

9. A compound of Formula I that is a compound of Formula IIb for use in the prevention or treatment of an HBV infection in a subject according to any of claims 1 or 3 to 8 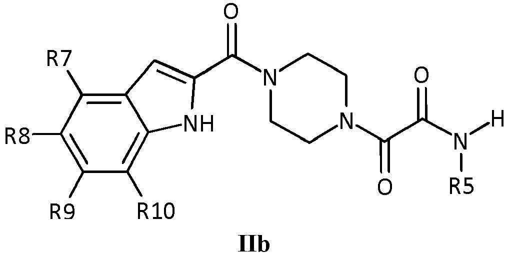 in which
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH3 and carboxy
- R7, R8, R9 and R10 are independently selected from the group comprising H, D, F, Cl, Br, I, CF₃, CF₂H, C1-C4-alkyl, CF₂CH₃, cyclopropyl and cyano,
wherein heteroaryl and heterocycloalkyl each has 1 or 2 heteroatoms each independently selected from N, O and S,
or a pharmaceutically acceptable salt thereof or a solvate of a compound of Formula IIb or the pharmaceutically acceptable salt thereof or a prodrug of a compound of Formula IIb or a pharmaceutically acceptable salt or a solvate thereof,
wherein the prodrug is selected from the group consisting of esters and amides.

10. A compound of Formula I that is a compound of Formula III for use in the prevention or treatment of an HBV infection in a subject according to claim 1 or 3 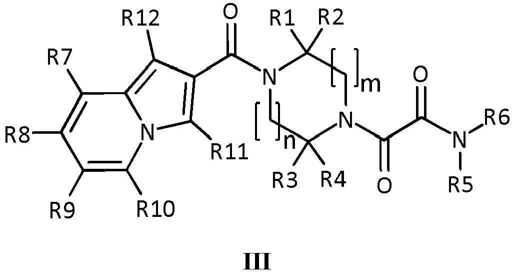 in which
- R1, R2, R3, and R4 are for each position independently selected from the group comprising H, D, and C1-C6-alkyl
- R5 and R6 are independently selected from the group comprising H, C6-aryl, C3-C5-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R5 and R6 are optionally connected to form a C4-C8-heterocyclyl ring
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S,
or a pharmaceutically acceptable salt thereof or a solvate of a compound of Formula III or the pharmaceutically acceptable salt thereof or a prodrug of a compound of Formula III or a pharmaceutically acceptable salt or a solvate thereof,
wherein the prodrug is selected from the group consisting of esters and amides.

11. A compound of Formula I that is a compound of Formula IIIa for use in the prevention or treatment of an HBV infection in a subject according to any of claims 1, 3 or 10 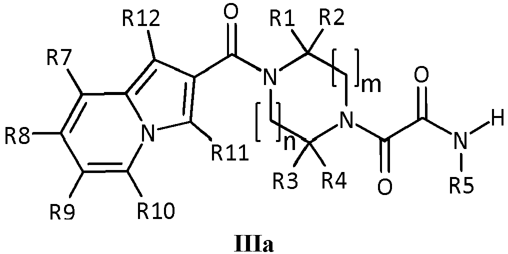 in which
- R1, R2, R3, and R4 are for each position independently selected from the group comprising H, D, and C1-C6-alkyl
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH3 and carboxy
- n is 0, 1, or 2
- m is 0, 1, or 2
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S,
or a pharmaceutically acceptable salt thereof or a solvate of a compound of Formula IIIa or the pharmaceutically acceptable salt thereof or a prodrug of a compound of Formula IIIa or a pharmaceutically acceptable salt or a solvate thereof,
wherein the prodrug is selected from the group consisting of esters and amides.

12. A compound of Formula I that is a compound of Formula IIIb for use in the prevention or treatment of an HBV infection in a subject according to any of claims 1, 3, 10 or 11 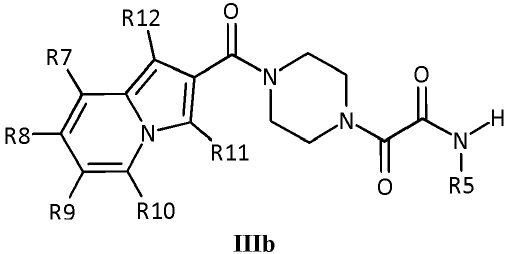 in which
- R5 is selected from the group comprising H, C6-aryl, C3-CS-heteroaryl, C1-C6-alkyl, C1-C6-haloalkyl, C3-C6-cycloalkyl, C3-C7-heterocycloalkyl, C2-C6-hydroxyalkyl, C1-C6-alkyl-O-C1-C6-alkyl, C1-C2-alkyl-C3-C5-cycloalkyl, and C1-C2-alkyl-C3-CS-heterocycloalkyl optionally substituted with 1, 2, or 3 groups each independently selected from OH, halo, phenyl, carboxyphenyl, C3-C7-heterocycloalkyl, C1-C6-alkyl, C1-C6-haloalkyl, C1-C6-hydroxyalkyl, C(=O)N(H)CH₃ and carboxy
- R7, R8, R9 and R10 are independently selected from the group comprising H, D, F, Cl, Br, I, CF₃, CF₂H, C1-C4-alkyl, CF₂CH₃, cyclopropyl and cyano
- R11 and R12 are independently selected from the group comprising H, D, F, Cl, Br, I, CF₃, CF₂H, C1-C4-alkyl, C2-C5-alkenyl, CF₂CH₃, cyclopropyl, cyano, and nitro, wherein heteroaryl and heterocycloalkyl each has 1 or 2 heteroatoms each independently selected from N, O and S,
or a pharmaceutically acceptable salt thereof or a solvate of a compound of Formula IIIb or the pharmaceutically acceptable salt thereof or a prodrug of a compound of Formula IIIb or a pharmaceutically acceptable salt or a solvate thereof,
wherein the prodrug is selected from the group consisting of esters and amides.

13. A pharmaceutical composition comprising a compound as defined in any of claims 1 to 12 or a pharmaceutically acceptable salt thereof or a solvate or a hydrate of said compound or the pharmaceutically acceptable salt thereof or a prodrug of said compound or a pharmaceutically acceptable salt or a solvate or a hydrate thereof, wherein the prodrug is selected from the group consisting of esters and amides together with a pharmaceutically acceptable carrier for use in the prevention or treatment of an HBV infection in a subject.

14. Method for the preparation of a compound of Formula I as defined in claim 1 by reacting a compound of Formula IV 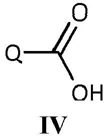 in which Q is as above-defined, with a compound of Formula V 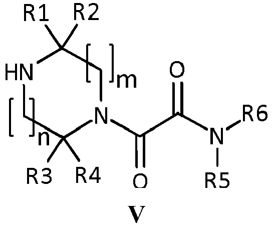 in which R1, R2, R3, R4, R5, R6, n and m are as defined in claim 1.

15. A method for the preparation of a compound of Formula I according to claim 14, wherein a compound of Formula IV 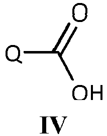 in which Q is as above-defined, reacts with a compound of Formula V 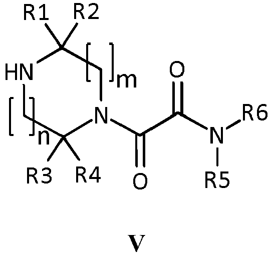 in which R1, R2, R3, R4, R5, R6, n and m are as defined in claim 3.

## Patentansprüche

1. Verbindung der Formel I, 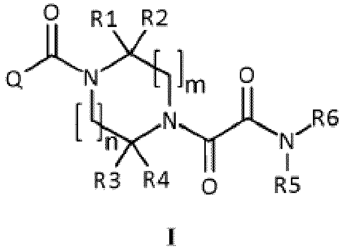 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C3-C5-Heteroaryl, C1-C6-Alkyl, C2-C6-Alkinyl, C1-C6-Halogenalkyl, C3-C7-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl, C1-C2-Alkyl-C3-C5-heteroaryl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C1-C4-Alkoxy, C1-C6-Alkyl-O-C1-C6-alkyl, C(=O)NH₂, C(=O)N(H)CH₃ und Carboxy
- R5 und R6 wahlweise verbunden sind, um einen C4-C8-Heterocyclylring zu bilden
- n 0, 1 oder 2 ist
- m 0, 1 oder 2 ist
- Q. Indol-2-yl ist, wahlweise substituiert mit 1, 2, 3 oder 4 Gruppen, unabhängig ausgewählt aus H, D, F, Cl, Br, I, CF₃, CF₂H, C1-C4-Alkyl, CF₂CH₃, Cyclopropyl, Cyano und Nitro, oder
Indolizin-2-yl, wahlweise substituiert mit 1, 2, 3, 4, 5 oder 6 Gruppen, unabhängig ausgewählt aus H, D, F, Cl, Br, I, CF₃, CF₂H, C1-C4-Alkyl, CF₂CH₃, Cyclopropyl, Cyano, C2-C5-Alkenyl und Nitro,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel I oder das pharmazeutisch verträgliche Salz davon oder ein Pro-Pharmakon einer Verbindung der Formel I oder ein pharmazeutisch verträgliches Salz oder ein Solvat davon zur Verwendung bei der Prävention oder Behandlung einer HBV-Infektion bei einem Subjekt,
wobei das Pro-Pharmakon ausgewählt ist aus der Gruppe bestehend aus Estern und Amiden.

2. Verbindung der Formel I zur Verwendung bei der Prävention oder Behandlung einer HBV-Infektion bei einem Subjekt nach Anspruch 1, 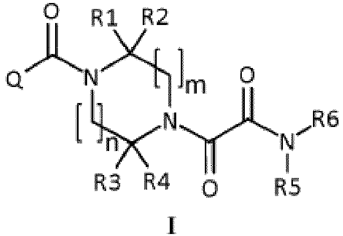 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C1-C6-Alkyl, C2-C6-Alkinyl, C1-C6-Halogenalkyl, C3-C7-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl, C1-C2-Alkyl-C3-C5-heteroaryl und C1-C2-Alkyl-C3-C5-heterocycloalkyl, wahlweise substituiert mit 1, 2 oder 3 Gruppen, jeweils unabhängig voneinander ausgewählt aus OH, C3-C7-Heterocycloalkyl, C1-C6-Alkyl, C1-C6-Hydroxyalkyl, C1-C4-Alkoxy, C1-C6-Alkyl-O-C1-C6-alkyl, C(=O)NH₂ und C(=O)N(H)CH₃
- R5 und R6 wahlweise verbunden sind, um einen C4-C8-Heterocyclylring zu bilden
- n 0, 1 oder 2 ist
- m 0, 1 oder 2 ist
- Q. Indol-2-yl ist, wahlweise substituiert mit 1, 2, 3 oder 4 Gruppen, unabhängig ausgewählt aus H, D, F, CI, Br, I, CF₃, CF₂H, C1-C4-Alkyl, CF₂CH₃, Cyclopropyl, Cyano und Nitro, oder Indolizin-2-yl, wahlweise substituiert mit 1, 2, 3, 4, 5 oder 6 Gruppen, unabhängig ausgewählt aus H, D, F, CI, Br, I, CF₃, CF₂H, C1-C4-Alkyl, CF₂CH₃, Cyclopropyl, Cyano, C2-C5-Alkenyl und Nitro,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel I oder das pharmazeutisch verträgliche Salz davon oder ein Pro-Pharmakon einer Verbindung der Formel I oder ein pharmazeutisch verträgliches Salz oder ein Solvat davon,
wobei das Pro-Pharmakon ausgewählt ist aus der Gruppe bestehend aus Estern und Amiden.

3. Verbindung der Formel I zur Verwendung bei der Prävention oder Behandlung einer HBV-Infektion bei einem Subjekt nach Anspruch 1, 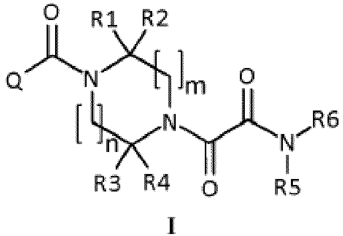 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-CS-cycloalkyl und C1-C2-Alkyl-C3-C5-heterocycloalkyl, wahlweise substituiert mit 1, 2 oder 3 Gruppen, jeweils unabhängig ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- R5 und R6 wahlweise verbunden sind, um einen C4-C8-Heterocyclylring zu bilden
- n 0, 1 oder 2 ist
- m 0, 1 oder 2 ist
- Q. Indol-2-yl ist, wahlweise substituiert mit 1, 2, 3 oder 4 Gruppen, unabhängig ausgewählt aus H, D, F, CI, Br, I, CF₃, CF₂H, C1-C4-Alkyl, CF₂CH₃, Cyclopropyl, Cyano und Nitro, oder Indolizin-2-yl, wahlweise substituiert mit 1, 2, 3, 4, 5 oder 6 Gruppen, unabhängig ausgewählt aus H, D, F, CI, Br, I, CF₃, CF₂H, C1-C4-Alkyl, CF₂CH₃, Cyclopropyl, Cyano, C2-C5-Alkenyl und Nitro, wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel I oder das pharmazeutisch verträgliche Salz davon oder ein Pro-Pharmakon einer Verbindung der Formel I oder ein pharmazeutisch verträgliches Salz oder ein Solvat davon,
wobei das Pro-Pharmakon ausgewählt ist aus der Gruppe bestehend aus Estern und Amiden.

4. Verbindung der Formel I, die eine Verbindung der Formel II ist, zur Verwendung bei der Prävention oder Behandlung einer HBV-Infektion bei einem Subjekt nach Anspruch 1 oder 3, 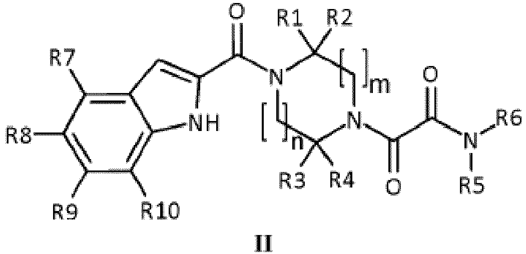 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C3-C5-Heteroaryl, C1-C6-Alkyl, C2-C6-Alkinyl, C1-C6-Halogenalkyl, C3-C7-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl, C1-C2-Alkyl-C3-C5-heteroaryl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C1-C4-Alkoxy, C1-C6-Alkyl-O-C1-C6-alkyl, C(=O)NH₂, C(=O)N(H)CH₃ und Carboxy
- R5 und R6 wahlweise verbunden sind, um einen C4-C8-Heterocyclylring zu bilden
- n 0, 1 oder 2 ist
- m 0, 1 oder 2 ist
- R7, R8, R9 und R10 unabhängig voneinander ausgewählt sind aus der Gruppe umfassend H, D, F, CI, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel II oder das pharmazeutisch verträgliche Salz davon oder ein Pro-Pharmakon einer Verbindung der Formel II oder ein pharmazeutisch verträgliches Salz oder ein Solvat davon,
wobei das Pro-Pharmakon ausgewählt ist aus der Gruppe bestehend aus Estern und Amiden.

5. Verbindung der Formel I, die eine Verbindung der Formel II ist, zur Verwendung bei der Prävention oder Behandlung einer HBV-Infektion bei einem Subjekt nach einem der Ansprüche 1, 3 oder 4, 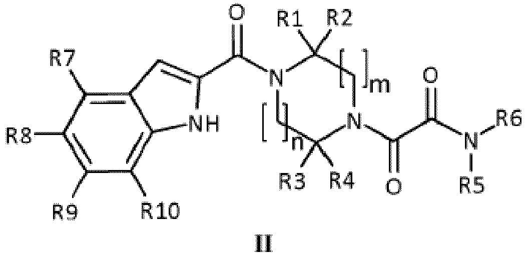 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- R5 und R6 wahlweise verbunden sind, um einen C4-C8-Heterocyclylring zu bilden
- n 0, 1 oder 2 ist
- m 0, 1 oder 2 ist
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel II oder das pharmazeutisch verträgliche Salz davon oder ein Pro-Pharmakon einer Verbindung der Formel II oder ein pharmazeutisch verträgliches Salz oder ein Solvat davon,
wobei das Pro-Pharmakon ausgewählt ist aus der Gruppe bestehend aus Estern und Amiden.

6. Verbindung der Formel I, die eine Verbindung der Formel IIa ist, zur Verwendung bei der Prävention oder Behandlung einer HBV-Infektion bei einem Subjekt nach einem der Ansprüche 1 oder 3 bis 5, 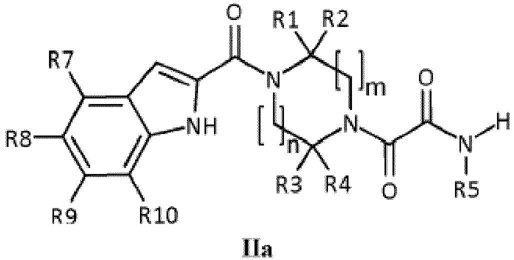 in welcher
- R1, R2, R3 und R4 für jede Position unabhängig voneinander ausgewählt sind aus der Gruppe umfassend H, D und C1-C6-Alkyl
- R5 ausgewählt ist aus der Gruppe umfassend H, C6-Aryl, C3-C5-Heteroaryl, C1-C6-Alkyl, C2-C6-Alkinyl, C1-C6-Halogenalkyl, C3-C7-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl, C1-C2-Alkyl-C3-C5-heteroaryl und C1-C2-Alkyl-C3-C5-heterocycloalkyl, wahlweise substituiert mit 1, 2 oder 3 Gruppen, jeweils unabhängig ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C1-C4-Alkoxy, C1-C6-Alkyl-O-C1-C6-alkyl, C(=O)NH₂, C(=O)N(H)CH₃ und Carboxy
- n 0, 1 oder 2 ist
- m 0, 1 oder 2 ist
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel IIa oder das pharmazeutisch verträgliche Salz davon oder ein Pro-Pharmakon einer Verbindung der Formel IIa oder ein pharmazeutisch verträgliches Salz oder ein Solvat davon,
wobei das Pro-Pharmakon ausgewählt ist aus der Gruppe bestehend aus Estern und Amiden.

7. Verbindung der Formel I, die eine Verbindung der Formel IIa ist, zur Verwendung bei der Prävention oder Behandlung einer HBV-Infektion bei einem Subjekt nach einem der Ansprüche 1 oder 3 bis 6, 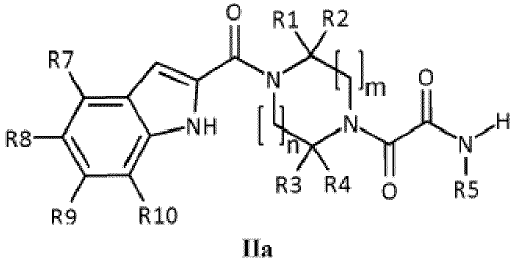 in welcher
- R1, R2, R3 und R4 für jede Position unabhängig voneinander ausgewählt sind aus der Gruppe umfassend H, D und C1-C6-Alkyl
- R5 ausgewählt ist aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- n 0, 1 oder 2 ist
- m 0, 1 oder 2 ist
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel Ila oder das pharmazeutisch verträgliche Salz davon oder ein Pro-Pharmakon einer Verbindung der Formel Ila oder ein pharmazeutisch verträgliches Salz oder ein Solvat davon,
wobei das Pro-Pharmakon ausgewählt ist aus der Gruppe bestehend aus Estern und Amiden.

8. Verbindung der Formel I, die eine Verbindung der Formel Ilb ist, zur Verwendung bei der Prävention oder Behandlung einer HBV-Infektion bei einem Subjekt nach einem der Ansprüche 1 oder 3 bis 7, 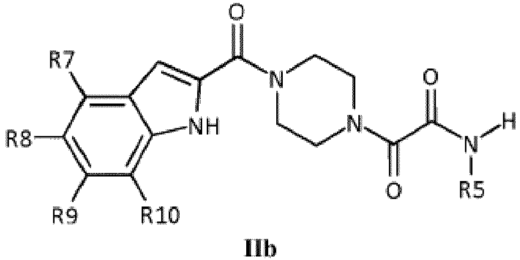 in welcher
- R5 ausgewählt ist aus der Gruppe umfassend H, C6-Aryl, C3-C5-Heteroaryl, C1-C6-Alkyl, C2-C6-Alkinyl, C1-C6-Halogenalkyl, C3-C7-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl, C1-C2-Alkyl-C3-C5-heteroaryl und C1-C2-Alkyl-C3-C5-heterocycloalkyl, wahlweise substituiert mit 1, 2 oder 3 Gruppen, jeweils unabhängig ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C1-C4-Alkoxy, C1-C6-Alkyl-O-C1-C6-alkyl, C(=O)NH₂, C(=O)N(H)CH₃ und Carboxy
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel IIb oder das pharmazeutisch verträgliche Salz davon oder ein Pro-Pharmakon einer Verbindung der Formel IIb oder ein pharmazeutisch verträgliches Salz oder ein Solvat davon,
wobei das Pro-Pharmakon ausgewählt ist aus der Gruppe bestehend aus Estern und Amiden.

9. Verbindung der Formel I, die eine Verbindung der Formel Ilb ist, zur Verwendung bei der Prävention oder Behandlung einer HBV-Infektion bei einem Subjekt nach einem der Ansprüche 1 oder 3 bis 8, 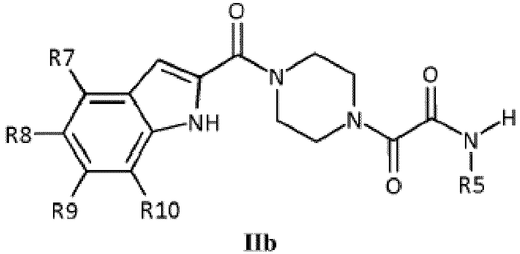 in welcher
- R5 ausgewählt ist aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel Ilb oder das pharmazeutisch verträgliche Salz davon oder ein Pro-Pharmakon einer Verbindung der Formel Ilb oder ein pharmazeutisch verträgliches Salz oder ein Solvat davon,
wobei das Pro-Pharmakon ausgewählt ist aus der Gruppe bestehend aus Estern und Amiden.

10. Verbindung der Formel I, die eine Verbindung der Formel III ist, zur Verwendung bei der Prävention oder Behandlung einer HBV-Infektion bei einem Subjekt nach Anspruch 1 oder 3, 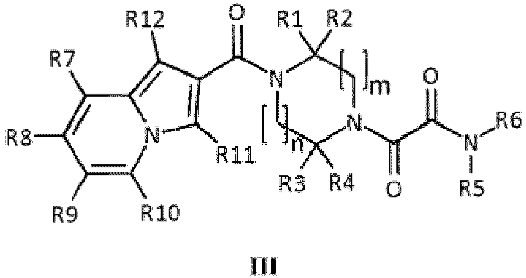 in welcher
- R1, R2, R3 und R4 für jede Position unabhängig voneinander ausgewählt sind aus der Gruppe umfassend H, D und C1-C6-Alkyl
- R5 und R6 unabhängig voneinander ausgewählt sind aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- R5 und R6 wahlweise verbunden sind, um einen C4-C8-Heterocyclylring zu bilden
- n 0, 1 oder 2 ist
- m 0, 1 oder 2 ist
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano
- R11 und R12 unabhängig voneinander ausgewählt sind aus der Gruppe umfassend H, D, F, Cl, Br, I, CF₃, CF₂H, C1-C4-Alkyl, C2-C5-Alkenyl, CF₂CH₃, Cyclopropyl, Cyano und Nitro,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung der Formel III oder das pharmazeutisch verträgliche Salz davon oder ein Pro-Pharmakon einer Verbindung der Formel III oder ein pharmazeutisch verträgliches Salz oder ein Solvat davon,
wobei das Pro-Pharmakon ausgewählt ist aus der Gruppe bestehend aus Estern und Amiden.

11. Verbindung der Formel I, die eine Verbindung der Formel Illa ist, zur Verwendung bei der Prävention oder Behandlung einer HBV-Infektion bei einem Subjekt nach einem der Ansprüche 1, 3 oder 10, 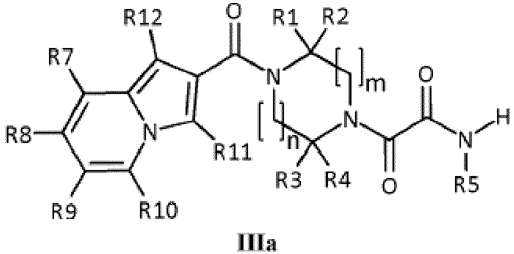 in welcher
- R1, R2, R3 und R4 für jede Position unabhängig voneinander ausgewählt sind aus der Gruppe umfassend H, D und C1-C6-Alkyl
- R5 ausgewählt ist aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- n 0, 1 oder 2 ist
- m 0, 1 oder 2 ist
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano
- R11 und R12 unabhängig voneinander ausgewählt sind aus der Gruppe umfassend H, D, F, Cl, Br, I, CF₃, CF₂H, C1-C4-Alkyl, C2-C5-Alkenyl, CF₂CH₃, Cyclopropyl, Cyano und Nitro,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung Formel Illa oder das pharmazeutisch verträgliche Salz davon oder ein Pro-Pharmakon einer Verbindung der Formel Illa oder ein pharmazeutisch verträgliches Salz oder ein Solvat davon,
wobei das Pro-Pharmakon ausgewählt ist aus der Gruppe bestehend aus Estern und Amiden.

12. Verbindung der Formel I, die eine Verbindung der Formel Illb ist, zur Verwendung bei der Prävention oder Behandlung einer HBV-Infektion bei einem Subjekt nach einem der Ansprüche 1, 3, 10 oder 11, 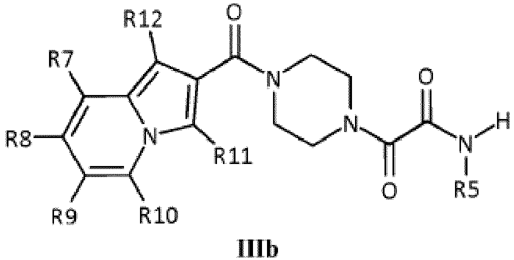 in welcher
- R5 ausgewählt ist aus der Gruppe umfassend H, C6-Aryl, C3-C5-Heteroaryl, C1-C6-Alkyl, C1-C6-Halogenalkyl, C3-C6-Cycloalkyl, C3-C7-Heterocycloalkyl, C2-C6-Hydroxyalkyl, C1-C6-Alkyl-O-C1-C6-alkyl, C1-C2-Alkyl-C3-C5-cycloalkyl und C1-C2-Alkyl-C3-C5-heterocycloalkyl, wahlweise substituiert mit 1, 2 oder 3 Gruppen, jeweils unabhängig voneinander ausgewählt aus OH, Halogen, Phenyl, Carboxyphenyl, C3-C7-Heterocycloalkyl, C1-C6-Alkyl, C1-C6-Halogenalkyl, C1-C6-Hydroxyalkyl, C(=O)N(H)CH₃ und Carboxy
- R7, R8, R9 und R10 unabhängig voneinander ausgewählt sind aus der Gruppe umfassend H, D, F, Cl, Br, I, CF₃, CF₂H, C1-C4-Alkyl, CF₂CH₃, Cyclopropyl und Cyano
- R11 und R12 unabhängig voneinander ausgewählt sind aus der Gruppe umfassend H, D, F, Cl, Br, I, CF₃, CF₂H, C1-C4-Alkyl, C2-C5-Alkenyl, CF₂CH₃, Cyclopropyl, Cyano und Nitro,
wobei Heteroaryl und Heterocycloalkyl jeweils 1 oder 2 Heteroatome aufweisen, die jeweils unabhängig voneinander ausgewählt sind aus N, O und S,
oder ein pharmazeutisch verträgliches Salz davon oder ein Solvat einer Verbindung Formel Illb oder das pharmazeutisch verträgliche Salz davon oder ein Pro-Pharmakon einer Verbindung der Formel Illb oder ein pharmazeutisch verträgliches Salz oder ein Solvat davon,
wobei das Pro-Pharmakon ausgewählt ist aus der Gruppe bestehend aus Estern und Amiden.

13. Pharmazeutische Zusammensetzung umfassend eine Verbindung, wie in einem der Ansprüche 1 bis 12 definiert, oder ein pharmazeutisch verträgliches Salz davon oder ein Solvat oder ein Hydrat der Verbindung oder des pharmazeutisch verträglichen Salzes davon oder ein Pro-Pharmakon der Verbindung oder eines pharmazeutisch verträglichen Salzes oder eines Solvats oder eines Hydrats davon, wobei das Pro-Pharmakon ausgewählt ist aus der Gruppe bestehend aus Estern und Amiden, zusammen mit einem pharmazeutisch verträglichen Trägerstoff zur Verwendung bei der Prävention oder Behandlung einer HBV-Infektion bei einem Subjekt.

14. Verfahren zur Herstellung einer Verbindung der Formel I, wie in Anspruch 1 definiert, durch Umsetzung einer Verbindung der Formel IV, 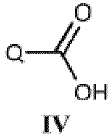 in welcher Q wie oben definiert ist, mit einer Verbindung der Formel V, 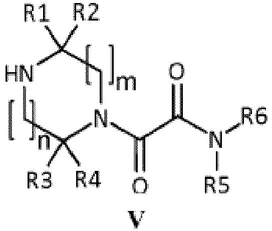 in welcher R1, R2, R3, R4, R5, R6, n und m wie in Anspruch 1 definiert sind.

15. Verfahren zur Herstellung einer Verbindung der Formel I nach Anspruch 14, wobei eine Verbindung der Formel IV, 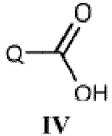 in welcher Q wie oben definiert ist, mit einer Verbindung der Formel V 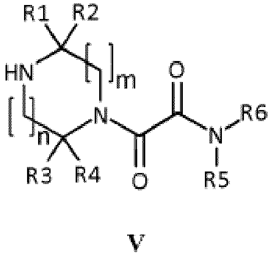 umgesetzt wird,
in welcher R1, R2, R3, R4, R5, R6, n und m wie in Anspruch 3 definiert sind.

## Revendications

1. Composé de la formule I 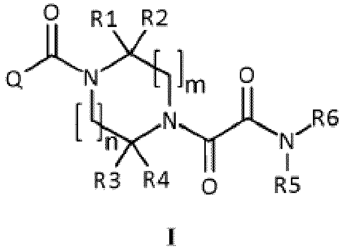 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, hétéroaryle en C3-C5, alkyle en C1-C6, alkynyle en C2-C6, haloalkyle en C1-C6, cycloalkyle en C3-C7, hétérocycloalkyle en C3-C7, hydroxyalkyle en C2-C6, alkyle-C1-C6-O-alkyle-C1-C6, alkyle-C1-C2-cycloalkyle-C3-C5, alkyle-C1-C2-hétéroaryle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, alkoxy en C1-C4, alkyle-C1-C6-O-alkyle-C1-C6, C(=O)NH₂, C(=O)N(H)CH₃ et carboxy
- R5 et R6 sont éventuellement reliés pour former un cycle hétérocyclyle en C4-C8
- n est égal à 0,1 ou 2
- m est égal à 0, 1 ou 2
- Q est un indol-2-yl, éventuellement substitué par 1, 2, 3 ou 4 groupes choisis indépendamment parmi H, D, F, Cl, Br, I, CF₃, CF₂H, alkyle en C1-C4, CF₂CH₃, cyclopropyle, cyano et nitro ; ou
un indolizin-2-yl, éventuellement substitué par 1, 2, 3, 4, 5 ou 6 groupes choisis indépendamment parmi H, D, F, Cl, Br, I, CF₃, CF₂H, alkyle en C1-C4, CF₂CH₃, cyclopropyle, cyano, alkényle en C2-C5 et nitro,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule I ou le sel pharmaceutiquement acceptable de celui-ci ou un promédicament d'un composé de formule I ou un sel pharmaceutiquement acceptable ou un solvant de celui-ci pour utilisation dans la prévention ou le traitement d'une infection par le VHB chez un sujet,
dans lequel le promédicament est choisi dans le groupe consistant en esters et amides.

2. Composé de la formule I pour utilisation dans la prévention ou le traitement d'une infection par le VHB chez un sujet selon la revendication 1 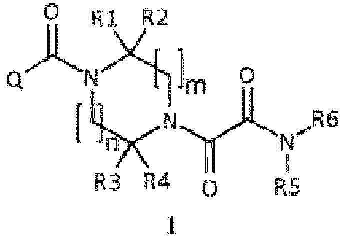 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, alkyle en C1-C6, alkynyle en C2-C6, haloalkyle en C1-C6, cycloalkyle en C3-C7, hétérocycloalkyle en C3-C7, hydroxyalkyle en C2-C6, alkyle-C1-C6-O-alkyle-C1-C6, alkyle-C1-C2-cycloalkyle-C3-C5, alkyle-C1-C2-hétéroaryle-C3-C5, et alkyle-C1-C2-hétérocycloalkyle-C3-C5 éventuellement substitué par 1, 2 ou 3 groupes choisis chacun indépendamment parmi OH, hétérocycloalkyle en C3-C7, alkyle en C1-C6, hydroxyalkyle en C1-C6, alkoxy en C1-C4, alkyle-C1-C6-O-alkyle-C1-C6, C(=O)NH₂, et C(=O)N(H)CH₃
- R5 et R6 sont éventuellement reliés pour former un cycle hétérocyclyle en C4-C8
- n est égal à 0,1 ou 2
- m est égal à 0, 1 ou 2
- Q est un indol-2-yl, éventuellement substitué par 1, 2, 3 ou 4 groupes choisis indépendamment parmi H, D, F, Cl, Br, I, CF₃, CF₂H, alkyle en C1-C4, CF₂CH₃, cyclopropyle, cyano et nitro ; ou indolizin-2-yl, éventuellement substitué par 1, 2, 3, 4, 5 ou 6 groupes choisis indépendamment parmi H, D, F, Cl, Br, I, CF₃, CF₂H, alkyle en C1-C4, CF₂CH₃, cyclopropyle, cyano, alkényle en C2-C5 et nitro,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvant d'un composé de la formule I ou le sel pharmaceutiquement acceptable de celui-ci ou un promédicament d'un composé de la formule I ou un sel pharmaceutiquement acceptable ou un solvant de celui-ci,
dans lequel le promédicament est choisi dans le groupe consistant en esters et amides.

3. Composé de la formule I pour utilisation dans la prévention ou le traitement d'une infection par le VHB chez un sujet selon la revendication 1 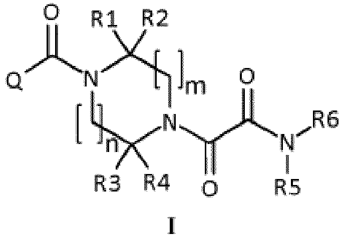 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- R5 et R6 sont éventuellement reliés pour former un cycle hétérocyclyle en C4-C8
- n est égal à 0,1 ou 2
- m est égal à 0, 1 ou 2
- Q est un indol-2-yl, éventuellement substitué par 1, 2, 3 ou 4 groupes choisis indépendamment parmi H, D, F, Cl, Br, I, CF₃, CF₂H, alkyle en C1-C4, CF₂CH₃, cyclopropyle, cyano et nitro ; ou
un indolizin-2-yl, éventuellement substitué par 1, 2, 3, 4, 5 ou 6 groupes choisis indépendamment parmi H, D, F, Cl, Br, I, CF₃, CF₂H, alkyle en C1-C4, CF₂CH₃, cyclopropyle, cyano, alkényle en C2-C5 et nitro,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvant d'un composé de la formule I ou le sel pharmaceutiquement acceptable de celui-ci ou un promédicament d'un composé de la formule I ou un sel pharmaceutiquement acceptable ou un solvant de celui-ci,
dans lequel le promédicament est choisi dans le groupe consistant en esters et amides.

4. Composé de la formule I qui est un composé de la formule Il pour utilisation dans la prévention ou le traitement d'une infection par le VHB chez un sujet selon la revendication 1 ou 3 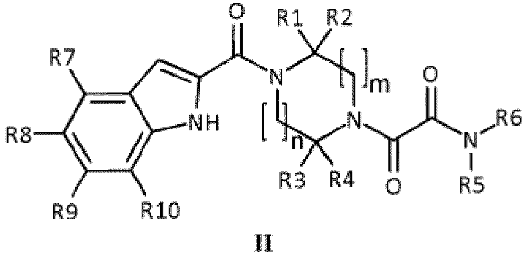 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, hétéroaryle en C3-C5, alkyle en C1-C6, alkynyle en C2-C6, haloalkyle en C1-C6, cycloalkyle en C3-C7, hétérocycloalkyle en C3-C7, hydroxyalkyle en C2-C6, alkyle-C1-C6-O-alkyle-C1-C6, alkyle-C1-C2-cycloalkyle-C3-C5, alkyle-C1-C2-hétéroaryle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, alkoxy en C1-C4, alkyle-C1-C6-O-alkyle-C1-C6, C(=O)NH₂, C(=O)N(H)CH₃ et carboxy
- R5 et R6 sont éventuellement reliés pour former un cycle hétérocyclyle en C4-C8
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvant d'un composé de la formule Il ou le sel pharmaceutiquement acceptable de celui-ci ou un promédicament d'un composé de la formule Il ou un sel pharmaceutiquement acceptable ou un solvant de celui-ci,
dans lequel le promédicament est choisi dans le groupe consistant en esters et amides.

5. Composé de la formule I qui est un composé de la formule Il pour utilisation dans la prévention ou le traitement d'une infection par le VHB chez un sujet selon l'une quelconque des revendications 1, 3 ou 4 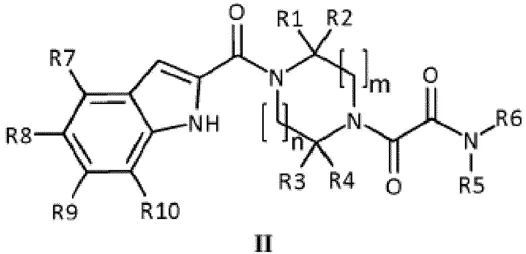 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- R5 et R6 sont éventuellement reliés pour former un cycle hétérocyclyle en C4-C8
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvant d'un composé de la formule Il ou le sel pharmaceutiquement acceptable de celui-ci ou un promédicament d'un composé de la formule Il ou un sel pharmaceutiquement acceptable ou un solvant de celui-ci,
dans lequel le promédicament est choisi dans le groupe consistant en esters et amides.

6. Composé de la formule I qui est un composé de la formule IIa pour utilisation dans la prévention ou le traitement d'une infection par le VHB chez un sujet selon l'une quelconque des revendications 1 ou 3à5 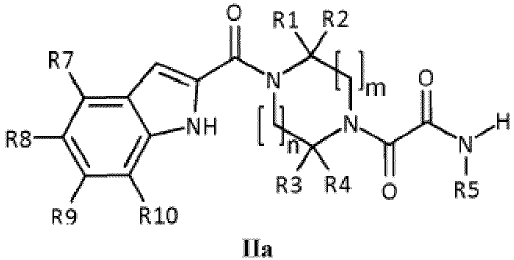 dans laquelle
- R1, R2, R3 et R4 sont pour chaque position indépendamment choisis dans le groupe comprenant H, D et alkyle en C1-C6
- R5 est choisi dans le groupe comprenant H, aryle en C6, hétéroaryle en C3-C5, alkyle en C1-C6, alkynyle en C2-C6, haloalkyle en C1-C6, cycloalkyle en C3-C7, hétérocycloalkyle en C3-C7, hydroxyalkyle en C2-C6, alkyle-C1-C6-O-alkyle-C1-C6, alkyle-C1-C2-cycloalkyle-C3-C5, alkyle-C1-C2-hétéroaryle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, alkoxy en C1-C4, alkyle-C1-C6-O-alkyle-C1-C6, C(=O)NH₂, C(=O)N(H)CH₃ et carboxy
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvant d'un composé de la formule IIa ou le sel pharmaceutiquement acceptable de celui-ci ou un promédicament d'un composé de la formule IIa ou un sel pharmaceutiquement acceptable ou un solvant de celui-ci,
dans lequel le promédicament est choisi dans le groupe consistant en esters et amides.

7. Composé de la formule I qui est un composé de la formule IIa pour utilisation dans la prévention ou le traitement d'une infection par le VHB chez un sujet selon l'une quelconque des revendications 1 ou 3à6 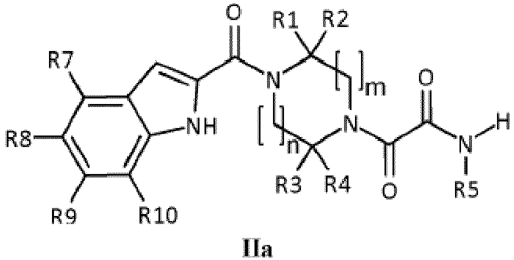 dans laquelle
- R1, R2, R3 et R4 sont pour chaque position indépendamment choisis dans le groupe comprenant H, D et alkyle en C1-C6
- R5 est choisi dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule IIa ou le sel pharmaceutiquement acceptable de celui-ci ou un solvate d'un composé de la formule IIa ou un sel pharmaceutiquement acceptable ou un solvant de celui-ci,
dans lequel le promédicament est choisi dans le groupe consistant en esters et amides.

8. Composé de la formule I qui est un composé de la formule Ilb pour utilisation dans la prévention ou le traitement d'une infection par le VHB chez un sujet selon l'une quelconque des revendications 1 ou 3à7 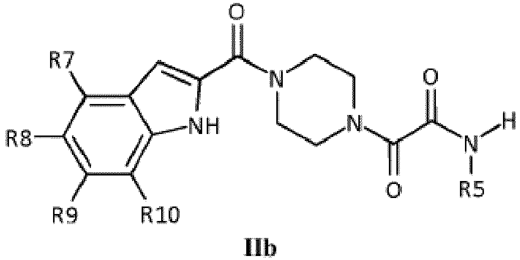 dans laquelle
- R5 est choisi dans le groupe comprenant H, aryle en C6, hétéroaryle en C3-C5, alkyle en C1-C6, alkynyle en C2-C6, haloalkyle en C1-C6, cycloalkyle en C3-C7, hétérocycloalkyle en C3-C7, hydroxyalkyle en C2-C6, alkyle-C1-C6-O-alkyle-C1-C6, alkyle-C1-C2-cycloalkyle-C3-C5, alkyle-C1-C2-hétéroaryle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, alkoxy en C1-C4, alkyle-C1-C6-O-alkyle-C1-C6, C(=O)NH₂, C(=O)N(H)CH₃ et carboxy
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule Ilb ou le sel pharmaceutiquement acceptable de celui-ci ou un solvate d'un composé de la formule IIb ou un sel pharmaceutiquement acceptable ou un solvant de celui-ci,
dans lequel le promédicament est choisi dans le groupe consistant en esters et amides.

9. Composé de la formule I qui est un composé de la formule Ilb pour utilisation dans la prévention ou le traitement d'une infection par le VHB chez un sujet selon l'une quelconque des revendications 1 ou 3à8 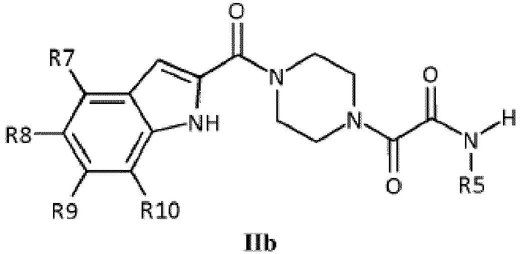 dans laquelle
- R5 est choisi dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule Ilb ou le sel pharmaceutiquement acceptable de celui-ci ou un solvate d'un composé de la formule Ilb ou un sel pharmaceutiquement acceptable ou un solvant de celui-ci,
dans lequel le promédicament est choisi dans le groupe consistant en esters et amides.

10. Composé de la formule I qui est un composé de la formule III pour utilisation dans la prévention ou le traitement d'une infection par le VHB chez un sujet selon la revendication 1 ou 3 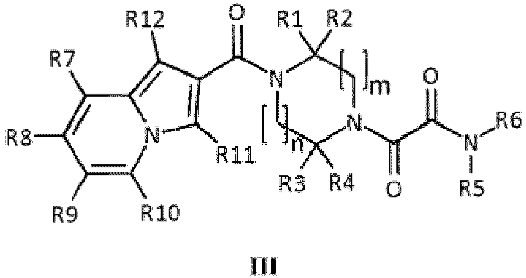 dans laquelle
- R1, R2, R3 et R4 sont pour chaque position indépendamment choisis dans le groupe comprenant H, D et alkyle en C1-C6
- R5 et R6 sont indépendamment choisis dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- R5 et R6 sont éventuellement reliés pour former un cycle hétérocyclyle en C4-C8
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
- R11 et R12 sont choisis indépendamment dans le groupe comprenant H, D, F, Cl, Br, I, CF₃, CF₂H, alkyle en C1-C4, alkényle en C2-C5, CF₂CH₃, cyclopropyle, cyano et nitro
dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule III ou le sel pharmaceutiquement acceptable de celui-ci ou un solvate d'un composé de la formule III ou un sel pharmaceutiquement acceptable ou un solvant de celui-ci,
dans lequel le promédicament est choisi dans le groupe consistant en esters et amides.

11. Composé de la formule I qui est un composé de la formule IIIa pour utilisation dans la prévention ou le traitement d'une infection par le VHB chez un sujet selon l'une quelconque des revendications 1, 3 ou 10 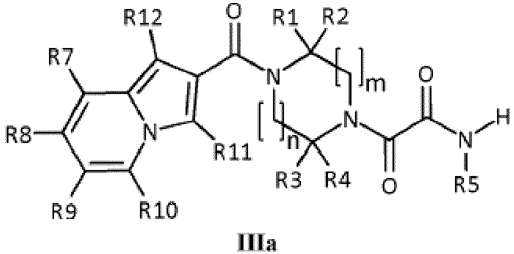 dans laquelle
- R1, R2, R3 et R4 sont pour chaque position indépendamment choisis dans le groupe comprenant H, D et alkyle en C1-C6
- R5 est choisi dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- n est égal à 0,1 ou 2
- m est égal à 0, 1 ou 2
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
- R11 et R12 sont choisis indépendamment dans le groupe comprenant H, D, F, Cl, Br, I, CF₃, CF₂H, alkyle en C1-C4, alkényle en C2-C5, CF₂CH₃, cyclopropyle, cyano et nitro dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule IIIa ou le sel pharmaceutiquement acceptable de celui-ci ou un solvate d'un composé de la formule IIIa ou un sel pharmaceutiquement acceptable ou un solvant de celui-ci,
dans lequel le promédicament est choisi dans le groupe consistant en esters et amides.

12. Composé de la formule I qui est un composé de la formule Illb pour utilisation dans la prévention ou le traitement d'une infection par le VHB chez un sujet selon l'une quelconque des revendications 1, 3, 10 ou 11 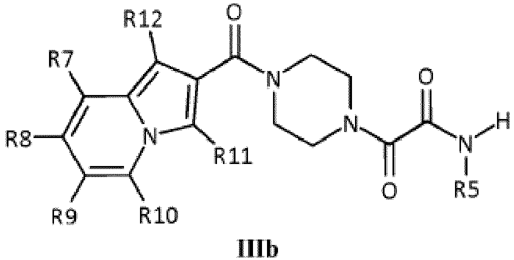 dans laquelle
- R5 est choisi dans le groupe comprenant H, aryle en C6, hétéroaryle en C3-C5, alkyle en C1-C6, haloalkyle en C1-C6, cycloalkyle en C3-C6, hétérocycloalkyle en C3-C7, hydroxyalkyle en C2-C6, alkyle-C1-C6-O-alkyle-O-C1-C6, alkyle-C1-C2-cycloalkyle-C3-C5, et alkyle-C1-C2-hétérocycloalkyle-C3-C5 éventuellement substitué par 1, 2 ou 3 groupes choisis chacun indépendamment parmi OH, halo, phényle, carboxyphényle, hétérocycloalkyle en C3-C7, alkyle en C1-C6, haloalkyle en C1-C6, hydroxyalkyle en C1-C6, C(=O)N(H)CH₃ et carboxy
- R7, R8, R9 et R10 sont choisis indépendamment dans le groupe comprenant H, D, F, Cl, Br, I, CF₃, CF₂H, alkyle en C1-C4, CF₂CH₃, cyclopropyle et cyano,
- R11 et R12 sont choisis indépendamment dans le groupe comprenant H, D, F, Cl, Br, I, CF₃, CF₂H, alkyle en C1-C4, alkényle en C2-C5, CF₂CH₃, cyclopropyle, cyano et nitro dans lequel l'hétéroaryle et l'hétérocycloalkyle possèdent chacun 1 ou 2 hétéroatomes choisis indépendamment parmi N, O et S,
ou un sel pharmaceutiquement acceptable de celui-ci ou un solvate d'un composé de la formule IIIb ou le sel pharmaceutiquement acceptable de celui-ci ou un solvate d'un composé de la formule Illb ou un sel pharmaceutiquement acceptable ou un solvant de celui-ci,
dans lequel le promédicament est choisi dans le groupe consistant en esters et amides.

13. Composition pharmaceutique comprenant un composé tel que défini dans l'une quelconque des revendications 1 à 12 ou un sel pharmaceutiquement acceptable de celui-ci ou un solvate ou un hydrate dudit composé ou le sel pharmaceutiquement acceptable de celui-ci ou un promédicament dudit composé ou un sel pharmaceutiquement acceptable ou un solvate ou un hydrate de celui-ci, dans lequel le promédicament est choisi dans le groupe consistant en esters et amides
conjointement avec un support pharmaceutiquement acceptable pour utilisation dans la prévention ou le traitement d'une infection par le VHB chez un sujet.

14. Procédé pour la préparation d'un composé de la formule I tel que défini dans la revendication 1 par réaction d'un composé de la formule IV 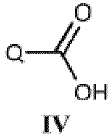 dans lequel Q est tel que défini ci-dessus, avec un composé de la formule V 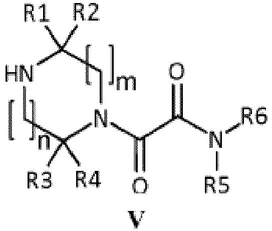 dans lequel R1, R2, R3, R4, R5, R6, n et m sont tels que définis dans la revendication 1.

15. Procédé pour la préparation d'un composé de la formule I selon la revendication 14, dans lequel un composé de la formule IV 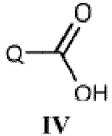 dans lequel Q est tel que défini ci-dessus, réagit avec un composé de la formule V 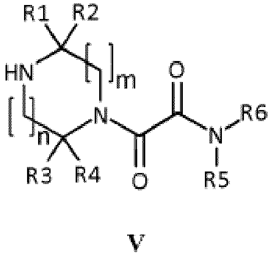 dans lequel R1, R2, R3, R4, R5, R6, n et m sont tels que définis dans la revendication 3.
